# EUROPEAN PATENT APPLICATION

(11) **EP 2 096 109 A1**
(43) Date of publication of application: **02.09.2009**
(21) Application number: 07832729.3
(22) Date of filing: 29.11.2007
(51) Int. Cl.: C07D 215/14, A61K 31/404, A61K 31/4184, A61K 31/4375, A61K 31/4439, A61K 31/4523, A61K 31/47, A61K 31/4706, A61K 31/4709, A61K 31/496, A61K 31/497, A61K 31/5377, A61K 31/538, A61K 31/541, A61P 3/10, A61P 43/00, C07D 209/08, C07D 215/26, C07D 215/40, C07D 215/58

(54) **CARBOXYLIC ACID DERIVATIVE**

(30) Priority: 01.12.2006 JP 2006325388
(71) Applicant: Astellas Pharma Inc., Tokyo 103-8411 (JP)
(72) Inventor: NEGORO, Kenji, Chuo-ku Tokyo 103-8411 (JP); OHNUKI, Kei, Chuo-ku Tokyo 103-8411 (JP); KUROSAKI, Toshio, Chuo-ku Tokyo 103-8411 (JP); IWASAKI, Fumiyoshi, Chuo-ku Tokyo 103-8411 (JP); YONETOKU, Yasuhiro, Chuo-ku Tokyo 103-8411 (JP); TSUCHIYA, Kazuyuki, Chuo-ku Tokyo 103-8411 (JP); ASAI, Norio, Chuo-ku Tokyo 103-8411 (JP); YOSHIDA, Shigeru, Chuo-ku Tokyo 103-8411 (JP); SOGA, Takatoshi, Chuo-ku Tokyo 103-8411 (JP); SUZUKI, Daisuke, Chuo-ku Tokyo 103-8411 (JP)
(74) Representative: Gille Hrabal Struck Neidlein Prop Roos
(86) International application number: PCT/JP2007/073014
(87) International publication number: WO 2008/066097

(57) **Abstract**

To provide a compound which is usable as a drug, in particular, an insulin secretagogue or a preventive or remedy for a disease in which GPR40 participates such as diabetes or the like. [MEANS FOR SOLVING PROBLEMS] If is found out that a novel carboxylic acid derivative, which is **characterized in that** a carboxylic acid is bonded via two atoms to a 6-membered monocyclic aromatic ring and this aromatic ring is further bonded via a linker to a nitrogen-containing bicyclic ring, or its salt has an excellent GPR40 agonist activity, Because of showing excellent effects of promoting insulin secretion and lowering blood glucose level, this carboxylic acid derivative is useful as an insulin secretagogue or a preventive or remedy for diabetes.

SEQUENCE LISTING

<110>  Astellas Pharma Inc.

<120>  carboxylic acid derivatives

<130>  A07037

<150>  JP2006/325388
<151>  2006-12-01

<160>  4

<170>  PatentIn version 3.1

<210>  1
<211>  25
<212>  DNA
<213>  Artificial

<220>
<223>  Description of Artifical Sequence:an artificailly synthesized pri
       mer seqence
<220>
<223>  Inventor: Negoro, Kenji; Ohnuki, Kei; Kurosaki, Toshio;
         Inventor: Iwasaki, Fumiyoshi; Yonetoku, Yasuhiro; Tsuchiya, Kazuyuki;
         Inventor: Asai, Norio; Yoshida, Shigeru; Soga, Takatoshi;
         Inventor: Suzuki, Daisuke
<400>  1
ggtctagaat ggacctgccc ccgca                                          25


<210>  2
<211>  27
<212>  DNA
<213>  Artificial

<220>
<223>  Description of Artifical Sequence:an artificailly synthesized pri
       mer seqence

<400>  2
ggtctagatt acttctggga cttgccc                                        27


<210>  3
<211>  903
<212>  DNA
<213>  Homo sapiens

<400>  3
atggacctgc ccccgcagct ctccttcggc ctctatgtgg ccgcctttgc gctgggcttc      60

ccgctcaacg tcctggccat ccgaggcgcg acggcccacg cccggctccg tctcacccct     120

agcctggtct acgccctgaa cctgggctgc tccgacctgc tgctgacagt ctctctgccc     180

ctgaaggcgg tggaggcgct agcctccggg gcctggcctc tgccggcctc gctgtgcccc     240

gtcttcgcgg tggcccactt cttcccactc tatgccggcg ggggcttcct ggccgccctg     300

agtgcaggcc gctacctggg agcagccttc cccttgggct accaagcctt ccggaggccg     360

tgctattcct ggggggtgtg cgcggccatc tgggccctcg tcctgtgtca cctgggtctg     420

gtctttgggt tggaggctcc aggaggctgg ctggaccaca gcaacacctc cctgggcatc     480

aacacaccgg tcaacggctc tccggtctgc ctggaggcct gggacccggc ctctgccggc     540

ccggcccgct tcagcctctc tctcctgctc ttttttctgc ccttggccat cacagccttc 600

tgctacgtgg gctgcctccg ggcactggcc cactccggcc tgacgcacag gcggaagctg 660

cgggccgcct gggtggccgg cggggccctc ctcacgctgc tgctctgcgt aggaccctac 720

aacgcctcca acgtggccag cttcctgtac cccaatctag gaggctcctg gcggaagctg 780

gggctcatca cgggtgcctg gagtgtggtg cttaatccgc tggtgaccgg ttacttggga 840

aggggtcctg gcctgaagac agtgtgtgcg gcaagaacgc aagggggcaa gtcccagaag 900

taa 903

<210> 4
<211> 300
<212> PRT
<213> Homo sapiens

<400> 4

Met Asp Leu Pro Pro Gln Leu Ser Phe Gly Leu Tyr Val Ala Ala Phe
1               5                   10                  15

Ala Leu Gly Phe Pro Leu Asn Val Leu Ala Ile Arg Gly Ala Thr Ala
            20                  25                  30

His Ala Arg Leu Arg Leu Thr Pro Ser Leu Val Tyr Ala Leu Asn Leu
        35                  40                  45

Gly Cys Ser Asp Leu Leu Leu Thr Val Ser Leu Pro Leu Lys Ala Val
    50                  55                  60

Glu Ala Leu Ala Ser Gly Ala Trp Pro Leu Pro Ala Ser Leu Cys Pro
65                  70                  75                  80

Val Phe Ala Val Ala His Phe Phe Pro Leu Tyr Ala Gly Gly Gly Phe
                85                  90                  95

Leu Ala Ala Leu Ser Ala Gly Arg Tyr Leu Gly Ala Ala Phe Pro Leu
            100                 105                 110

Gly Tyr Gln Ala Phe Arg Arg Pro Cys Tyr Ser Trp Gly Val Cys Ala
        115                 120                 125

Ala Ile Trp Ala Leu Val Leu Cys His Leu Gly Leu Val Phe Gly Leu
    130                 135                 140

Glu Ala Pro Gly Gly Trp Leu Asp His Ser Asn Thr Ser Leu Gly Ile
145                 150                 155                 160

Asn Thr Pro Val Asn Gly Ser Pro Val Cys Leu Glu Ala Trp Asp Pro
                165                 170                 175

Ala Ser Ala Gly Pro Ala Arg Phe Ser Leu Ser Leu Leu Leu Phe Phe

180                 185                 190

Leu Pro Leu Ala Ile Thr Ala Phe Cys Tyr Val Gly Cys Leu Arg Ala
        195                 200                 205

Leu Ala His Ser Gly Leu Thr His Arg Arg Lys Leu Arg Ala Ala Trp
    210                 215                 220

Val Ala Gly Gly Ala Leu Leu Thr Leu Leu Leu Cys Val Gly Pro Tyr
225                 230                 235                 240

Asn Ala Ser Asn Val Ala Ser Phe Leu Tyr Pro Asn Leu Gly Gly Ser
                245                 250                 255

Trp Arg Lys Leu Gly Leu Ile Thr Gly Ala Trp Ser Val Val Leu Asn
            260                 265                 270

Pro Leu Val Thr Gly Tyr Leu Gly Arg Gly Pro Gly Leu Lys Thr Val
        275                 280                 285

Cys Ala Ala Arg Thr Gln Gly Gly Lys Ser Gln Lys
    290                 295                 300

## Description

### FIELD OF THE INVENTION

This invention relates to a pharmaceutical, particularly a novel carboxylic acid derivative or a pharmaceutically acceptable salt thereof which is useful as an insulin secretion promoter or a preventive or therapeutic agent for diabetes mellitus.

### BACKGROUND OF THE INVENTION

Diabetes mellitus is a disease having chronic hyperglycemia as the main symptom, which is developed by the absolute or relative shortage of insulin action. It is roughly divided into insulin-dependent diabetes mellitus (IDDM) and non insulin-dependent diabetes mellitus (NIDDM) based on its clinical characteristics. In the non insulin-dependent diabetes mellitus (NIDDM), reduction of insulin secretion from pancreatic β cells is one of the main causes of the onset of the disease, and hyperglycemia after meals by initial stage insulin secretion disorders is particularly observed.

Recently, it was confirmed by large scale clinical tests that correction of hyperglycemia after meals is important for the onset and progress suppression of diabetic complications. In addition, it has been reported that arteriosclerosis is developed during a period of hyperglycemia alone, and that continuation of slight hyperglycemia after meals increases mortality rate caused by cardiovascular diseases and the like. This suggests that even when it is slight, hyperglycemia after meals is an independent risk factor of cardiovascular death. Based on the above findings, necessity of a drug therapy for hyperglycemia after meals has been recognized.

At present, a sulfonylurea (SU) agent is the main current as an insulin secretion promoter, but it is known that this is apt to cause hypoglycemia and induces secondary invalidity due to exhaustion of the pancreas under a long period of time of its administration. In addition, though the SU agent is effective in controlling blood glucose level during meals, it is difficult to suppress hyperglycemia after meals.

GPR40 is a G protein-coupled receptor identified as a fatty acid receptor, which is highly expressed in β cells of the pancreas, and it has been reported that this is concerned in the insulin secretion action of fatty acids (Non-patent Reference 1).
Accordingly, a GPR40 receptor agonist is expected to be effective in correcting hyperglycemia after meals based on its insulin secretion promoting action, and therefore is useful as a preventive or therapeutic agent for insulin-dependent diabetes mellitus (IDDM), non insulin-dependent diabetes mellitus (NIDDM) and their boundary type (abnormal glucose resistance and fasting blood glucose level) slight diabetes mellitus.

In Patent Reference 1, it is reported that the compound shown by formula (A) including a broad range of compounds have a GPR40 receptor regulating action and are useful as an insulin secretion promoting agent and a preventive or therapeutic agent for diabetes mellitus. However, the ring P corresponding to the nitrogen-containing bicyclic ring of this application is limited to aromatic rings. 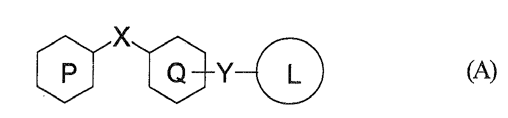 (In the formula, ring P represents an aromatic ring which may have a substituent group, and ring Q an aromatic ring which may further have a substituent group other than 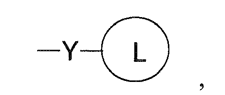 X and Y are spacers, and 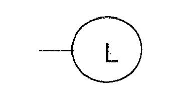 a group capable of releasing a cation.)

In Patent Reference 2, it is reported that the compounds shown by formula (B) have a GPR40 receptor regulating action and are useful as an insulin secretion promoting agent and a preventive or therapeutic agent for diabetes mellitus. However, the ring S 1 corresponding to the nitrogen-containing bicyclic ring of this application is limited to benzene ring. 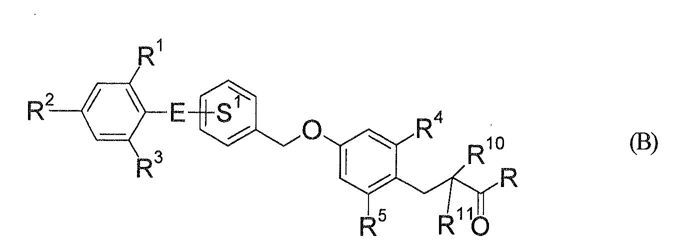 (See said official gazette for the symbols in the formula.)

In Patent Reference 3, it is reported that the compounds shown by formula (C) have a GPR40 receptor regulating action and are useful as an insulin secretion promoting agent and a preventive or therapeutic agent for diabetes mellitus. However, the ring S¹ corresponding to the nitrogen-containing bicyclic ring of this application is limited to benzene ring or pyridine ring. 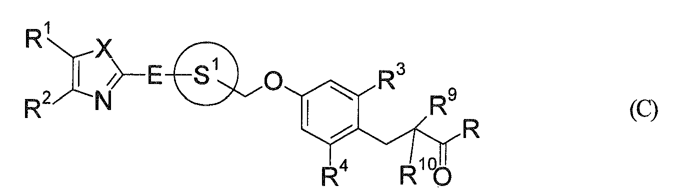 (In the formula, S¹ means benzene ring or pyridine ring. See said official gazette for other symbols.)

In Patent Reference 4, it is reported that the compounds shown by formula (D) have a GPR40 receptor regulating action and are useful as an insulin secretion promoting agent and a preventive or therapeutic agent for diabetes mellitus. However, the part corresponding to the nitrogen-containing bicyclic ring of this application is limited to benzene ring. 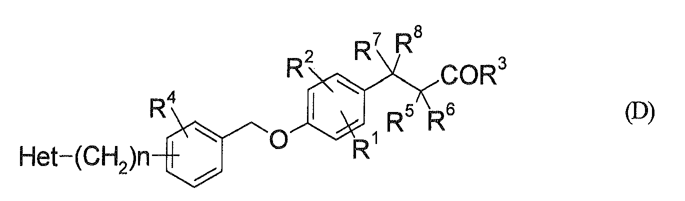 (See said official gazette for the symbols in the formula.)

In Patent Reference 5, it is reported that the compound shown by formula (E) including a broad range of compounds have a GPR40 receptor regulating action and are useful as an insulin secretion promoting agent and a preventive or therapeutic agent for diabetes mellitus. However, there is no illustrative disclosure on the nitrogen-containing bicyclic ring of this application in the ring A which corresponds to the nitrogen-containing bicyclic ring of this application. 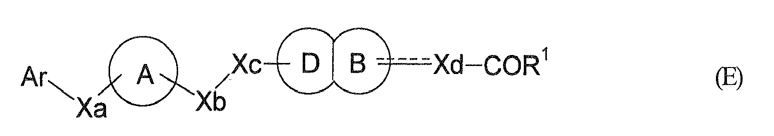 (See said official gazette for the symbols in the formula.)

In Patent Reference 6, it is reported that the compound shown by formula (F) including a broad range of compounds have a GPR40 receptor regulating action and are useful as an insulin secretion promoting agent and a preventive or therapeutic agent for diabetes mellitus. However, there is no illustrative disclosure on the nitrogen-containing bicyclic ring of this application in the ring B which corresponds to the nitrogen-containing bicyclic ring of this application. 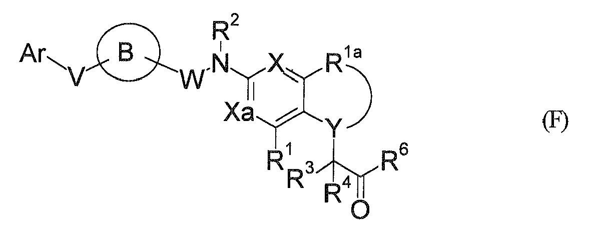 (See said official gazette for the symbols in the formula.)

In Patent Reference 7, it is reported that the compound shown by formula (G) including a broad range of compounds have a GPR40 receptor regulating action and are useful as a preventive or therapeutic agent for diabetes mellitus, obesity and the like. However, the Y corresponding to the nitrogen-containing bicyclic ring of this application is limited aryl or heteroaryl. 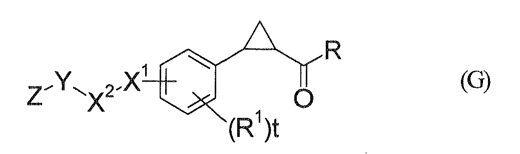 (In the formula, X¹ represents -NH-, and X² a -C(R⁵)₂-, and Y an aryl or hetero aryl. See said official gazette for other symbols.)

In Patent Reference 8, it is reported that the compound shown by formula (H) including a broad range of compounds have a GPR40 receptor regulating action and are useful as an insulin secretion promoting agent and a preventive or therapeutic agent for diabetes mellitus, high blood pressure and the like. However, the P corresponding to the nitrogen-containing bicyclic ring of this application is limited to aromatic ring, hetero aromatic ring, (C₃-C₈) hetero cycloalkylene or (C₃-C₈) cycloalkylene.

Q-L¹-P-L²-M-X-L³-A (H)

(A in the formula means -CO₂H or the like; and L³ a bond, (C₁-C₅) alkylene or (C₂-C₅) hetero alkylene; X a CR³R⁴,N(R⁵), O or S(O)ₙ; M a hetero aromatic ring, (C₅-C₈) cycloalkylene, aryl (C₁-C₄) alkylene or hetero aryl (C₁-C₄) alkylene; L² a bond, (C₁-C₆) alkylene, (C₂-C₆) alkylene, oxymethylene, O or the like; P an aromatic ring, hetero aromatic ring, (C₃-C₈) hetero cycloalkylene or (C₃-C₈) cycloalkylene. See said official gazette for other symbols.)

In Patent Reference 9, it is reported that the compounds shown by formula (J) have a PPAR receptor agonist action and are useful as a preventive or therapeutic agent for diabetes mellitus and the like. However, there is no illustrative disclosure on the compounds of the instant application. 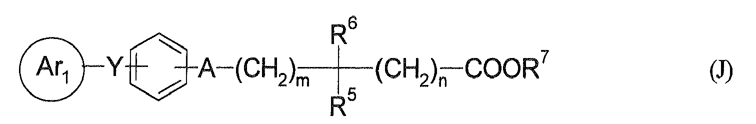 (See said official gazette for the symbols in the formula.)

In Patent Reference 10, it is reported that the compounds shown by formula (K) have a PPAR receptor agonist action and are useful as an insulin secretion promoting agent and a preventive or therapeutic agent for diabetes mellitus and the like. However, the compound (J) has a 1,3-dicarbonyl structure. 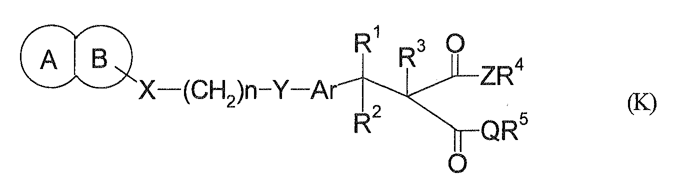 (See said official gazette for the symbols in the formula.)

In Patent Reference 11, it is reported that the compounds shown by formula (L) have a PPAR receptor agonist action and are useful as a preventive or therapeutic agent for diabetes mellitus and the like. However, there is no illustrative disclosure on the nitrogen-containing bicyclic ring of this application regarding the Z which corresponds to the nitrogen-containing bicyclic ring of this application. 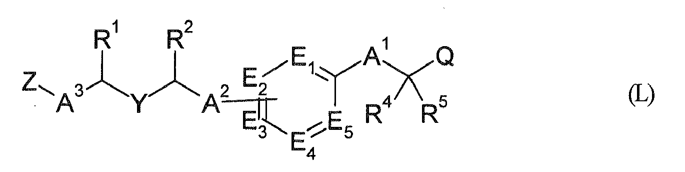 (In the formula, Q means C(O)OR⁶ or R^{6A}; and A¹ a bond, CH₂, O or S; A² and A³ each independently CH₂, O or S; Y a bond, C₁-C₆ alkyl or C₃-C₆ cycloalkyl; and Z an aryl, 5- to 10-membered hetero aryl, bi-aryl or bi-heteroaryl. See said official gazette for other symbols)

Non-patent Reference 1: Nature, (England), 2003, vol. 422, p. 173 - 176
Patent Reference 1: International Publication No. 2004/041266
Patent Reference 2: International Publication No. 2005/063729
Patent Reference 3: International Publication No. 2005/063725
Patent Reference 4: International Publication No. 2005/095338
Patent Reference 5: International Publication No. 2004/106276
Patent Reference 6: International Publication No. 2005/087710
Patent Reference 7: International Publication No. 2005/051890
Patent Reference 8: International Publication No. 2005/086661
Patent Reference 9: International Publication No. 2005/040102
Patent Reference 10: International Publication No. 2004/048338
Patent Reference 11: International Publication No. 2005/19151

### DISCLOSURE OF THE INVENTION

### PROBLEMS THAT THE INVENTION IS TO SOLVE

The invention aims at providing a novel compound which has a GPR40 receptor agonist action and is useful as an insulin secretion promoting agent and a preventive or therapeutic agent for diabetes mellitus.

### MEANS FOR SOLVING THE PROBLEMS

When the inventors have conducted intensive studies on compounds having GPR40 receptor agonist action, it was found that novel carboxylic acid derivatives or salts thereof, **characterized in that** carboxylic acid is linked to a 6-membered monocyclic aromatic ring via two atoms and said aromatic ring is linked to a nitrogen-containing bicyclic ring via a linker, have excellent GPR40 receptor agonist action. By further finding that these carboxylic acid derivatives have excellent insulin secretion promoter action and can strongly suppress blood glucose increase after glucose tolerance test, the invention has been accomplished.
That is, the invention relates to a carboxylic acid derivative represented by the following formula (I) or a pharmaceutically acceptable salt thereof. 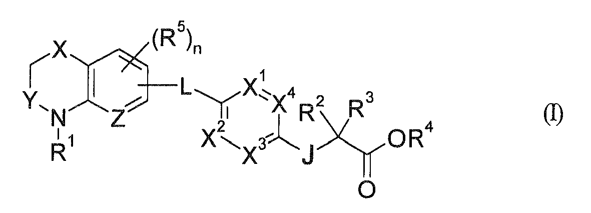 (Symbols in the formula represent the following meanings;
R¹: -H, lower alkyl, halogeno-lower alkyl, cycloalkyl, aryl, heterocyclic group, lower alkylene-R^{A}, -C(O)R^{B}, -CO₂R^{B} or -S(O)ₚR^{B},
with the proviso that the lower alkylene, aryl and heterocyclic group in R¹ may be respectively substituted,
R^{A}: cycloalkyl, aryl, heterocyclic group, -S(O)ₚR⁰, -S(O)ₚ-aryl, -S(O)ₚ-heterocyclic group, -C(O)R⁰, -C(O)-aryl, -C(O)-heterocyclic group, -CO₂R⁰, -OR⁰, -O-aryl, -O-heterocyclic group, -N(R⁰)₂, -N(R⁰)-aryl, -N(R⁰)-heterocyclic group, -CO(R⁰)(aryl)₂, -C(O)N(R⁰)-cycloalkyl or -C(O)N(R⁰)-aryl,
with the proviso that the aryl and heterocyclic group in R^{A} may be respectively substituted, R^{B}: lower alkyl, halogeno-lower alkyl, cycloalkyl, aryl, heterocyclic group, lower alkylene-cycloalkyl, lower alkylene-aryl, lower alkylene-heterocyclic group, lower alkylene-OR⁰, lower alkylene-O-aryl or lower alkylene-S(O)₂NH₂,
with the proviso that the aryl and heterocyclic group in R^{B} may be respectively substituted, R⁰: -H or lower alkyl,
n and p: the same or different from each other and each represents 0, 1 or 2,
J: -C(R⁶)(R⁷)-, -O- or -S-,
R², R³, R⁶ and R⁷: the same or different from one another and each represents -H, halogen, lower alkyl, -OR⁰ or aryl,
with the proviso that R² and R³, R³ and R⁶ and R⁶ and R⁷ may together form a lower alkylene,
R⁴_{:} -H or lower alkyl,
X: single bond, -CH₂-, -(CHO₂)₂-, -O-, -S-, -S(O)- or -S(O)₂-,
Y: -CH₂- or -C(O)-,
Z: C(-*), C(R⁸), N or N(O), with the proviso that the * in Z means binding to L,
X¹ and X²: the same or different from each other and each represents C(R⁹), N or N(O),
X³ and X⁴: the same or different from each other and each represents C(R¹⁰), N or N(O),
R⁵: lower alkyl, halogen, halogeno-lower alkyl, -OR⁰ or -O-halogeno-lower alkyl,
R⁸, R⁹ and R¹⁰: the same or different from one another and each represents -H, lower alkyl, halogen, halogeno-lower alkyl, -OR⁰ or -O-halogeno-lower alkyl,
with the proviso that R⁶ and R¹⁰ may together form a lower alkylene, -O-lower alkylene or lower alkylene-O-,
L: -O-lower alkylene, lower alkylene-O-, -N(R¹¹)-lower alkylene, lower alkylene-N(R¹¹)-, -O-lower alkylene-O-, -N(R¹¹)-lower alkylene-N(R¹¹)-, -O-lower alkylene-N(R¹¹)- or -N(R¹¹)-lower alkylene-O-, and
R¹¹: -H, lower alkyl or -C(O)R⁰. The same shall apply hereinafter.)

In addition, this application also relates to a pharmaceutical, particularly a GPR40 agonist which uses a compound represented by the general formula (I) or a salt thereof as the active ingredient.
Further, this application also relates to the use of a compound represented by the formula (I) or a pharmaceutically acceptable salt thereof for the production of a GPR40 agonist, an insulin secretion promoter or a preventive and/or therapeutic agent for diabetes mellitus, and a method for preventing and/or treating diabetes mellitus, which comprises administering an effective amount of the compound represented by the formula (I) or a pharmaceutically acceptable salt thereof to a patient.
That is,
(1) a pharmaceutical composition, which comprises a compound described by the formula (I) or a pharmaceutically acceptable salt thereof and a pharmaceutically acceptable carrier,
(2) the pharmaceutical composition described in (1), which is a GPR40 agonist,
(3) the pharmaceutical composition described in (1), which is an insulin secretion promoter,
(4) the pharmaceutical composition described in (1), which is an agent for preventing and/or treating diabetes mellitus,
(5) use of a compound described by the formula (I) or a pharmaceutically acceptable salt thereof, for producing a GPR40 agonist, an insulin secretion promoter or a preventive and/or therapeutic agent for diabetes mellitus, and
(6) a method for preventing and/or treating diabetes mellitus, which comprises administering an effective amount of a compound described by the formula (I) or a salt thereof to a patient.

### ADVANTAGE OF THE INVENTION

The compound of the invention has excellent GPR40 receptor agonist activity and therefore is useful as an insulin secretion promoter and a preventive or therapeutic agent for diabetes mellitus (insulin-dependent diabetes mellitus (IDDM), non insulin-dependent diabetes mellitus (NIDDM) and their boundary type (abnormal glucose resistance and fasting blood glucose level) slight diabetes mellitus) and the like diseases in which GPR40 is concerned.

### BEST MODE FOR CARRYING OUT THE INVENTION

The following describes the invention in detail.
According to the definitions in this specification, unless otherwise noted, the "lower alkyl" and "lower alkylene" mean straight or branched hydrocarbon chains preferably having from 1 to 6 carbon atoms (to be referred to as C₁₋₆ hereinafter).

Preferred as the "lower alkyl" is a C₁₋₆ alkyl (methyl, ethyl, n-propyl, isopropyl, n-butyl, isobutyl, sec-butyl, tert-butyl, n-pentyl and n-hexyl groups and the like). More preferred is a C₁₋₄ alkyl, and particularly preferred are methyl, ethyl, n-propyl or isopropyl.

Preferred as the "lower alkylene" is a C₁₋₆ alkylene (methylene, ethylene, trimethylene, tetramethylene, pentamethylene, hexamethylene, propylene, methylmethylene, ethylethylene, 1,2-dimethylethylene and 1,1,2,2-tetramethylethylene groups and the like). More preferred is a C₁₋₅ alkylene, and particularly preferred are methylene, ethylene or trimethylene.

The "halogen" means fluoro, chloro, bromo and iodo.
The "halogeno-lower alkyl" means a C₁₋₆ alkyl substituted by one or more halogens (fluoromethyl, difluoromethyl, trifluoromethyl, 2,2,2-trifluoroethyl, pentafluoroethyl and hexafluoropropyl groups and the like). Preferred is a lower alkyl substituted by 1 to 5 halogens, and more preferred is trifluoromethyl.

The "cycloalkyl" is a C₃₋₁₀ saturated hydrocarbon ring group which may have a bridge. Preferred is a C₃₋₈ cycloalkyl, more preferred is cyclopropyl, cyclobutyl, cyclopentyl, cyclohexyl, cycloheptyl or cyclooctyl, and particularly preferred is cyclopropyl, cyclobutyl, cyclopentyl or cyclohexyl.

The "cycloalkenyl" is a C₃₋₁₀ cycloalkenyl which may have a bridge and two or more double bonds. Preferred is cyclopentenyl, cyclopentadienyl, cyclohexenyl or cyclohexadienyl. More preferred is a C₅₋₁₀ cycloalkenyl, and particularly preferred is cyclopentenyl or cyclohexenyl.

The "aryl" is a C₆₋₁₄ monocyclic to tricyclic aromatic hydrocarbon ring group, which includes ring groups condensed with C₅₋₈ alkane and C₅₋₈ alkene. Preferred is phenyl, naphthyl, tetrahydronaphthyl, indenyl or fluorenyl, more preferred is phenyl or naphthyl, and further more preferred is phenyl.

The "heterocyclic group" means a ring group consisting of i) a monocyclic 3- to 8-membered (preferably 5- to 7-membered) hetero ring which contains 1 to 4 hetero atoms selected from O, S and N or ii) a bicyclic 8- to 14-membered (preferably 9- to 11-membered) hetero ruing or tricyclic 11- to 20-membered (preferably 12- to 15-membered) hetero ring, which contains 1 to 5 hetero atoms selected from O, S and N and is formed by the condensation of said monocyclic hetero ring with 1 or 2 rings selected from the group consisting of monocyclic hetero ring, benzene ring, C₅₋₈ cycloalkane and C₅₋₈ cycloalkene. The S or N as a ring atom may be oxidized to form oxide or dioxide.
Preferred as the "heterocyclic group" is aziridinyl, azetidinyl, pyrrolidinyl, piperidinyl, piperazinyl, homopiperazinyl, oxiranyl, oxetanyl, tetrahydrofuranyl, tetrahydropyranyl, morpholinyl, homomorpholinyl, tetrahydrothienyl, tetrahydrothiopyranyl, thiomorpholinyl, pyrrolyl, imidazolyl, pyrazolyl, triazolyl, tetrazolyl, pyridinyl, pyrimidinyl, pyrazinyl, furyl, thienyl, oxazolyl, oxadiazolyl, thiazolyl, thiadiazolyl, indolyl, benzimidazolyl, quinolyl, quinazolyl, quinoxalinyl, naphthyridinyl, benzofuranyl, benzothienyl, benzoxazolyl, benzothiazolyl, dihydroindolyl, dihydrobenzimidazolyl, dihydrobenzofuranyl, tetrahydroquinolyl, benzodioxolyl, dihydrobenzoxazinyl, tetrahydronaphthyridinyl, dihydropyridoxazinyl, carbazolyl or quinuclidinyl, more preferred is pyrrolidinyl, piperidinyl, piperazinyl, tetrahydrofuranyl, tetrahydropyranyl, morpholinyl, pyridinyl, pyrimidinyl, pyrazinyl, furyl, thienyl, oxazolyl, oxadiazolyl, thiazolyl, thiadiazolyl, indolyl, benzimidazolyl, quinolyl, quinazolyl, quinoxalinyl, benzofuranyl, benzothienyl, benzoxazolyl or benzothiazolyl, and particularly preferred is piperidinyl, morpholinyl, pyrazinyl, pyridyl, pyrimidinyl, pyrazinyl, furyl, thienyl, oxazolyl, thiazolyl, indolyl, benzofuranyl, benzothienyl or quinolyl.

The term "may be substituted" means "no substitution" or "has 1 to 5 substituent groups which are the same or different from one another". In this connection, when it has two or more substituent groups, these substituent groups may be the same or different from one another.

As the "aryl" and "heterocyclic group" in R¹ which may be respectively substituted; the "aryl" and "heterocyclic group" in R^{A} which may be respectively substituted; and the "aryl" and "heterocyclic group" in R^{B} which may be respectively substituted, preferred are groups selected from the following group G.
Group G: halogen, -CN, lower alkyl, halogeno-lower alkyl, -OR⁰, -O-halogeno-lower alkyl, oxo, aryl, heterocyclic group, -N(R⁰)₂, -N(R⁰)C(O)R⁰, -N(R⁰)S(O)₂-aryl, lower alkylene-OR⁰, -O-lower alkylene-OR⁰, lower alkylene-aryl and lower alkylene-heterocyclic group. However, the aryl and heterocyclic group in group G may be substituted by halogen, lower alkyl, halogeno-lower alkyl, -OR⁰ or -O-halogeno-lower alkyl.

Preferred as the substituent group of the "lower alkylene" which may be substituted in R¹ is a group selected from halogen and -OR⁰.

Preferred embodiments of the invention are shown in the following.
(a) Preferred as R¹ is lower alkyl, halogeno-lower alkyl, aryl, heterocyclic group, lower alkylene-OR⁰, lower alkylene-aryl, lower alkylene-heterocyclic group or lower alkylene-O-aryl. However, the aryl and heterocyclic group may be substituted by a group selected from halogen, -CN, lower alkyl, halogeno-lower alkyl, -OR⁰ and -O-halogeno-lower alkyl. More preferred as R¹ is phenyl, pyridyl, lower alkylene-phenyl or lower alkylene-O-phenyl. However, phenyl and pyridyl may be substituted by a group selected from halogen, -CN, lower alkyl, halogeno-lower alkyl, -OR⁰ and -O-halogeno-lower alkyl.
(b) Preferred as X is -CH₂- or -O-, and more preferred is -CH₂-.
(c) Preferred as Y is -CH₂-.
(d) Preferred as Z is CH, C(lower alkyl), C(-*) or N, and more preferred is CH, C(lower alkyl) or N.
(e) Preferred as L is -O-lower alkylene, -NH-lower alkylene, -lower alkylene-O- or lower alkylene-NH-, and more preferred is *-CH₂-NH- or *-CH₂-O-. However, * means binding to the nitrogen-containing bicyclic ring group to which R¹ is bonded.
(f) Preferred as X¹ and X² are the same or different from each other and each is CH or N, and more preferred is CH.
(g) Preferred as Preferred as X³ and X⁴ are the same or different from each other and each is CH or C(halogen), more preferably one is CH and the other is C(halogen).
(h) Preferred as J is -C(R⁶)(R⁷)-.
(i) Preferred as R², R³, R⁶ and R⁷ is -H. Alternatively, as another embodiment, preferably R² and R⁷ is -H and R³ and R⁶ together form lower alkylene, more preferably R² and R7 is -H and R³ and R⁶ together form methylene.
(j) Preferred as R⁴ is -H.
(l) Preferred as R⁵ is halogen or lower alkyl.
(m) Preferred as n is 0 pr 1, more preferably 0.
(n) When the nitrogen-containing bicyclic ring group to which R¹ is bonded is 1,2,3,4-tetrahydroquinolyl, preferred as the substituting position of L is 5- or 7-position, more preferably 5-position. When the nitrogen-containing bicyclic ring group to which R¹ is bonded is 5,6,7,8-tetrahydronaphthyridyl, preferred as the substituting position of L is 2- or 4-position.
As another preferred embodiment, a compound consisting of the combination of respective preferred groups described in the aforementioned (a) to (n) is preferable.

In addition, another preferred embodiments of the compound of the invention shown by the general formula (I) are shown in the following.
(1) A compound described by the formula (I), wherein J is -C(R⁶)(R⁷)-.
(2) Thecompound described in (1), wherein X and Y are -CH₂-.
(3) The compound described in (2), wherein L is *-CH₂-NH- or *-CH₂-O- (however, * means binding to the nitrogen-containing bicyclic ring group to which R¹ is bonded).
(4) The compound described in (3), wherein Z is CH, C(lower alkyl), C(*) (however, * means binding to L) or N.
(5) The compound described in (4), wherein n is 0; or n is 1, and R⁵ is halogen or lower alkyl.
(6) The compound described in (5), wherein X¹ and X² are the same or different from each other and each is CH or N, and X³ and X⁴ are the same or different from each other and each is CH or C(halogen).
(7) The compound described in (6), wherein R², R³, R⁶ and R⁷ are H.
(8) The compound described in (7), wherein R⁴ is -H.
(9) The compound described in (8), wherein R¹ is lower alkyl, halogeno-lower alkyl, aryl, heterocyclic group, lower alkylene-OR⁰, lower alkylene-aryl, lower alkylene-heterocyclic group or lower alkylene-O-aryl (however, the aryl and heterocyclic group in R¹ may be substituted by a group selected from halogen, -CN, lower alkyl, halogeno-lower alkyl, -OR⁰ and -O-halogeno-lower alkyl).
(10) The compound described by the formula (I), which is selected from the group consisting of 3-[2-fluoro-4-({[1-(2-phenylethyl)-1,2,3,4-tetrahydroquinolin-5-yl]methyl}amino)phenyl]propanoic acid, 3-[2-fluoro-4-({[1-(2-phenoxyethyl)-1,2,3,4-tetrahydroquinolin-5-yl]methyl}amino)phenyl]propanoic acid, 3-(2-fluoro-4-{[(8-methyl-1-propyl-1,2,3,4-tetrahydroquinolin-7-yl)methyl]amino}phenyl)propanoic acid, 3-[2-fluoro-4-({[8-methyl-1-(2-phenylethyl)-1,2,3,4-tetrahydroquinolin-7-yl]methyl}amino)phenyl]propanoic acid, 3-(2-fluoro-4-{[(8-methyl-1-phenyl-1,2,3,4-tetrahydroquinolin-7-yl)methyl]amino}phenyl)propanoic acid, 3-(2-fluoro-4-{[(8-phenyl-5,6,7,8-tetrahydro-1,8-naphthyridin-2-yl)methyl]amino}phenyl)propanoic acid, 3-{2-fluoro-4-[({1-[2-(4-methoxyphenyl)ethyl]-1,2,3,4-tetrahydroquinolin-5-yl}methyl)amino]phenyl}propanoic acid, 3-{2-fluoro-4-[({1-[2-(4-fluorophenoxy)ethyl]-1,2,3,4-tetrahydroquinolin-5-yl}methyl)amino]phenyl}propanoic acid, 3-{4-[({1-[2-(3-chlorophenoxy)ethyl]-1,2,3,4-tetrahydroquinolin-5-yl}methyl)amino]-2-fluorophenyl}propanoic acid, 3-[2-fluoro-4-({[1-(3-fluorophenyl)-8-methyl-1,2,3,4-tetrahydroquinolin-7-yl]methyl}amino)phenyl]propanoic acid, 3-{2-fluoro-4-({[8-methyl-1-(3-methylphenyl)-1,2,3,4-tetrahydroquinolin-7-yl]methyl}amino)phenyl}propanoic acid, and 3-[4-({[1-(3,4-difluorophenyl)-8-methyl-1,2,3,4-tetrahydroquinolin-7-yl]methyl}amino)-2-fluorophenyl]propanoic acid,
or pharmaceutically acceptable salts thereof.

Depending on the kinds of substituent groups, there are cases in which other tautomers and geometrical isomers are present in the compound of the invention. In this specification, only one form of these isomers is described in some cases, but these isomers are included in the invention and separated isomers or mixtures thereof are also included therein.
Also, the compound (I) sometimes has an asymmetric atom and axial asymmetry, and (R) form, (S) form and the like optical isomers based thereon can be present. The invention includes all of the mixtures and isolated counterparts of these optical isomers.
Further, pharmacologically acceptable prodrugs of the compound (I) are also included in the invention. The pharmacologically acceptable prodrugs are compounds which have groups that can be converted into amino group, OH, CO₂H and the like of the invention by solvolysis or under a physiological condition. As the groups which form prodrugs, the groups described for example in Prog. Med., 5, 2157 - 2161 (1985) and "Iyakuhin no Kaihatsu(Development of Medicines)"Hirokawa Shoten, 1990) vol. 7 Bunshi Sekkei (Molecular Design), 163 - 1981, and the like can be exemplified.

In addition, the compounds of the invention form acid addition salts or salts with bases in some cases depending on the kinds of substituent groups, and such salts are included in the invention with the proviso that they are pharmaceutically acceptable salts. Illustratively, acid addition salts with hydrochloric acid, hydrobromic acid, hydriodic acid, sulfuric acid, nitric acid, phosphoric acid and the like inorganic acids or with formic acid, acetic acid, propionic acid, oxalic acid, malonic acid, succinic acid, fumaric acid, maleic acid, lactic acid, malic acid, tartaric acid citric acid, methanesulfonic acid, ethanesulfonic acid, p-toluenesulfonic acid, aspartic acid, glutamic acid and the like organic acids, salts with sodium, potassium, magnesium, calcium, aluminum and the like inorganic bases or with methylamine, ethylamine, ethanolamine, lysine, ornithine and the like organic bases, ammonium salts and the like can be exemplified.
Further, the invention also includes various hydrates and solvates and polymorphic substances of the compounds of the invention and pharmaceutically acceptable salts thereof. In addition, the invention also includes compounds labeled with various radioisotopes or non-radioactive isotopes.

### (Production methods)

The compounds of the invention and pharmaceutically acceptable salts thereof can be produced by employing various conventionally known synthesis methods, making use of the characteristics based on their basic backbones or kinds of substituent groups. In that case, depending on the kinds of functional group, it is sometimes effective in view of production techniques to replace said functional group with an appropriate protecting group (a group which can be easily converted into said functional group) at the stage of materials to intermediates. Examples of such functional group include amino group, hydroxyl group , carboxyl group and the like, and as their protecting groups, the protecting groups described, for example, in "Protective Groups in Organic Synthesis", edited by Greene and Wuts (3rd edition, 1999) can be exemplified, and these may be optionally selected and used in response to the reaction conditions. In such a method, the desired compound can be obtained by introducing said protecting group to carry out the reaction and then removing the protecting group in response to the necessity.
In addition, prodrugs of the compound (I) can be produced in the same manner as the case of the aforementioned protecting groups, by introducing a specified group at the stage of materials to intermediates or carrying out the reaction using the obtained compound (I). The reaction can be carried out by employing usual esterification, amidation, dehydration and the like methods conventionally known by those skilled in the art.
The following describes typical production methods of the compound of the invention. Each production method can also be carried out by referring to the references put to said descriptions. In this connection, the production methods of the invention are not limited to the examples shown in the following.

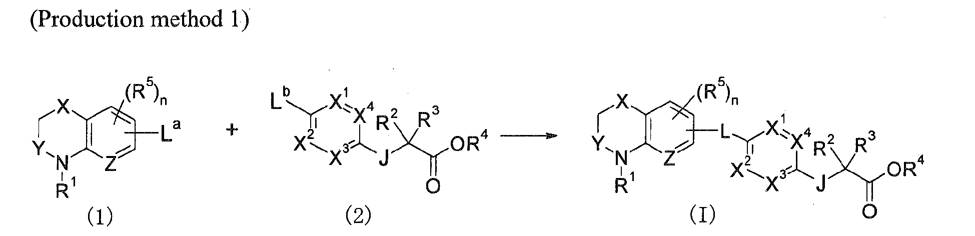

(In the formulae, one of L^{a} and L^{b} means -OH or -N(R^{p})H, and the other a lower alkylene-OH-, -O-lower alkylene-OH or -N(R¹¹)-lower alkylene-OH, and R^{p} a protecting group.)
This production method is a method in which the compound (I) of the invention is obtained by allowing a compound (1) to react with a compound (2). The protecting group of R^{p} is not particularly limited with the proviso that it can be used as the protecting group of Mitsunobu reaction, but 2-nitrobenzenesulfonyl group or the like can for example be used. The method described in the aforementioned "Protective Groups in Organic Synthesis" can be used for the deprotection of R^{p}.
The reaction is carried out using equivalent amounts of the compound (1) and compound (2), or one of them in an excess amount, in a reaction-inert solvent in the presence of triphenylphosphine, tributylphosphine or the like phosphine and diethyl azodicarboxylate, di-t-butyl azodicarboxylate, 1,1'-(azodicarbonyl)dipiperidine or the like azo reagent, by stirring them generally from 0.1 hour to 5 days under cooling to heat reflux, preferably at from 0°C to 80°C. As the solvent, for example, benzene, toluene, xylene and the like aromatic hydrocarbons, diethyl ether, tetrahydrofuran (THF), dioxane, dimethoxyethane and the like ethers or dichloromethane, 1,2-dichloroethane, chloroform and the like halogenated hydrocarbons can be used.

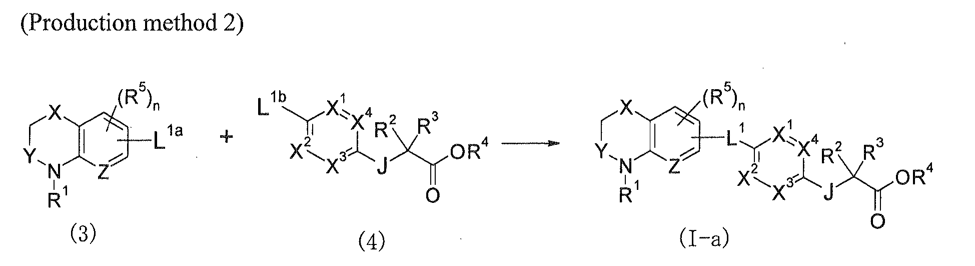

(Symbols in the formulae represent the following.
L^{1a} and L^{1b}: one is -OH and the other is lower alkylene-Lv, -O-lower alkylene-Lv or -N(R¹¹)-lower alkylene-Lv.
L¹: lower alkylene-O-, -O-lower alkylene, -O-lower alkylene-O-, -N(R¹¹)-lower alkylene-O-or -O-lower alkylene-N(R¹¹)-.
Lv: leaving group. The same shall apply hereinafter.)
This production method is a method in which a compound (I-a) of the invention is obtained by allowing a compound (3) to react with a compound (4). In this connection, as the leaving group of Lv, halogen, methanesulfonyloxy, p-toluenesulfonyloxy and the like can for example be cited.
The reaction is carried out using equivalent amounts of the compound (3) and compound (4), or one of them in an excess amount, in a reaction-inert solvent in the presence of a base, by stirring them generally from 0.1 hour to 5 days under cooling to heat reflux, preferably at from 0°C to 80°C. As the solvent, it is not particularly limited, but for example, aromatic hydrocarbons, ethers, halogenated hydrocarbons, N,N-dimethylformamide (DMF), dimethylacetamide (DMA), N-methylpyrrolidone (NMP), dimethyl sulfoxide (DMSO), ethyl acetate, acetonitrile or mixtures thereof can be cited. As the base, triethylamine, N,N-diisopropylethylamine, 1,8-diazabicyclo[5.4.0]-7-undecene, n-butyl lithium and the like organic bases and sodium carbonate, potassium carbonate, sodium hydride, potassium tert-butoxide and the like inorganic bases can be exemplified. In some cases, it is desirable to carry out this reaction in the presence of tetra-t-butylammonium chloride or the like phase-transfer catalyst.

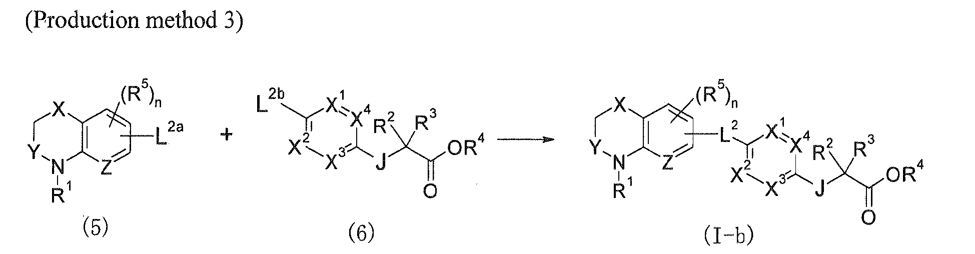

(Symbols in the formulae represent the following.
L^{2a} and L^{2b}: one is -N(R¹¹)-H and the other is lower alkylene-Lv, -O-lower alkylene-Lv or -N(R¹¹)-lower alkylene-Lv.
L²: lower alkylene-N(R¹¹)-, -N(R¹¹)-lower alkylene, -O-lower alkylene-N(R¹¹)-,
-N(R¹¹)-lower alkylene-O- or -N(R¹¹)-lower alkylene-N(R¹¹)-.
The same shall apply hereinafter.)
This production method is a method in which a compound (I-b) of the invention is obtained by allowing a compound (5) to react with a compound (6).
The reaction is carried out using equivalent amounts of the compound (5) and compound (6), or one of them in an excess amount, in a reaction-inert solvent or under no solvent, by stirring them generally from 0.1 hour to 5 days under cooling to heat reflux, preferably at from 0°C to 80°C. In this case, the solvent is not particularly limited, but for example, aromatic hydrocarbons, ethers, halogenated hydrocarbons, N,N-dimethylformamide, dimethylacetamide, ethyl acetate, acetonitrile or mixtures thereof can be cited. In view of effecting smooth advance of the reaction, it is sometimes advantageous to carry out the reaction in the presence of triethylamine, N,N-diisopropylethylamine, N-methylmorpholine or the like organic base or sodium hydride, potassium carbonate, sodium carbonate, cesium carbonate, potassium hydroxide or the like inorganic base.

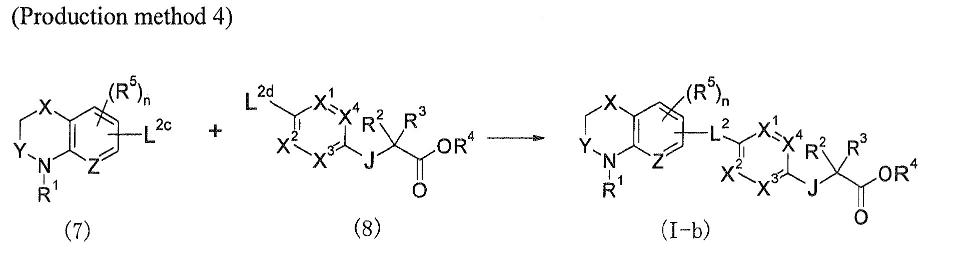

(Symbols in the formulae represent the following.
L^{2c} and L^{2d}: one is -N(R¹¹)H and the other is -CHO, C₁₋₅ alkylene-CHO, -O-C₁₋₅ alkylene-CHO or -N(R¹¹)-C₁₋₅ alkylene-CHO. The same shall apply hereinafter.)
This production method is a method in which the compound (I-b) of the invention is obtained by allowing a compound (7) to react with a compound (8).
The reaction is carried out using equivalent amounts of the compound (7) and compound (8), or one of them in an excess amount, in a reaction-inert solvent in the presence of a reducing agent, by stirring them generally from 0.1 hour to 5 days at from -45°C to under heat reflux, preferably at from 0°C to room temperature. In this case, the solvent is not particularly limited, but for example, methanol, ethanol and the like alcohols, ethers, halogenated hydrocarbons, aromatic hydrocarbons, acetonitrile or a mixture thereof and the like can be cited. As the reducing agent, sodium borohydride cyanide, sodium triacetoxy borohydride, sodium borohydride and the like can be exemplified. It is desirable in some cases to carry out the reaction in the presence of Molecular Sieves or the like dehydrating agent or acetic acid, hydrochloric acid, titanium(IV) isopropoxide complex or the like acid. Depending on the reaction, when the imine compound formed in the reaction system as the intermediate can be stably isolated, a reduction reaction may be separately carried out after obtaining said imine compound. In addition, it can be carried out in methanol, ethanol, ethyl acetate or the like solvent in the presence or absence of acetic acid, hydrochloric acid or the like acid, using a reduction catalyst (e.g., palladium carbon, Raney nickel or the like) instead of the aforementioned treatment with a reducing agent. In this case, it is desirable to carry out the reaction at from 0°C to under heating in an atmosphere of hydrogen under from ordinary pressure to 50 atmospheric pressure.

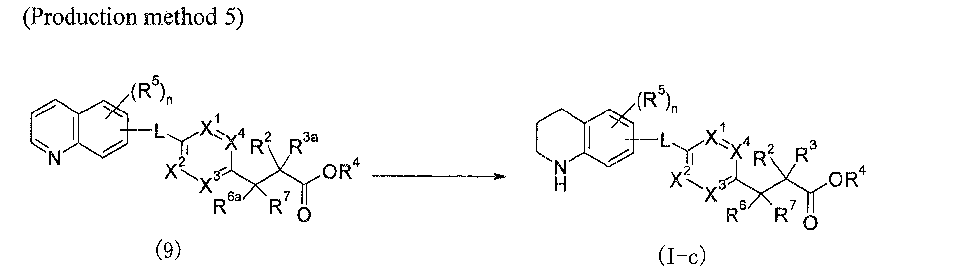

(In the formulae, R^{3a} and R^{6a} respectively mean R³ and R⁶ or a lower alkylene or a bond as one body of R^{3a} and R^{6a}. The same shall apply hereinafter.)
This production method is a method in which a compound (I-c) of the invention is obtained by reducing the quinoline ring of a compound (9).
The reaction can be carried out under cooling to heating, in a solvent such as alcohols, acetic acid or the like in the presence of nickel chloride and sodium borohydride or sodium cyanoborohydride.
When R^{3a} and R^{6a} together form a bond, reduction of double bond can be simultaneously carried out.

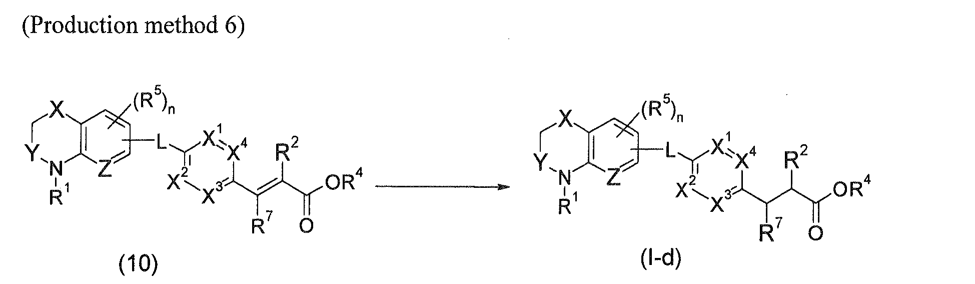

This production method is a method in which a compound (I-d) of the invention is obtained by reducing a double bond of a compound (10).
The reaction is carried out in a solvent such as methanol, ethanol, 2-propanol and the like alcohols, diethyl ether, tetrahydrofuran, dioxane, dimethoxyethane and the like ethers, water, ethyl acetate, N,N-dimethylformamide and the like, by stirring the compound (10) in the presence of a metal catalyst generally from 1 hour to 5 days in an atmosphere of hydrogen. This reaction is generally carried out under cooling to under heating, preferably at room temperature. As the metal catalyst, palladium carbon, palladium black, palladium hydroxide and the like palladium catalysts, platinum plate, platinum oxide and the like platinum catalysts, reduced nickel, Raney nickel and the like nickel catalysts, tetrakistriphenylphospnine chlororhodium and the like rhodium catalysts, reduced iron and the like iron catalysts and the like are suitably used.
Alternatively, this can be carried out under cooling to under heating, in a solvent such as methanol, ethanol and the like alcohols, acetic acid and the like, in the presence of nickel chloride and sodium borohydride or sodium cyanoborohydride.

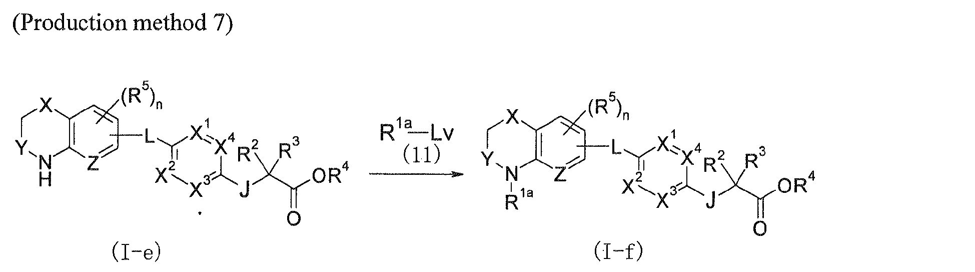

(R^{1a} means lower alkyl, halogeno-lower alkyl, cycloalkyl, aryl, heterocyclic group or lower alkylene-R^{A}. The same shall apply hereinafter.)
This production method is a method in which a compound (I-f) of the invention is obtained by allowing a compound (I-e) to react with a compound (11).
The reaction can be carried out in the same manner as in the production method 3.

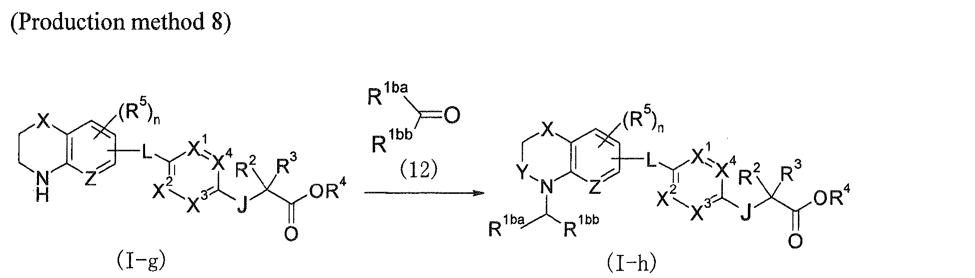

(In the formulae, R^{1ba} and R^{1bb} mean residual part of lower alkyl, halogeno-lower alkyl or lower alkylene-R^{A}, formed in (I-h) together with the carbon atoms to which are bonded.
The same shall apply hereinafter.)
This production method is a method in which a compound (I-h) of the invention is obtained by allowing the compound (I-g) to react with a compound (12).
The reaction can be carried out in the same manner as in the production method 4.

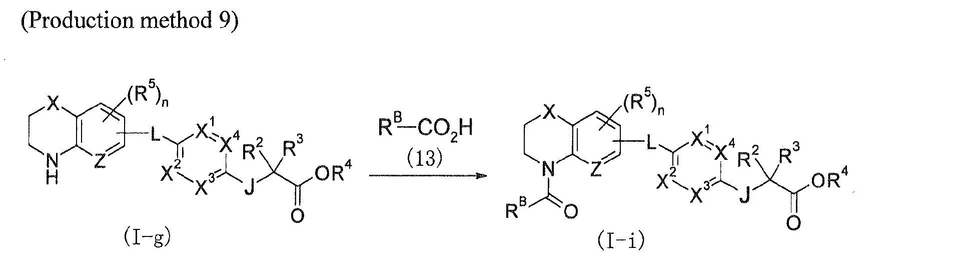

This production method is a method in which a compound (I-i) of the invention is obtained by allowing the compound (I-g) to react with a compound (13).
The reaction is carried out using equivalent amounts of the compound (I-g) and compound (13), or one of them in an excess amount, in a reaction-inert solvent in the presence of a condensing agent, by stirring them generally from 0.1 hour to 5 days at from under cooling to under heating, preferably at from -20°C to 60°C. In this case, the solvent is not particularly limited, but for example, aromatic hydrocarbons, halogenated hydrocarbons, ethers, N,N-dimethylformamide, dimethyl sulfoxide, ethyl acetate, acetonitrile, pyridine or water or a mixture thereof can be cited. As the condensing agent, 1-(3-dimethylaminopropyl)-3-ethylcarbodiimide, dicyclohexylcarbodiimide, 1,1'-carbonyldiimidazole, diphenylphosphoric acid azide, phosphorus oxychloride and the like can be exemplified, though limited thereto. In some cases, it is desirable for the reaction to use an additive agent (e.g., 1-hydroxybenzotriazole or the like). In view of effecting smooth advance of the reaction, it is sometimes advantageous to carry out the reaction in the presence of triethylamine, N,N-diisopropylethylamine, N-methylmorpholine or the like organic base or potassium carbonate, sodium carbonate, potassium hydroxide or the like inorganic base.
In addition, a method in which the carboxylic acid (13) is converted into a reactive derivative and then allowed to react with the amine compound (I-g) can also be used. As the reactive derivative of carboxylic acid, an acid halide obtained by reacting with phosphorus oxychloride, thionyl chloride or the like halogenation agent, a mixed acid anhydride obtained by reacting with isobutyl chloroformate or the like, an active ester obtained by condensing with 1-hydroxybenzotriazole or the like and the like can be exemplified. Reaction of these reactive derivatives with the compound (I-g) can be carried out at from under cooling to under heating, preferably from -20°C to 60°C, in a reaction-inert solvent such as halogenated hydrocarbons, aromatic hydrocarbons, ethers and the like.

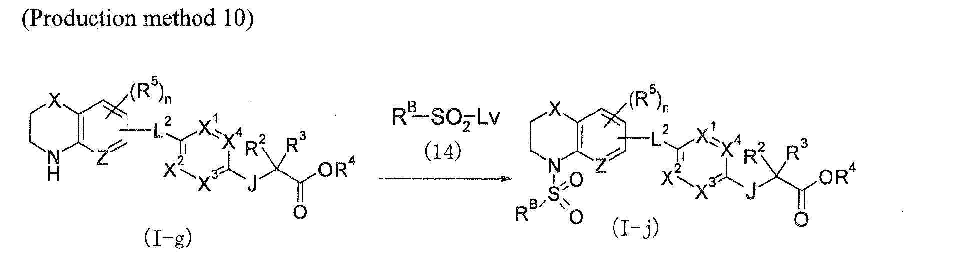

This production method is a method in which a compound (I-j) of the invention is obtained by allowing the compound (I-g) to react with a compound (14).
The reaction is carried out using equivalent amounts of the compound (I-g) and compound (14), or one of them in an excess amount, in a reaction-inert solvent such as halogenated hydrocarbons, aromatic hydrocarbons, ethers and the like, at from under cooling to under heating, preferably from -20°C to 60°C. In view of effecting smooth advance of the reaction, it is sometimes advantageous to carry out the reaction in the presence of triethylamine, N,N-diisopropylethylamine, N-methylmorpholine or the like organic base or potassium carbonate, sodium carbonate, potassium hydroxide or the like inorganic base.

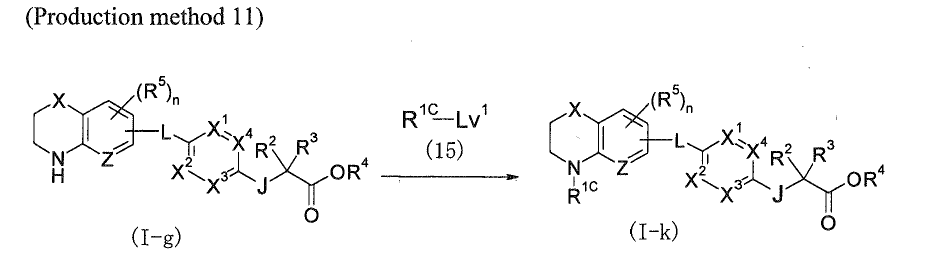

(R^{1c} means aryl or aromatic heterocyclic group, and Lv¹ a leaving group. The same shall apply hereinafter.)
This production method is a method in which a compound (I-k) of the invention is obtained by allowing the compound (I-g) to react with a compound (15). As the Lv¹, for example, halogen, trifluoromethane sulfonyloxy and the like can be cited.
The reaction is carried out under cooling to under heating, using equivalent amounts of the compound (I-g) and compound (15), or one of them in an excess amount, in a reaction-inert solvent such as aromatic hydrocarbons, ethers and the like in the presence of a palladium catalyst, a phosphine ligand and a base. As the palladium catalyst, palladium acetate or dibenzylidene acetone palladium can for example be used, and as the phosphine ligand, 2,2'-bis(diphenylphosphino)-1,1'-binaphthyl (BINAP), dicyclohexyl(2',4',6'-triisopropylbiphenyl-2-yl)phosphine and the like for example, and as the base, cesium carbonate, potassium phosphate and the like for example.

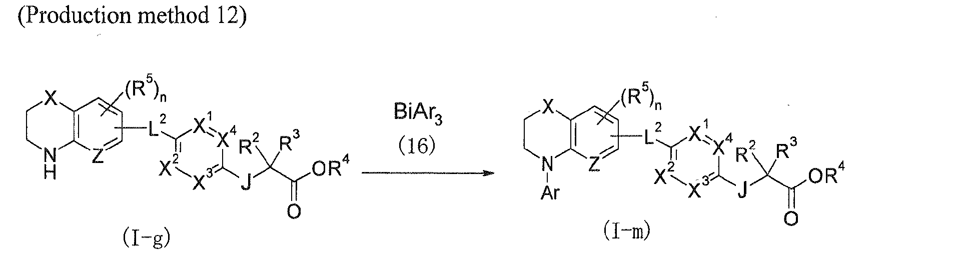

(Ar means aryl. The same shall apply hereinafter.)
This production method is a method in which a compound (I-m) of the invention is obtained by allowing the compound (I-g) to react with a compound (16).
The reaction is carried out under cooling to under heating, using equivalent amounts of the compound (I-g) and compound (16), or one of them in an excess amount, in a reaction-inert solvent such as aromatic hydrocarbons, halogenated hydrocarbons, DMF and the like in the presence of copper acetate.

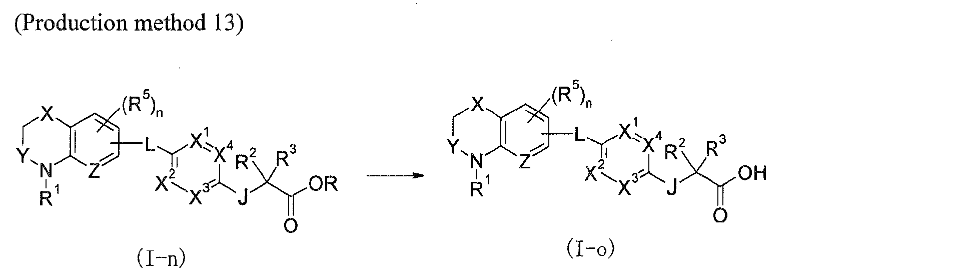

(In the formula, R means lower alkyl. The same shall apply hereinafter)
This production method is a method in which a compound (I-o) of the invention is obtained by hydrolyzing a compound (I-n).
The reaction an be carried out by the method described in the aforementioned "Protective Groups in Organic Synthesis". For example, it can be carried out under cooling to under heating, in a reaction-inert solvent such as aromatic hydrocarbons, ethers, halogenated hydrocarbons, alcohols, DMF, DMA, NMP, DMSO, pyridine, water and the like, in the presence of an acid such as sulfuric acid, hydrochloric acid, hydrobromic acid or the like mineral acid, formic acid, acetic acid or the like organic acid or the like; or in the presence of a base such as lithium hydroxide, sodium hydroxide, potassium hydroxide, potassium carbonate, sodium carbonate, cesium carbonate, ammonia or the like.

In addition, some of the compounds represented by the formula (I) can also be produced from the compounds of the invention obtained in the above manner, by optionally combining alkylation, amidation, oxidation, reduction, hydrolysis and the like steps which can be generally employed by those skilled in the art.

### (Production methods of material compounds)

The materials to be used in the production of the compounds of the invention can be produced by employing, for example, the methods described in the Production Examples which are described later, conventionally known methods or methods obvious for those skilled in the art, or modified methods thereof.

The compounds of the invention are isolated and purified as free compounds or pharmaceutically acceptable salts, hydrates, solvates or polymorphic substances thereof. Pharmaceutically acceptable salt of the compound (I) of the invention can also be producing by subjecting to a general salt formation reaction.
The isolation and purification are carried out by employing extraction, fractional crystallization, various types of fractional chromatography and the like general chemical operations.
Various types of isomers can be separated by selecting appropriate material compounds or making use of the difference in the physicochemical properties between isomers. For example, optical isomers can be separated into stereochemically pure isomers by a general optical resolution method (e.g., a fractional crystallization for introducing into optically active diastereomer salts with a base or acid, a chiral column or the like chromatography or the like). In addition, these can also be produced from appropriate optically active material compounds.

Pharmacological activities of the compounds of the invention were confirmed by the test methods shown below.

### Test method 1: GPR40 agonist activity measurement

### i) Cloning of human GPR40

In accordance with the following procedure, complete length sequence of GPR40 was obtained by a PCR method using a human genomic DNA (Clontech) as the template.
An oligonucleotide consisting of the nucleotide sequence represented by SEQ ID NO:1 was used as the forward primer, and an oligonucleotide consisting of the nucleotide sequence represented by SEQ ID NO:2 as the reverse primer. In this connection, a nucleotide sequence containing a *Xba*I recognition sequence is added to the %7 end of each of the aforementioned forward primer and reverse primer. In the PCR, a cycle consisting of 94°C (15 seconds)/55°C (30 seconds)/72°C (1 minute) was repeated 30 times using a Taq DNA polymerase (Ex Taq DNA polymerase; Takara Bio) in the presence of 5% dimethyl sulfoxide (DMSO). As a result, a DNA fragment of about 0.9 kbp was amplified. This DNA fragment was digested with *Xba*I and then inserted into the *Xba*I site of a plasmid pEF-BOS-dhfr (Nucleic Acids Research, 18, 5322, 1990) to obtain a plasmid pEF-BOS-dhfr-GPR40.
Nucleotide sequence of GPR40 gene in the plasmid pEF-BOS-dhfr-GPR40 was determined by the dideoxy terminator method using a DNA sequencer (ABI 377 DNA Sequencer; Applied Biosystems). Nucleotide sequence of the GPR40 gene was as the nucleotide sequence represented by SEQ ID NO:3. The nucleotide sequence represented by SEQ ID NO:3 was possessed of an open reading frame (ORF) of 903 bases, and the amino acid sequence deduced from this ORF (300 amino acids) was as the amino acid sequence represented by SEQ ID NO:4.

### ii) Preparation of GPR40 stable expression cell

A CHO dhfr cell (a dihydrofolate reductase (dhfr) gene-deficient CHO cell) was used as the cell for expressing GPR40 protein. In addition, the plasmid pEF-BOS-dhfr-GPR40 obtained in the aforementioned i) was used as the expression plasmid for expressing the GPR40 protein. The CHO dhfr cell in 10% fetal calf serum (FCS)-containing αMEM medium was inoculated into a 6 well plate and cultured overnight to a stage of 80 to 90% confluent, and then gene transfer of 2 µg per well of the plasmid pEF-BOS-dhfr-GPR40 was carried out using a transfection reagent (Lipofectamine 2000; Invitrogen). After 24 hours of the culturing since the gene transfer, the cells were diluted and inoculated again. In that case, the αMEM medium containing 10% FCS was changed to αMEM medium which contains 10% FCS but does not contain nucleic acids. After 20 days of culturing, the thus formed colonies of cells were individually recovered and cultured to obtain CHO cells stably expressing GPR40. Cells showing high reactivity for integral ligands oleic acid and linoleic acid were selected from them.

### iii) GPR40 agonist activity measurement

This test was measured by FLIPR (registered trade mark, Molecular Device) using change in the intracellular calcium concentration as the index. The test method is shown below.
A human GPR40-expressed CHO cell strain was inoculated in 6 x 10³ cells per well portions into a 384 well black plate (Becton-Dickinson). A Calcium-3 assay kit (Molecular Device) was used as the luminescence pigment and dissolved in 10 ml per bottle of HBSS-HEPES buffer (pH 7.4, 1 x HBSS, 20 mM HEPES, Invitrogen). A 35.68 mg portion of probenecid (Sigma) was dissolved in 250 µl of 1 M NaOH and then adjusted by adding 250 µl of HBSS-HEPES buffer. The fluorescence pigment solution was prepared by mixing 16 ml of HBSS-HEPES buffer, 640 µl of the fluorescence pigment and 32 µl of probenecid, per plate. The medium in the plate was discarded, and the fluorescence pigment solution was dispensed in 40 µl per well portions and incubated at room temperature for 2 hours. Each compound to be tested was dissolved in DMSO, diluted with HBSS-HEPES buffer and then dispensed in 10 µl portions into the plate to start the reaction and measure change in the intracellular calcium concentration by FLIPR. EC₅₀ values of the compounds to be tested were calculated from the dose-response curve of fluorescence intensity changes one minute after the measurement.
The test results are shown in Table 1. Ex indicates compound numbers of Examples which are described later.

**[Table 1]**

| Ex | EC₅₀ (µM) |
|---|---|
| 57 | 0.097 |
| 73 | 0.073 |
| 74 | 0.075 |
| 86 | 0.019 |
| 87 | 0.082 |
| 89 | 0.62 |
| 104 | 0.42 |
| 176 | 0.091 |
| 188 | 0.036 |
| 211 | 0.93 |
| 215 | 0.32 |
| 424 | 0.13 |
| 437 | 0.73 |
| 453 | 0.025 |
| 455 | 0.36 |

### Test method 2: Insulin secretion promoting action using MIN6 cell

In this test, the insulin secretion promoting action of compounds to be tested was examined using a mouse pancreatic β cell strain, MIN6 cell. The test method is shown below.
The MIN6 cell was inoculated onto a 96 well plate to a density of 5 x 10⁴ cells/well (200 µl). As the medium, DMEM medium (25 mM glucose) containing 10% FBS, 55 µM of 2-mercaptoethanol, 100 U/ml of penicillin and 100 µg/ml of streptomycin was used. Two days thereafter, the medium was removed using an aspirator, and the plate was once washed with 200 µl of KRB-HEPES (116 mM NaCl, 4.7 mM KCl, 1.2 mM KH₂PO₄, 1.2 mM MgSO₄, 0.25 mM CaCl₂, 25 mM NaHCO₃, 0.005% FFA Free BSA, 24 mM HEPES (pH 7.4)) containing 2.8 mM glucose, which had been warmed up to 37°C, and again filled with 200 µl of the same buffer and incubated at 37°C for 1 hour. After removing the aforementioned buffer using an aspirator, this was again washed with the buffer (200 µl), and then a predetermined concentration of each compound to be tested was added to KRB-HEPES containing 2.8 mM or 22.4 mM of glucose, added in 100 µl portions to respective wells and incubated at 37°C for 2 hours. The aforementioned samples were collected and diluted 100 times, and the insulin concentration was determined using an insulin RIA kit (Amersham). The activity value was show by a relative activity value (%) at the time of the addition of 1 µM of each compound, based on the control (DMSO) 100%.
The test results are shown in Table 2. As a result, it was confirmed that the compounds of the invention have excellent insulin secretion promoting action.

**[Table 2]**

| Ex | Insulin secretion promoting action (%) |
|---|---|
| 57 | 167 |
| 104 | 162 |

### Test method 3: Normal mice single administration oral glucose tolerance test

This test examined on the blood glucose suppressive action of compounds to be tested after glucose loading using normal mice. Male ICR mice (6 weeks of age) were reared for 1 week in advance, subjected to overnight fasting and then used as animals to be tested. Each compound to be tested was prepared into a 0.5% methyl cellulose suspension and orally administered at a dose of 10 mg/kg 30 minutes before the glucose (2 g/kg) loading. In the control group, 0.5% methyl cellulose was administered. Blood glucose decreasing ratio (%) at the time of 30 minutes of glucose loading was calculated based on the control group.
The test results are shown in Table 3. As a result, it was confirmed that the compounds of the invention have excellent blood glucose decreasing action.

**[Table 3]**

| Ex | Blood glucose decreasing ratio (%) |
|---|---|
| 89 | 21 |
| 104 | 20 |
| 188 | 24 |
| 453 | 24 |

In addition, it was confirmed on several compounds of the invention that they have excellent pharmacokinetics and are sufficiently separated from the cytochrome P450 (CYP) inhibitory action in which the GPR40 agonist action becomes a cause of drug interaction and the HERG inhibitory action in which the same becomes a cause of Qt prolongation.

From the results of the aforementioned respective tests, it was confirmed that the compounds of the invention have excellent GPR40 agonist action. Based on this, these are useful as an insulin secretion promoter and a preventive or therapeutic agent for diabetes mellitus (insulin-dependent diabetes mellitus (IDDM), non insulin-dependent diabetes mellitus (NIDDM) and their boundary type (abnormal glucose resistance and fasting blood glucose level) slight diabetes mellitus) and the like diseases.

The pharmaceutical preparation which comprises one or two or more of the compounds (I) of the invention or salts thereof as the active ingredient can be prepared by generally used methods using medicinal carriers, fillers and the like which are generally used in said field.
The administration may be either oral administration by tablets, pills, capsules, granules, powders, solutions and the like, or parenteral administration by intraarticular, intravenous, intramuscular and the like injections, suppositories, eye drops, eye ointments, solutions for percutaneous use, ointments, patches for percutaneous use transmucosal solutions, transmucosal patches, inhalations and the like.

As the solid composition for oral administration by the invention, tablets, powders, granules and the like are used. In such a solid composition, one or two more active substances are mixed with at least one inert filler such as lactose, mannitol, glucose, hydroxypropylcellulose, microcrystalline cellulose, starch, polyvinyl pyrrolidone and/or aluminum magnesium silicate or the like. In accordance with the usual way, the composition may contain inert additives such as magnesium stearate and the like lubricants, carboxymethylstarch sodium and the like disintegrators, stabilizes and solubilizing agents. As occasion demands, the tablets or pills may be coated with a sugar coating or a gastric or enteric coating.
As the liquid composition for oral administration, pharmaceutically acceptable emulsions, solutions, suspensions, syrups, elixirs and the like are included, which contain generally used inert diluents such as purified water or ethanol. In addition to the inert diluents, said liquid composition may contain solubilizing agents, moistening agents, suspending agents and the like auxiliary agents, sweeteners, correctives, aromatics and antiseptics.
As the injections for parenteral administration, sterile aqueous or non-aqueous solutions, suspensions and emulsions are included. As the aqueous solvent, for example, distilled water for injection and physiological saline are included. Examples of the non-aqueous solvent include propylene glycol, polyethylene glycol, olive oil or the like plant oil, ethanol or the like alcohols, polysorbate 80 (the name in Pharmacopeia) and the like. Such a composition may further contain tonicity agents, antiseptics, moistening agents, emulsifying agents, dispersing agents, stabilizing agents and solubilizing agents. These are sterilized by, for example, filtration through a bacteria retaining filter, formulation of bactericides or irradiation. In addition, these can also be used by producing a sterile solid compositions and dissolving or suspending them in sterile water or a sterile solvent for injection prior to use.

As the external preparations, ointments, plasters, creams, jellies, cataplasmas, sprays, lotions, eye drops, eye ointments and the like are included. These contain generally used ointment base, lotion base, aqueous or non-aqueous solutions, suspensions, emulsions and the like. For example, polyethylene glycol, propylene glycol, white petrolatum, white beeswax, polyoxyethylene hydrogenated castor oil, glycerol monostearate, stearyl alcohol, cetyl alcohol, lauromacrogol, sorbitan sesquioleate and the like can be cited as the ointment or lotion base.
Inhalations, transnasal preparations and the like transmucosal preparations are used in a solid, liquid or semisolid form and can be produced in accordance with conventionally known methods. For example, a conventionally known filler, as well as a pH adjusting agent, an antiseptic, a surfactant, a lubricant, a stabilizer, a thickener and the like, may be optionally added. An appropriate device for inhalation or blowing can be used for the administration. For example, using a measured administration inhalation device or the like conventionally known device or a sprayer, a compound can be administered alone or as a powder of a formulated mixture, or as a solution or suspension by a combination with a medicinally acceptable carrier. The dry powder inhaler or the like may be for single or multiple administration use, and a dry powder or a powder-containing capsule can be used. Alternatively, it may be a pressurized aerosol spray or the like form which uses chlorofluproalkane, hydrofluoroalkane or carbon dioxide or the like suitable gas.

In the case of oral administration, daily dose is generally from about 0.001 to 100 mg/kg body weight, preferably from 0.1 to 30 mg/kg, more preferably from 0.1 to 10 mg/kg, and this is administered once or dividing into 2 to 4 times. When intravenously administered, it is suitable that the daily dose is from about 0.0001 to 10 mg/kg body weight, and this is administered once a day or dividing it into two or more times. In addition, as a transmucosal preparation, a daily dose of from about 0.001 to 100 mg/kg body weight is administered once a day or dividing it into two or more times. The dose is optionally decided in response to individual cases, taking symptom, age, sex and the like into consideration.

The compounds of the invention can be used concomitantly with various therapeutic or preventive agents for diseases in which the aforementioned compounds of the invention are considered to be effective. Said concomitant use may be effected by simultaneous administration or by administering individually continuously or at a desired interval of time. The simultaneous administration preparations may be a combination drug or separately prepared.

### EXAMPLES

The following describes production methods of the compounds (I) of the invention further in detail based on examples. The compounds of the invention are not limited to the compounds described in the following examples. Also, production methods of the material compounds are shown in production examples.

### Production Example 1

Under ice-cooling, sodium hydride (about 40% mineral oil was added, 7.0 g) was added to a THF (120 ml) solution of ethyl diethylphosphonoacetate (38 ml) and stirred under ice-cooling for 30 minutes. A THF (200 ml) solution of 2-fluoro-4-methylbenzaldehyde (20 g) was added dropwise under ice-cooling to the reaction mixture and stirred under ice-cooling for 1 hour. Water was added to the reaction mixture, followed by extraction with ethyl acetate. The organic layer was washed with saturated sodium chloride aqueous solution and then dried with anhydrous magnesium sulfate. The desiccant was removed, the solvent was evaporated under a reduced pressure, and then the residue was purified by a silica gel column chromatography (hexane-ethyl acetate) to obtain ethyl (2E)-3-(2-fluoro-4-methylphenyl)acrylate (30 g) as a colorless oil.

### Production Example 2

A mixture of ethyl (2E)-3-(2-fluoro-4-methylphenyl)acrylate (30 g), N-bromosuccinimide (31 g), 2,2'-azobisisobutyronitrile (1.2 g) and carbon tetrachloride (360 ml) was stirred for 10 hours under heating reflux. After separating the insoluble matter by filtration, the solvent was evaporated under a reduced pressure. By purifying the residue by a silica gel column chromatography (hexane-ethyl acetate), ethyl (2E)-3-[4-(bromomethyl)-2-fluorophenyl]acrylate (42 g) was obtained as a colorless solid.

### Production Example 3

N-Bromosuccinimide (30.76 g) and 2,2'-azoisobutyronitrile (645 mg) were added to a carbon tetrachloride (500 ml) solution of ethyl (2E)-3-(2-fluoro-4-methylphenyl)acrylate (16.36 g), and stirred for 21 hours under heating reflux. The reaction mixture was concentrated under a reduced pressure, ethyl acetate was added to the residue, followed by washing with water, saturated sodium thiosulfate aqueous solution, water and saturated sodium chloride aqueous solution in that order and subsequent drying with anhydrous magnesium sulfate. After removing the desiccant, the solvent was evaporated under a reduced pressure, the thus obtained yellow oil (24.91 g) was dissolved in acetone (581 ml) and water (119 ml) followed by the addition of silver nitrate (34.69 g), and the reaction mixture was stirred under shade at room temperature for 15 hours. The precipitate was removed by filtration, and ethyl acetate was added to the thus obtained filtrate. The organic layer was washed with saturated sodium bicarbonate aqueous solution, water and saturated sodium chloride aqueous solution in that order and dried with anhydrous magnesium sulfate. After removing the desiccant, the solvent was evaporated under a reduced pressure and the thus obtained residue was purified by a silica gel column chromatography (hexane-ethyl acetate) to obtain ethyl (2E)-3-[2-fluoro-4-(hydroxymethyl)phenyl]acrylate (4.70g) as a yellow oil.

### Production Example 4

In an atmosphere of nitrogen and under cooling on an ice-methanol bath, nickel(II) chloride hexahydrate (1.06 g) was added to an ethanol (40 ml) and THF (40 ml) solution of ethyl (2E)-3-[2-fluoro-4-(hydroxymethyl)phenyl]acrylate (4.00g), followed by the addition of sodium borohydride (1.35 g) in small portions. The reaction mixture was stirred under ice-cooling for 1.5 hours and then warmed up to room temperature and stirred as such for 1.5 hours. Under ice-cooling, 10% citric acid aqueous solution (100 ml) was added to the reaction mixture, followed by extraction with ethyl acetate. The organic layer was washed with water and saturated sodium chloride aqueous solution in that order and dried with anhydrous magnesium sulfate. By removing the desiccant and then evaporating the solvent under a reduced pressure, ethyl 3-[2-fluoro-4-(hydroxymethyl)phenyl]propanoate (3.81 g) was obtained as a pale yellow oil.

### Production Example 5

1,1,1-Triacetoxy-1,1-dihydro-1,2-benzoiodoxol-3(1H)-one (6.50 g) was added to a dichloromethane (35 ml) solution of ethyl 3-[2-fluoro-4-(hydroxymethyl)phenyl]propanoate (1.73 g) and stirred at room temperature for 1 hour. The reaction mixture was poured into saturated sodium bicarbonate aqueous solution (100 ml) and extracted with chloroform. The organic layer was washed with saturated sodium chloride aqueous solution and then dried with anhydrous magnesium sulfate. After removal of the desiccant, the solvent was evaporated under a reduced pressure, and the thus obtained residue was purified by a silica gel column chromatography (hexane-ethyl acetate) to obtain ethyl 3-(2-fluoro-4-(formylphenyl)propanoate (1.41 g) as a pale yellow oil.

### Production Example 6

Under ice-cooling, thionyl chloride (0.75 ml) was added to a methanol (26 ml) solution of rel-(1R,2R)-2-(4-aminophenyl)cyclopropanecarboxylic acid (1.30 g) and stirred at room temperature for 3 hours. After evaporation of the solvent under a reduced pressure, methanol and subsequent saturated sodium bicarbonate aqueous solution were added to the residue, and the solvent was evaporated under a reduced pressure. The residue was extracted with ethyl acetate. The organic layer was washed with saturated sodium chloride aqueous solution and then dried with anhydrous magnesium sulfate. The desiccant was removed and the solvent was evaporated under a reduced pressure. By drying the residue under a reduced pressure, methyl rel-(1R,2R)-2-(4-aminophenyl)cyclopropanecarboxylate (1.25 g) was obtained as a pale brown oil.

### Production Example 7

Under ice-cooling, 2-nitrobenzenesulfonyl chloride (2.46 g) was added to a pyridine (15 ml) solution of methyl rel-(1R,2R)-2-(4-aminophenyl)cyclopropanecarboxylate (1.93 g) and stirred at room temperature for 12 hours. After evaporating the solvent under a reduced pressure, water and ethyl acetate were added to the residue, and the insoluble matter was separated by filtration. After separation of layers of the filtrate, the organic layer was washed with 10% citric acid aqueous solution and saturated sodium chloride aqueous solution in that order, and dried with anhydrous magnesium sulfate. The desiccant was removed, the solvent was evaporated under a reduced pressure, and then the residue was purified by a silica gel column chromatography (hexane-ethyl acetate) to obtain methyl rel-(1R,2R)-2-(4-{[(2-nitrophenyl)sulfonyl]amino}phenyl)cylopropanecarboxylate (3.30 g) as a pale yellow oil.

### Production Example 8

Di-t-butyl dicarbonate (10.4 g) was added to a dioxane (26 ml) solution of ethyl 3-(4-amino-2-fluorophenyl)propanoate (10.0 g) and stirred at 90°C for 20 hours. This reaction mixture was spontaneously cooled down to room temperature, and the solvent was evaporated under a reduced pressure. Ethyl acetate was added to the residue, followed by washing with saturated sodium bicarbonate aqueous solution and saturated sodium chloride aqueous solution. The organic layer was dried with anhydrous magnesium sulfate, and then the desiccant was removed and the solvent was evaporated under a reduced pressure. By purifying the residue by a silica gel column chromatography (hexane-ethyl acetate), ethyl 3-{4-[(tert-butoxycarbonyl)amino]2-fluorophenyl}propanoate (14.9 g) was obtained as a colorless oil.

### Production Example 9

A mixture of 1,2,3,4-tetrahydroquinolin-5-ylmethanol (5.29 g)di-tert-butyl dicarbonate (10.6 g) and dioxane (50 ml) was stirred at 80°C for 12 hours. The solvent was evaporated under a reduced pressure, and then the residue was purified by a silica gel column chromatography (hexane-ethyl acetate) to obtain tert-butyl 5-(hydroxymethyl)-3,4-dihydroquinoline-1(2H)carboxylate (7.64 g) as a pale yellow oil.

### Production Example 10

Under ice-cooling, sodium borohydride (3.2 g) was added to an ethanol (160 ml) solution of tert-butyl 8-formyl-3,4-dihydroquinoline-1(2H)-carboxylate and stirred under ice-cooling for 40 minutes. Water was added to the reaction mixture, the solvent was evaporated under a reduced pressure, and then the residue was extracted with ethyl acetate. The organic layer was washed with saturated sodium chloride aqueous solution and then dried with anhydrous magnesium sulfate. The desiccant was removed and the solvent was evaporated under a reduced pressure. By purifying the residue by a silica gel column chromatography, tert-butyl 8-(hydroxymethyl)-3,4-dihydroquinoline-1(2H)-carboxylate (10 g) was obtained as a colorless oil.

### Production Example 11

In an atmosphere of hydrogen, 10% palladium-activated carbon (4.50 g) was added to a methanol (325 ml) and THF (325 ml) solution of methyl 4-[(1E)-3-ethoxy-3-oxoprop-1-en-1-yl]-3-nitrobenzoate (11.4 g) and stirred at room temperature for 20 hours. The insoluble matter was separated by filtration, and 1 M hydrochloric acid (190 ml) was added to the filtrate and stirred at room temperature for 5 hours. The solvent was evaporated under a reduced pressure, and the thus formed solid was collected by filtration to obtain methyl 2-oxo-1,2,3,4-tetrahydroquinoline-7-carboxylate (34.8 g) as a pale green solid.

### Production Example 12

A mixture of methyl 2-oxo-1,2,3,4-tetrahydroquinoline-7-carboxylate (500 mg), iodobenzene (0.42 ml), copper(I) iodide (44 mg), 1,10-phenanthroline (44 mg), 40% potassium fluoride-alumina (1.77 g) and toluene (15 ml) was heated under reflux for 1 day. Iodobenzene (0.41 ml) and copper(I) iodide (44 mg) were added to the reaction mixture and further stirred under heating reflux for 2 days. The reaction mixture was cooled down to room temperature, the insoluble matter was separated by filtration, and the solvent was evaporated under a reduced pressure. By purifying the residue by a silica gel column chromatography (chloroform-methanol), methyl 2-oxo-1-phenyl-1,2,3,4-tetrahydroquinoline-7-carboxylate (150 mg) was obtained as a pale yellow solid.

### Production Example 13

To a THF (5 ml) and methanol (1 ml) solution of methyl 2-oxo-1-phenyl-1,2,3,4-tetrahydroquinoline-7-carboxylate (530 mg), 1 M sodium hydroxide aqueous solution (5.7 ml) was added and stirred at room temperature for 1 week. After evaporation of the solvent under a reduced pressure, 1 M hydrochloric acid was added to the residue to adjust to pH 3 to 4, and the thus formed solid was collected by filtration. By drying under a reduced pressure, 2-oxo-1-phenyl-1,2,3,4-tetrahydroquinoline-7-carboxylic acid (385 mg) was obtained as a white solid.

### Production Example 14

Under ice-cooling, isobutyl chloroformate (0.21 ml) was added dropwise to a THF (5 ml) mixture of 2-oxo-1-phenyl-1,2,3,4-tetrahydroquinoline-7-carboxylic acid (385 mg) and 4-methylmorpholine (0.19 ml) and stirred at the same temperature for 30 minutes, followed by the addition of sodium borohydride (82 mg) and methanol (1 ml). Water was added to the reaction mixture, followed by extraction with chloroform. After drying the organic layer with anhydrous magnesium sulfate, the desiccant was removed and the solvent was evaporated under a reduced pressure. The residue was purified by a silica gel column chromatography (chloroform-methanol) to obtain 7-(hydroxymethyl)-1-phenyl-3,4-dihydroquinolin-2(1H)-one (306 mg) was obtained as a white solid.

### Production Example 15

A mixture of 7-dimethoxymethyl-1,2,3,4-tetrahydro-1,8-naphthyridine (500 mg), bromobenzene (0.55 ml), tris(dibenzylideneacetone)dipalladium (44 mg), dicyclohexyl(2',4',6'-triisopropylbiphenyl-2-yl)phosphine (46 mg), potassium phosphate (2.04 g) and dimethoxyethane (10 ml) was stirred at 90°C for 12 hours. The reaction mixture was spontaneously cooled to room temperature and extracted with ethyl acetate by adding water. The organic layer was washed with saturated sodium chloride aqueous solution and then dried with anhydrous magnesium sulfate. By removing the desiccant and evaporating the solvent under a reduced pressure, 7-(dimethoxymethyl)-1-phenyl-1,2,3,4-tetrahydro-1,8-naphthyridine (1.14 g) was obtained as a black oil.

### Production Example 16

3 M Hydrochloric acid (20 ml) was added to a THF (10 ml) solution of 7-(dimethoxymethyl)-1-phenyl-1,2,3,4-tetrahydro-1,8-naphthyridine (1.14 g) and stirred at room temperature for 2 hours. 3 M Sodium hydroxide aqueous solution (20 ml) was added, extracted with ethyl acetate, and the organic layer was dried with anhydrous magnesium sulfate. Under ice-cooling, sodium borohydride (76 mg) was added to a methanol (10 ml) solution of the residue and stirred for 10 minutes. Water was added to the reaction mixture, and extracted with ethyl acetate. The organic layer was dried with anhydrous magnesium sulfate, the desiccant was removed and the solvent was evaporated under a reduced pressure. By purifying the residue by a silica gel column chromatography (hexane-ethyl acetate), (8-phenyl-5,6,7,8-tetrahydro-1,8-naphthyridin-2-yl)methanol (556 mg) was obtained as a pale yellow oil.

### Production Example 17

Concentrated sulfuric acid (32.0 ml) was added to a mixture of 3-amino-2-methylbenzoic acid (25.0 g), glycerol (49.9 g), boric acid (7.5 g) and nitrobenzene (13.6 g) and stirred at 160°C for 12 hours. This reaction mixture was spontaneously cooled to room temperature and allowed to stand still for 1 day by adding water (110 ml) and sodium hydroxide (59.5 g). This reaction mixture was subjected to decantation, and the supernatant alone was transferred. Acetic acid (37.9 ml) was added to the thus obtained supernatant. The thus formed solid was washed with water and then dried under a reduced pressure. Methanol (250 ml) and concentrated sulfuric acid (9.1 ml) were added to the thus obtained black solid, and 2 days of stirring was carried out under heating reflux. After spontaneous cooling of the reaction mixture to room temperature, sodium bicarbonate (3 5.9 g) was added in small portions, and the solvent was evaporated under a reduced pressure. Ethyl acetate was added to the residue, the insoluble matter was separated by filtration, and the filtrate was washed with saturated sodium chloride aqueous solution. After drying the organic layer with anhydrous magnesium sulfate, the desiccant was removed and the solvent was evaporated under a reduced pressure. By purifying the residue by a silica gel column chromatography (hexane-ethyl acetate), methyl 8-methylquinoline-7-carboxylate (10.6 g) was obtained as a pale yellow solid.

### Production Example 18

Under cooling on an ice-methanol bath, sodium borohydride (1.50 g) was added in small portions to a methanol (80 ml) solution of methyl 8-methylquinoline-7-carboxylate (8.00 g) and nickel (II) chloride hexahydrate (2.84 g) and stirred for 1 hour. Saturated ammonium chloride aqueous solution was added to the reaction solution and the solvent was evaporated under a reduced pressure. Water and ethyl acetate were added to the residue, the insoluble matter was removed by celite filtration and the filtrate was extracted with ethyl acetate. The organic layer was washed with saturated sodium chloride aqueous solution and dried with anhydrous magnesium sulfate. By removing the descant and evaporating the solvent under a reduced pressure, methyl 8-methyl-1,2,3,4-tetrahydroquinoline-7-carboxylate was obtained as a pale yellow oil.

### Production Example 19

A mixture of methyl 1,2,3,4-tetrahydroquinoline-7-carboxylate (3.28 g), triphenylbismuthin (11.3 g), copper(II) acetate (3.12 g) and dichloroethane (30 ml) was stirred at 80°C for 12 hours. The insoluble matter was removed by celite filtration and then the solvent was evaporated under a reduced pressure. By purifying the residue by a silica gel column chromatography (hexane-ethyl acetate), methyl 1-phenyl-1,2,3,4-tetrahydroquinoline-7-carboxylate (4.36 g) was obtained as a pale yellow oil.

### Production Example 20

Sodium triacetoxyborohydride (4.3 g) was added at room temperature to a mixture of 1,2,3,4-tetrahydroquinolin-8-ol (1.0 g), 2-methylpropanal (1.2 ml) and dichloroethane (10 ml) and stirred at room temperature for 6 hours. Water added to the reaction mixture, followed by extraction with ethyl acetate. The organic layer was washed with saturated sodium chloride aqueous solution and then dried with anhydrous magnesium sulfate. The desiccant was removed, the solvent was evaporated under a reduced pressure, and then the residue was purified by a silica gel column chromatography (hexane-ethyl acetate) to obtain 1-isobutyl-1,2,3,4-tetrahydroquinolin-8-ol (1.4 g) as a brown oil.

### Production Example 21

N,N-diisopropylethylamine (2.0 ml) and benzyl bromide (1.00 ml) were added to a DMF (20 ml) solution of 6-nitro-3,4-dihydro-2H-1,4-benzoxazine (1.00 g), and the reaction mixture was stirred at 60°C for 2 days. The reaction mixture was spontaneously cooled to room temperature, followed by the addition of water (80 ml) and subsequent extraction with ethyl acetate. The organic layer was washed with saturated sodium chloride aqueous solution and then dried with anhydrous magnesium sulfate. After removing he desiccant, the solvent was evaporated under a reduced pressure, and the thus obtained residue was purified by a silica gel column chromatography (hexane-ethyl acetate) to obtain 4-benzyl-6-nitro-3,4-dihydro-2H-1,4-benzoxazine (1.49 g) as an orange solid.

### Production Example 22

Reduced iron (1.51 g) and ammonium chloride (290 mg) were added to an ethanol (24 ml) and water (6 ml) solution of 4-benzyl-6-nitro-3,4-dihydro-2H-1,4-benzoxazine (1.46 g), and the reaction mixture was stirred for 2 hours under heating reflux. The reaction mixture was spontaneously cooled to room temperature and filtered over celite. By washing with ethanol, the thus obtained filtrate was concentrated under a reduced pressure. Chloroform and saturated sodium bicarbonate aqueous solution were added to the residue, followed by extraction with chloroform. The organic layer was dried with anhydrous magnesium sulfate, and after removing he desiccant, the solvent was evaporated under a reduced pressure. By purifying the thus obtained residue by a silica gel column chromatography (chloroform-methanol), 4-benzyl-3,4-dihydro-2H-1,4-benzoxazine-6-amine (1.19 g) was obtained as a light brown solid.

### Production Example 23

Under an atmosphere of nitrogen, a THF (5.6 ml) solution of methyl 8-methyl-1-propyl-1,2,3,4-tetrahydroquinoline-7-carboxylate was added dropwise at 0°C to a THF (30 ml) suspension of lithium aluminum hydride (410 mg) and stirred for 30 minutes. By adding water (1.67 ml) and 15% sodium hydroxide aqueous solution (0.40 ml), 1 hour of stirring was carried out at room temperature. After removing the insoluble matter by filtration, the filtrate was concentrated under a reduced pressure to obtain (8-methyl-1-propyl-1,2,3,4-tetrahydroquinolin-7-yl)methanol (1.52 g) as a colorless oil.

### Production Example 24

A mixture of (1-phenyl-1,2,3,4-tetrahydroquinolin-7-yl)methanol (2.0 g), manganese dioxide (3.6 g) and chloroform (40 ml) was stirred at 60°C for 12 hours. After removing the insoluble matter by celite filtration, the solvent was evaporated under a reduced pressure. By purifying the residue by a silica gel column chromatography (hexane-ethyl acetate), 1-phenyl-1,2,3,4-tetrahydroquinoline-7-carbaldehyde (1.6 g) was obtained as a yellow oil.

### Production Example 25

Under ice-cooling, sodium hydride (about 40% mineral oil was added, 1.2 g) was added to a mixture of ethyl (2E)-3-[4-(bromomethyl)-2-fluorophenyl]acrylate (8.0 g), 8-quinolinol (4.2 g) and DMF (150 ml) and stirred under ice-cooling for 2 hours. Water was added to the reaction mixture, followed by extraction with ethyl acetate. The organic layer was washed with saturated sodium chloride aqueous solution and then dried with anhydrous magnesium sulfate. After removing the desiccant, the solvent was evaporated under a reduced pressure. By purifying the residue by a silica gel column chromatography (hexane-ethyl acetate), ethyl (2E)-3-{2-fluoro-4-[(quinolin-8-yloxy)methyl]phenyl}acrylate (0.32 g) was obtained as a yellow solid.

### Production Example 26

Under ice-cooling, sodium hydride (about 40% of mineral oil was added, 0.19 g) was added to a mixture of 7-(bromomethyl)quinoline hydrochloride (0.45 g), ethyl 3-(2-fluoro-4-hydroxyphenyl)propanoate (0.55 g) and DMF (5 ml) and stirred under ice-cooling for 2 hours. Water was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. By purifying the residue by a silica gel column chromatography (hexane-ethyl acetate), ethyl 3-[2-fluoro-4-(quinolin-7-ylmethoxy) phenyl]propanoate (0.32 g) was obtained as a colorless solid.

### Production Example 27

10% palladium-activated carbon (80mg) was added to a methanol (10 ml) and THF (5 ml) solution of methyl (6-{[(1-benzyl-1,2,3,4-tetrahydroquinolin-8-yl)methyl]amino}-1-benzofuran-3-yl)acetate (659 mg) and stirred at room temperature for 8 hours in an atmosphere of hydrogen. The catalyst was removed by celite filtration and the filtrate was concentrated under a reduced pressure. By purifying the thus obtained residue by a silica gel column chromatography (hexane-ethyl acetate), methyl (6-amino-2,3-dihydro-1-benzofuran-3-yl)acetate (389 mg) was obtained as a pale pink oil.

### Production Example 28

Under ice-cooling, 4 M hydrogen chloride dioxane solution (6.0 ml) was added to a THF (1.5 ml) solution of ethyl (2E)-3-(4-{[tert-butoxycarbonyl)(1-propyl-1,2,3,4-tetrahydroquinolin-8-yl)amino]methyl}-2-fluorophenyl)acrylate (1.23 g) and stirred under ice-cooling for 12 hours. After evaporation of the solvent under a reduced pressure, saturated sodium bicarbonate aqueous solution was added to the residue, followed by extraction with ethyl acetate. The organic layer was washed with saturated sodium chloride aqueous solution and then dried with anhydrous magnesium sulfate. The desiccant was removed and the solvent was evaporated under a reduced pressure. By drying the residue under a reduced pressure, ethyl (2E)-3-(4-{[(1-propyl-1,2,3,4-tetrahydroquinolin-8-yl)amino]methyl}-2-fluorophenyl)acrylate (0.98 g) was obtained as a yellow oil.

### Production Example 29

Under ice-cooling, sodium cyanoborohydride (1.54 g) was added to an acetic acid (65 ml) solution of methyl rel-(1R,2R)-2-(4-{[(2-nitrophenyl)sulfonyl](quinolin-8-ylmethyl)amino}phenyl)cyclopropanecarboxylate (4.24 g) and stirred at room temperature for 1 hour. The reaction mixture was neutralized by adding saturated sodium bicarbonate aqueous solution and sodium carbonate, followed by extraction with ethyl acetate. The organic layer was washed with water and saturated sodium chloride aqueous solution in that order and then dried with anhydrous magnesium sulfate. The desiccant was removed and the solvent was evaporated under a reduced pressure. By purifying the residue by a silica gel column chromatography (hexane-ethyl acetate), methyl rel-(1R,2R)-2-(4-{[(2-nitrophenyl)sulfonyl](1,2,3,4-tetrahydroquinolin-8-ylmethyl)amino}phenyl)cyclopropanecarboxylate (3.41 g) was obtained as a pale yellow amorphous solid.

### Production Example 30

Under ice-cooling, trifluoroacetic anhydride (0.31 ml) was added to a dichloromethane (10 ml) solution of tert-butyl rel-8-[({4-[(1R,2R)-2-(methoxycarbonyl)cyclopropyl]phenyl}amino)methyl]-3,4-dihydroquinoline-1(2H)-carboxylate (800 mg) and stirred under ice-cooling for 2 hours. Saturated sodium bicarbonate aqueous solution was added to the reaction solution, followed by extraction with ethyl acetate. The organic layer was washed with saturated sodium chloride aqueous solution and then dried with anhydrous magnesium sulfate. The desiccant was removed and the solvent was evaporated under a reduced pressure. By drying the residue under a reduced pressure, tert-butyl rel-8-{[{4-[(1R,2R)-2-(methoxycarbonyl)cyclopropyl]phenyl}(trifluoroacetyl)amino]methyl}-3,4-dihydroquinoline-1(2H)-carboxylate (940 mg) was obtained as a pale yellow oil.

### Production Example 31

A mixture of ethyl 3-{4-[(tert-butoxycarbonyl)(1,2,3,4-tetrahydroquinolin-7-ylmethyl)amino]-2-fluorophenyl}propanoate (670 mg), 1-bromo-2-methylbenzene (0.35 ml), tris(dibenzylideneacetone)dipalladium (26.9 mg), dicyclohexyl(2',4',6'-triisopropylbiphenyl-2-yl)phosphine (28.0 mg), potassium phosphate (1.25 g) and dimethoxyethane (6.7 ml) was stirred for 2 days under heating reflux. The reaction mixture was spontaneously cooled to room temperature and extracted with ethyl acetate by adding water. The organic layer was washed with saturated sodium chloride aqueous solution and then dried with anhydrous magnesium sulfate. The desiccant was removed, the solvent was evaporated under a reduced pressure, and the residue was purified by a silica gel column chromatography (hexane-ethyl acetate) to obtain ethyl 3-{4-[(tert-butoxycarbonyl){[1-(2-methylphenyl)-(1,2,3,4-tetrahydroquinolin-7-ylmethyl)amino]-2-fluorophenyl}propanoate (527 mg) as a yellow oil.

### Production Example 32

Under ice-cooling, sodium borohydride (67 mg) was added to a mixture of ethyl 3-[2-fluoro-4-([(2-nitrophenyl)sulfonyl]{[1-(2-oxo-2-phenylethyl)-1,2,3,4-tetrahydroquinolin-5-yl]methyl}amino)phenyl]propanoate (1.17 g), ethanol (12 ml) and THF (6 ml) and stirred under ice-cooling for 5 hours. Water was added to the reaction solution, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-[2-fluoro-4-({[1-(2-hydroxy-2-phenylethyl)-1,2,3,4-tetrahydroquinolin-5-yl]methyl}[(2-nitrophenyl)sulfonyl]amino)phenyl]propanoate (1.15 g) as a colorless oil.

### Production Example 33

Under ice-cooling, mercaptoacetic acid (0.36 ml) and lithium hydroxide monohydrate (430 mg) were added to a DMF (20 ml) solution of methyl (6-{[(1-benzyl-1,2,3,4-tetrahydroquinolin-8-yl)methyl][(2-nitrophenyl)sulfonyl]amino}-1-benzofuran-3-yl)acetate (1.56 g), warmed up to room temperature and stirred for 3 hours. Saturated sodium bicarbonate aqueous solution was added to the reaction mixture, followed by extraction with ethyl acetate. The organic layer was washed with saturated sodium chloride aqueous solution and then dried with anhydrous magnesium sulfate. After removing the desiccant, the solvent was evaporated under a reduced pressure and the thus obtained residue was purified by a silica gel column chromatography (hexane-ethyl acetate) to obtain methyl (6-{[(1-benzyl-1,2,3,4-tetrahydroquinolin-8-yl)methyl]amino}-1-benzofuran-3-yl)acetate (670 mg) as a pale yellow syrup.

### Production Example 34

1 M Tetrabutylammonium fluoride THF solution (1.18 ml) was added to a THF (4.7 ml) solution of ethyl 3-{4-[(tert-butoxycarbonyl){[1-(2-{[tert-butyl(dimethyl)silyl]oxy}ethyl)-1,2,3,4-tetrahydroquinolin-7-yl]methyl}amino]-2-fluorophenyl}propanoate (291 mg) and stirred at room temperature for 1 day. The solvent was evaporated under a reduced pressure and saturated ammonium chloride aqueous solution was added to the residue, followed by extraction with ethyl acetate. The organic layer was washed with saturated sodium chloride aqueous solution and then dried with anhydrous magnesium sulfate. After removing the desiccant, the solvent was evaporated under a reduced pressure and the thus obtained residue was purified by a silica gel column chromatography (hexane-ethyl acetate) to obtain ethyl 3-{4-[(tert-butoxycarbonyl){[1-(2-hydroxyethyl)-1,2,3,4-tetrahydroquinolin-7-yl]methyl}amino]-2-fluorophenyl }propanoate (234 mg) as colorless oil.

### Production Example 35

1,1'-(Azodicarbonyl)dipiperidine (390 mg) was added under ice-cooling to a mixture of ethyl 3-[2-fluoro-4-({[1-(2-hydroxyethyl)-1,2,3,4-tetrahydroquinolin-5-yl]methyl}[(2-nitrophenyl)sulfonyl]amino)phenyl]propanoate (600 mg), 2-fluorophenol (230 mg), tributylphosphine (0.38 ml) and THF (6 ml) and stirred at room temperature for 3 days. After separation of the insoluble matter by filtration, the solvent was evaporated under a reduced pressure. By purifying the residue by a silica gel column chromatography (hexane-ethyl acetate), ethyl 3-[2-fluoro-4-{({1-[2-(2-fluorophenoxy)ethyl]-1,2,3,4-tetrahydroquinolin-5-yl}methyl)[(2-nitrophenyl)sulfonyl]amino}phenyl)propanoate (610 mg) was obtained as a yellow oil.

### Production Example 36

4 M Hydrogen chloride dioxane solution (13 ml) was added to a dioxane (2 ml) solution of ethyl 3-{4-[{[1-(2-tert-butoxy-2-oxoethyl)-8-methyl-1,2,3,4-tetrahydroquinolin-7-yl]methyl}(trifluoroacetyl)amino]-2-fluorophenyl}propanoate (1.50 g) and stirred at room temperature for 6 hours. The solvent was evaporated under a reduced pressure and pH was adjusted to 7 by adding water and saturated sodium bicarbonate aqueous solution, followed by extraction with ethyl acetate. The organic layer was washed with saturated sodium chloride aqueous solution and then dried with anhydrous magnesium sulfate. The desiccant was removed, the solvent was evaporated under a reduced pressure and the residue was purified by a silica gel column chromatography (chloroform-methanol) to obtain [7-({[4-(3-ethoxy-3-oxopropyl)-3-fluorophenyl](trifluoroacetyl)amino}methyl)-8-methyl-3,4-dihydroquinolin-1(2H)-yl]acetic acid (1.40 g) as a yellow oil.

### Production Example 37

To a DMF (5 ml) solution of [7-({[4-(3-ethoxy-3-oxopropyl)-3-fluorophenyl](trifluoroacetyl)amino}methyl)-8-methyl-3,4-dihydroquinolin-1(2H)-yl]acetate (429 mg), morpholine (0.17 ml), 1-hydroxybenzotriazole monohydrate (138 mg) and N,N-diisopropylethylamine 0.34 ml), 1-(3-dimethylaminopropyl)-3-ethylcarbodiimide hydrochloride (376 mg) was added and stirred at room temperature for 3 days. Water was added thereto, followed by extraction with ethyl acetate. The organic layer was washed with saturated sodium bicarbonate aqueous solution and saturated sodium chloride aqueous solution and then dried with anhydrous magnesium sulfate. The desiccant was removed and the solvent was evaporated under a reduced pressure to obtain ethyl 3-[2-fluoro-4-[{[8-methyl-1-(2-morpholin-4-yl-2-oxoethyl)-1,2,3,4-tetrahydroquinolin-7-yl]methyl}(trifluoroacetyl)amino]phenyl}propanoate (356 mg) as a yellow oil.

### Production Example 38

Methanesulfonyl chloride (0.24 ml) was added dropwise to a mixture of ethyl 3-[2-fluoro-4-[{[1-(2-hydroxyethyl)-8-methyl-1,2,3,4-tetrahydroquinolin-7-yl]methyl}(trifluoroacetyl)amino]phenyl}propanoate (1.20 g), triethylamine (0.45 ml) and ethyl acetate (15 ml) and stirred at room temperature for 3 hours. The insoluble matter was separated by filtration and the solvent was evaporated under a reduced pressure. The residue was purified by a silica gel column chromatography (hexane-ethyl acetate) to obtain ethyl 3-(2-fluoro-4-{[(8-methyl-1-{2-[(methylsulfonyl)oxy]ethyl}-1,2,3,4-tetrahydroquinolin-7-yl)methyl](trifluoroacetyl)amino}phenyl)propanoate (1.13 g) as a colorless oil.

### Production Example 39

Piperidine (0.48 ml) was added to a DMF (10 ml) solution of ethyl 3-(2-fluoro-4-{[(8-methyl-1-{2-[(methylsulfonyl)oxy]ethyl}-1,2,3,4-tetrahydroquinolin-7-yl)methyl](trifluoroacetyl)amino}phenyl)propanoate (574 mg) and potassium iodide (162 mg) and stirred at 70°C for 1 day. Water was added thereto, followed by extraction with ethyl acetate. The organic layer was dried with anhydrous magnesium sulfate, the desiccant was removed and the solvent was evaporated under a reduced pressure. The residue was purified by a silica gel column chromatography (chloroform-methanol) to obtain ethyl 3-{2-fluoro-4-[{[8-methyl-1-(2-piperidin-1-ylethyl)-1,2,3,4-tetrahydroquinolin-7-yl]methyl}(trifluoroacetyl)amino]phenyl}propanoate (515 mg) as a colorless oil.

### Production Example 40

While stirring, concentrated sulfuric acid (30 ml) was added to a mixture of 3-amino-4-chlorobenzoic acid (25.25 g), sodium 3-nitrobenzenesulfonate (37.0 g) and boric acid (9.20 g). Thereafter, glycerol (37 ml) was added thereto, and temperature of the mixture was gradually increased to around 155°C while mixing with a glass rod. Thereafter, by attaching a condenser, the reaction mixture was stirred at 160°C for 3 hours and then spontaneously cooled to room temperature. Under ice-cooling, water (200 ml) was added to the reaction mixture, sodium hydroxide (ca. 50 g) was added in small portions thereto, followed by 30 minutes of stirring as such. Next, acetic acid (50 ml) and water (100 ml) were added thereto under ice-cooling, and the thus formed solid was collected by filtration and washed with water (approximately 100 ml). Methanol (200 ml) and toluene (200 ml) were added to the thus obtained paste-like solid, followed by concentration under a reduced pressure (the same operation was repeated twice). By drying the thus obtained residue under a reduced pressure, a dark brown solid (42.78 g) was obtained. Under ice-cooling, concentrated sulfuric acid (30 ml) was slowly added to a methanol (500 ml) suspension of the thus obtained dark brown solid, and the reaction mixture was stirred for 19 hours under heating reflux and then spontaneously cooled to room temperature. The reaction mixture was concentrated under a reduced pressure, and saturated sodium bicarbonate aqueous solution (700 ml) and chloroform (200 ml) were added in small portions to the thus obtained residue under ice-cooling. Sodium bicarbonate (20 g) was further added in small portions, followed by 10 minutes of stirring under ice-cooling. The mixture was filtered through celite, the insoluble matter was removed and then the filtrate was extracted with chloroform. By purifying the thus obtained residue by a silica gel column chromatography (hexane-ethyl acetate), methyl 8-chloroquinoline-5-carboxylate (22.97 g) was obtained as a pale yellow solid.

### Production Example 41

A mixture of (2-bromoethoxy)benzene (25.00 g), ethyl 1,2,3,4-tetrahydroquinoline-5-carboxylate (6.24 g), diisopropylethylamine (43 ml), potassium iodide (10.00 g) and DMF (60 ml) was stirred at 120°C for 21 hours. The reaction mixture was concentrated under a reduced pressure and water was added to the residue, followed by extraction with ethyl acetate. The organic layer was washed with saturated sodium chloride aqueous solution and then dried with anhydrous magnesium sulfate. The desiccant was removed, the solvent was evaporated under a reduced pressure, and the thus obtained residue was purified by a silica gel column chromatography (hexane-ethyl acetate) to obtain ethyl 1-(2-phenoxyethyl)-1,2,3,4-tetrahydroquinoline-5-carboxylate (9.67 g) as a pale yellow oil.

### Production Example 42

A DMSO (15 ml) solution of sulfur trioxide-pyridine complex (2.57 g) was added dropwise at room temperature to a mixture of [1-(2-phenoxyethyl)-1,2,3,4-tetrahydroquinolin-5-yl]methanol (9.67 g) and DMSO (15 ml) and stirred for 10 minutes. Water (120 ml) was added to the reaction mixture, followed by extraction with diethyl ether. The organic layer was washed with water and saturated sodium chloride aqueous solution in that order and then dried with anhydrous magnesium sulfate. The desiccant was removed and then the solvent was evaporated under a reduced pressure. The thus obtained residue was purified by a silica gel column chromatography (hexane-ethyl acetate) to obtain 1-(2-phenoxyethyl)-1,2,3,4-tetrahydroquinoline-5-carbaldehyde (1.44 g) as a yellow oil.

### Production Example 43

(2-Bromoethoxy)benzene (2.60 g), N,N-diisopropylethylamine (4.5 ml) and potassium iodide (850 mg) were added to a DMF (20 ml) solution of methyl 8-methyl-1,2,3,4-tetrahydroquinoline-5-carboxylate (1.05 g) and the reaction mixture was stirred at 120°C for 16 hours and then spontaneously cooled to room temperature. (2-Bromoethoxy)benzene (7.80 g), N,N-diisopropylethylamine (14 ml) and potassium iodide (850 mg) were added to the reaction mixture and the reaction mixture was again stirred at 130°C for 23 hours and then spontaneously cooled to room temperature. (2-Bromoethoxy)benzene (3.90 g), N,N-diisopropylethylamine (7 ml) and potassium iodide (850 mg) were added to the reaction mixture and the reaction mixture was again stirred at 150°C for 5 hours and then spontaneously cooled to room temperature. Water (100 ml) was added to the reaction mixture, followed by extraction with ethyl acetate.
The organic layer was washed with saturated sodium chloride aqueous solution and then dried with anhydrous magnesium sulfate. The desiccant was removed and the solvent was evaporated under a reduced pressure. The thus obtained residue was purified by a silica gel column chromatography (hexane-ethyl acetate) to obtain a yellow solid (1.75 g). Lithium aluminum hydride (200 mg) was added under ice-cooling to a THF (30 ml) solution of the thus obtained yellow solid, warmed up to room temperature and then stirred for 1.5 hours. Under ice-cooling, water (1.0 ml) was slowly added dropwise to the reaction mixture and stirred for 5 minutes. After adding anhydrous sodium sulfate to the reaction mixture, celite filtration was carried out, followed by washing with THF. The filtrate was concentrated under a reduced pressure and the thus obtained residue was purified by a silica gel column chromatography (hexane-ethyl acetate) to obtain [8-methyl-1-(2-phenoxyethyl)1,2,3,4-tetrahydroquinolin-5-yl]methanol (1.48 g) as a pale yellow syrup.

### Production Example 44

3 M Hydrochloric acid (5 ml) was added to a THF (3 ml) solution of 7-(dimethoxymethyl)-1-propyl-1,2,3,4-tetrahydro-1,8-naphthyridine (207 mg) and stirred at room temperature for 1.5 hours. 3 M Sodium hydroxide aqueous solution (5 ml) was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The thus obtained residue was dissolved in methanol (10 ml) and sodium borohydride (16 mg) was added thereto under ice-cooling, followed by 10 minutes of stirring. Water (20 ml) was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The thus obtained residue was purified by a silica gel column chromatography (hexane-ethyl acetate) to obtain (8-propyl-5,6,7,8-tetrahydro-1,8-naphthyridin-2-yl)methanol (144 mg) as a yellow oil.

### Production Example 45

Benzoyl chloride (2.5 ml) was added under ice-cooling to a pyridine (30 ml) solution of tert-butyl 7-(hydroxymethyl)-8-methyl-3,4-dihydroquinoline-1(2H)-carboxylate and stirred at room temperature for 12 hours. After 10 minutes of stirring by adding water, the solvent was evaporated under a reduced pressure. Water was added to the residue, followed by extraction with ethyl acetate. The organic layer was washed with 1 M hydrochloric acid, saturated sodium bicarbonate aqueous solution and saturated sodium chloride aqueous solution and then dried with anhydrous magnesium sulfate. The desiccant was removed, the solvent was evaporated under a reduced pressure and then the thus obtained residue was purified by a silica gel column chromatography (hexane-ethyl acetate) to obtain tert-butyl 7-[(benzoyloxy)methyl]-8-methyl-3,4-dihydroquinoline-1(2H)-carboxylate (3.95 g) as a pale yellow oil.

### Production Example 46

Toluene (13.2 ml) was added to (8-methyl-1,2,3,4-tetrahydroquinolin-7-yl)methyl benzoate (880 mg), 1-bromo-2-methylbenzene (0.57 ml), palladium(II) acetate (35 mg), tri-tert-butylphosphine (0.94 ml) and sodium tert-butoxide (460 mg). This mixture was allowed to undergo the reaction at 150°C for 18 hours in a sealed tube using a microwave reactor (Biotage). The reaction mixture was spontaneously cooled down to room temperature, the insoluble matter was separated by filtration and the solvent was evaporated under a reduced pressure. The residue was purified by a silica gel column chromatography (hexane-ethyl acetate) to obtain (8-methyl-1-(2-methylphenyl)-1,2,3,4-tetrahydroquinolin-7-yl)methyl benzoate (658 mg) as a pale yellow oil.

### Production Example 47

1 M Sodium hydroxide aqueous solution (3.5 ml) was added to a methanol (6.4 ml) and THF (6.4 ml) solution of (8-methyl-1-(2-methylphenyl)-1,2,3,4-tetrahydroquinolin-7-yl)methyl benzoate (640 mg) and stirred at room temperature for 4 hours. The solvent was evaporated under a reduced pressure and then the residue was purified by a silica gel column chromatography (hexane-ethyl acetate) to obtain [8-methyl-1-(2-methylphenyl)-1,2,3,4-tetrahydroquinolin-7-yl)methanol (364 mg) as a white solid.

### Production Example 48

Potassium carbonate (718 mg) and 3-hydroxy-3-methylbutyl 4-methylbenzenesulfonate (1.16 g) were added to a DMF (7 ml) solution of methyl 1-(4-hydroxyphenyl)-8-methyl-1,2,3,4-tetrahydroquinoline-7-carboxylate (700 mg), and the reaction mixture was stirred at 70°C for 24 hours and then spontaneously cooled down to room temperature. Water (30 ml) was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. By purifying the thus obtained residue by a silica gel column chromatography (hexane-ethyl acetate), methyl 1-[4-(3-hydroxy-3-methylbutoxy)phenyl]-8-methyl-1,2,3,4-tetrahydroquinoline-7-carboxylate (935 mg) was obtained as a colorless oil.

### Production Example 49

Under ice-cooling, m-chloroperbenzoic acid (650 mg) was added to a chloroform (15 ml) solution of ethyl 3-{2-fluoro-4-[{[4-(2-phenoxyethyl)-3,4-dihydro-2H-1,4-benzothiazin-8-yl]methyl}(trifluoroacetyl)amino]phenyl}propanoate (472 mg), warmed up to room temperature and then stirred for 1.5 hours. 10% Sodium hydrogen sulfite aqueous solution (40 ml) was added to the reaction mixture, followed by extraction with chloroform. The organic layer was washed with saturated sodium bicarbonate aqueous solution and then dried with anhydrous magnesium sulfate. The desiccant was removed and the solvent was evaporated under a reduced pressure. By purifying the thus obtained residue by a silica gel column chromatography (chloroform-methanol), ethyl 3-{4-[{[1,1-dioxido-4-(2-phenoxyethyl)-3,4-dihydro-2H-1,4-benzothiazin-8-yl]methyl}(trifluoroacetyl)amino]-2-fluorophenyl}propanoate (364 mg) was obtained as a pale yellow amorphous solid.

The compounds of Production Examples 50 to 197 shown in the tables which are described later were produced in the same manner as in the methods of Production Examples 1 to 49. Structures, production methods and physicochemical data of the production example compounds are shown in Tables 4 to 31.

### Example 1

Under ice-cooling, 1 M sodium hydroxide aqueous solution (1.21 ml) was added to a mixture of ethyl 3-(2-fluoro-4-{[1-(2-phenylethyl)-1,2,3,4-tetrahydroquinolin-7-yl]methoxy}phenyl)propanoate (186 mg), methanol (3 ml) and THF (3 ml) and stirred at room temperature for 1 hour. 10% Citric acid aqueous solution was added to the reaction solution to adjust to pH 5 to 6, followed by extraction with ethyl acetate. The organic layer was washed with saturated sodium bicarbonate aqueous solution and then dried with anhydrous magnesium sulfate. After removing the desiccant and evaporating the solvent under a reduced pressure, the residue was purified by a silica gel column chromatography (hexane-ethyl acetate), 1 M sodium hydroxide aqueous solution (0.26 ml) was added to a methanol (3 ml) and THF (3 ml) solution of the thus obtained colorless oil (119 mg) and the solvent was evaporated under a reduced pressure. The residue was crystallized by adding 2-propanol-diisopropyl ether to the residue, collected by filtration and then dried by heating under a reduced pressure to obtain sodium 3-(2-fluoro-4-{[1-(2-phenylethyl)-1,2,3,4-tetrahydroquinolin-7-yl]methoxy}phenyl)propanoate (95 mg) as pale yellow crystals.

### Example 2

1 M Sodium hydroxide aqueous solution (5 ml) was added to a methanol (3 ml) and THF (10 ml) solution of ethyl 3-{2-fluoro-4-[{[1-(4-hydroxybutyl)-8-methyl-1,2,3,4-tetrahydroquinolin-7-yl]methyl}(trifluoroacetyl)amino}propanoate (430 mg) and stirred at room temperature for 12 hours. After dropwise addition of 1 M hydrochloric acid (5 ml) and subsequent extraction with chloroform, the organic layer was dried with anhydrous magnesium sulfate. After removing the desiccant and evaporating the solvent under a reduced pressure, 4 M hydrogen chloride dioxane solution (1 ml) was added to a THF (10 ml) solution of the thus obtained yellow oil (411 mg) and the solvent was evaporated under a reduced pressure. After adding THF-diisopropyl ether to the residue, the thus formed solid was collected by filtration and dried by heating under a reduced pressure to obtain 3-[2-fluoro-4-({[1-(4-hydroxybutyl)-8-methyl-1,2,3,4-tetrahydroquinolin-7-yl]methyl}amino)-phenyl]propanoic acid dihydrochloride (404 mg) as a white solid.

### Example 3

Under ice-cooling, lithium hydroxide monohydrate (184 mg) was added to a mixture of ethyl 3-[2-fluoro-4-([(2-phenylethyl)sulfonyl]{[1-(2-phenylethyl)-1,2,3,4-tetrahydroquinolin-8-yl]methyl}amino)phenyl]propanoate (707 mg), mercaptoacetic acid (0.152 ml) and DMF (10 ml) and, after rising the temperature to room temperature, stirred for 2 hours. Saturated sodium bicarbonate aqueous solution was added to the reaction mixture, followed by extraction with ethyl acetate. The organic layer was washed with water and saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate), and 1 M sodium hydroxide aqueous solution (2.27 ml) was added under ice-cooling to a mixture of the thus obtained oil (349 mg), methanol (3 ml) and THF (3 ml) and stirred at room temperature for 5 hours. After evaporating the solvent under a reduced pressure, 10% citric acid aqueous solution was added to the residue to adjust to pH 5 to 6, followed by extraction with ethyl acetate. The organic layer was washed with saturated sodium chloride aqueous solution and then dried with anhydrous magnesium sulfate. After removing the desiccant and evaporating the solvent under a reduced pressure, 1 M sodium hydroxide aqueous solution (0.74 ml) was added to the residue and the solvent was evaporated under a reduced pressure. The residue was crystallized by adding ethanol to the residue, and the thus formed crystals were dissolved by heating and then spontaneously cooled to room temperature and recrystallized. By drying with heating under a reduced pressure after collection by filtration, sodium 3-[2-fluoro-4-({[1-(2-phenylethyl)-1,2,3,4-tetrahydroquinolin-8-yl]methyl}amino)phenyl]propanoate (122 mg) was obtained as colorless crystals.

### Example 4

Benzyl bromide (0.53 ml) was added to a mixture of ethyl 3-(2-fluoro-4-{[(8-methyl-1,2,3,4-tetrahydroquinolin-7-yl)methyl](trifluoroacetyl)amino}phenyl)propanoate (1.00 g), diisopropylethylamine (1.12 ml) and DMF (10 ml) and stirred at 70°C for 12 hours. Water was added to the reaction solution, followed by extraction with ethyl acetate. The organic layer was washed with saturated sodium bicarbonate aqueous solution and saturated sodium chloride aqueous solution and then dried with anhydrous magnesium sulfate. After removing the desiccant and evaporating the solvent under a reduced pressure, the residue was purified by a silica gel column chromatography (hexane-ethyl acetate), and 1 M sodium hydroxide aqueous solution (5 ml) was added to a methanol (5 ml) and THF (10 ml) mixture of the thus obtained yellow oil (1.09 g), and stirred at room temperature for 12 hours. After adding 1 M hydrochloric acid (5 ml) dropwise and subsequent extraction with chloroform, the organic layer was dried with anhydrous magnesium sulfate. After removing the desiccant and evaporating the solvent under a reduced pressure, 4 M hydrogen chloride dioxane solution (1 ml) was added to a THF (10 ml) solution of the thus obtained yellow oil (1.07 g), and the solvent was evaporated under a reduced pressure. After adding THF to the residue, the thus formed solid was collected by filtration and dried by heating under a reduced pressure to obtain 3-(4-{[(1-benzyl-8-methyl-1,2,3,4-tetrahydroquinolin-7-yl)methyl]amino}-2-fluorophenyl)propanoic acid dihydrochloride (823 mg) as a white solid.

### Example 5

1,1'-(Azodicarbonyl)dipiperidine (741 mg) was added at room temperature to a mixture of ethyl 3 -{2-fluoro-4-[{[1-(2-hydroxyethyl)-8-methyl-1,2,3,4-tetrahydroquinolin-7-yl]methyl}(trifluoroacetyl)amino]phenyl}propanoate (1.00 g), 2-chlorophenol (504 mg), tributylphosphine (0.73 ml) and THF (10 ml) and stirred at room temperature for 2 days. After separating the insoluble matter by filtration, the solvent was evaporated under a reduced pressure. The thus obtained residue was purified by a silica gel column chromatography (hexane-ethyl acetate), and 1 M sodium hydroxide aqueous solution (5.0 ml) was added under ice-cooling to a mixture of the thus obtained pale yellow oil (741 mg), methanol (5 ml) and THF (5 ml) and stirred at room temperature for 12 hours. 1 M Hydrochloric acid (5 ml) was added to the reaction solution, followed by extraction with ethyl acetate. The organic layer was washed with saturated sodium chloride aqueous solution and then dried with anhydrous magnesium sulfate. After removing the desiccant and evaporating the solvent under a reduced pressure, the thus obtained residue was dissolved in THF, followed by the addition of 4 M hydrogen chloride dioxane solution (1 ml) and then diethyl ether, the thus formed solid was collected by filtration. By drying with heating under a reduced pressure, 3-{4-[({1-[2-(2-chlorophenoxy)ethyl]-8-methyl-1,2,3,4-tetrahydroquinolin-7-yl}methyl)amino]-2-fluorophenyl}propanoic acid dihydrochloride (587 mg) was obtained as a pale yellow solid.

### Example 6

Toluene (8 ml) was added to a mixture of ethyl 3-(2-fluoro-4-{[(8-methyl-1,2,3,4-tetrahydroquinolin-7-yl)methyl](trifluoroacetyl)amino}phenyl)propanoate (500 mg), 1-bromo-4-fluorobenzene (0.15 ml), tris(dibenzylideneacetone)dipalladium (49 mg), dicyclohexyl(2',4',6'-triisopropylbiphenyl-2-yl)phosphine (51 mg) and potassium phosphate (910 mg). This mixture was allowed to undergo the reaction at 170°C for 60 minutes in a sealed tube using a microwave reactor (Biotage). The reaction mixture was spontaneously cooled down to room temperature, the insoluble matter was separated by filtration and the filtrate was evaporated under a reduced pressure. The thus obtained residue was purified by a silica gel column chromatography (hexane-ethyl acetate), and 1 M sodium hydroxide aqueous solution (3.0 ml) was added to a THF (3 ml)-ethanol (3 ml) solution of the thus obtained pale yellow syrup (241 mg) and the reaction mixture was stirred at room temperature for 15 hours. 1 M Hydrochloric acid (3.0 ml) and water (10 ml) were added to the reaction mixture, followed by extraction with chloroform. After drying the organic layer with anhydrous magnesium sulfate, the desiccant was removed and the solvent was evaporated under a reduced pressure. The thus obtained residue was dissolved in THF and 4M hydrogen chloride dioxane solution was added thereto, followed by concentration under a reduced pressure. The thus obtained residue was solidified with diethyl ether, collected by filtration and then dried with heating under a reduced pressure to obtain 3-[2-fluoro-4-({[1-(4-fluorophenyl)-8-methyl-1,2,3,4-tetrahydroquinolin-7-yl]methyl}amino)phenyl]propanoic acid hydrochloride (172 mg) as a pale yellow solid.

### Example 7

Acetic anhydride (0.06 ml) was added to a pyridine (2 ml) solution of isopropyl {6-[(1,2,3,4-tetrahydroquinolin-8-ylmethyl)(trifluoroacetyl)amino]2,3-dihydro-1-benzofuran-3-yl}acetate, and the reaction mixture was stirred at room temperature for 2 days. Saturated sodium bicarbonate aqueous solution was added to the reaction mixture, followed by extraction with ethyl acetate. The organic layer was washed with saturated sodium chloride aqueous solution and dried with anhydrous magnesium sulfate, and then the desiccant was removed and the solvent was evaporated under a reduced pressure to obtain a pale yellow syrup (229 mg). The thus obtained pale yellow syrup (229 mg) was dissolved in THF (2 ml)-ethanol (2 ml), 1 M sodium hydroxide aqueous solution (3.0 ml) was added thereto and the reaction mixture was stirred at room temperature for 2 days. To the reaction mixture were added 1 M hydrochloric acid (3.0 ml) and water (20 ml), followed by extraction with chloroform. After drying the organic layer with anhydrous magnesium sulfate, the desiccant was removed and the solvent was evaporated under a reduced pressure. The thus obtained residue was purified by a silica gel column chromatography (chloroform-methanol), and 1 M sodium hydroxide aqueous solution (0.39 ml) was added to a THF (5 ml)-methanol (5 ml) solution of the thus obtained pale yellow syrup (145 mg), followed by concentration under a reduced pressure. The thus obtained residue was crystallized with 2-propanol-diethyl ether, collected by filtration and then dried with heating under a reduced pressure to obtain sodium (6-{[(1-acetyl-1,2,3,4-tetrahydroquinolin-8-yl)methyl]amino}2,3-dihydro-1-benzofuran-3-yl)acetate (104 mg) as slightly yellow crystals.

### Example 8

Under ice-cooling, sodium borohydride (70 mg) was added to a THF (5 ml)-ethanol (5 ml) solution of ethyl 3-{2-fluoro-4-[{[8-methyl-1-(2-oxo-2-phenylethyl)-1,2,3,4-tetrahydroquinolin-7-yl]methyl}(trifluoroacetyl)amino]phenyl}propanoate, and the reaction mixture was warmed up to room temperature and stirred for 2 hours. Under ice-cooling, 1 M hydrochloric acid (10 ml) was added to the reaction mixture and stirred for 5 minutes. Thereafter, saturated sodium bicarbonate aqueous solution (20 ml) was added thereto, followed by extraction with chloroform. After drying the organic layer with anhydrous magnesium sulfate, the desiccant was removed and the solvent was evaporated under a reduced pressure to obtain a yellowish brown syrup (501 mg). 1 M Sodium hydroxide aqueous solution (3.0 ml) was added to a THF (5 ml)-ethanol (5 ml) solution of the thus obtained yellowish brown syrup (501 mg) and the reaction mixture was stirred at room temperature for 3 hours. 1 M Hydrochloric acid (3.0 ml) and water (20 ml) were added to the reaction mixture, followed by extraction with chloroform. After drying the organic layer with anhydrous magnesium sulfate, the desiccant was removed and the solvent was evaporated under a reduced pressure. The thus obtained residue was dissolved in THF (15 ml) and 4 M hydrogen chloride dioxane solution (2 ml) was added thereto, followed by concentration under a reduced pressure. Diethyl ether (20 ml) was added to the thus obtained residue and the resulting solid was collected by filtration, washed with diethyl ether and then dried with heating under a reduced pressure to obtain 3-[2-fluoro-4-({[1-(2-hydroxy-2-phenylethyl)-8-methyl-1,2,3,4-tetrahydroquinolin-7-yl]methyl}amino)phenyl]propanoic acid dihydrochloride (437 mg).

### Example 9

DMF (15 ml) was added to ethyl 3-(2-fluoro-4-{[(8-methyl-1,2,3,4-tetrahydroquinolin-7-yl)methyl](trifluoroacetyl)amino}phenyl)propanoate (1.00 g), 4-(2-chloroethyl)morpholine hydrochloride (1.99 g), potassium phosphate (4.55 g) and potassium iodide (711 mg). This mixture was allowed to undergo the reaction at 175°C for 60 minutes in a sealed tube using a microwave reactor (Biotage). Water was added to the reaction mixture, followed by extraction with ethyl acetate. After washing the organic layer with saturated sodium chloride aqueous solution, the organic layer was dried anhydrous magnesium sulfate. The desiccant was removed, the solvent was evaporated under a reduced pressure and then the residue was purified by a silica gel column chromatography (hexane-ethyl acetate), and 1 M sodium hydroxide aqueous solution (5 ml) was added to a methanol (5 ml) and THF (10 ml) mixture of the thus obtained yellow oil (220 mg) and stirred at room temperature for 12 hours. After dropwise addition of 1 M hydrochloric acid (5 ml) and subsequent extraction with chloroform, the organic layer was dried with anhydrous magnesium sulfate. The desiccant was removed, the solvent was evaporated under a reduced pressure and then 4 M hydrogen chloride dioxane solution (1 ml) was added to a THF (10 ml) solution of the thus obtained yellow oil (170 mg) and the solvent was evaporated under a reduced pressure. THF-diisopropyl ether was added to the thus formed solid was collected by filtration and then dried with heating under a reduced pressure to obtain 3-[2-fluoro-4-({[8-methyl-1-(2-morpholin-4-ylethyl)-1,2,3,4-tetrahydroquinolin-7-yl]methyl}amino)phenyl]propanoic acid trihydrochloride (183 mg) as a white solid.

### Example 10

Sodium triacetoxyborohydride (490 mg) was added at room temperature to a mixture of methyl rel-(1R,2R)-2-{4-[(1,2,3,4-tetrahydroquinolin-8-ylmethyl)(trifluoroacetyl)amino]phenyl}cyclopropanecarboxylate (333 mg), phenylacetaldehyde (0.30 ml), acetic acid (2 drops) and dichloromethane (3 ml) and stirred at room temperature for 6 hours. Phenylacetaldehyde (0.30 ml) was added to the reaction mixture and further stirred at room temperature for 12 hours. After cooling the reaction mixture with ice, methanol (1.0 ml) and sodium borohydride (87 mg) were added thereto and stirred under ice-cooling for 3 hours to consume excess amount of phenylacetaldehyde. Saturated sodium bicarbonate aqueous solution was added to the reaction mixture, followed by extraction with ethyl acetate. After washing the organic layer with saturated sodium chloride aqueous solution, the organic layer was dried anhydrous magnesium sulfate. The desiccant was removed, the solvent was evaporated under a reduced pressure and then the residue was purified by a silica gel column chromatography (hexane-ethyl acetate), and 1 M sodium hydroxide aqueous solution (4.7 ml) was added under ice-cooling to a mixture of the thus obtained colorless oil (506 mg), methanol (5 ml) and THF (5 ml) and stirred at room temperature for 3 hours. The solvent was evaporated under a reduced pressure and then 10% citric acid aqueous solution was added to the residue to adjust to pH 5 to 6. After extraction with ethyl acetate and subsequent washing of the organic layer with saturated sodium chloride aqueous solution, the organic layer was dried anhydrous magnesium sulfate. The desiccant was removed, the solvent was evaporated under a reduced pressure and then the residue was purified by a silica gel column chromatography (hexane-ethyl acetate), and the thus obtained oil was purified by a fractional HPLC (Kanto Kagaku, Mightysil RP-18 GP (20 x 250 mm, 5 µm)) to obtain an oil. The thus obtained oil was dissolved in acetonitrile, and the solid formed by adding water was collected by filtration and then dried with heating under a reduced pressure to obtain rel-(1R,2R)-2-[4-({[1-(2-phenylethyl)-1,2,3,4-tetrahydroquinolin-8-yl]methyl}amino)phenyl]cyclopropanecarboxylic acid (35 mg) as a pale yellow solid.

### Example 11

Titanium(IV) isopropoxide (0.91 ml) was added to a dichloromethane (8 ml) solution of 4-benzyl-3,4-dihydro-2H-1,4-benzoxazine-6-amine (370 ml) and ethyl 3-(2-fluoro-4-formylphenyl)propanoate (356 mg) and stirred at room temperature for 15 hours. Ethanol (8 ml) was added under ice cooling to the reaction mixture, followed by the addition of sodium borohydride (90 mg), and stirred as such for 1 hour. Under ice-cooling, 1 M hydrochloric acid (10 ml) was added dropwise to the reaction mixture and stirred at room temperature for 1 hour. The liquid property was adjusted to pH 9 to 10 with saturated sodium bicarbonate aqueous solution, and the precipitate was removed by celite filtration. The filtrate was extracted with chloroform, the organic layer was dried with anhydrous magnesium sulfate, and then the desiccant was removed and the solvent was evaporated under a reduced pressure. The thus obtained residue was purified by a silica gel column chromatography (hexane-ethyl acetate) to obtain a yellow syrup (371 mg). 1 M Sodium hydroxide aqueous solution (4.0 ml) was added to a THF (6 ml)-ethanol (6 ml) solution of the thus obtained yellow syrup (371 mg) and stirred at room temperature for 12 hours. The reaction mixture was mixed with 1 M hydrochloric acid (4.0 ml) and water (20 ml) and extracted with chloroform. After drying the organic layer with anhydrous magnesium sulfate, the desiccant was removed and the solvent was evaporated under a reduced pressure. The thus obtained residue was purified by a silica gel column chromatography (chloroform-methanol), and the thus obtained light yellow amorphous solid (344 mg) was dissolved in THF (5 ml)-ethanol (5 ml) and, after adding 1 M sodium hydroxide aqueous solution (0.80 ml), concentrated under a reduced pressure. By solidifying the thus obtained residue with water-2-propanol-diethyl ether, sodium 3-(4-{[(4-benzyl-3,4-dihydro-2H-1,4-bensoxazin-6-yl)amino]methyl}2-fluorophenyl)propanoate (316 mg) was obtained as a light yellow solid.

### Example 12

Cyclopropanecarbonyl chloride (0.06 ml) was added to a pyridine (2 ml) solution of isopropyl {6-[(1,2,3,4-tetrahydroquinolin-8-ylmethyl)(trifluoroacetyl)amino]-2,3-dihydro-1-benzofuran-3-yl}acetate (200 mg), and the reaction mixture was stirred at room temperature for 2 days. Saturated sodium bicarbonate aqueous solution (20 ml) was added to the reaction mixture, followed by extraction with ethyl acetate. The organic layer was washed with saturated sodium chloride aqueous solution and dried with anhydrous magnesium sulfate, and then the desiccant was removed and the solvent was evaporated under a reduced pressure to obtain a light yellow syrup (241 mg). The thus obtained light yellow syrup (241 mg) was dissolved in THF (2 ml)-ethanol (2 ml), 1 M sodium hydroxide aqueous solution (3.0 ml) was added thereto, and the reaction mixture was stirred at room temperature for 2 days. The reaction mixture was mixed with 1 M hydrochloric acid (3.0 ml) and water (20 ml) and extracted with chloroform. After drying the organic layer with anhydrous magnesium sulfate, the desiccant was removed and the solvent was evaporated under a reduced pressure. The thus obtained residue was purified by a silica gel column chromatography (chloroform-methanol), and the thus obtained light yellow amorphous solid (171 mg) was dissolved in THF (5 ml)-methanol (5 ml) and, after adding 1 M sodium hydroxide aqueous solution (0.42 ml), concentrated under a reduced pressure. The thus obtained residue was crystallized from 2-propanol-diethyl ether, collected by filtration and then dried with heating under a reduced pressure to obtain sodium [6-({[1-(cyclopropylcarbonyl)-1,2,3,4-tetrahydroquinolin-8-yl]methyl}amino)-2,3-dihydro-1-benzofuran-3-yl]acetate (158 mg) as light yellow crystals.

### Example 13

Benzenesulfonyl chloride (0.09 ml) was added to a pyridine (2 ml) solution of isopropyl {6-[(1,2,3,4-tetrahydroquinolin-8-ylmethyl)(trifluoroacetyl)amino]-2,3-dihydro-1-benzofuran-3-yl} acetate (200 mg), and the reaction mixture was stirred at room temperature for 2 days. Saturated sodium bicarbonate aqueous solution (20 ml) was added to the reaction mixture, followed by extraction with ethyl acetate. The organic layer was washed with saturated sodium chloride aqueous solution and dried with anhydrous magnesium sulfate, and then the desiccant was removed and the solvent was evaporated under a reduced pressure to obtain a light yellow syrup (243 mg). The thus obtained light yellow syrup (243 mg) was dissolved in THF (2 ml)-ethanol (2 ml), 1 M sodium hydroxide aqueous solution (3.0 ml) was added thereto, and the reaction mixture was stirred at room temperature for 2 days. The reaction mixture was mixed with 1 M hydrochloric acid (3.0 ml) and water (20 ml) and extracted with chloroform. After drying the organic layer with anhydrous magnesium sulfate, the desiccant was removed and the solvent was evaporated under a reduced pressure. The thus obtained residue was purified by a silica gel column chromatography (chloroform-methanol), and the thus obtained light yellow amorphous solid (193 mg) was dissolved in THF (5 ml)- methanol (5 ml) and, after adding 1 M sodium hydroxide aqueous solution (0.41 ml), concentrated under a reduced pressure. The thus obtained residue was solidified with 2-propanol-diethyl ether, collected by filtration and then dried with heating under a reduced pressure to obtain sodium [6-({[1-(phenylsulfonyl)-1,2,3,4-tetrahydroquinolin-8-yl]methyl}amino)-2,3-dihydro-1-benzofuran-3-yl]acetate (165 mg) as a light yellow solid.

### Example 14

Under ice-cooling, sodium triacetoxyborohydride (440 mg) was added to a mixture of ethyl 3-[2-fluoro-4-((1,2,3,4-tetrahydroquinolin-8-ylmethoxy)phenyl)propanoate (250 mg), phenylacetaldehyde (840 mg), acetic acid (2 drops) and dichloroethane (5 ml) and stirred at room temperature for 5 hours. Saturated sodium bicarbonate aqueous solution was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate), and 1 M sodium hydroxide aqueous solution was added at room temperature to a methanol (3 ml) solution of the thus obtained oil (310 mg) and stirred at room temperature for 3 hours. The reaction solution was adjusted to pH 5 to 6 by adding 10% citric acid aqueous solution, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain a colorless oil (96 mg). 1 M Sodium hydroxide aqueous solution (0.22 ml) was added to an ethanol solution (2 ml) of the thus obtained colorless oil, and the solvent was evaporated under a reduced pressure. Diethyl ether was added to the residue, and the thus formed solid was collected by filtration and dried with heating under a reduced pressure to obtain sodium 3-(2-fluoro-4-{[1-(2-phenylethyl)-1,2,3,4-tetrahydroquinolin-8-yl]methoxy}phenyl)propanoate (66 mg) as a light yellow amorphous solid.

### Example 15

Ethyl 3-{4-[(1,2,3,4-tetrahydroquinolin-8-yloxy)methyl]phenyl}propanoate (14 mg) was dissolved in a dichloroethane-acetic acid mixed solution (1 ml, 9:1 (v/v)), added to N-(4-formylphenyl)acetamide (13 ml) and stirred at room temperature for 30 minutes. Sodium triacetoxyborohydride (25 mg) was added to the solution and stirred at room temperature for 3 days. Thereafter, the solvent was evaporated under a reduced pressure and an extraction operation was carried out by adding saturated sodium bicarbonate aqueous solution and chloroform. The organic layer was concentrated, and the residue was dissolved in ethanol (1 ml) and stirred at 60°C for 18 hours by adding 1 M sodium hydroxide aqueous solution (0.16 ml). After spontaneous cooling, 1 M hydrochloric acid (0.16 ml) was added thereto and stirred at room temperature for 10 minutes, followed by concentration. By purifying the residue by a fractional HPLC (Waters, SunFire Prep C₁₈OBD (19 x 100 mm, 5 µm)), 3-[4-({[1-(4-acetamidobenzyl)-1,2,3,4-tetrahydroquinolin-8-yl]oxy}methyl)phenyl]propanoic acid (2.8 mg) was obtained.

### Example 16

2-Chlorobenzoic acid (13 mg) was dissolved in dichloroethane (0.5 ml), mixed with oxalyl chloride (9 µl) and DMF-dichloroethane mixed solution (5 µl, 1:1 (v/v)) and stirred at room temperature for 30 minutes. Thereafter, ethyl 3-[4-(2,3-dihydro-1H-indol-7-ylmethoxy)-2-fluorophenyl]propanoate (14 mg) was dissolved in dichloroethane (0.3 ml) and added thereto together with triethylamine (0.025 ml) and stirred at 40°C for 18 hours. After spontaneous cooling to room temperature, an extraction operation was carried out by adding saturated sodium bicarbonate aqueous solution and chloroform to the solution. The organic layer was concentrated, and the residue was dissolved in ethanol (1 ml), mixed with 1 M sodium hydroxide aqueous solution (0.2 ml) and stirred at room temperature for 3 hours and then at 45°C for 18 hours. After spontaneous cooling, 1 M hydrochloric acid (0.2 ml) was added thereto and stirred at room temperature for 10 minutes, followed by concentration. By purifying the residue by a fractional HPLC (Waters, SunFire Prep C₁₈OBD (19 x 100 mm, 5 µm)), 3-(4-{[1-(2-chlorobenzoyl)-2,3-dihydro-1H-indol-7-yl]methoxy}-2-fluorophenyl)propanoic acid (8.8 mg) was obtained.

### Example 17

Ethyl 3-{4-[(1,2,3,4-tetrahydroquinolin-8-yloxy)methyl]phenyl}propanoate (14 mg) was dissolved in DMF (0.5 ml) and added to 1-(bromomethyl)-2-fluorobenzene (23 mg), further added to N,N-diisopropylethylamine (0.042 ml) and stirred at 60°C for 18 hours. After spontaneous cooling, an extraction operation was carried out by adding saturated sodium bicarbonate aqueous solution and chloroform to the solution. The organic layer was concentrated, and the residue was dissolved in ethanol (1 ml), mixed with 1 M sodium hydroxide aqueous solution (0.2 ml) and stirred at 50°C for 18 hours. After spontaneous cooling, 1 M hydrochloric acid (0.2 ml) was added thereto and stirred at room temperature for 10 minutes, followed by concentration. By purifying the residue by a fractional HPLC (Waters, SunFire Prep C₁₈OBD (19 x 100 mm, 5 µm)), 3-[4-({[1-(2-fluorobenzyl)-1,2,3,4-tetrahydroquinolin-8-yl]oxy}methyl)phenyl]propanoic acid (10.9 mg) was obtained.

### Example 18

Ethyl 3-[4-(2,3-dihydro-1H-indol-7-ylmethoxy)-2-fluorophenyl]propanoate (14 mg) was dissolved in pyridine (0.5 ml), added to 3-methylbenzenesulfonyl chloride (15 mg) and stirred at room temperature for 4 days. Thereafter, an extraction operation was carried out by adding saturated sodium bicarbonate aqueous solution and chloroform to the solution. The organic layer was concentrated, and the residue was dissolved in ethanol (1 ml), mixed with 1 M sodium hydroxide aqueous solution (0.2 ml) and stirred at 50°C for 18 hours. After spontaneous cooling, 1 M hydrochloric acid (0.2 ml) was added thereto and stirred at room temperature for 10 minutes, followed by concentration. By purifying the residue by a fractional HPLC (Waters, SunFire Prep C₁₈OBD (19 x 100 mm, 5 µm)), 3-[2-fluoro-4-({1-[(3-methylphenyl)sulfonyl]-2,3-dihydro-1H-indol-7-yl}methoxyphenyl)propanoic acid (8.4 mg) was obtained.

### Example 19

1,1'-(Azodicarbonyl)dipiperidine (0.43 g) was added under ice-cooling to a mixture of 1-(2-phenylethyl)-1,2,3,4-tetrahydroquinolin-8-ol (0.44 g), ethyl 3-[2-fluoro-4-(hydroxymethyl)phenyl]propanoate (0.30 g), tributylphosphine (0.43 ml) and THF (3.0 ml) and stirred at room temperature for 2 days. After separation of the insoluble matter by filtration, the solvent was evaporated under a reduced pressure. By purifying the residue by a silica gel column chromatography (hexane-ethyl acetate), ethyl 3-[2-fluoro-4-({1-(2-phenylethyl)-1,2,3,4-tetrahydroquinolin-8-yl]oxy}methyl)phenyl]propanoate (0.16 g) was obtained as a colorless oil.

### Example 20

1,1'-(Azodicarbonyl)dipiperidine (0.43 g) was added under ice-cooling to a mixture of tert-butyl 5-(hydroxymethyl)-3,4-dihydroquinoline-1(2H)-carboxylate (0.930 g), ethyl 3-(2-fluoro-4-hydroxyphenyl) propanoate (1.12 g), tributylphosphine (1.31 ml) and THF (9.0 ml) and stirred at room temperature for 12 hours. After separation of the insoluble matter by filtration, the solvent was evaporated under a reduced pressure. By purifying the residue by a silica gel column chromatography (hexane-ethyl acetate), tert-butyl 5-{[4-(3-ethoxy-3-oxopropyl)-3-fluorophenoxy]methyl}-3,4-dihydroquinoline-1(2H)-carboxylate (1.08 g) was obtained as a colorless oil.

### Example 21

Acetic acid (0.14 ml) was added to a dichloroethane (5 ml) solution of tert-butyl 7-formyl-8-methyl-3,4-dihydroquinoline-1(2H)-carboxylate (480 mg) and ethyl 3-(4-amino-2-fluorophenyl)propanoate (368 mg) and stirred at room temperature for 5 hours. Sodium triacetoxyborohydride (480 mg) was added to the reaction mixture and stirred at room temperature for 30 minutes. By adding ethyl acetate, washed with saturated sodium bicarbonate aqueous solution and saturated sodium chloride aqueous solution. After drying the organic layer with anhydrous magnesium sulfate, the desiccant was removed and the solvent was evaporated under a reduced pressure. By purifying the residue by a silica gel column chromatography (hexane-ethyl acetate), tert-butyl 7-({[4-(3-ethoxy-3-oxopropyl)-3-fluorophenyl]amino}methyl)-8-methyl-3,4-dihydroquinoline-1(2H)-carboxylate (740 mg) was obtained as a colorless oil.

### Example 22

Titanium(IV) isopropoxide (2.50 ml) was added to a dichloroethane (25 ml) solution of methyl (6-amino-2,3-dihydro-1-benzofuran-3-yl)acetate (1.468 g) and tert-butyl 8-formyl-3,4-dihydroquinoline-1(2H)-carboxylate (1.85 g) and stirred at room temperature for 5 hours. Sodium triacetoxyborohydride (480 mg) was added to the reaction mixture, and the reaction mixture was stirred at room temperature for 3 days. Under ice-cooling, ethanol (25 ml) was added to the reaction mixture and then sodium borohydride (270 mg) was added thereto, and stirred as such for 1 hour. Under ice-cooling, 10% citric acid aqueous solution (20 ml) was added to the reaction mixture and, after warming up to room temperature, stirred for 15 minutes. Thereafter, the liquid property was adjusted to pH 9 to 10 by adding saturated sodium bicarbonate aqueous solution and the precipitate was removed by celite filtration. The filtrate was extracted with ethyl acetate and the organic layer was dried with anhydrous magnesium sulfate. After removing the desiccant, the solvent was evaporated under a reduced pressure and the thus obtained residue was purified by a silica gel column chromatography (hexane-ethyl acetate) to obtain isopropyl [6-({[1-(tert-butoxycarbonyl)-1,2,3,4-tetrahydroquinolin-8-yl]methyl}amino)-2,3-dihydro-1-benzofuran-3-yl]acetate as a colorless amorphous solid.

### Example 23

Sodium triacetoxyborohydride (1.20 g) and acetic acid (0.19 ml) were added at room temperature to a mixture of ethyl 3-(2-fluoro-4-{[(1-propyl-1,2,3,4-tetrahydroquinolin-8-yl)amino]methyl}phenyl)propanoate (0.45 g), 37% formaldehyde aqueous solution (0.91 g) and dichloroethane (4.5 ml) and stirred at room temperature for 1 hour. Saturated sodium bicarbonate aqueous solution was added to the reaction mixture, followed by extraction with ethyl acetate. The organic layer was washed with saturated sodium bicarbonate aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-(2-fluoro-4-{[methyl(1-propyl-1,2,3,4-tetrahydroquinolin-8-yl)amino]methyl}phenyl)propanoate (0.38 g) as a colorless oil.

### Example 24

Under ice-cooling on an ice-methanol bath, sodium borohydride (0.41 g) was added to a mixture of methyl (2E)-3-{4-[(quinolin-8-yloxy)methyl]phenyl}acrylate (1.16 g), nickel(II) hexahydrate (0.26 g) and methanol (20 ml) and stirred under ice-cooling for 1.5 hours. 10% Citric acid aqueous solution was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain methyl 3-{4-[(1,2,3,4-tetrahydroquinolin-8-yloxy)methyl]phenyl}propanoate (0.82 g) as a colorless oil.

### Example 25

Under ice-cooling on an ice-methanol bath, sodium borohydride (210 mg) was added to a mixture of ethyl 3-[2-fluoro-4-(quinolin-7-ylmethoxy)phenyl]propanoate (320 mg), nickel(II) hexahydrate (65 mg) and methanol (5 ml) and stirred under ice-cooling for 2 hours. Water was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-[2-fluoro-4-(1,2,3,4-tetrahydroquinolin-7-ylmethoxy) phenyl]propanoate (170 mg) as a colorless oil.

### Example 26

Under ice-cooling on an ice-methanol bath, sodium borohydride (110 mg) was added to a mixture of ethyl (2E)-3-(2-fluoro-4-{[(1-propyl-1,2,3,4-tetrahydroquinolin-8-yl)amino]methyl}phenyl)acrylate (980 mg), nickel(II) hexahydrate (180 mg), methanol (10 ml) and THF (10 ml) and stirred under ice-cooling for 5 hours. Water was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-(2-fluoro-4-{[(1-propyl-1,2,3,4-tetrahydroquinolin-8-yl)amino]methyl}phenyl)propanoate (900 mg) as a colorless oil.

### Example 27

Under ice-cooling, 4 M hydrogen chloride dioxane solution (15 ml) was added to a THF (15 ml) solution of tert-butyl 8-{[4-(3-ethoxy-3-oxopropyl)-3-fluorophenoxy]methyl}-3,4-dihydroquinolin-1(2H)-yl]carboxylate (3.2 g) and stirred at room temperature for 4 hours. The solvent was evaporated under a reduced pressure and then saturated sodium bicarbonate aqueous solution was added to the residue, followed by extraction with ethyl acetate. The organic layer was washed with saturated sodium chloride aqueous solution and then dried with anhydrous magnesium sulfate. The desiccant was removed and the solvent was evaporated under a reduced pressure The residue was purified by a silica gel column chromatography (hexane-ethyl acetate) to obtain ethyl 3-[2-fluoro-4-(1,2,3,4-tetrahydroquinolin-8-ylmethoxy)phenyl]propanoate (1.8 g) as a colorless oil.

### Example 28

Under ice-cooling, sodium triacetoxyborohydride (1.6 g) was added to a mixture of methyl 3-{4-[(1,2,3,4-tetrahydroquinolin-8-yloxy)methyl]phenyl}propanoate (0.82 g), benzaldehyde (0.38 ml), acetic acid (0.43 ml) and dichloroethane (10 ml) and stirred at room temperature for 9 hours. Benzaldehyde (0.38 ml) and sodium triacetoxyborohydride (0.80 g) were added to the reaction mixture and stirred at room temperature for 12 hours. Saturated sodium bicarbonate aqueous solution was added to the reaction mixture, followed by extraction with ethyl acetate. The organic layer was washed with water and saturated sodium chloride aqueous solution in that order and then dried with anhydrous magnesium sulfate. The desiccant was removed and the solvent was evaporated under a reduced pressure The residue was purified by a silica gel column chromatography (hexane-ethyl acetate) to obtain methyl 3-(4-{[(1-benzyl-1,2,3,4-tetrahydroquinolin-8-yl)oxy]methyl}phenyl)propanoate (0.82 g) as a colorless amorphous solid.

### Example 29

4 M Hydrogen chloride dioxane solution (15 ml) was added at room temperature to ethyl 3-(4-{(tert-butoxycarbonyl)[(1-methyl-1,2,3,4-tetrahydroquinolin-7-yl)methyl]amino}-2-fluorophenyl)propanoate (648 mg) and stirred for 30 minutes. The solvent was evaporated under a reduced pressure and saturated sodium bicarbonate aqueous solution was added, followed by extraction with ethyl acetate. The organic layer was washed with saturated sodium chloride aqueous solution and then dried with anhydrous magnesium sulfate. The desiccant was removed and the solvent was evaporated under a reduced pressure to obtain ethyl 3-(2-fluoro-4-{[(1-methyl-1,2,3,4-tetrahydroquinolin-7-yl)methyl]amino}phenyl)propanoate (496 mg) as a brown oil.

### Example 30

A mixture of ethyl 3-{2-fluoro-4-[(1,2,3,4-tetrahydroquinolin-8-yloxy)methyl]phenyl}propanoate (400 mg), 1-(bromomethyl)-4-methoxybenzene (680 mg), N,N-diisopropylethylamine (0.58 ml) and DMF (4 ml) was stirred at 80°C for 12 hours. Water was added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-[2-fluoro-4-({[1-(4-methoxybenzyl)-1,2,3,4-tetrahydroquinolin-8-yl]oxy}methyl)phenyl]propanoate (240 mg) as a colorless oil.

### Example 31

Benzoyl chloride (0.13 ml) was added at room temperature to a pyridine (2 ml) solution of ethyl 3-[2-fluoro-4-(1,2,3,4-tetrahydroquinolin-8-ylmethoxy)phenyl]propanoate (200 mg) and stirred at room temperature for 3 hours. After evaporation of the solvent under a reduced pressure, 10% citric acid aqueous solution was added to the residue, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-{4-[(1-benzoyl-1,2,3,4-tetrahydroquinolin-8-yl)methoxy]-2-fluorophenyl}propanoate (220 mg) as a light yellow oil.

### Example 32

Benzenesulfonyl chloride (0.14 ml) was added at room temperature to a pyridine (2 ml) solution of ethyl 3-[2-fluoro-4-(1,2,3,4-tetrahydroquinolin-8-ylmethoxy)phenyl}propanoate (200 mg) and stirred at room temperature for 12 hours. After evaporation of the solvent under a reduced pressure, 10% citric acid aqueous solution was added to the residue, followed by extraction with ethyl acetate. The organic layer was washed with saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-(2-fluoro-4-{[1-(phenylsulfonyl)-1,2,3,4-tetrahydroquinolin-8-yl]methoxy}phenyl]propanoate (230 mg) as a colorless oil.

### Example 33

Acetic anhydride (0.254 ml) was added at room temperature to a pyridine (5 ml) solution of ethyl 3-(2-fluoro-4-{[(1-propyl-1,2,3,4-tetrahydroquinolin-8-yl)amino]methyl}phenyl)propanoate (450 mg) and stirred at room temperature for 3 days. 10% Citric acid aqueous solution was added to the reaction solution, followed by extraction with ethyl acetate. The organic layer was washed with 10% citric acid aqueous solution and saturated sodium chloride aqueous solution in that order and then dried with anhydrous magnesium sulfate. The desiccant was removed and the solvent was evaporated under a reduced pressure. The residue was purified by a silica gel column chromatography (hexane-ethyl acetate) to obtain ethyl 3-(4-{[acetyl(1-propyl-1,2,3,4-tetrahydroquinolin-8-yl)amino]methyl}-2-fluorophenyl)propanoate (430 mg) as a colorless oil.

### Example 34

A mixture of ethyl 3-[2-fluoro-4-(1,2,3,4-tetrahydroquinolin-8-ylmethoxy)phenyl]propanoate (380 mg), triphenylbismuthine (520 mg), copper(II) acetate (140 mg) and dichloroethane (3 ml) was stirred at 100°C for 12 hours. After removing the insoluble matter by celite filtration, the solvent was evaporated under a reduced pressure. The residue was purified by a silica gel column chromatography (hexane-ethyl acetate) to obtain ethyl 3-{2-fluoro-4-[(1-phenyl-1,2,3,4-tetrahydroquinolin-8-yl)methyl]phenyl}propanoate (120 mg) as a light yellow oil.

### Example 35

In an atmosphere of hydrogen, a mixture of ethyl 3-[2-fluoro-4-({[1-(2-methoxy-2-phenylvinyl)-1,2,3,4-tetrahydroquinolin-5-yl]methyl}amino)phenyl]propanoate (200 mg), 10% palladium-activated carbon (44 mg), ethanol (1.5 ml) and THF (1.5 ml) was stirred at room temperature for 6 hours. After removing the insoluble matter by celite filtration, the solvent was evaporated under a reduced pressure. The residue was purified by a silica gel column chromatography (hexane-ethyl acetate) to obtain ethyl 3-[2-fluoro-4-({[1-(2-methoxy-2-phenylethyl)-1,2,3,4-tetrahydroquinolin-5-yl]methyl}amino)phenyl]propanoate (130 mg) as a light yellow oil.

### Example 36

1 M Sodium hydroxide aqueous solution (9.0 ml) was added to a THF (10 ml) and ethanol (10 ml) solution of diethyl methyl[4-({[1-(2-phenylethyl)-1,2,3,4-tetrahydroquinolin-5-yl]methyl}amino)benzyl]malonate (955 mg) and stirred at room temperature for 15 hours. 1 M Hydrochloric acid (9.0 ml) and water (30 ml) were added to the reaction mixture, followed by extraction with chloroform, and the organic layer was dried with anhydrous magnesium sulfate. The desiccant was removed and the solvent was evaporated under a reduced pressure The thus obtained residue was dissolved in THF (10 ml) and ethanol (10 ml), mixed with 5 M sodium hydroxide aqueous solution (5.0 ml), stirred at 70°C for 8 hours and then spontaneously cooled to room temperature. 1 M Hydrochloric acid (25 ml) and water (30 ml) were added to the reaction mixture, followed by extraction with chloroform, and the organic layer was dried with anhydrous magnesium sulfate. The desiccant was removed and the solvent was evaporated under a reduced pressure The thus obtained residue was dissolved in dioxane (20 ml) and stirred at 120°C for 2 hours. The reaction mixture was stirred at 130°C for 17 hours and then spontaneously cooled to room temperature. The reaction mixture was concentrated under a reduced pressure, and the thus obtained residue was purified by a silica gel column chromatography (chloroform-methanol) to obtain a brown solid (454 mg). The thus obtained brown solid was dissolved in THF (10 ml) and ethanol (10 ml) and 1 M sodium hydroxide aqueous solution (1.02 ml) was added thereto, followed by concentration under a reduced pressure. Diethyl ether (15 ml) was added to the thus obtained residue and stirred for 1 hour. The solid was collected by filtration, washed with diethyl ether and then dried at 60°C under a reduced pressure to obtain sodium 2-methyl-3-[4-({[1-(2-phenoxyethyl)-1,2,3,4-tetrahydroquinolin-5-yl]methyl}amino)phenyl]propanoate (400 mg) as a light brown solid.

### Example 37

3 M Hydrochloric acid (5 ml) was added to a THF (10 ml) solution of 7-(dimethoxymethyl)-1-(4-fluorophenyl)-1,2,3,4-tetrahydro-1,8-naphthyridine (665 mg) and stirred at room temperature for 2 hours. 3 M Sodium hydroxide aqueous solution (5 ml) was added to the reaction mixture, followed by extraction with ethyl acetate, and the organic layer was dried with anhydrous magnesium sulfate. The desiccant was removed and the solvent was evaporated under a reduced pressure The thus obtained residue was dissolved in dichloroethane (10 ml), mixed with ethyl 3-(4-amino-2-fluorophenyl)propanoate (465 mg), acetic acid (0.18 ml) and sodium triacetoxyborohydride (606 mg) and stirred at room temperature for 30 minutes. Saturated sodium bicarbonate aqueous solution (30 ml) was added to the reaction mixture, followed by extraction with ethyl acetate. The organic layer was saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-[2-fluoro-4-({[8-(4-fluorophenyl)-5,6,7,8-tetrahydro-1,8-naphthyridin-2-yl]methyl}amino)phenyl]propanoate (735 mg) as a yellow solid.

### Example 38

Tributylphosphine (3.10 ml) and 1,1'-(azodicarbonyl)dipiperidine (3.13 g) were added to a THF (20 ml) solution of [8-methyl-1-(2-phenoxyethyl)-1,2,3,4-tetrahydroquinolin-5-yl]methanol (1.48 g) and ethyl 3-(2-fluoro-4-{[(2-nitrophenyl)sulfonyl]amino}phenyl)propanoate (2.17 g), and the reaction mixture was stirred at room temperature for 2 days. The precipitate was separated by filtration and washed with THF, and the filtrate was mixed with silica gel (10 g) and concentrated under a reduced pressure. By purifying the thus obtained carrier by a basic silica gel (Fuji Silysia Chemical) column chromatography (hexane-ethyl acetate), a yellow syrup (4.79 g) was obtained. Under ice-cooling, mercaptoacetic acid (1.05 ml) and lithium hydroxide monohydrate (1.25 g) were added to a DMF (35 ml) solution of the thus obtained yellow syrup and, after warming up to room temperature, stirred for 2 hours. Saturated sodium bicarbonate aqueous solution (50 ml) and water (50 ml) were added to the reaction mixture, followed by extraction with ethyl acetate. The organic layer was washed with saturated sodium chloride aqueous solution and then dried with anhydrous magnesium sulfate. The desiccant was removed and the solvent was evaporated under a reduced pressure By purifying the thus obtained residue by a basic silica gel (Fuji Silysia Chemical) column chromatography (hexane-ethyl acetate), a light yellow syrup (2.24 g) was obtained. By further purifying the thus obtained light yellow syrup by an ODS column chromatography (acetonitrile-water), ethyl 3-[2-fluoro-4-({[8-methyl-1-(2-phenoxyethyl)-1,2,3,4-tetrahydroquinolin-5-yl]methyl}amino)phenyl]propanoate (1.32 g) was obtained as a colorless solid.

### Example 39

Tributylphosphine (0.45 ml) and 1,1'-(azodicarbonyl)dipiperidine (3.13 460 mg) were added to a THF (20 ml) solution of [1-(2-phenoxyethyl)-1,2,3,4-tetrahydroquinolin-5-yl]methanol (400 mg) and ethyl 3-(2-fluoro-4-hydroxyphenyl)propanoate (450 mg), and the reaction mixture was stirred at room temperature for 12 hours. The precipitate was separated by filtration and then the solvent was evaporated under a reduced pressure. By purifying the thus obtained residue by a silica gel column chromatography (hexane-ethyl acetate), a white solid (393 mg) was obtained. 1 M Sodium hydroxide aqueous solution (3.0 ml) was added to a mixture of the thus obtained white solid, ethanol (5 ml) and THF (5 ml) and stirred at room temperature for 20 hours. 1 M Hydrochloric acid (3.0 ml) was added to the reaction solution, followed by extraction with ethyl acetate. The organic layer was washed with saturated sodium chloride aqueous solution and then dried with anhydrous magnesium sulfate. The desiccant was removed and the solvent was evaporated under a reduced pressure. 1 M Sodium hydroxide aqueous solution (0.823 ml) was added to a THF (5 ml) solution of the thus obtained residue and the solvent was evaporated under a reduced pressure. Diethyl ether was added to the thus obtained residue and the thus formed solid was collected by filtration. By drying with heating under a reduced pressure, sodium 3-(2-fluoro-4-{[1-(2-phenoxyethyl)-1,2,3,4-tetrahydroquinolin-5-yl]methoxy}phenyl)propanoate (328 mg) was obtained as a white solid.

### Example 40

Under ice-cooling, lithium hydroxide monohydrate (150 mg) was added to a mixture of ethyl 3-[2-fluoro-4-([(2-nitrophenyl)sulfonyl]{[1-(3-phenylpropyl)-1,2,3,4-tetrahydroquinolin-5-yl]methyl}amino)phenyl]propanoate (591 mg), mercaptoacetic acid (0.13 ml) and DMF (6 ml) and, after warming up to room temperature, stirred for 2 hours. Saturated sodium bicarbonate aqueous solution was added to the reaction solution, followed by extraction with ethyl acetate. The organic layer was washed with saturated sodium bicarbonate aqueous solution and saturated sodium chloride aqueous solution and then dried with anhydrous magnesium sulfate. The desiccant was removed and the solvent was evaporated under a reduced pressure. The residue was purified by a silica gel column chromatography (hexane-ethyl acetate) to obtain ethyl 3-[2-fluoro-4-({[1-(3-phenylpropyl)-1,2,3,4-tetrahydroquinolin-5-yl]methyl}amino)phenyl]propanoate (401 mg) as a light yellow oil.

In the same manner as in the methods of Examples 1 to 40, the Example compounds 41 to 478 shown in the following tables were produced using respectively corresponding materials. Structures and production methods of respective Example compounds are shown in Tables 32 to 109, and physicochemical data thereof in Tables 110 to 133.

Also, structures of other compounds of the invention are shown in Tables 134 to 136. These can be easily synthesized using the aforementioned production methods, the methods described in Examples and the methods obvious to those skilled in the art, or modified methods thereof.

In addition, the following abbreviations are used in the following tables. PEx: production example, Ex: Example, No: compound number, EI: m/z value of EI-MS ((M)⁺ unless otherwise noted), CI: m/z value of CI-MS ((M+H)⁺ unless otherwise noted), ESI+: m/z value of ESI-MS (anion) ((M-H)⁻ unless otherwise noted), FAB+: m/z value of FAB-MS (cation) ((M+H)⁺ unless otherwise noted), FAB-: m/z value of FAB-MS (anion) ((M-H)⁻ unless otherwise noted), NMR1: δ (ppm) of ¹H NMR in DMSO-d₆, NMR2: δ (ppm) of ¹H NMR in CDCl₃, NMR3: δ (ppm) of ¹H NMR in CD₃OH, PSyn: production method (The number means that the production example compound was produced using a corresponding material in the same manner as in the production example compound having the number as a production example number. The case of having E before the number means that the production example compound was produced using a corresponding material in the same manner as in the Example compound having the number as an Example number.), Syn: production method (The number means that the Example compound was produced using a corresponding material in the same manner as in the Example compound having the number as an Example number. The case of having P before the number means that the Example compound was produced using a corresponding material in the same manner as in the production example compound having the number as a production example number.), Me: methyl, Et: ethyl, iPr: isopropyl, Boc: tert-butoxycarbonyl, Ns: 2-nitrobenzenesulfonyl. Also, the HCl in the structural formulae represents hydrochloride, and the number before HCl represents molar ratio. For example, 2HCl represents dihydrochloride.) Also, 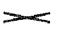 represents double bond of E or Z.

**[Table 4]**

| PEx | PSyn | Structure | Data |
|---|---|---|---|
| 1 | 1 | 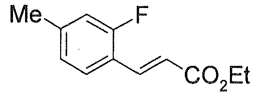 | EI:208 |
| 50 | 1 | 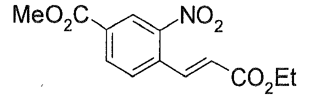 | FAB+:280 |
| 2 | 2 | 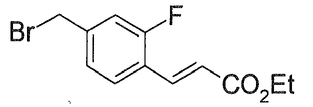 | EI:289 |
| 3 | 3 | 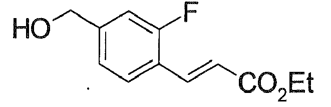 | FAB+:225 |
| 4 | 4 | 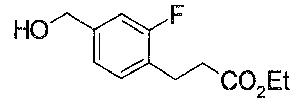 | EI:226 |
| 5 | 5 | 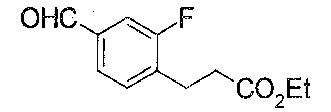 | ESI+:225 |
| 6 | 6 | 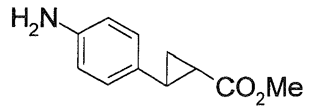 | EI:191 |
| 7 | 7 | 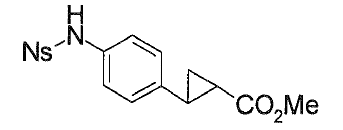 | FAB+:377 |
| 8 | 8 | 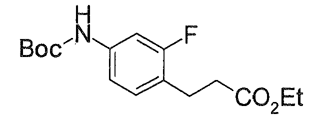 | ESI+:312 |
| 9 | 9 | 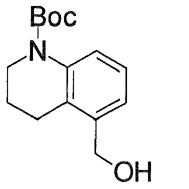 | FAB+:264 |

**[Table 5]**

| | | | |
|---|---|---|---|
| 10 | 10 | 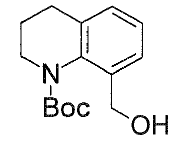 | FAB+264 |
| 51 | 10 | 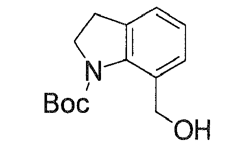 | EI:249 |
| 11 | 11 | 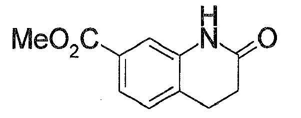 | FAB+:206 |
| 12 | 12 | 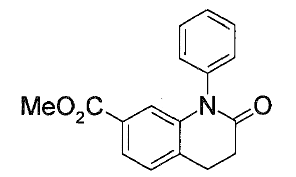 | ESI+:282 |
| 13 | 13 | 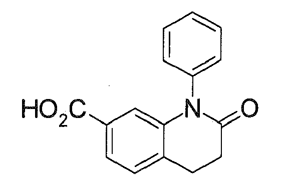 | ESI+:268 |
| 14 | 14 | 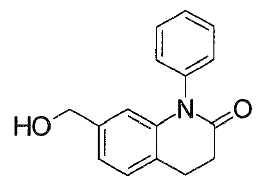 | ESI+:254 |
| 15 | 15 | 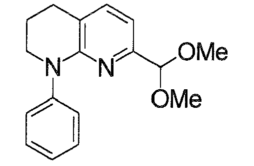 | ESI+:285 |

**[Table 6]**

| | | | |
|---|---|---|---|
| 16 | 16 | 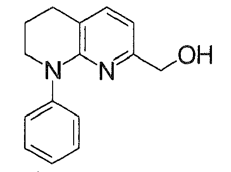 | ESI+:241 |
| 17 | 17 | 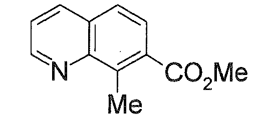 | ESI+:202 |
| 18 | 18 | 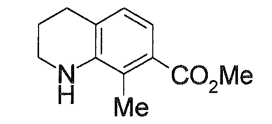 | ESI+:206 |
| 19 | 19 | 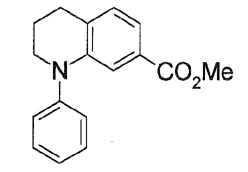 | FAB+:268 |
| 52 | 19 | 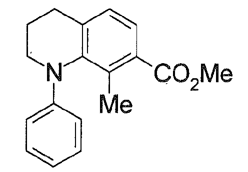 | ESI+:282 |
| 20 | 20 | 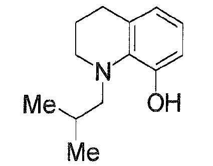 | EI:205 |
| 53 | 20 | 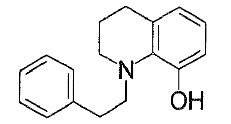 | ESI+:254 |
| 54 | 20 | 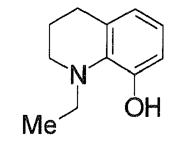 | ESI+:178 |

**[Table 7]**

| | | | |
|---|---|---|---|
| 55 | 20 | 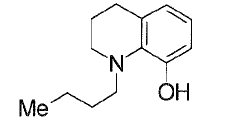 | EI:205 |
| 56 | 20 | 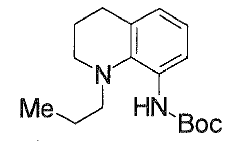 | FAB+:291 |
| 57 | 20 | 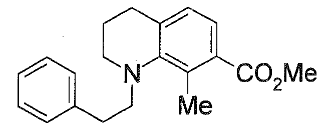 | ESI+:310 |
| 21 | 21 | 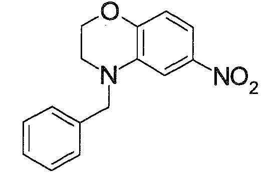 | ESI+:271 |
| 58 | 21 | 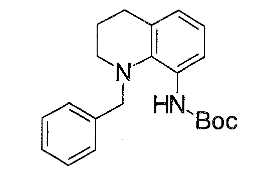 | FAB+:339 |
| 59 | 21 | 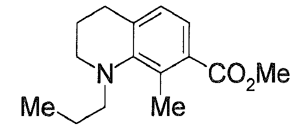 | ESI+:248 |
| 22 | 22 | 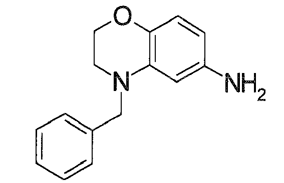 | EI:240 |
| 23 | 23 | 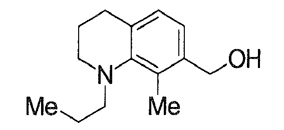 | ESI+:220 |

**[Table 8]**

| | | | |
|---|---|---|---|
| 60 | 23 | 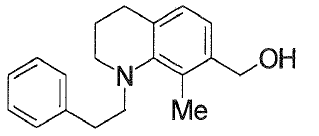 | ESI+:282 |
| 61 | 23 | 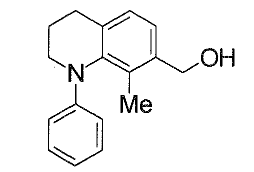 | ESI+:254 |
| 62 | 23 | 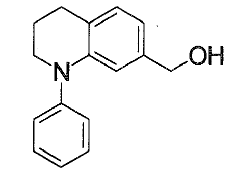 | FAB+:240 |
| 24 | 24 | 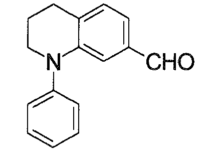 | EI:237 |
| 63 | 24 | 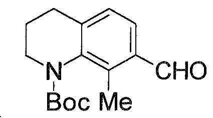 | EI:275 |
| 25 | 25 | 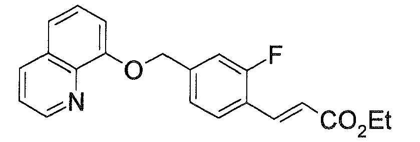 | FAB+:352 |
| 64 | 25 | 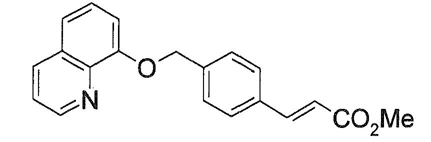 | FAB+:320 |
| 65 | 25 | 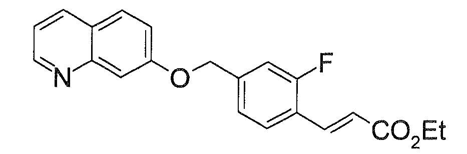 | ESI+:352 |

**[Table 9]**

| | | | |
|---|---|---|---|
| 66 | 25 | 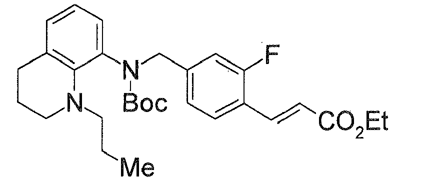 | FAB+:497 |
| 67 | 25 | 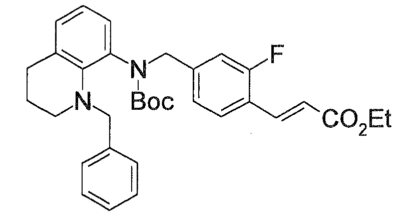 | FAB+:545 |
| 26 | 26 | 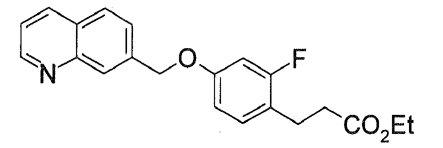 | FAB+:354 |
| 68 | 26 | 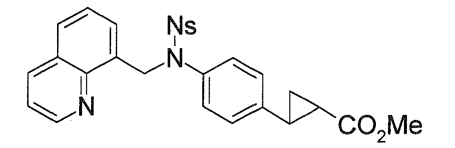 | FAB+:518 |
| 69 | 26 | 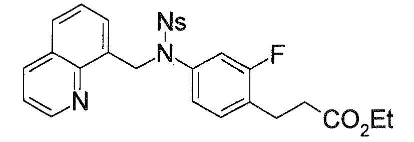 | FAB+:538 |
| 70 | 26 | 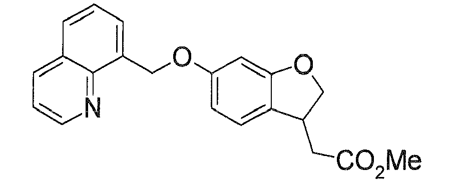 | FAB+:350 |
| 71 | 26 | 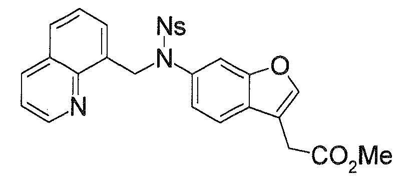 | ESI+:532 |

**[Table 10]**

| | | | |
|---|---|---|---|
| 72 | 26 | 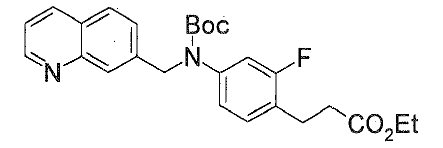 | ESI+:453 |
| 73 | E20 | 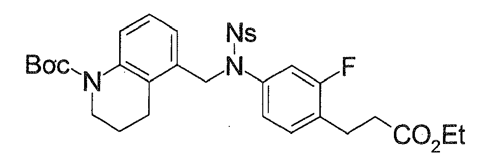 | FAB+:641 (M)+ |
| 74 | E20 | 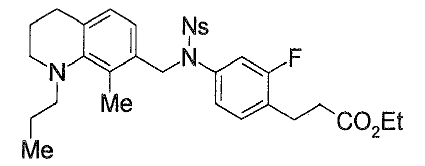 | ESI+:598 |
| 75 | E20 | 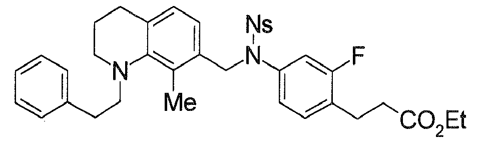 | ESI+:660 |
| 76 | E20 | 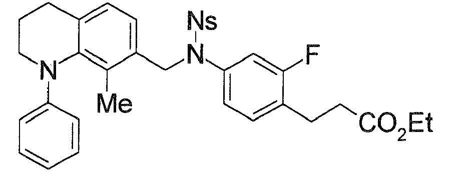 | ESI+:632 |
| 77 | E20 | 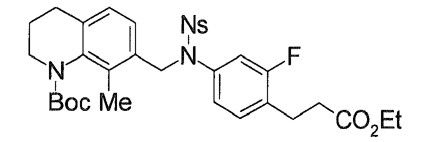 | FAB+:655 |
| 78 | E20 | 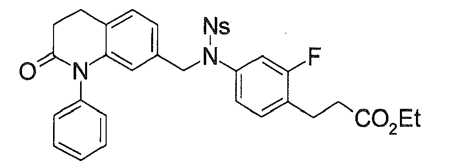 | ESI+:632 |
| 79 | E20 | 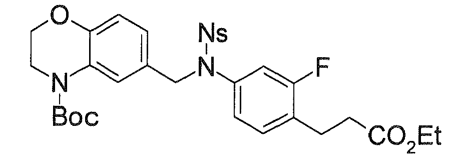 | ESI-:642 |

**[Table 11]**

| | | | |
|---|---|---|---|
| 80 | E20 | 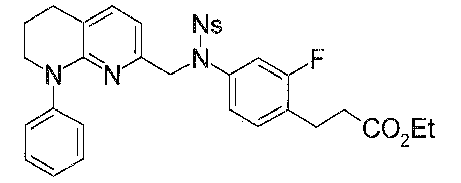 | ESI+:619 |
| 27 | 27 | 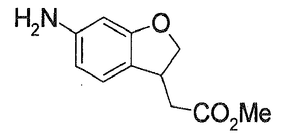 | EI:207 |
| 28 | 28 | 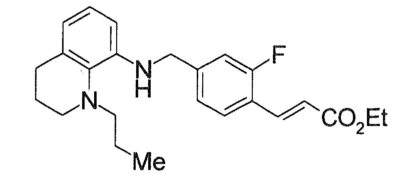 | FAB+:397 |
| 81 | 28 | 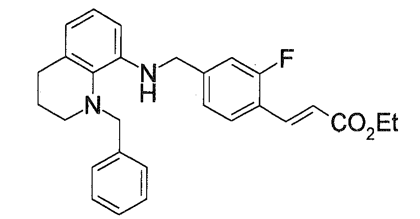 | FAB+:445 |
| 82 | 28 | 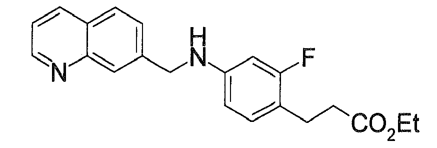 | ESI+:353 |
| 83 | E23 | 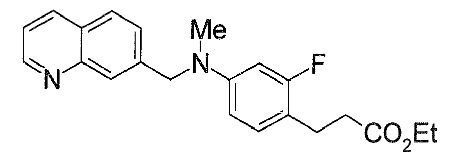 | ESI+:367 |
| 84 | E25 | 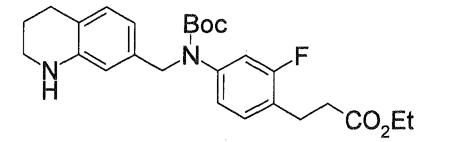 | ESI+:457 |

**[Table 12]**

| | | | |
|---|---|---|---|
| 29 | 29 | 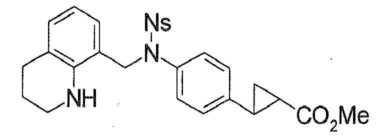 | ESI-:520 |
| 85 | 29 | 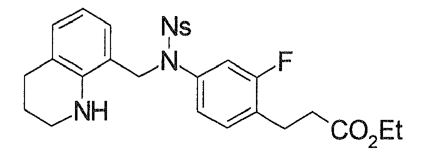 | FAB+:541 (M)+ |
| 86 | 29 | 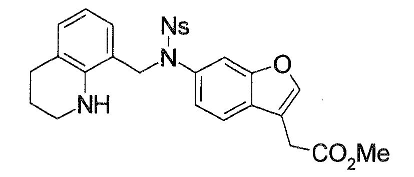 | ESI+:536 |
| 30 | 30 | 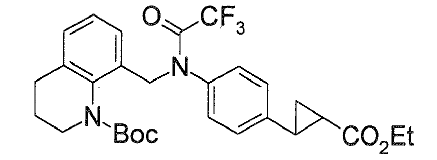 | FAB+:533 |
| 87 | 30 | 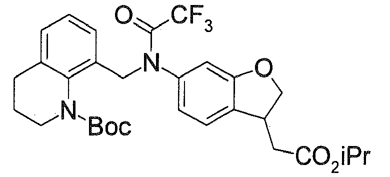 | ESI+:577 |
| 88 | 30 | 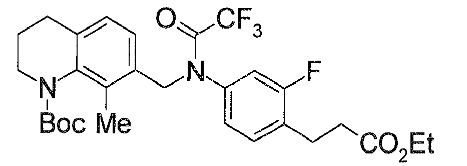 | FAB+:567 |
| 89 | E27 | 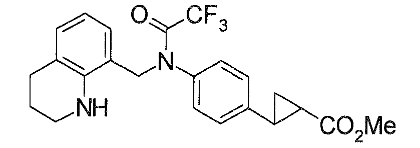 | ESI+:433 |

**[Table 13]**

| | | | |
|---|---|---|---|
| 90 | E27 | 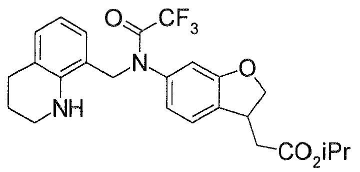 | ESI+:477 |
| 91 | E27 | 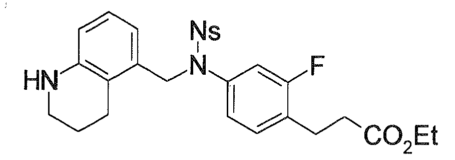 | FAB+:542 |
| 92 | E27 | 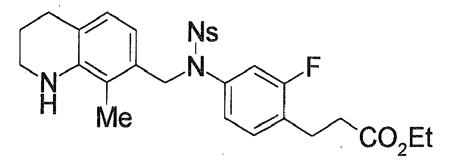 | ESI+:556 |
| 93 | E27 | 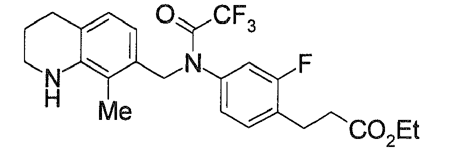 | ESI+:467 |
| 94 | E27 | 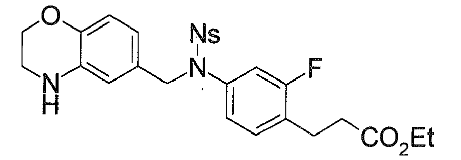 | ESI+:544 |
| 95 | E28 | 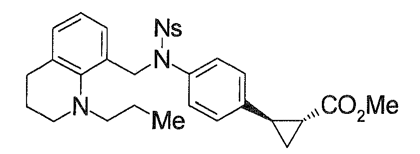 | FAB-:563 (M)- |
| 96 | E28 | 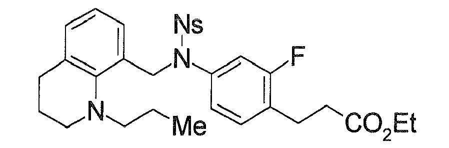 | FAB+:584 |

**[Table 14]**

| | | | |
|---|---|---|---|
| 97 | E28 | 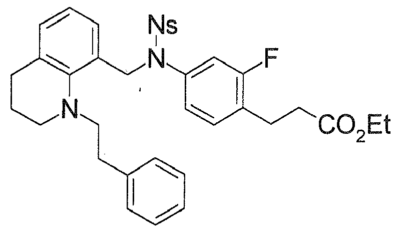 | FAB+:646 |
| 98 | E28 | 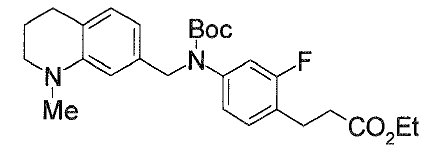 | ESI+:471 |
| 99 | E28 | 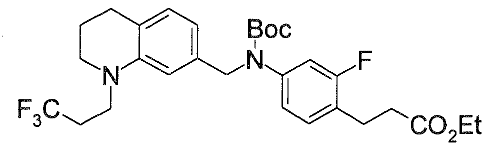 | ESI+:553 |
| 100 | E28 | 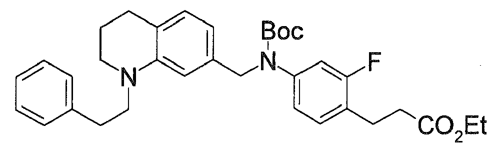 | ESI+:561 |
| 101 | E28 | 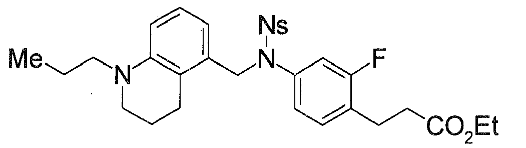 | FAB+:584 |
| 102 | E28 | 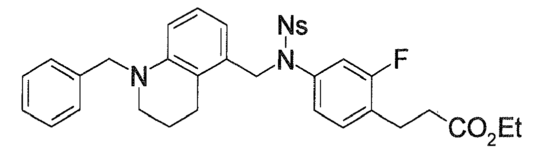 | FAB+:631 (M)+ |
| 103 | E28 | 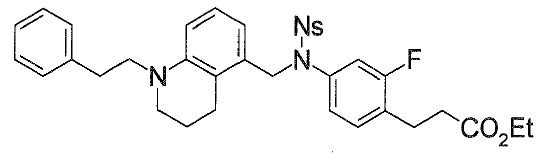 | FAB+:646 |

**[Table 15]**

| | | | |
|---|---|---|---|
| 104 | E28 | 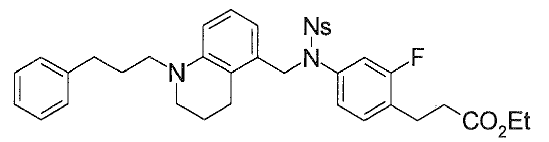 | ESI+:660 |
| 105 | E28 | 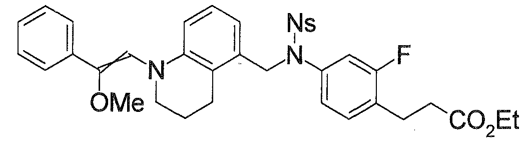 | ESI+:674 |
| 106 | E30 | 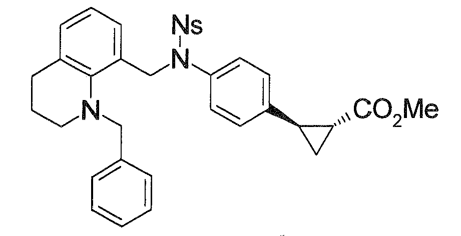 | ESI+:612 |
| 107 | E30 | 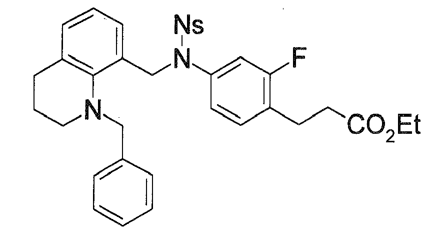 | FAB+:632 |
| 108 | E30 | 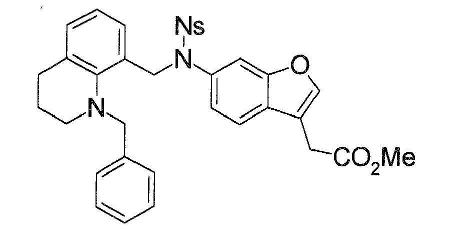 | ESI+:626 |
| 109 | E30 | 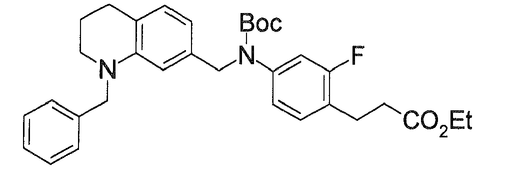 | ESI+:547 |

**[Table 16]**

| | | | |
|---|---|---|---|
| 110 | E30 | 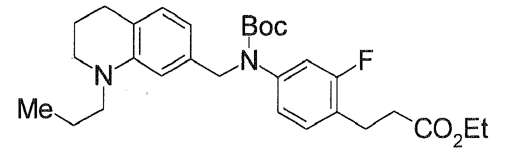 | ESI+:499 |
| 111 | E30 | 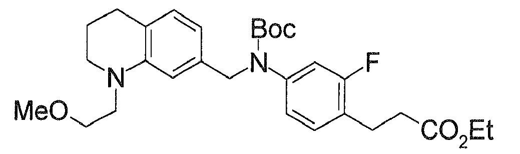 | ESI+:515 |
| 112 | E30 | 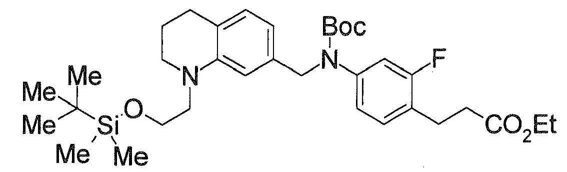 | ESI+:615 |
| 113 | E30 | 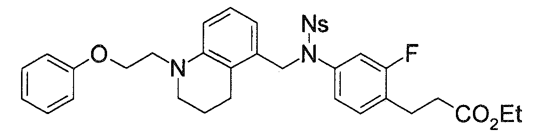 | FAB+:662 |
| 114 | E30 | 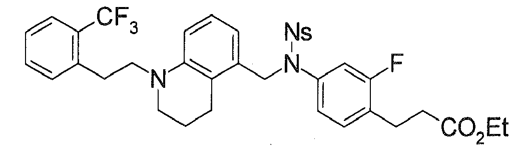 | FAB+:714 |
| 115 | E30 | 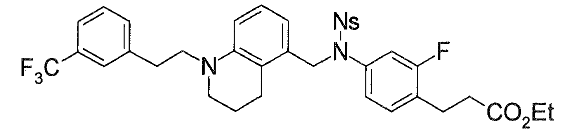 | FAB+:714 |
| 116 | E30 | 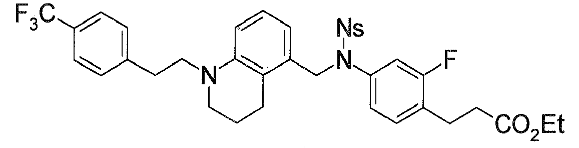 | FAB+:714 |
| 117 | E30 | 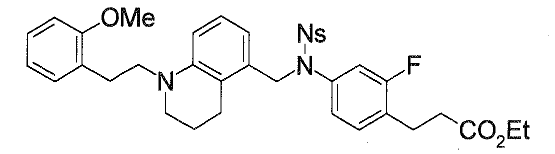 | ESI+:676 |

**[Table 17]**

| | | | |
|---|---|---|---|
| 118 | E30 | 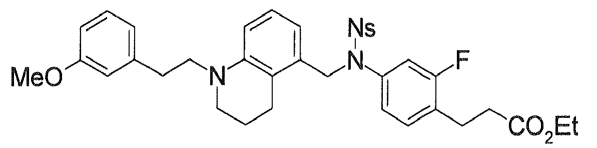 | ESI+:676 |
| 119 | E30 | 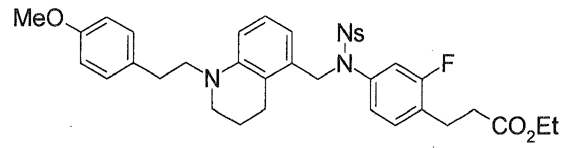 | ESI+:676 |
| 120 | E30 | 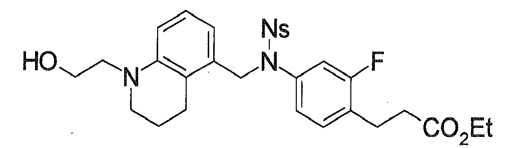 | FAB+:586 |
| 121 | E30 | 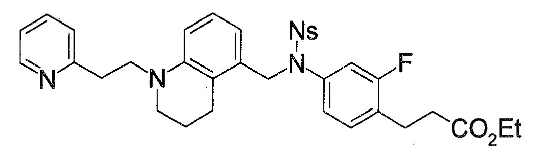 | ESI+:647 |
| 122 | E30 | 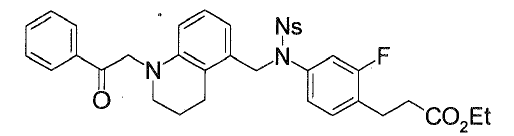 | FAB+:660 |
| 123 | E30 | 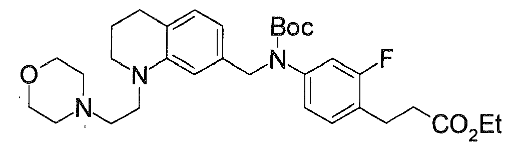 | ESI+:570 |
| 124 | E30 | 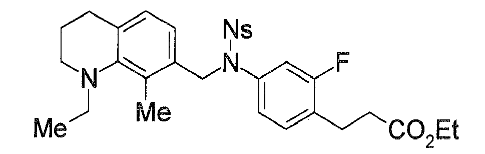 | ESI+:584 |
| 125 | E30 | 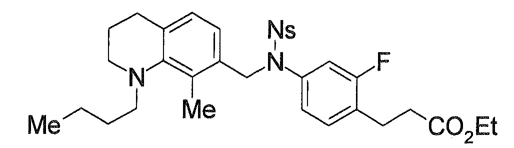 | ESI+:612 |

**[Table 18]**

| | | | |
|---|---|---|---|
| 126 | E30 | 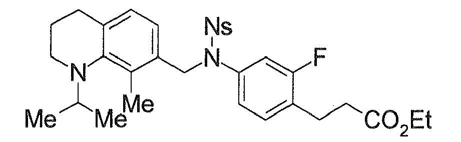 | ESI+:598 |
| 127 | E30 | 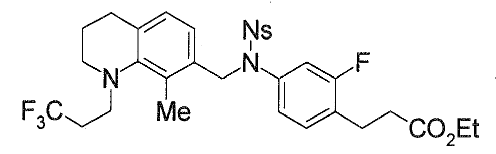 | ESI+:652 |
| 128 | E30 | 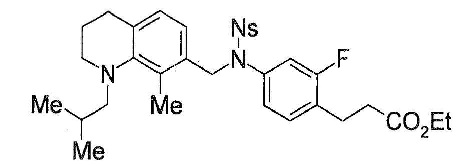 | ESI+:612 |
| 129 | E30 | 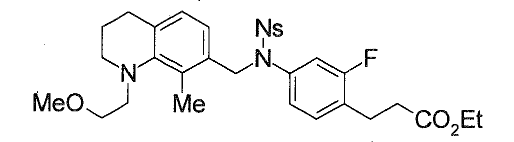 | ESI+:614 |
| 130 | E30 | 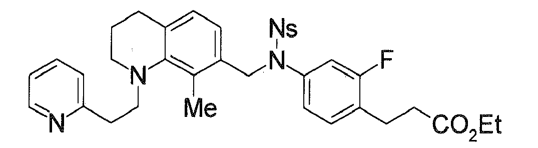 | ESI+:661 |
| 131 | E30 | 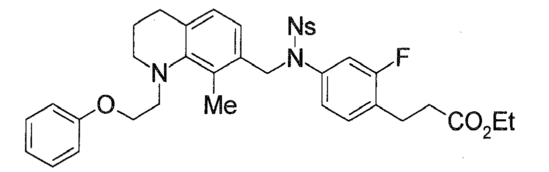 | FAB+:676 |
| 132 | E30 | 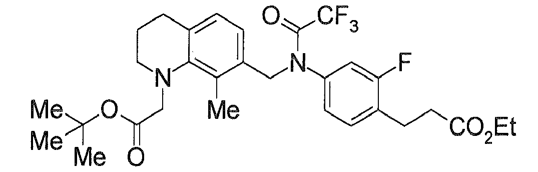 | ESI+:581 |
| 133 | E30 | 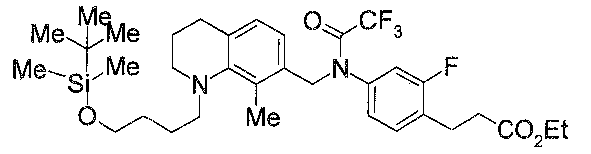 | ESI+:653 |

**[Table 19]**

| | | | |
|---|---|---|---|
| 134 | E30 | 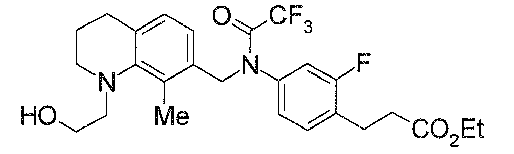 | FAB+:511 |
| 135 | E30 | 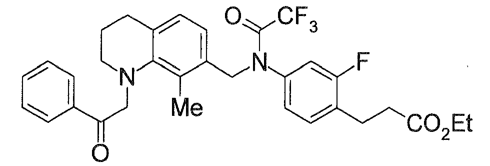 | ESI+:585 |
| 136 | E30 | 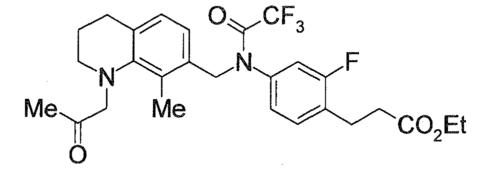 | ESI+:523 |
| 137 | E30 | 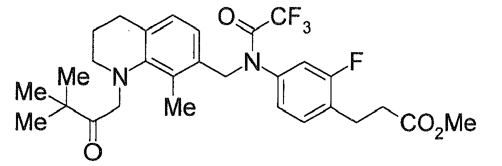 | ESI+:565 |
| 138 | E31 | 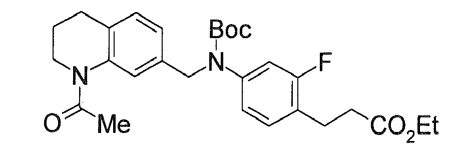 | ESI+:399 (M-Boc+H)+ |
| 139 | E31 | 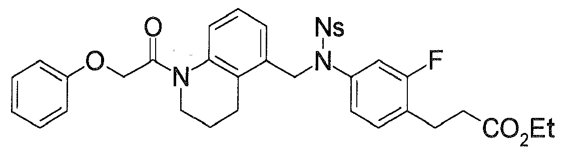 | ESI+:676 |
| 140 | E31 | 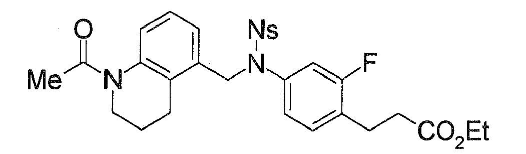 | FAB+:584 |
| 141 | E32 | 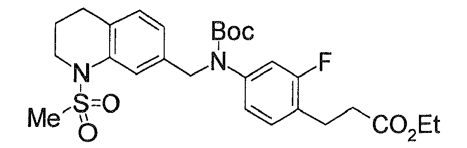 | FAB-:533 |

**[Table 20]**

| | | | |
|---|---|---|---|
| 142 | E34 | 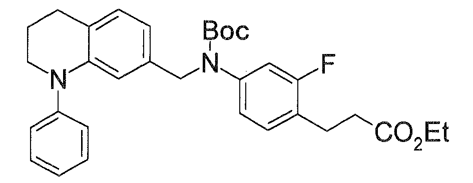 | ESI+:533 |
| 143 | E34 | 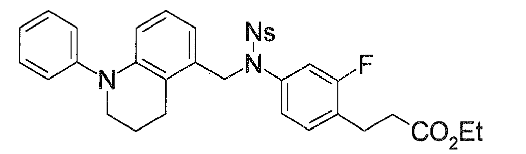 | ESI+:618 |
| 31 | 31 | 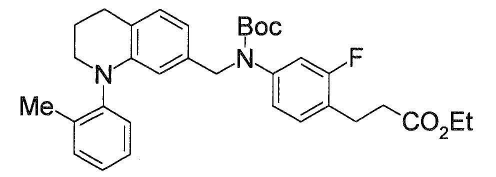 | ESI+:547 |
| 144 | 31 | 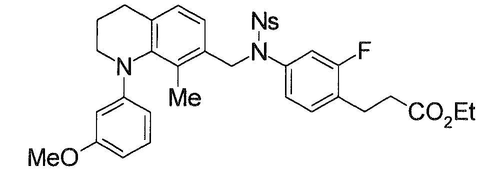 | FAB+:662 |
| 145 | 31 | 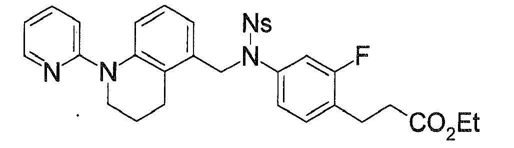 | ESI+:619 |
| 146 | 31 | 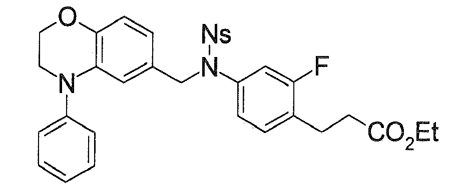 | ESI+:620 |
| 32 | 32 | 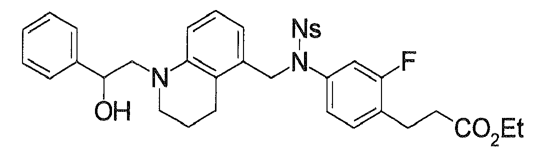 | FAB+:662 |

**[Table 21]**

| | | | |
|---|---|---|---|
| 33 | 33 | 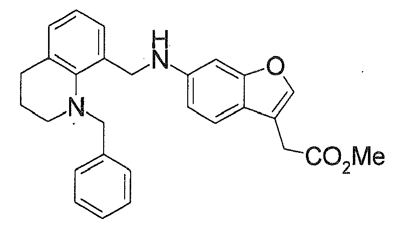 | ESI+:441 |
| 147 | 33 | 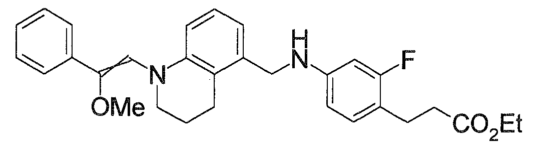 | ESI+:489 |
| 34 | 34 | 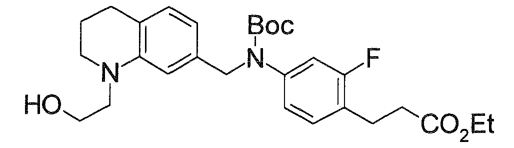 | ESI+:501 |
| 148 | 34 | 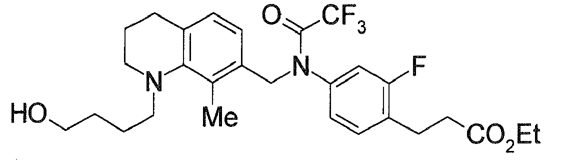 | ESI+:539 |
| 35 | 35 | 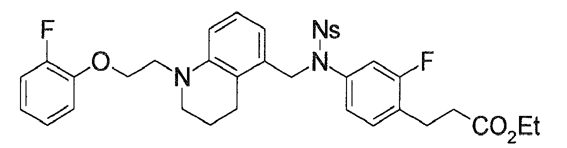 | ESI+:680 |
| 149 | 35 | 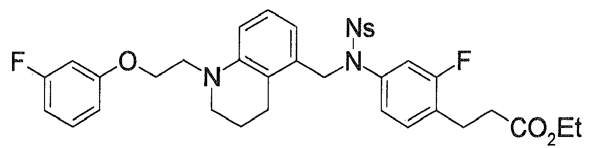 | ESI+:680 |
| 150 | 35 | 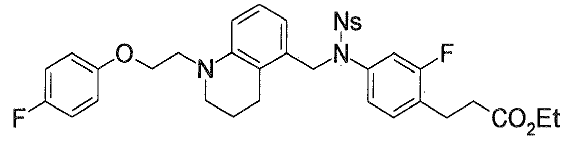 | ESI+:680 |
| 151 | 35 | 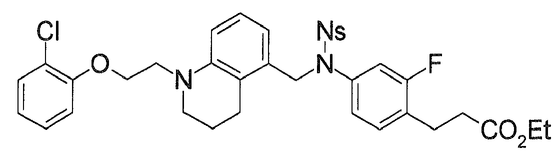 | ESI+:696 |

**[Table 22]**

| | | | |
|---|---|---|---|
| 152 | 35 | 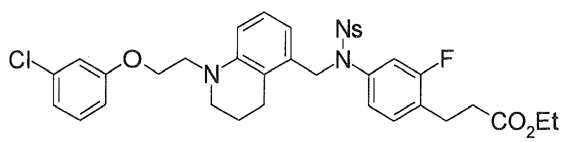 | ESI+:696 |
| 153 | 35 | 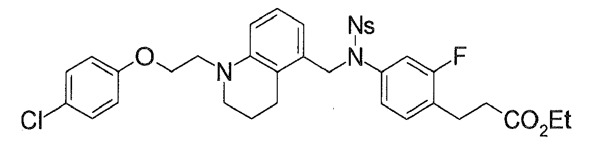 | ESI+:696 |
| 154 | 35 | 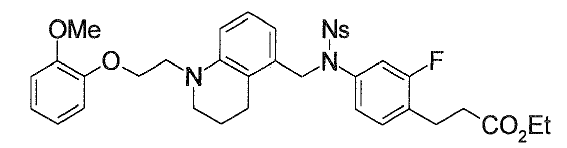 | FAB+:692 |
| 155 | 35 | 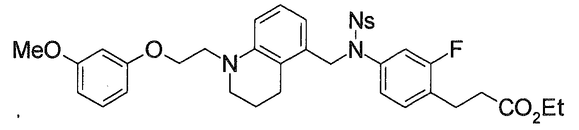 | FAB+:692 |
| 156 | 35 | 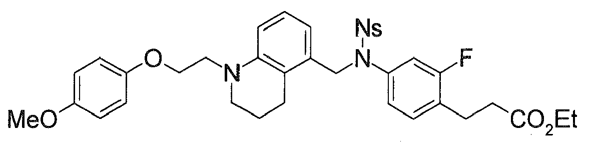 | FAB+:692 |
| 36 | 36 | 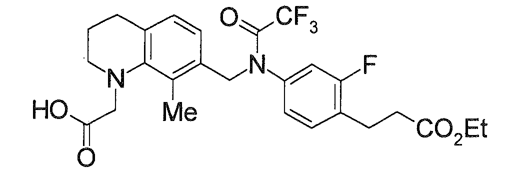 | ESI+:525 |
| 37 | 37 | 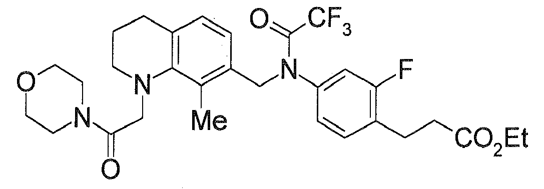 | ESI+:594 |
| 38 | 38 | 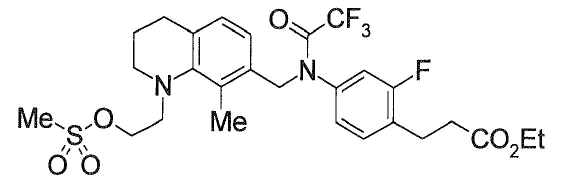 | ESI+:589 |

**[Table 23]**

| | | | |
|---|---|---|---|
| 39 | 39 | 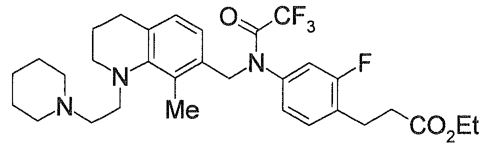 | ESI+:578 |
| 157 | 39 | 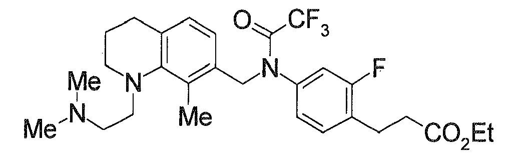 | ESI+:538 |
| 40 | 40 | 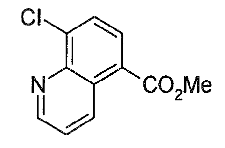 | EI:221 |
| 158 | 18 | 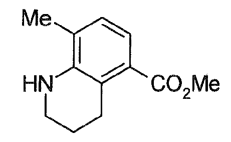 | ESI+:206 |
| 159 | 18 | 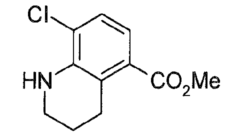 | ESI+:226 |
| 160 | 24 | 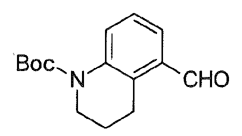 | EI:261 |
| 41 | 41 | 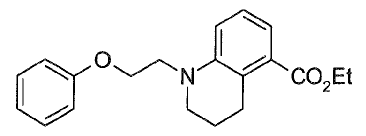 | ESI+:326 |
| 161 | 23 | 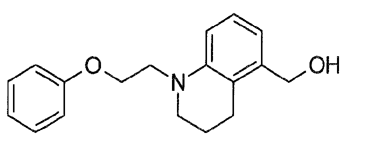 | EI:283 |

**[Table 24]**

| | | | |
|---|---|---|---|
| 42 | 42 | 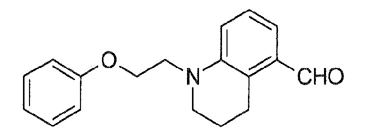 | EI:281 |
| 43 | 43 | 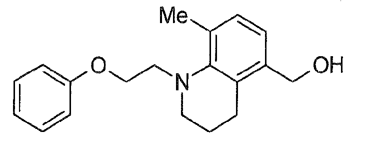 | ESI+:298 |
| 162 | 43 | 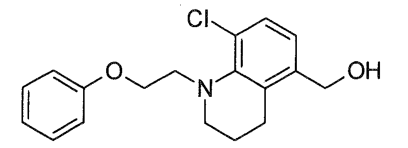 | ESI+:318 |
| 163 | 41 | 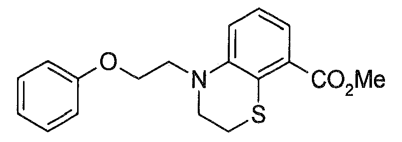 | ESI+:298 [(M-OMe)+] |
| 164 | 23 | 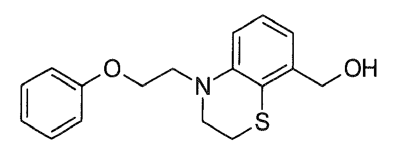 | ESI+:284 [(M-OH)+] |
| 165 | 15 | 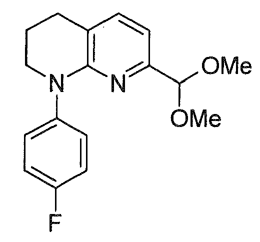 | CI:302 |
| 166 | 15 | 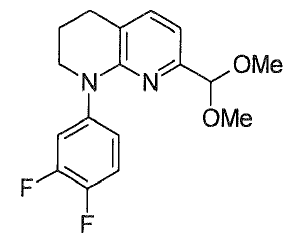 | EI:320 |

**[Table 25]**

| | | | |
|---|---|---|---|
| 167 | 15 | 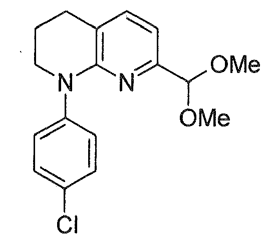 | EI:318 |
| 168 | 15 | 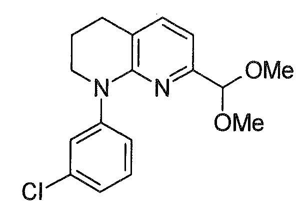 | CI:318 |
| 169 | 15 | 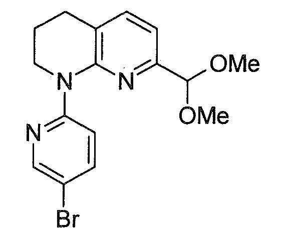 | CI:364,366 |
| 170 | 15 | 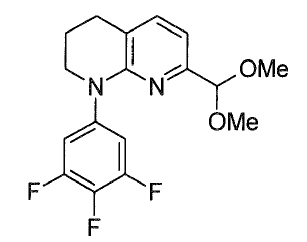 | EI:338 |

**[Table 26]**

| | | | |
|---|---|---|---|
| 171 | 21 | 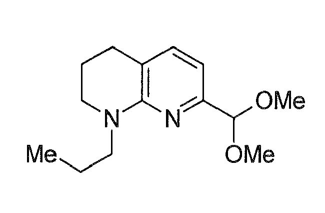 | NMR2:0.90(3H,t,J =7.4Hz),1.60(2H,t q,J=7.3,7.4Hz),1.9 0(2H,tt,J=5.6,6.4H z),2.70(2H,t,J=6.4 Hz),3.37(2H,t,J=5. 6Hz),3.40(6H,s),3. 57(2H,t,J=7.3Hz),5 .12(1H,s),6.61(1H, d,J=7.3Hz),7.08(1 H,d,J=7.3Hz) |
| 44 | 44 | 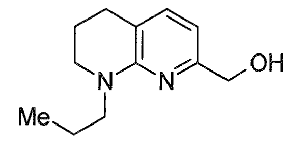 | ESI+:207 |
| 172 | 18 | 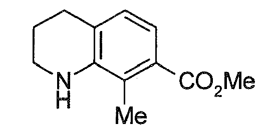 | ESI+:206 |
| 173 | 23 | 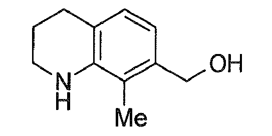 | ESI+:178 |
| 174 | 9 | 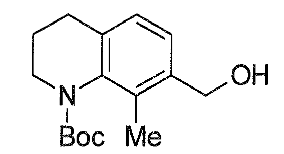 | FAB+:278 |
| 45 | 45 | 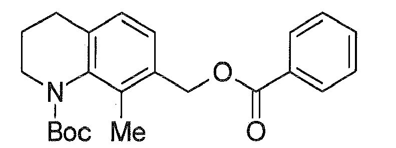 | FAB+:382 |

**[Table 27]**

| | | | |
|---|---|---|---|
| 175 | 28 | 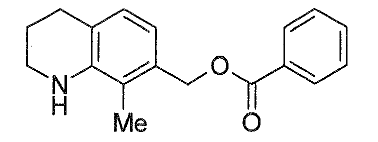 | ESI+:282 |
| 46 | 46 | 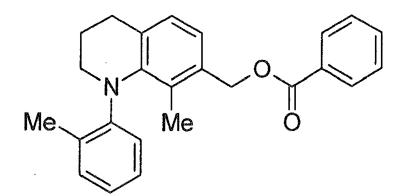 | ESI+:372 |
| 47 | 47 | 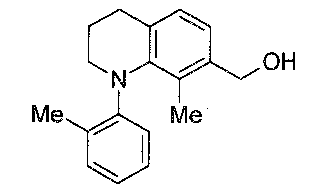 | ESI+:268 |
| 176 | 46 | 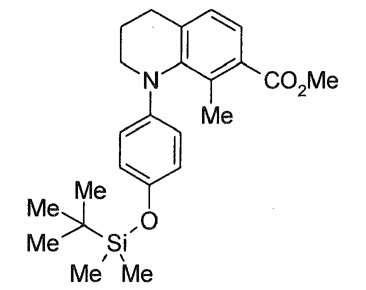 | ESI+:412 |
| 177 | 46 | 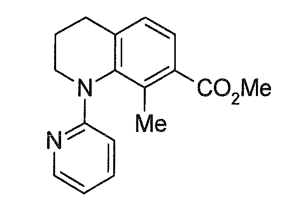 | ESI+:283 |
| 178 | 41 | 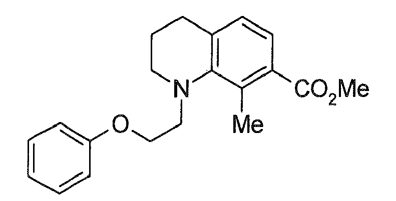 | ESI+:326 |

**[Table 28]**

| | | | |
|---|---|---|---|
| 179 | 34 | 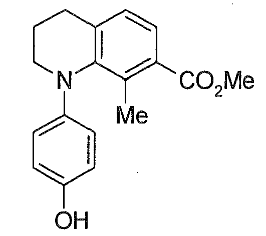 | ESI+:298 |
| 48 | 48 | 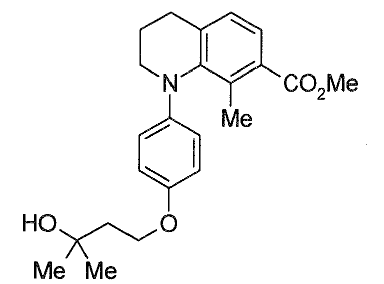 | ESI+:384 |
| 180 | 23 | 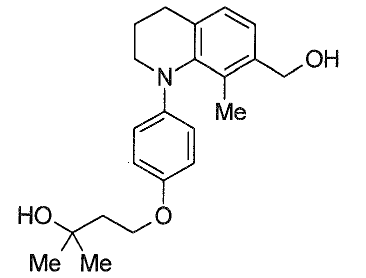 | ESI+:356 |
| 181 | 24 | 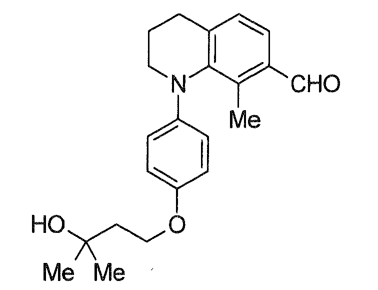 | ESI+:354 |

**[Table 29]**

| | | | |
|---|---|---|---|
| 182 | 23 | 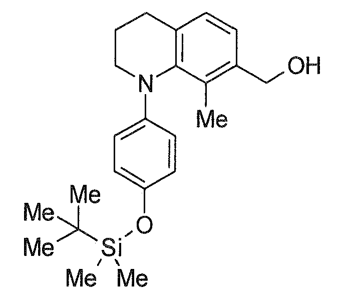 | FAB+:383 [(M)+] |
| 183 | 23 | 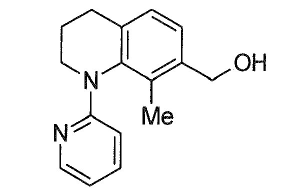 | ESI+:255 |
| 184 | 23 | 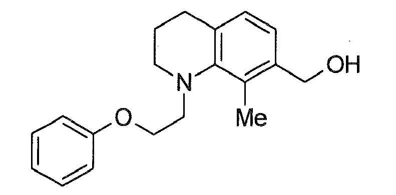 | ESI+:298 |
| 185 | E20 | 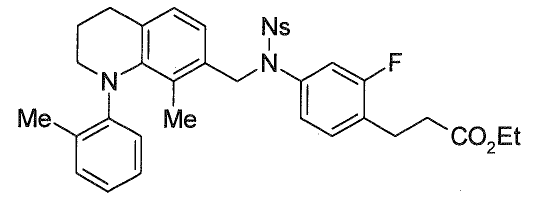 | ESI+:646 |
| 186 | E20 | 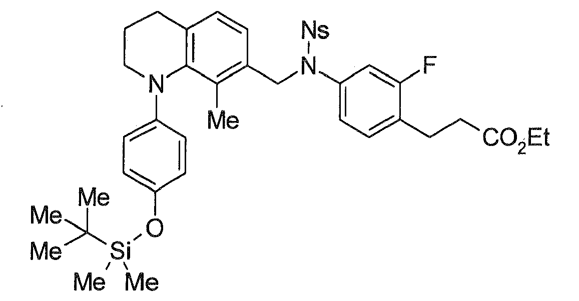 | ESI+:762 |

**[Table 30]**

| | | | |
|---|---|---|---|
| 187 | 33 | 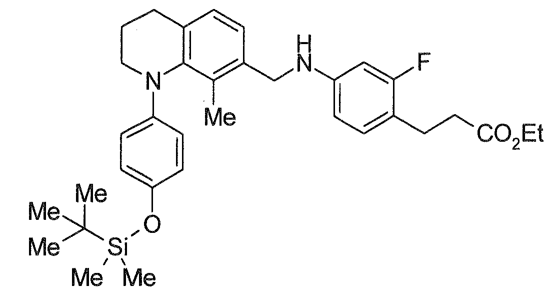 | ESI+:577 |
| 188 | E20 | 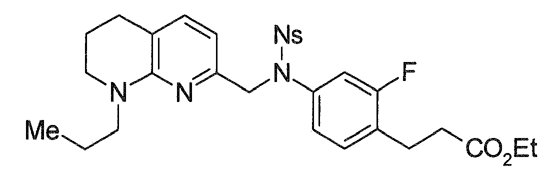 | ESI+:585 |
| 189 | E20 | 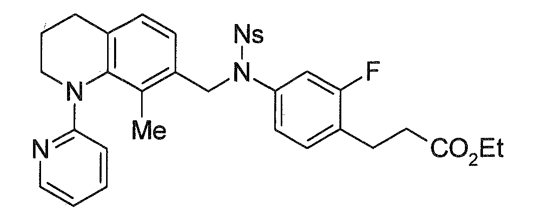 | ESI+:633 |
| 190 | 30 | 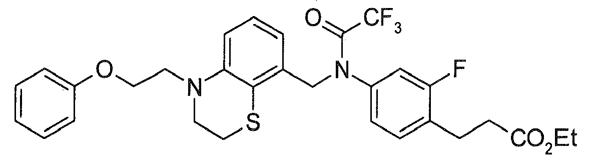 | ESI+:591 |
| 49 | 49 | 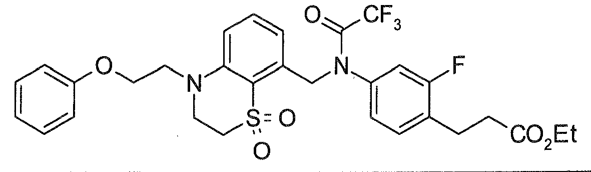 | ESI+:623 |
| 191 | 41 | 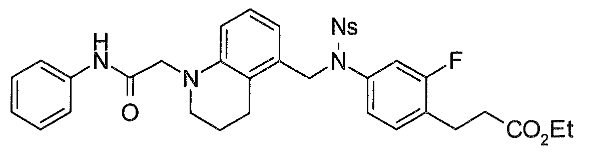 | ESI+:675 |

**[Table 31]**

| | | | |
|---|---|---|---|
| 192 | E21 | 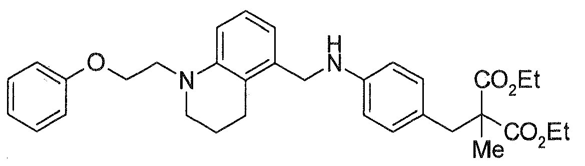 | ESI+:545 |
| 193 | 30 | 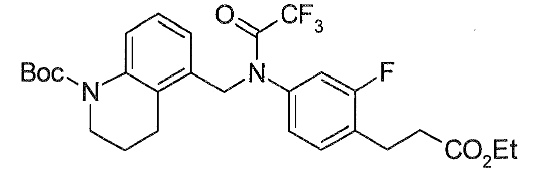 | FAB+:553 |
| 194 | 36 | 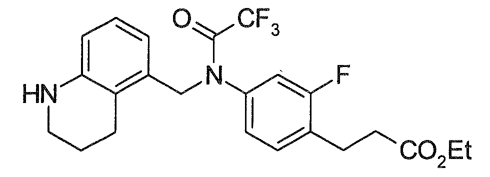 | FAB+:453 |
| 195 | 41 | 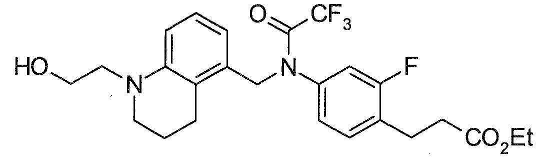 | ESI+:497 |
| 196 | 35 | 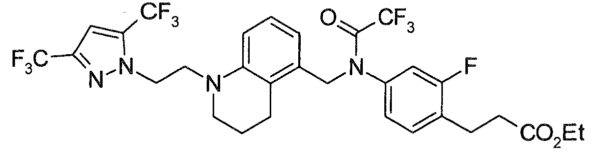 | ESI+:683 |
| 197 | 35 | 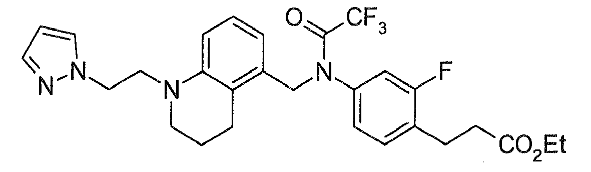 | ESI+:547 |

**[Table 32]**

| Ex | Structure |
|---|---|
| 41 | 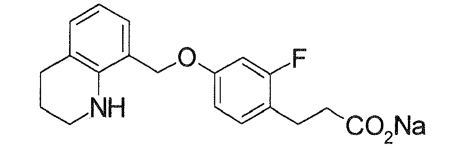 |
| 14 | 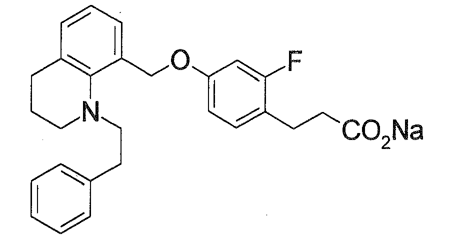 |
| 42 | 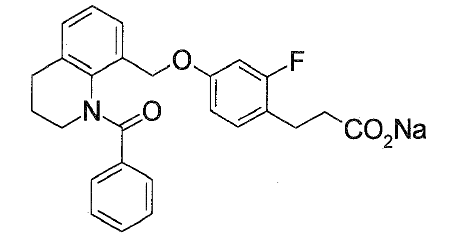 |
| 43 | 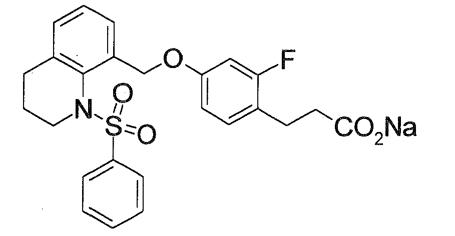 |
| 44 | 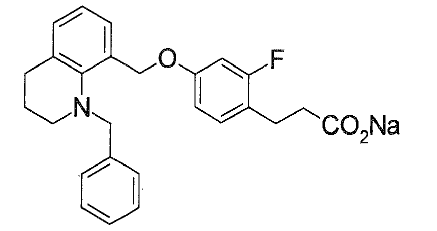 |

**[Table 33]**

| | |
|---|---|
| 45 | 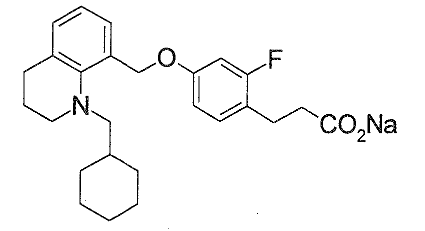 |
| 46 | 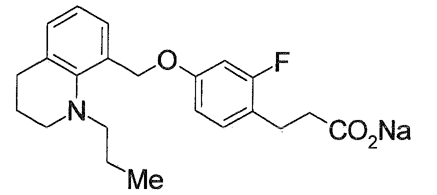 |
| 47 | 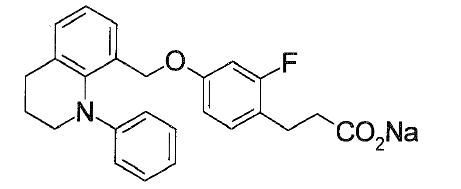 |
| 48 | 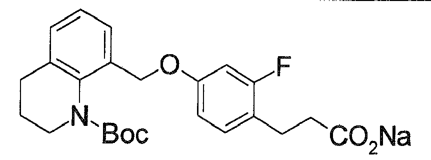 |
| 1 | 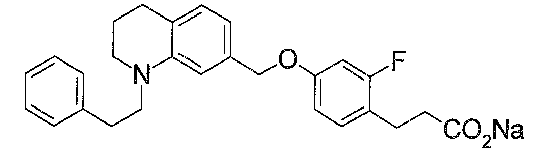 |
| 49 | 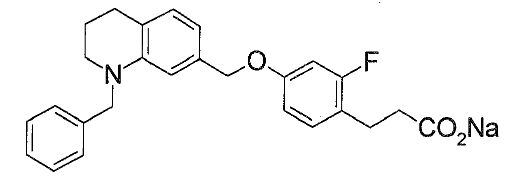 |

**[Table 34]**

| | |
|---|---|
| 10 | 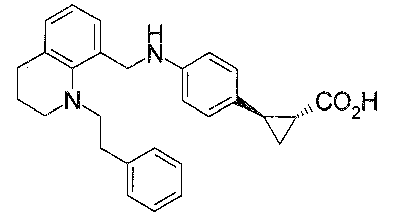 |
| 50 | 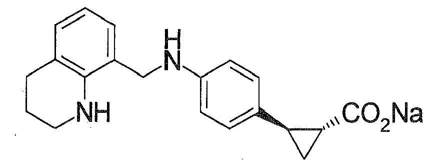 |
| 51 | 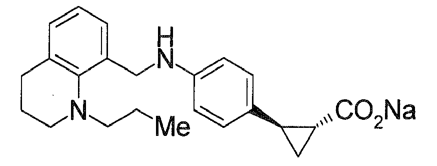 |
| 52 | 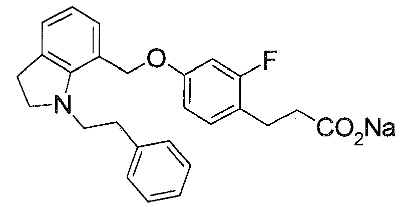 |
| 53 | 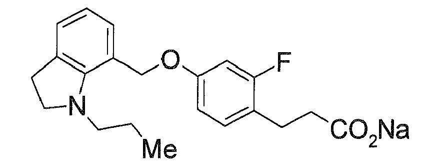 |
| 54 | 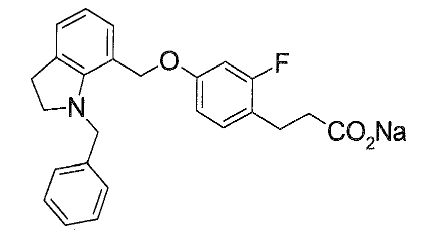 |

**[Table 35]**

| | |
|---|---|
| 55 | 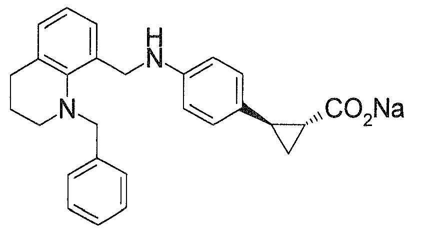 |
| 56 | 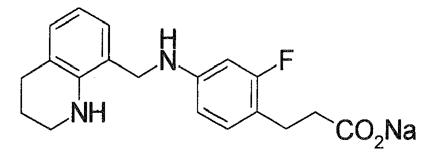 |
| 3 | 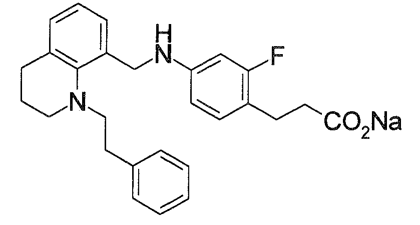 |
| 57 | 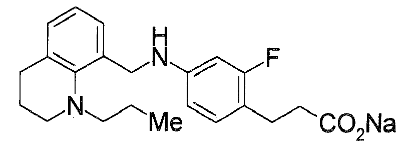 |
| 58 | 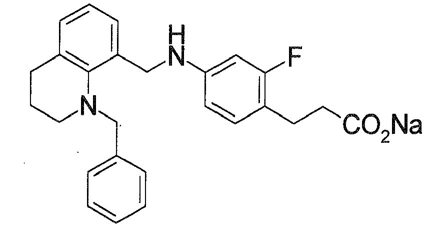 |
| 59 | 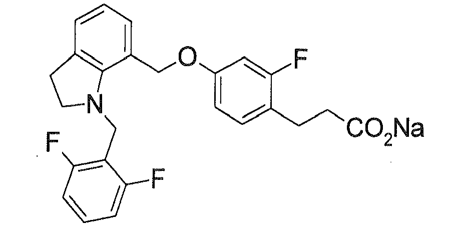 |

**[Table 36]**

| | |
|---|---|
| 60 | 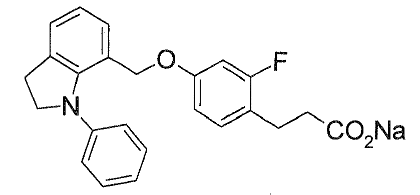 |
| 61 | 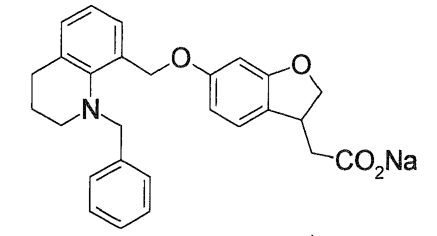 |
| 62 | 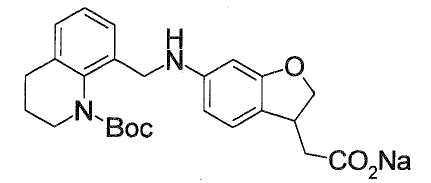 |
| 63 | 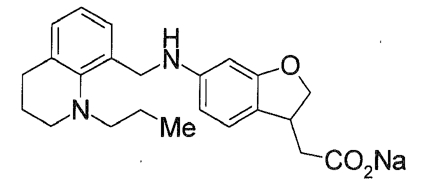 |
| 7 | 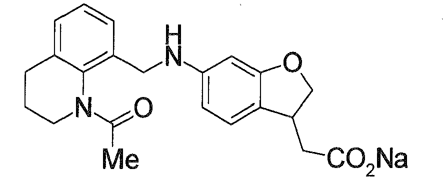 |
| 13 | 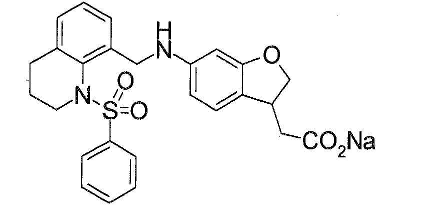 |

**[Table 37]**

| | |
|---|---|
| 12 | 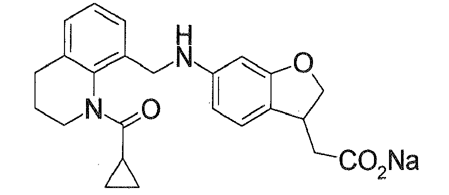 |
| 64 | 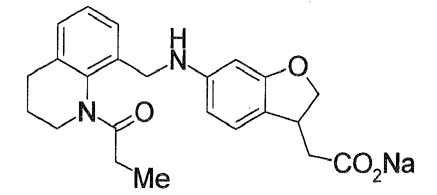 |
| 65 | 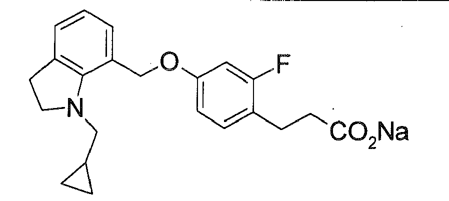 |
| 66 | 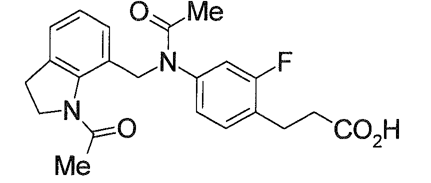 |
| 67 | 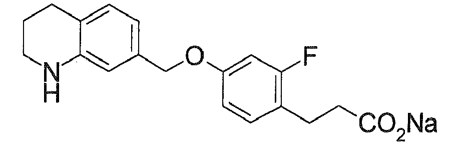 |
| 68 | 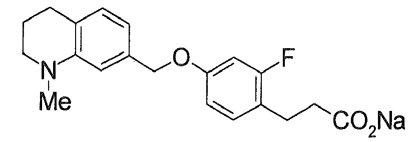 |
| 69 | 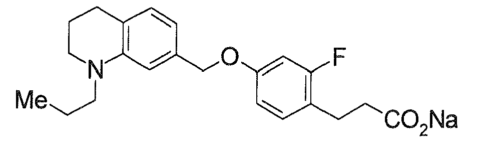 |

**[Table 38]**

| | |
|---|---|
| 70 | 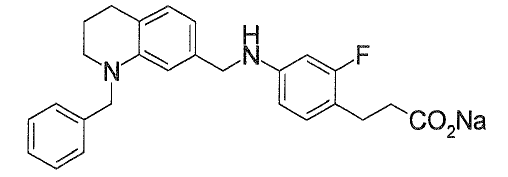 |
| 71 | 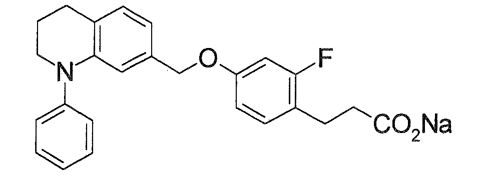 |
| 72 | 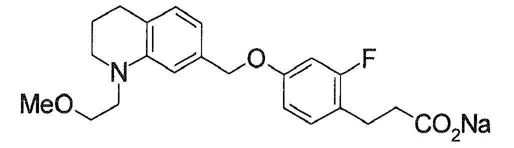 |
| 73 | 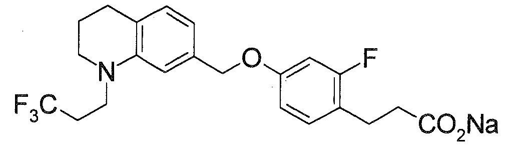 |
| 74 | 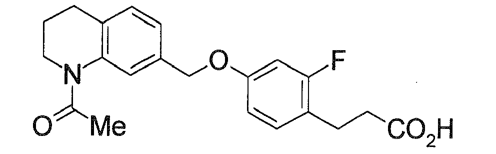 |
| 75 | 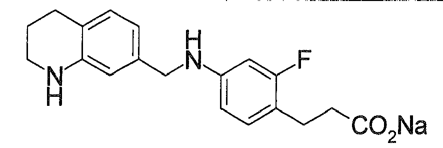 |
| 76 | 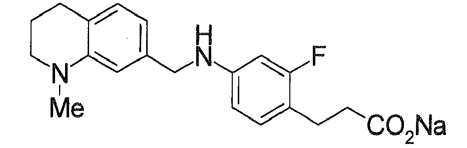 |
| 77 | 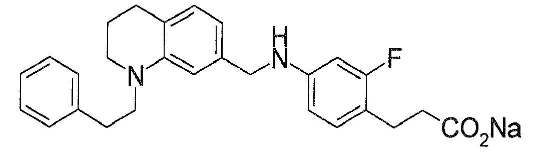 |

**[Table 39]**

| | |
|---|---|
| 78 | 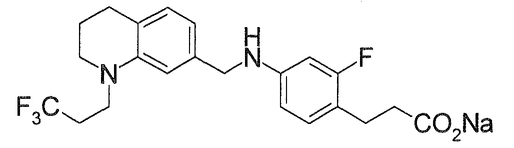 |
| 79 | 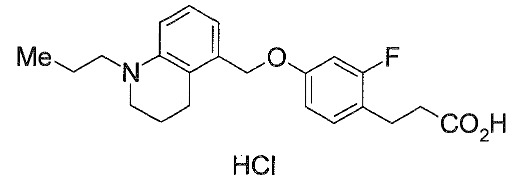 |
| 80 | 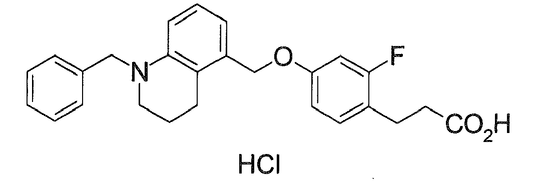 |
| 81 | 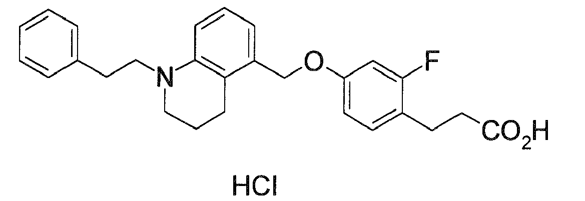 |
| 82 | 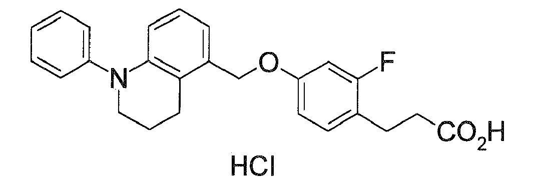 |
| 83 | 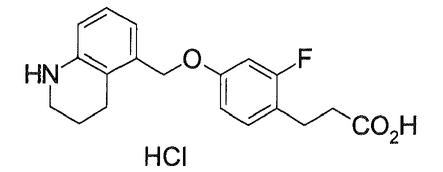 |
| 84 | 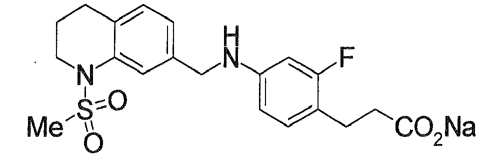 |

**[Table 40]**

| | |
|---|---|
| 85 | 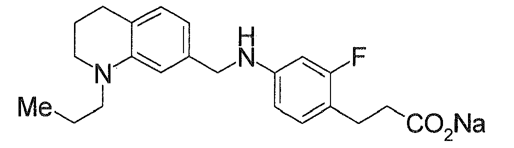 |
| 86 | 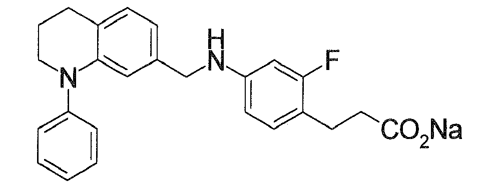 |
| 87 | 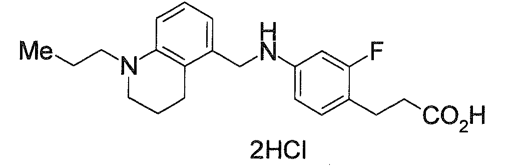 |
| 88 | 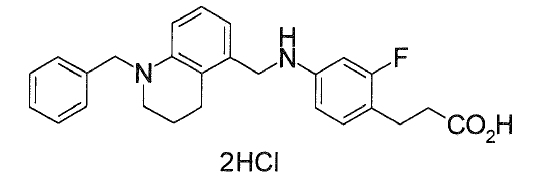 |
| 89 | 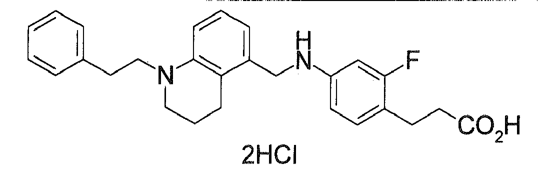 |
| 90 | 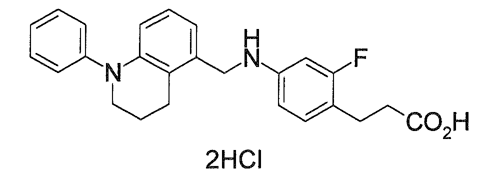 |
| 91 | 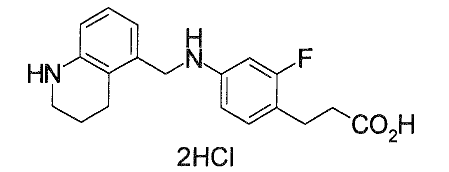 |

**[Table 41]**

| | |
|---|---|
| 92 | 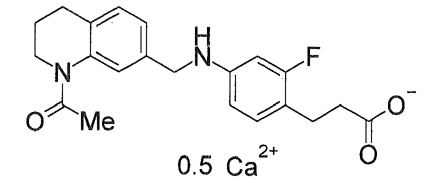 |
| 93 | 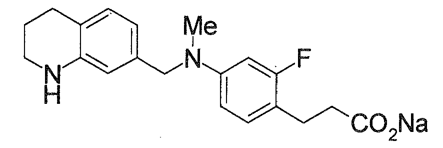 |
| 94 | 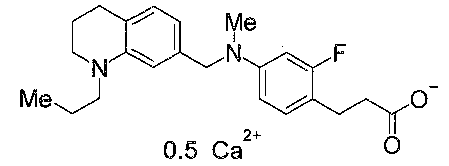 |
| 95 | 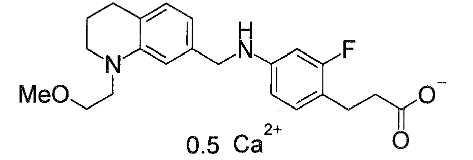 |
| 96 | 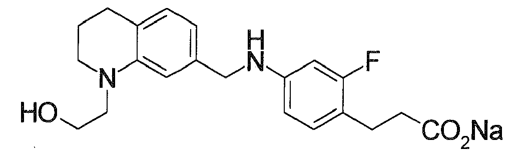 |
| 97 | 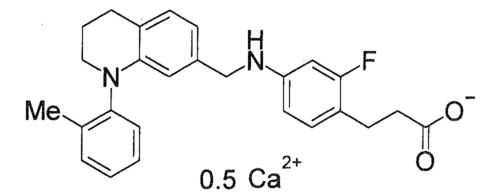 |
| 98 | 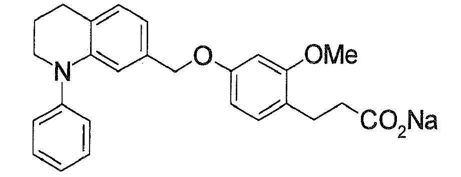 |

**[Table 42]**

| | |
|---|---|
| 99 | 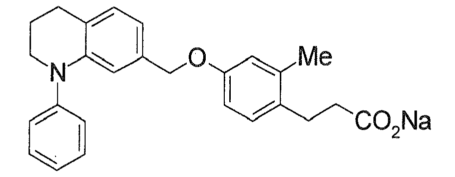 |
| 100 | 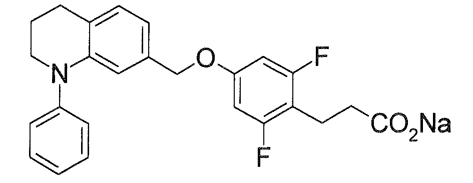 |
| 101 | 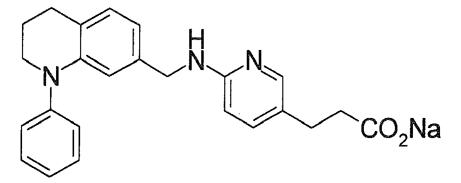 |
| 102 | 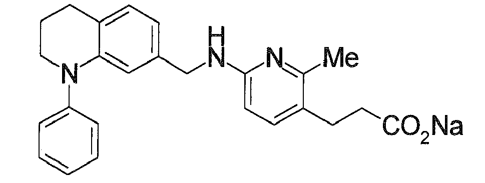 |
| 103 | 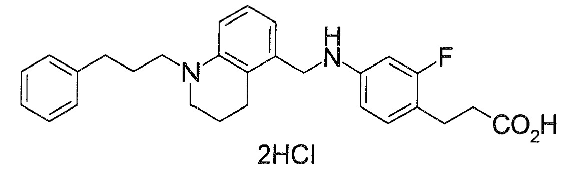 |
| 104 | 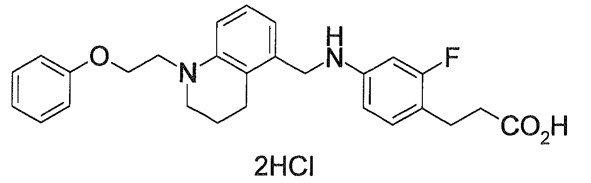 |
| 105 | 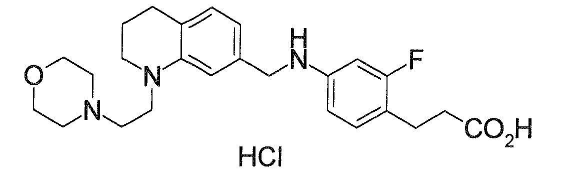 |

**[Table 43]**

| | |
|---|---|
| 106 | 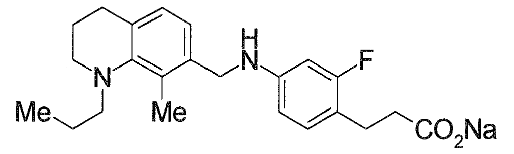 |
| 107 | 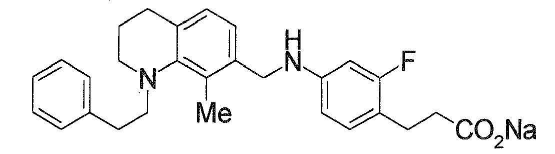 |
| 108 | 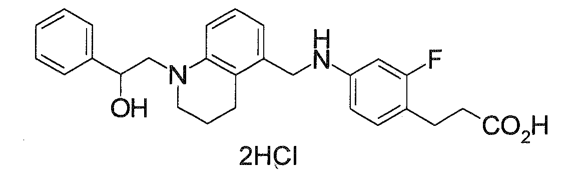 |
| 109 | 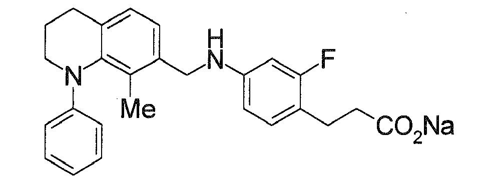 |
| 110 | 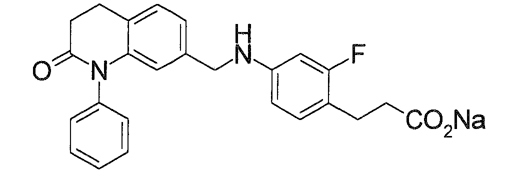 |
| 111 | 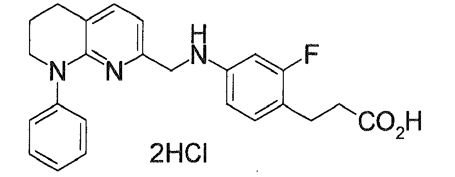 |
| 112 | 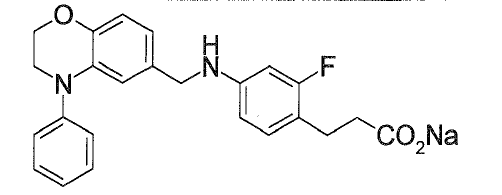 |

**[Table 44]**

| | |
|---|---|
| 113 | 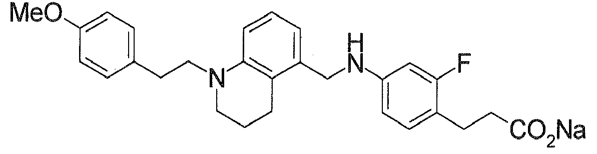 |
| 114 | 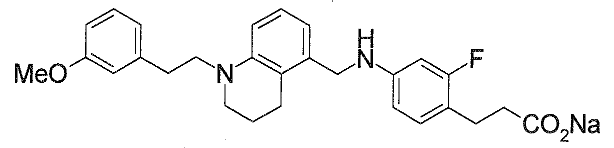 |
| 115 | 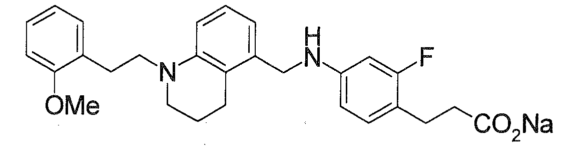 |
| 116 | 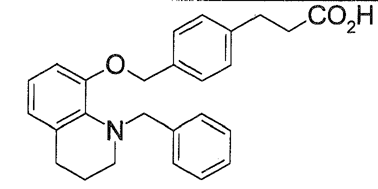 |
| 117 | 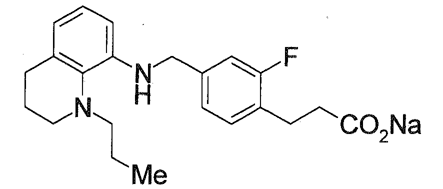 |
| 11 | 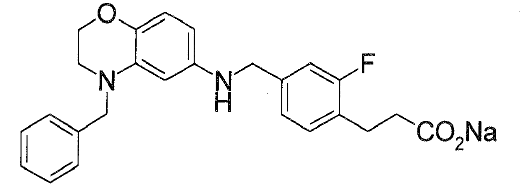 |
| 118 | 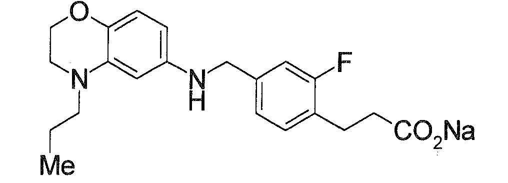 |

**[Table 45]**

| | |
|---|---|
| 119 | 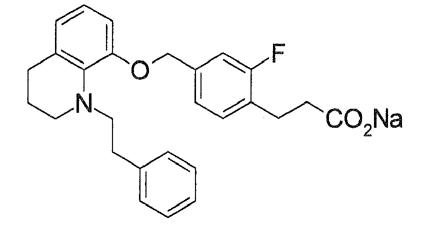 |
| 120 | 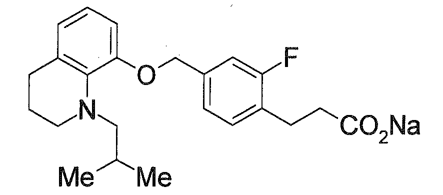 |
| 121 | 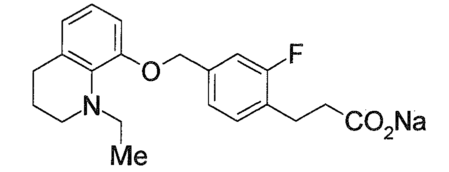 |
| 122 | 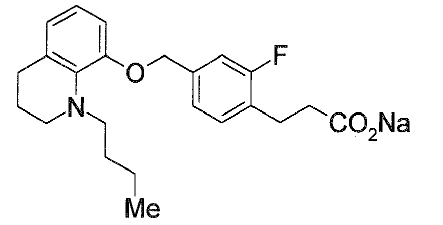 |
| 123 | 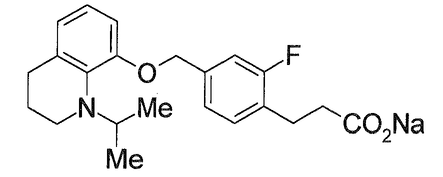 |
| 124 | 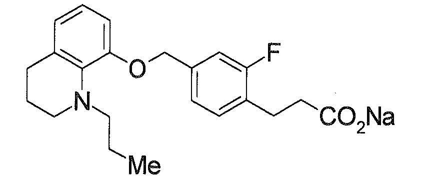 |

**[Table 46]**

| | |
|---|---|
| 125 | 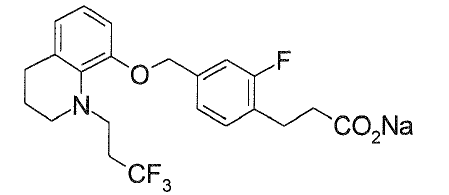 |
| 126 | 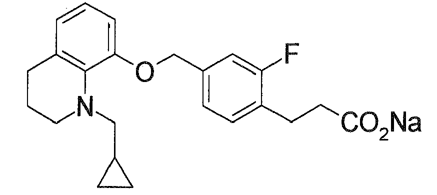 |
| 127 | 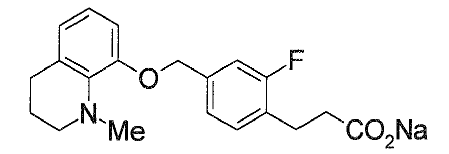 |
| 128 | 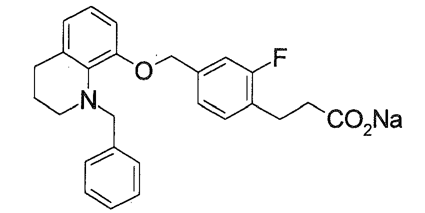 |
| 129 | 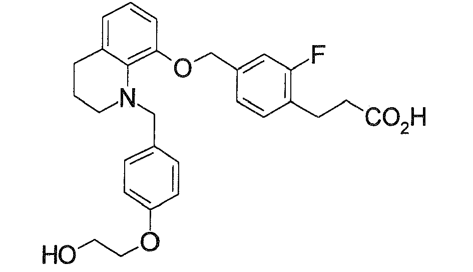 |
| 130 | 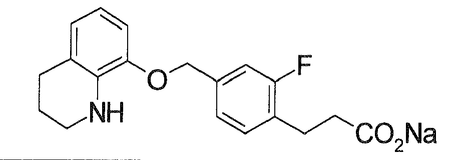 |

**[Table 47]**

| | |
|---|---|
| 131 | 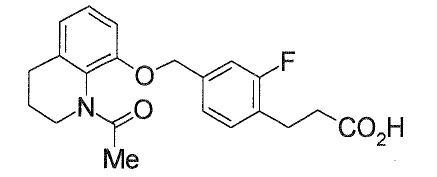 |
| 132 | 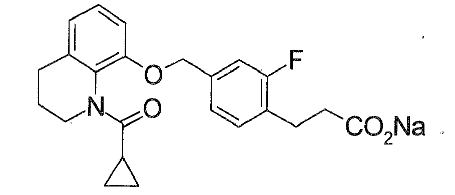 |
| 133 | 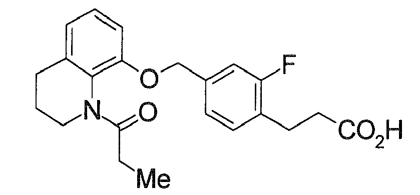 |
| 134 | 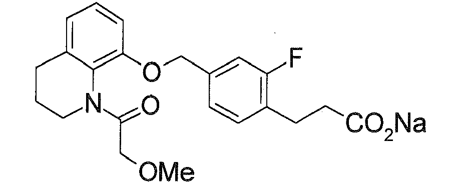 |
| 135 | 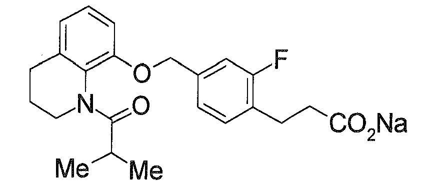 |
| 136 | 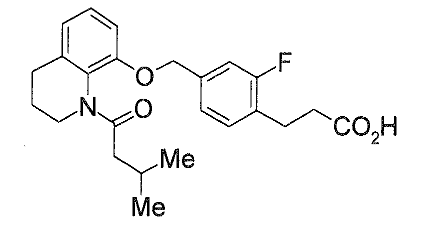 |

**[Table 48]**

| | |
|---|---|
| 137 | 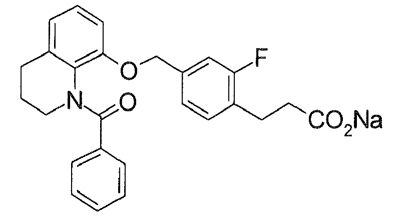 |
| 138 | 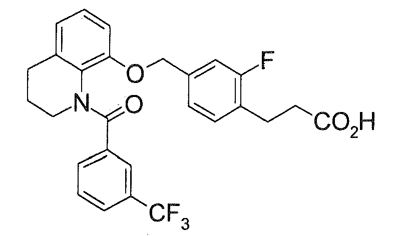 |
| 139 | 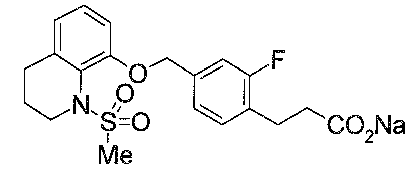 |
| 140 | 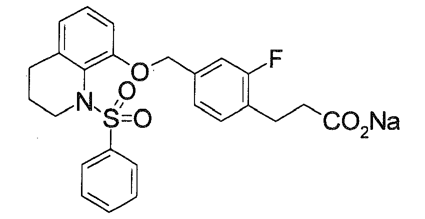 |
| 141 | 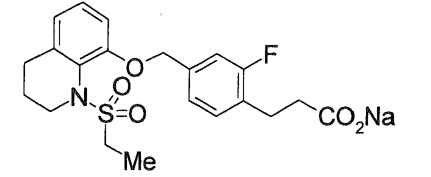 |
| 142 | 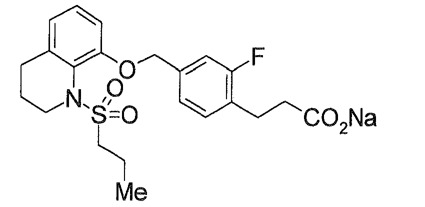 |

**[Table 49]**

| | |
|---|---|
| 143 | 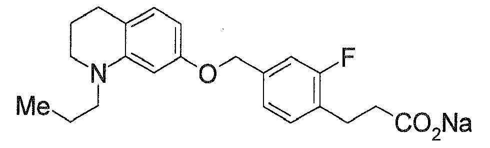 |
| 144 | 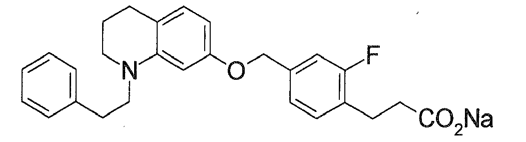 |
| 145 | 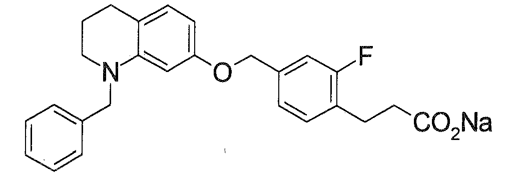 |
| 146 | 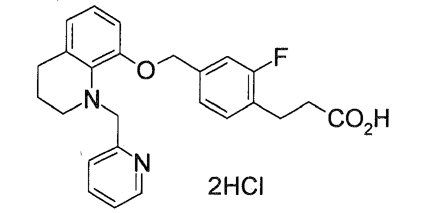 |
| 147 | 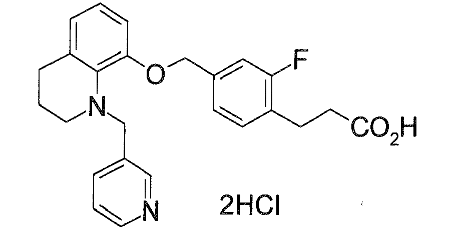 |
| 148 | 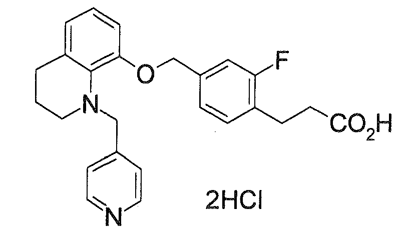 |

**[Table 50]**

| | |
|---|---|
| 149 | 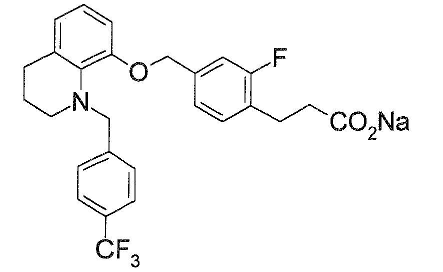 |
| 150 | 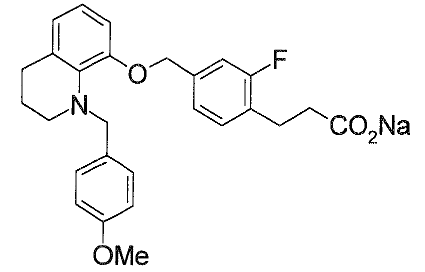 |
| 151 | 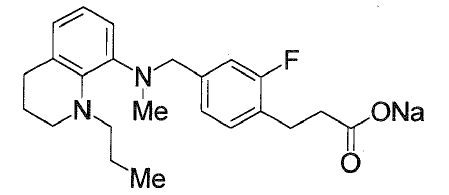 |
| 152 | 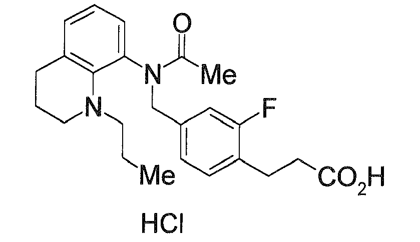 |
| 153 | 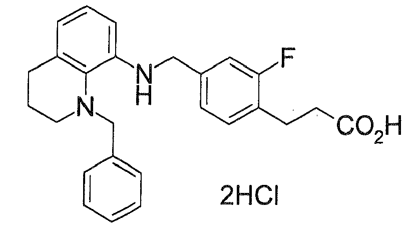 |

**[Table 51]**

| | |
|---|---|
| 154 | 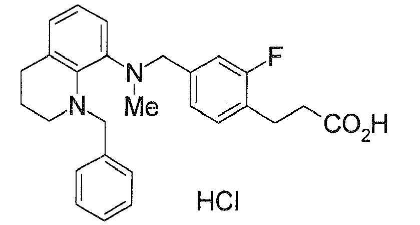 |
| 155 | 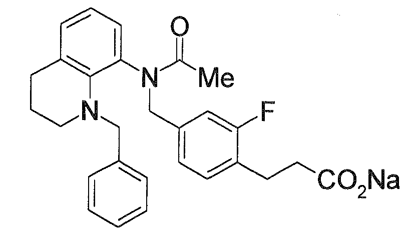 |
| 156 | 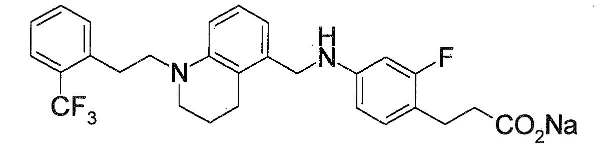 |
| 157 | 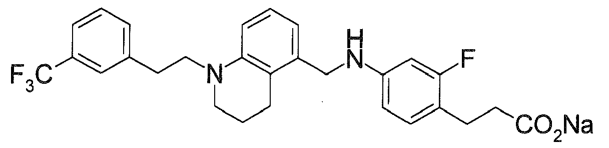 |
| 158 | 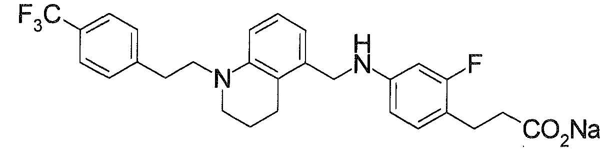 |
| 159 | 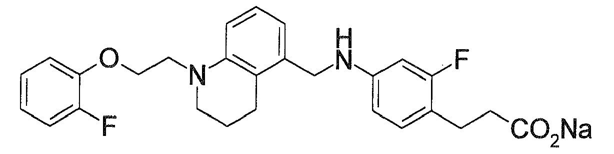 |
| 160 | 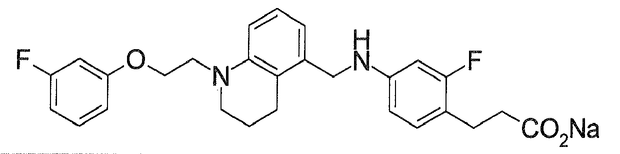 |

**[Table 52]**

| | |
|---|---|
| 161 | 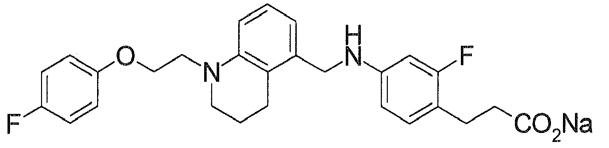 |
| 162 | 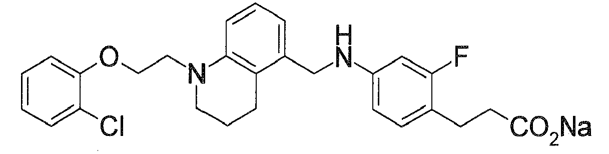 |
| 163 | 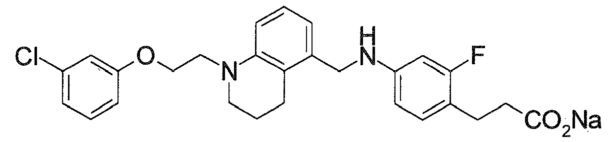 |
| 164 | 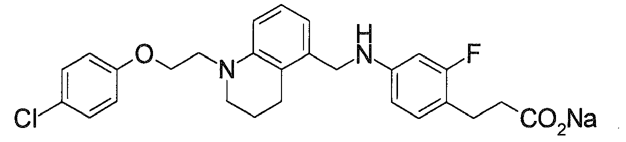 |
| 165 | 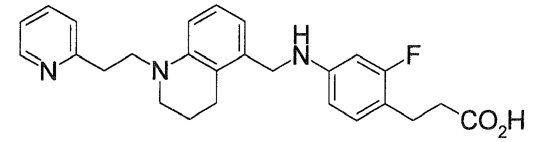 |
| 166 | 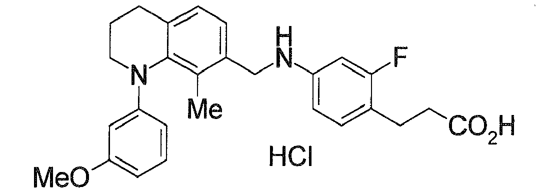 |
| 167 | 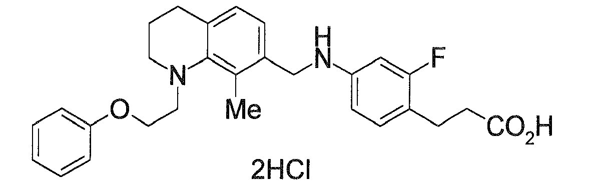 |
| 168 | 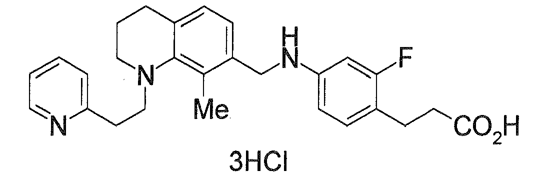 |

**[Table 53]**

| | |
|---|---|
| 169 | 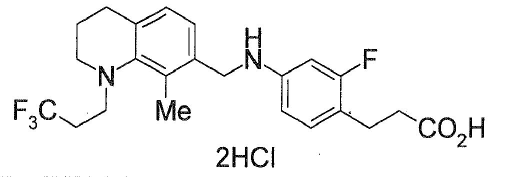 |
| 170 | 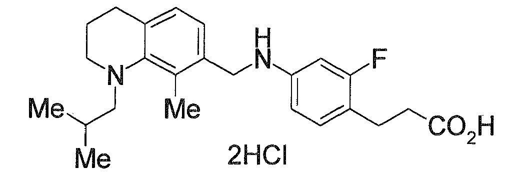 |
| 171 | 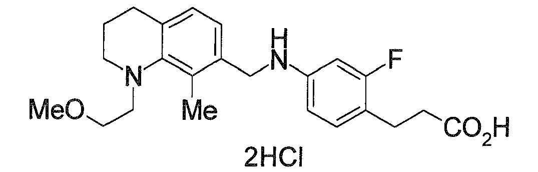 |
| 172 | 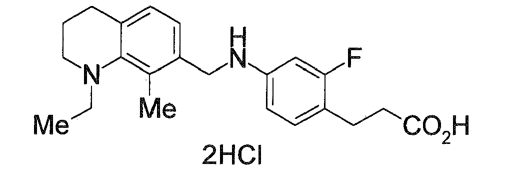 |
| 173 | 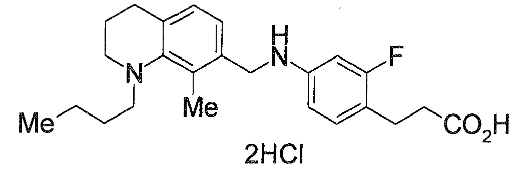 |
| 174 | 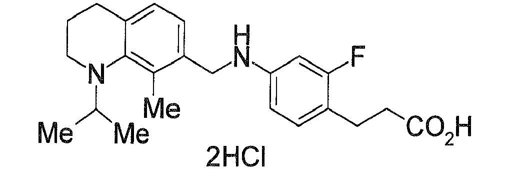 |
| 175 | 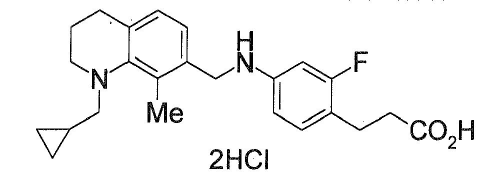 |

**[Table 54]**

| | |
|---|---|
| 176 | 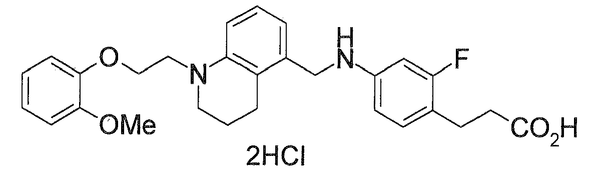 |
| 177 | 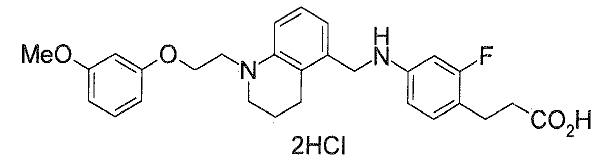 |
| 178 | 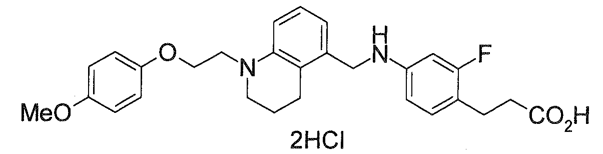 |
| 179 | 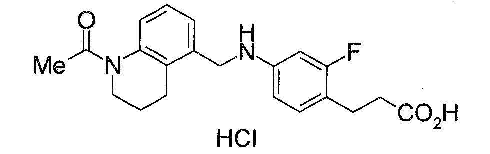 |
| 180 | 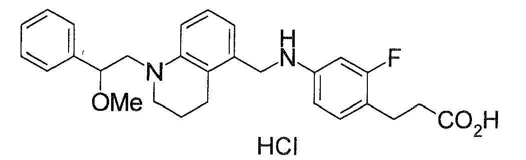 |
| 181 | 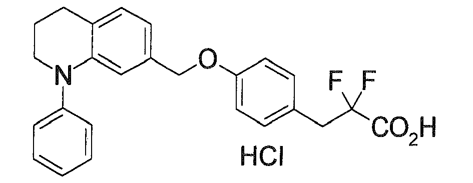 |
| 182 | 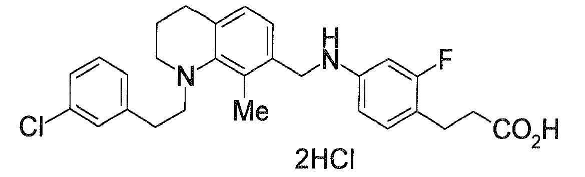 |

**[Table 55]**

| | |
|---|---|
| 183 | 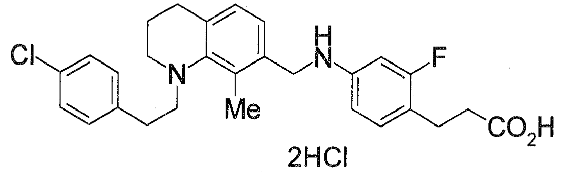 |
| 184 | 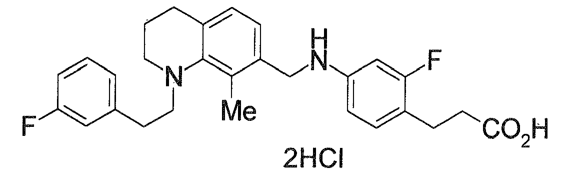 |
| 185 | 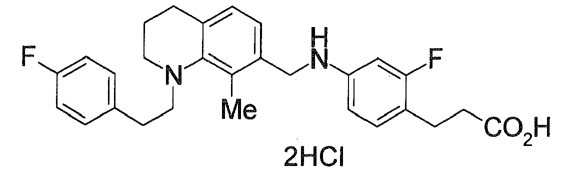 |
| 186 | 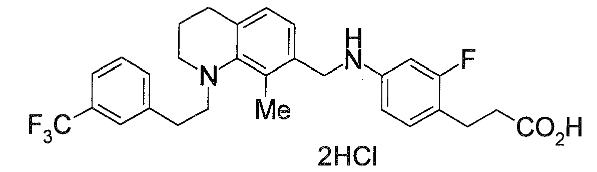 |
| 187 | 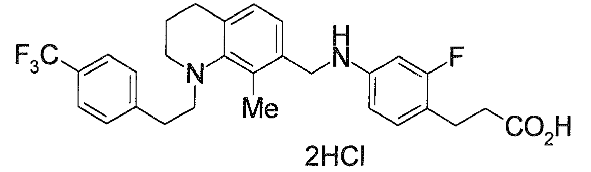 |
| 188 | 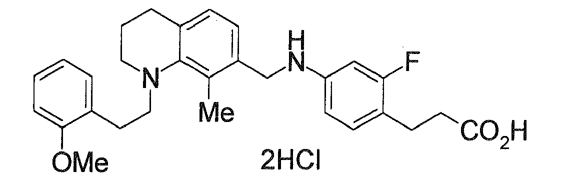 |
| 189 | 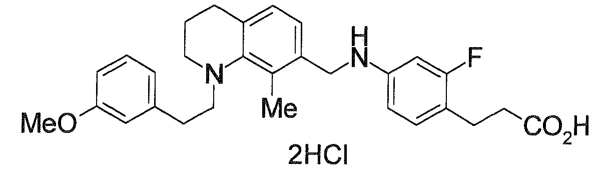 |

**[Table 56]**

| | |
|---|---|
| 190 | 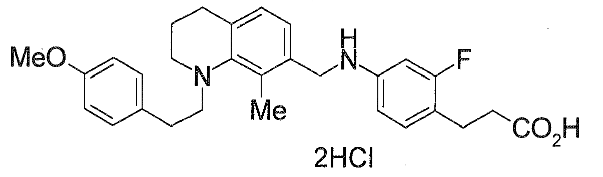 |
| 5 | 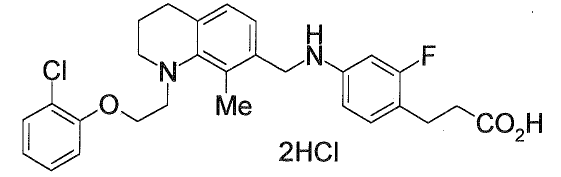 |
| 191 | 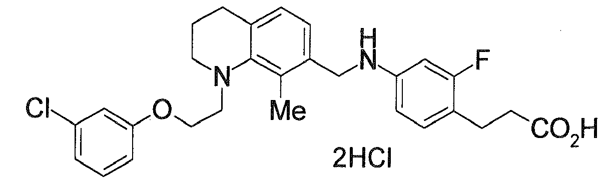 |
| 192 | 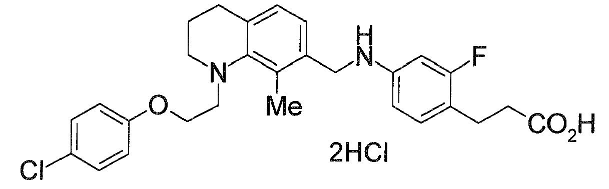 |
| 193 | 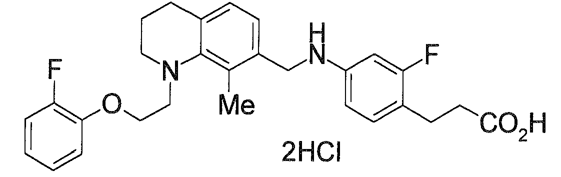 |
| 194 | 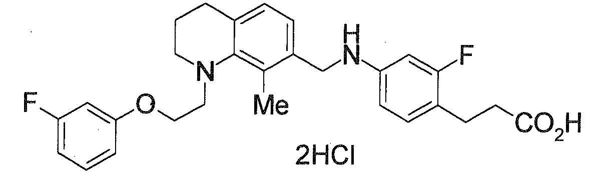 |
| 195 | 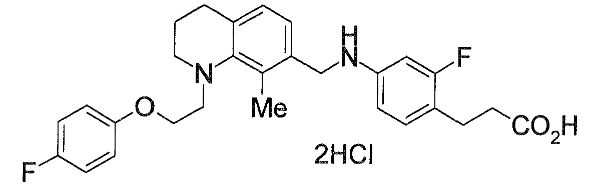 |

**[Table 57]**

| | |
|---|---|
| 196 | 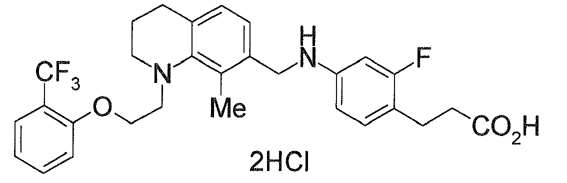 |
| 197 | 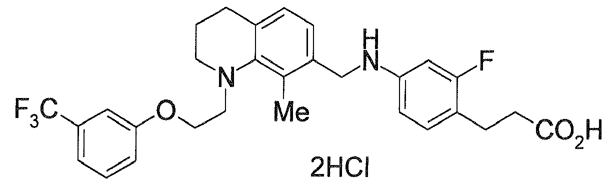 |
| 198 | 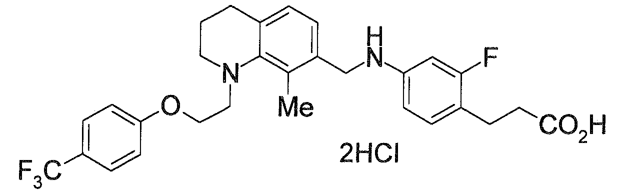 |
| 199 | 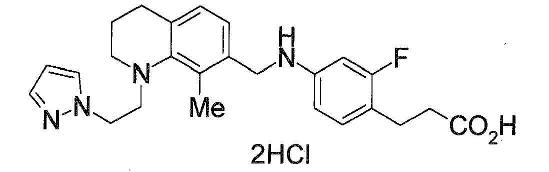 |
| 200 | 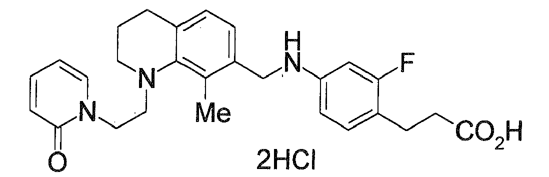 |
| 201 | 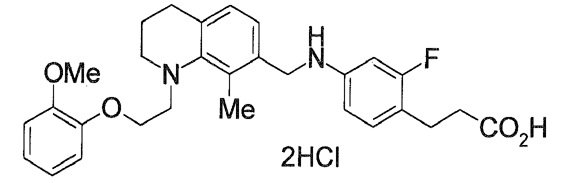 |
| 202 | 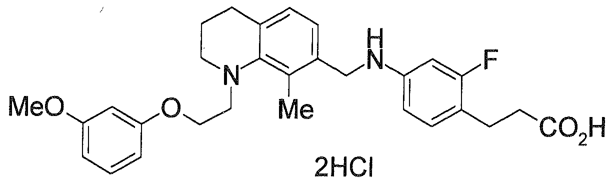 |

**[Table 58]**

| | |
|---|---|
| 203 | 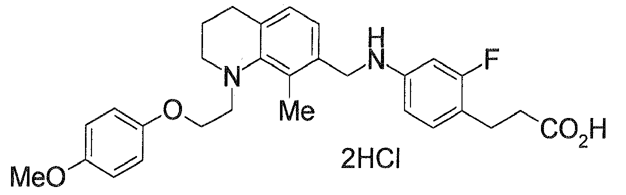 |
| 204 | 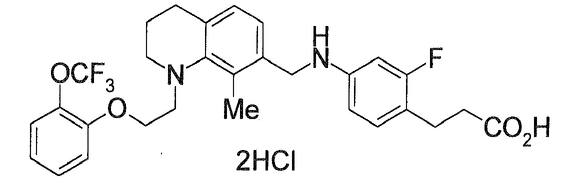 |
| 205 | 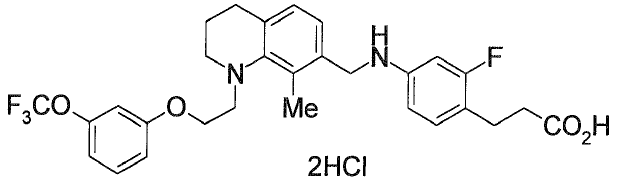 |
| 206 | 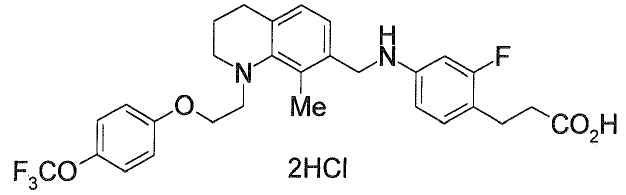 |
| 207 | 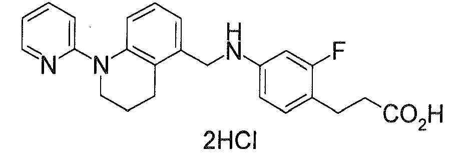 |
| 208 | 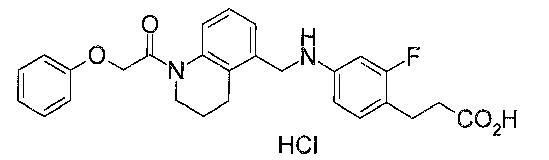 |
| 6 | 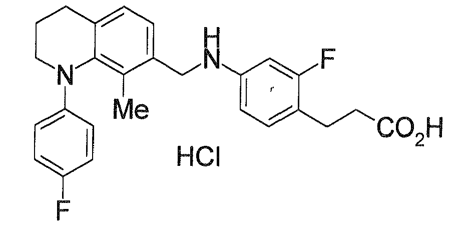 |

**[Table 59]**

| | |
|---|---|
| 209 | 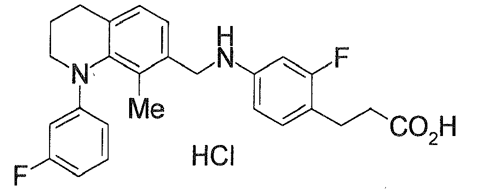 |
| 210 | 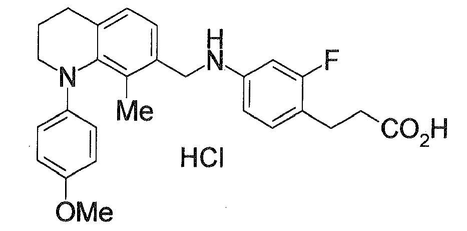 |
| 211 | 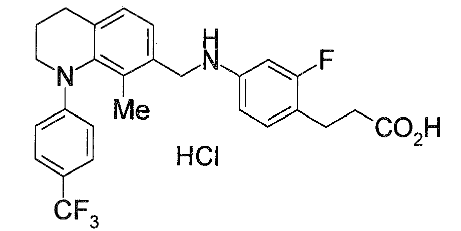 |
| 212 | 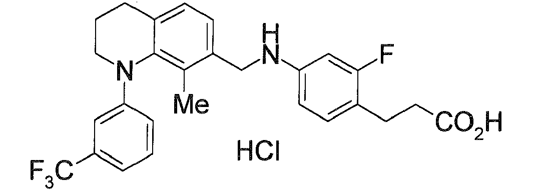 |
| 213 | 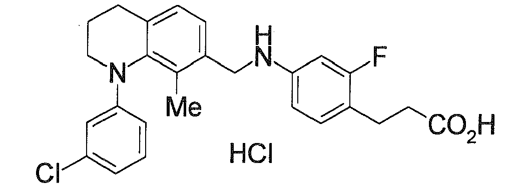 |
| 214 | 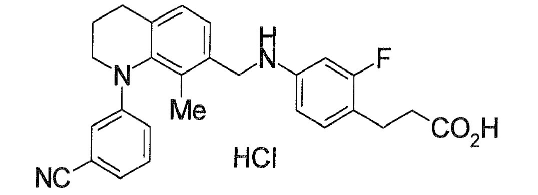 |

**[Table 60]**

| | |
|---|---|
| 215 | 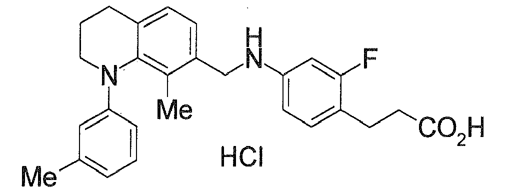 |
| 216 | 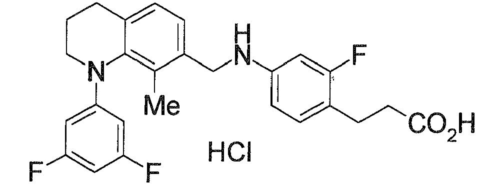 |
| 217 | 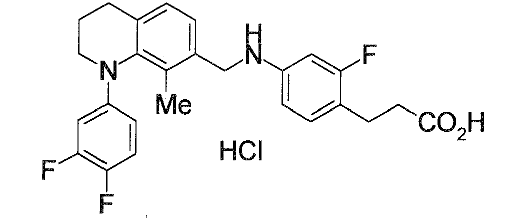 |
| 218 | 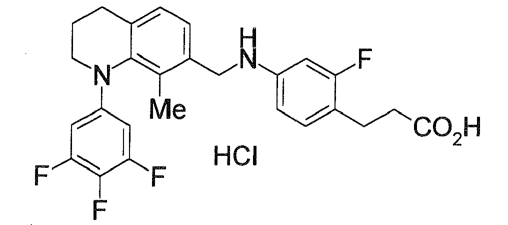 |
| 219 | 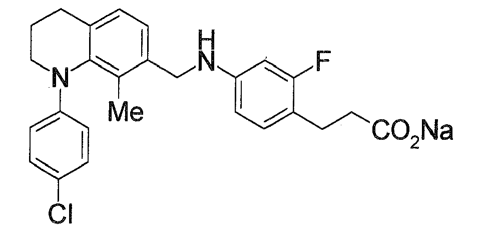 |
| 220 | 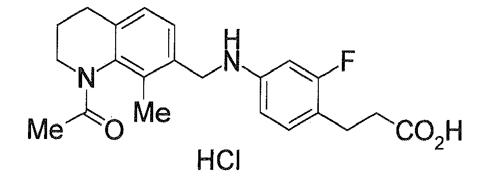 |

**[Table 61]**

| | |
|---|---|
| 221 | 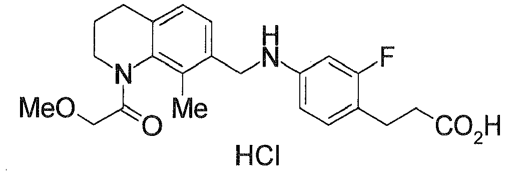 |
| 8 | 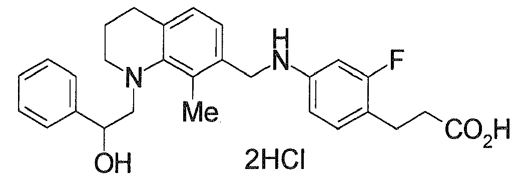 |
| 222 | 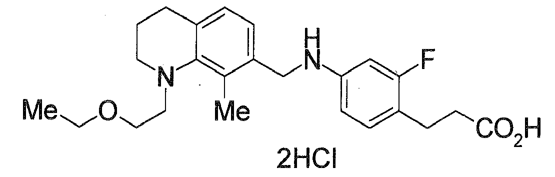 |
| 223 | 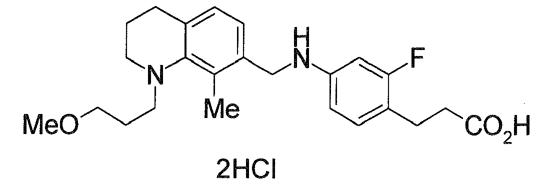 |
| 224 | 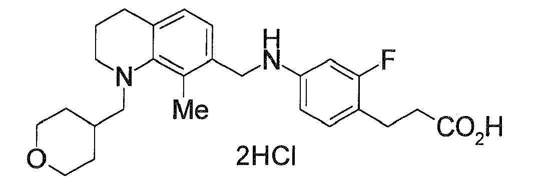 |
| 225 | 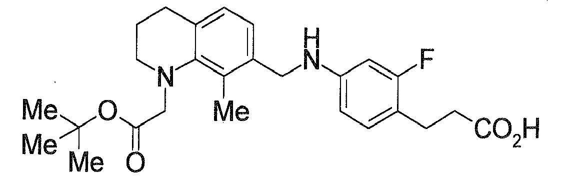 |
| 4 | 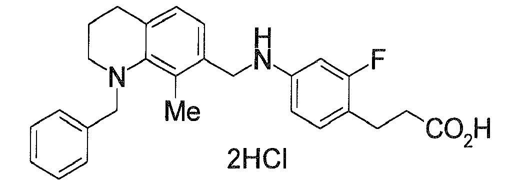 |

**[Table 62]**

| | |
|---|---|
| 226 | 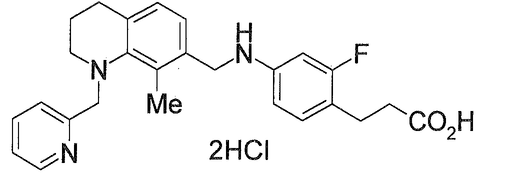 |
| 227 | 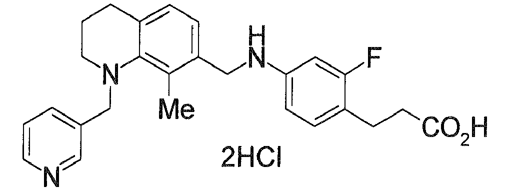 |
| 9 | 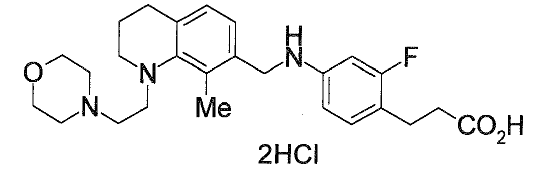 |
| 2 | 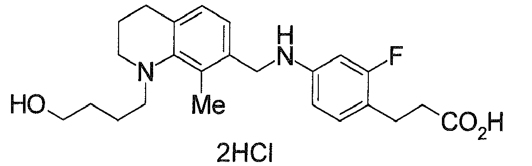 |
| 228 | 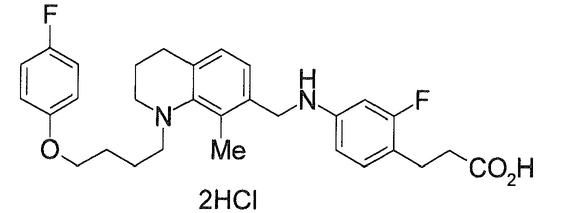 |
| 229 | 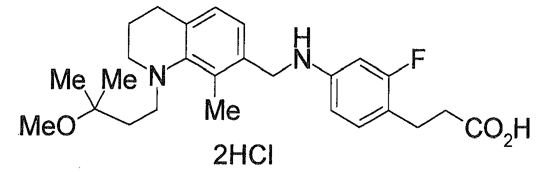 |
| 230 | 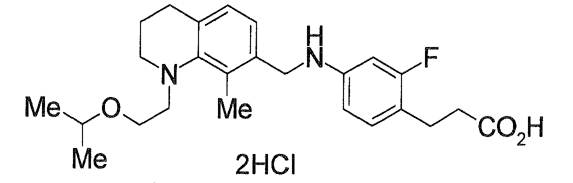 |

**[Table 63]**

| | |
|---|---|
| 231 | 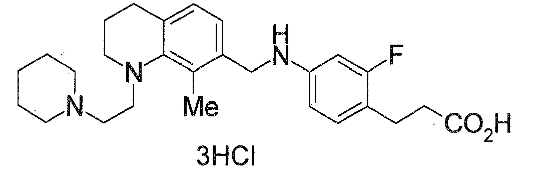 |
| 232 | 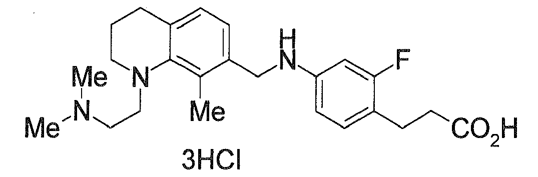 |
| 233 | 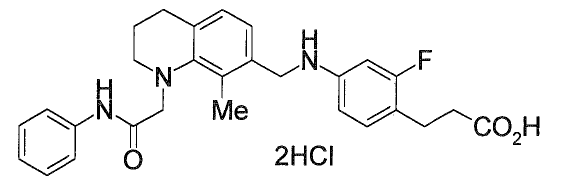 |
| 234 | 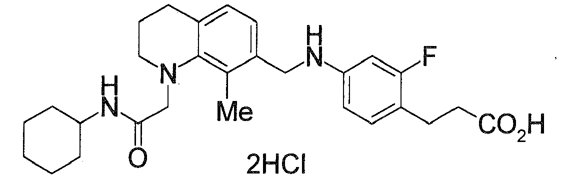 |
| 235 | 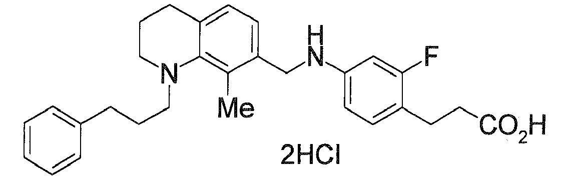 |
| 236 | 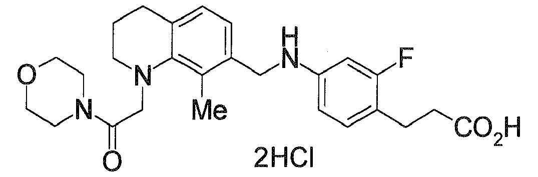 |
| 237 | 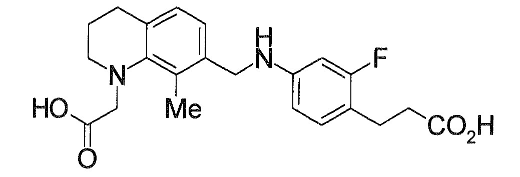 |

**[Table 64]**

| | |
|---|---|
| 238 | 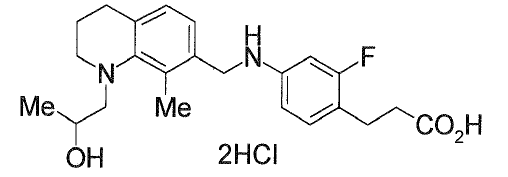 |
| 239 | 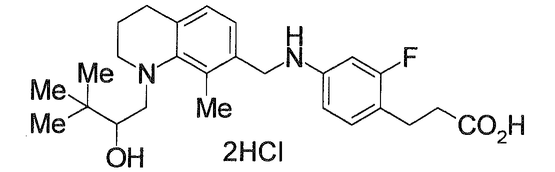 |
| 240 | 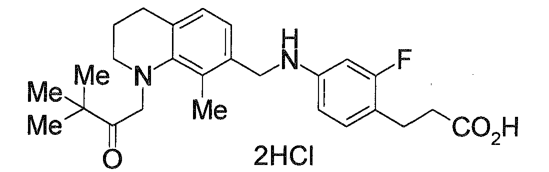 |
| 241 | 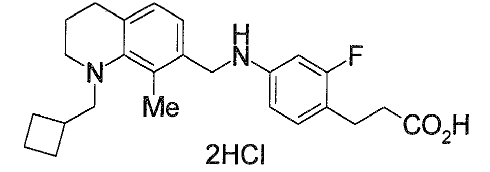 |
| 242 | 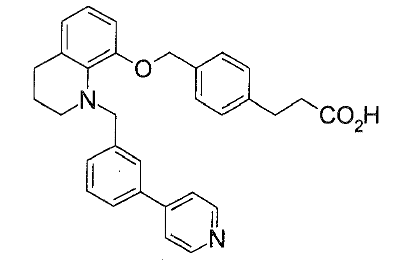 |
| 243 | 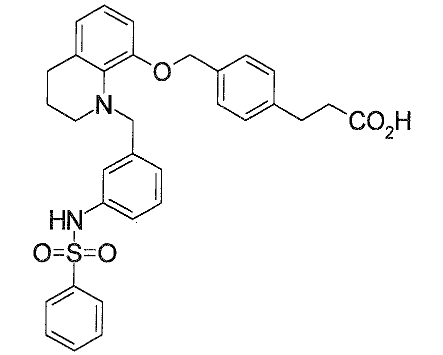 |

**[Table 65]**

| | |
|---|---|
| 15 | 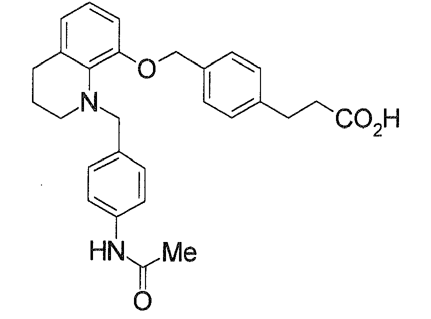 |
| 244 | 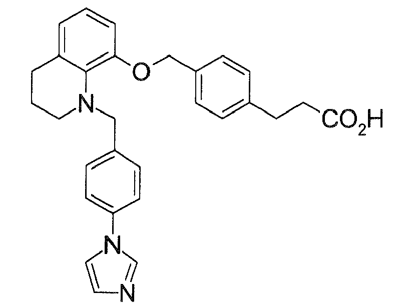 |
| 245 | 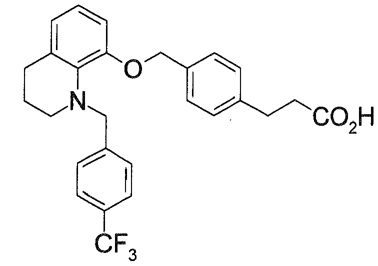 |
| 246 | 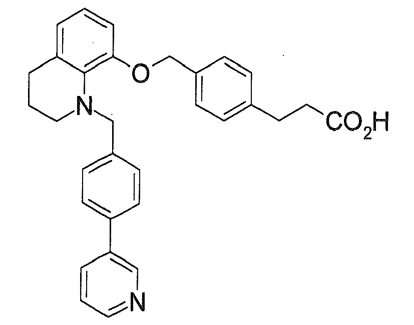 |

**[Table 66]**

| | |
|---|---|
| 247 | 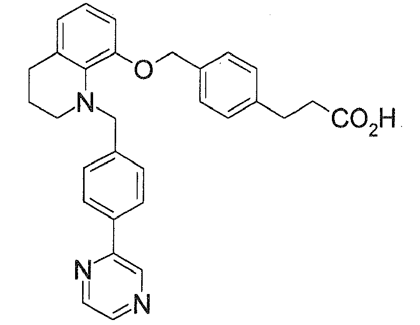 |
| 248 | 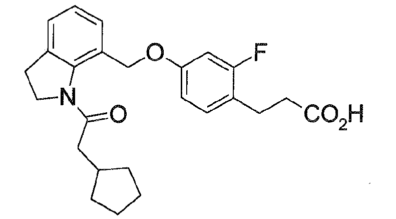 |
| 249 | 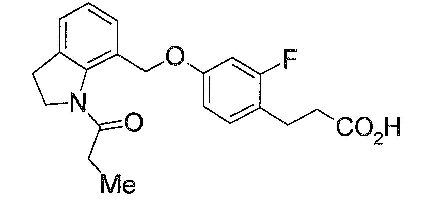 |
| 250 | 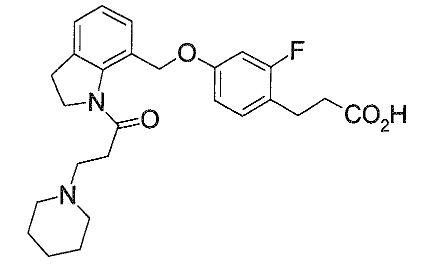 |
| 251 | 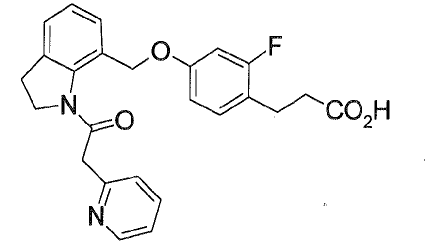 |

**[Table 67]**

| | |
|---|---|
| 252 | 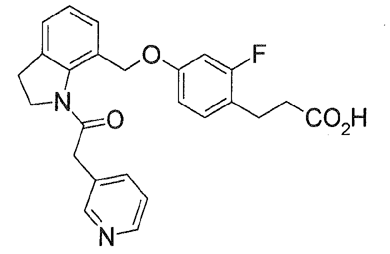 |
| 253 | 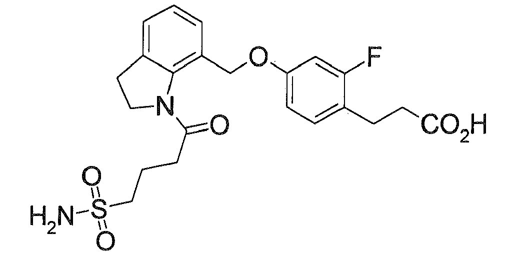 |
| 254 | 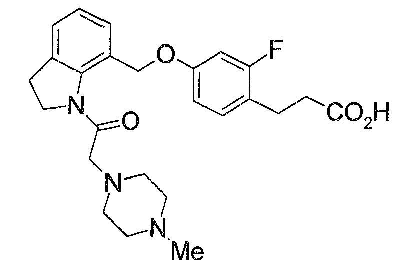 |
| 255 | 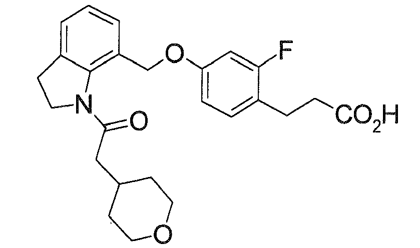 |
| 256 | 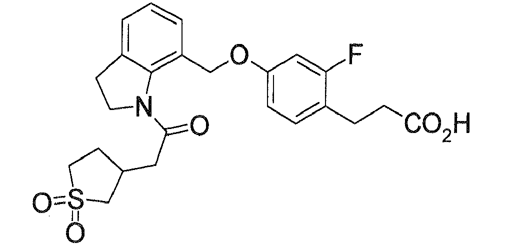 |

**[Table 68]**

| | |
|---|---|
| 257 | 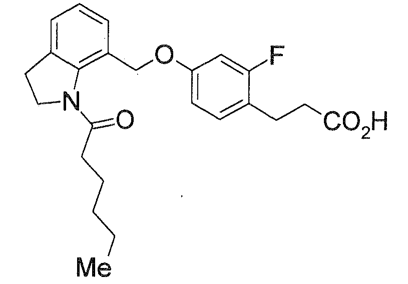 |
| 258 | 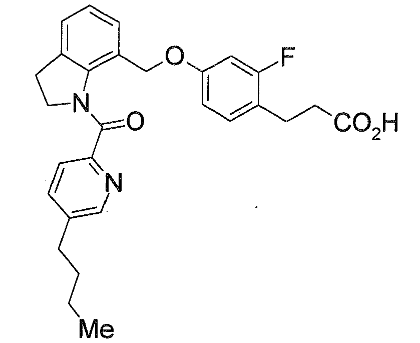 |
| 259 | 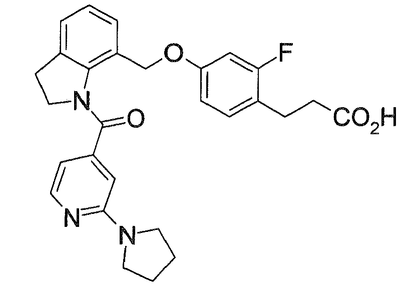 |
| 260 | 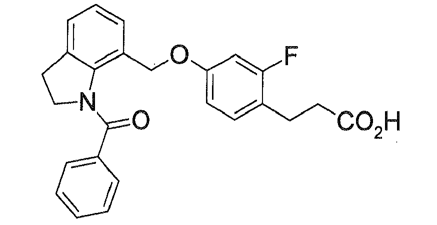 |

**[Table 69]**

| | |
|---|---|
| 16 | 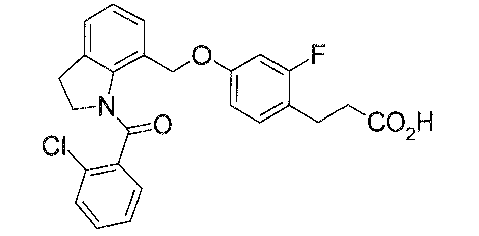 |
| 261 | 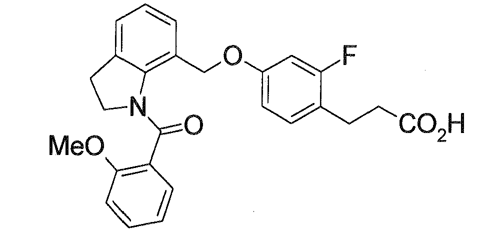 |
| 262 | 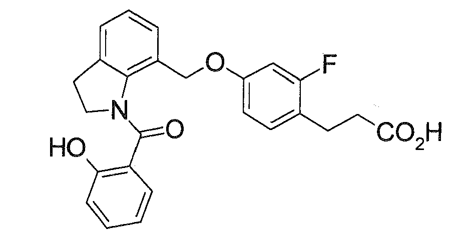 |
| 263 | 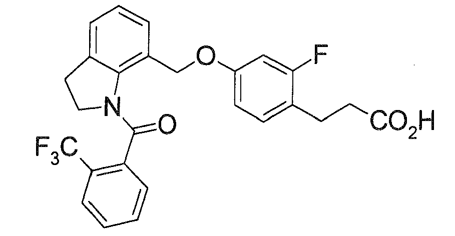 |
| 264 | 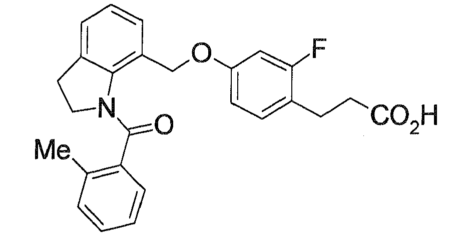 |

**[Table 70]**

| | |
|---|---|
| 265 | 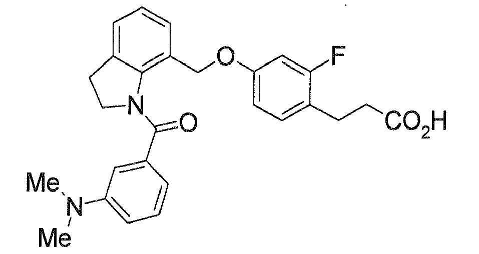 |
| 266 | 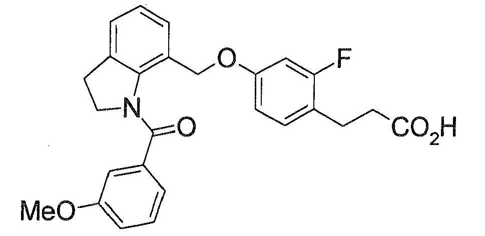 |
| 267 | 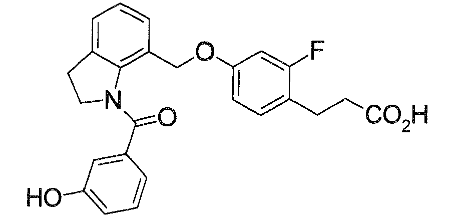 |
| 268 | 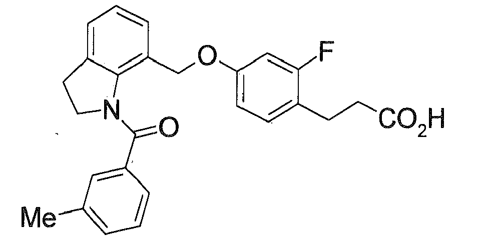 |
| 269 | 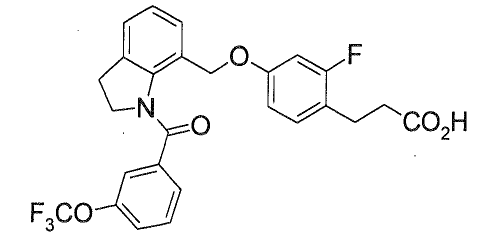 |

**[Table 71]**

| | |
|---|---|
| 270 | 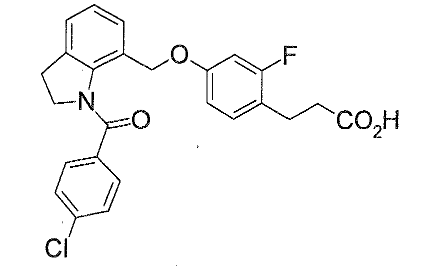 |
| 271 | 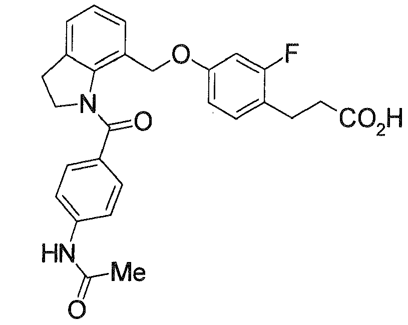 |
| 272 | 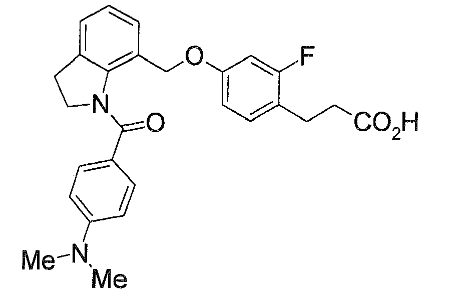 |
| 273 | 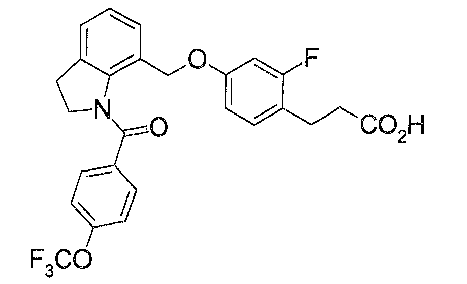 |

**[Table 72]**

| | |
|---|---|
| 274 | 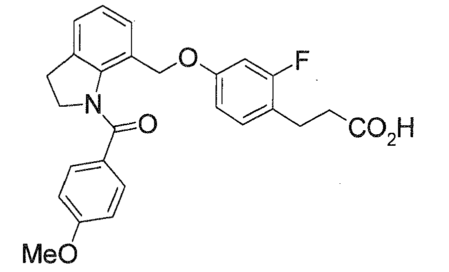 |
| 275 | 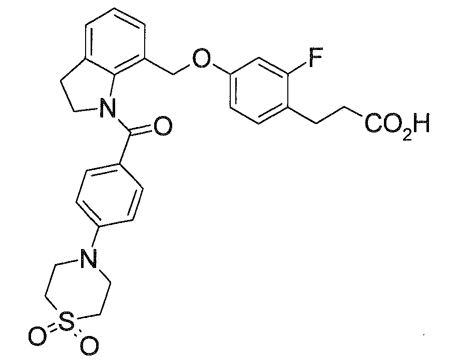 |
| 276 | 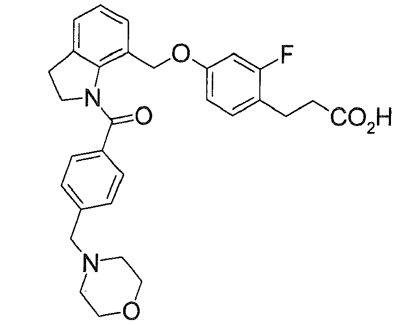 |
| 277 | 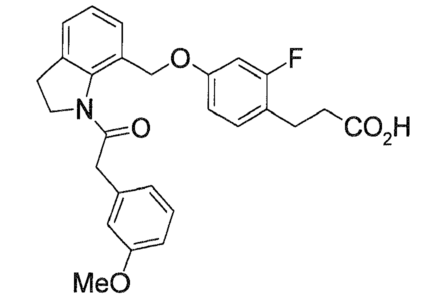 |

**[Table 73]**

| | |
|---|---|
| 278 | 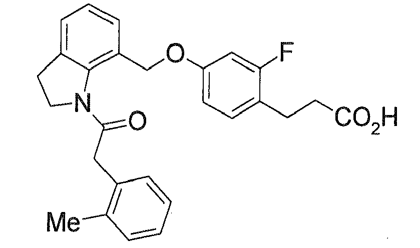 |
| 279 | 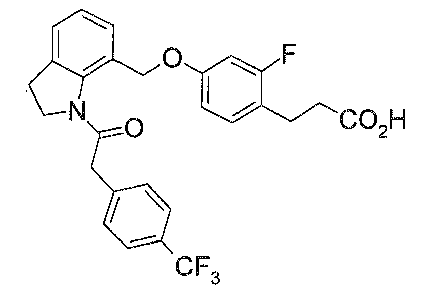 |
| 280 | 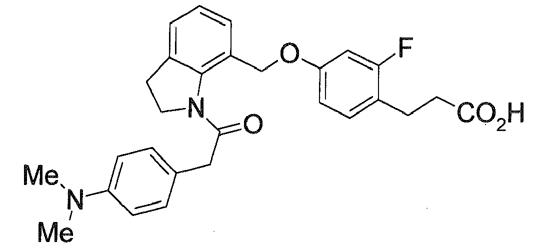 |
| 281 | 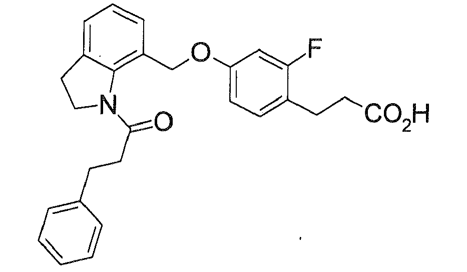 |

**[Table 74]**

| | |
|---|---|
| 282 | 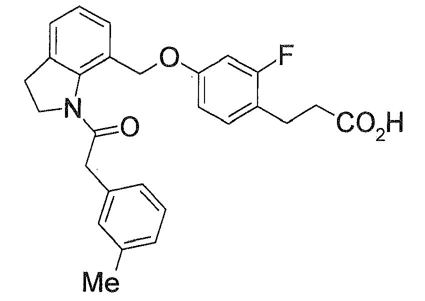 |
| 283 | 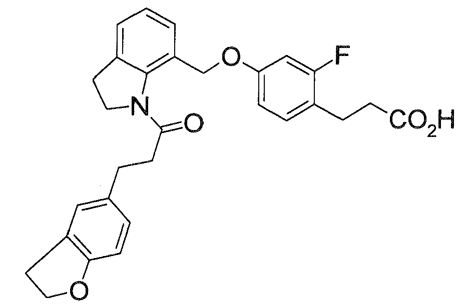 |
| 284 | 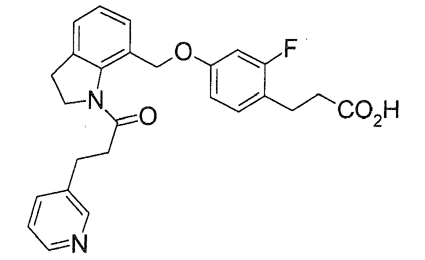 |
| 285 | 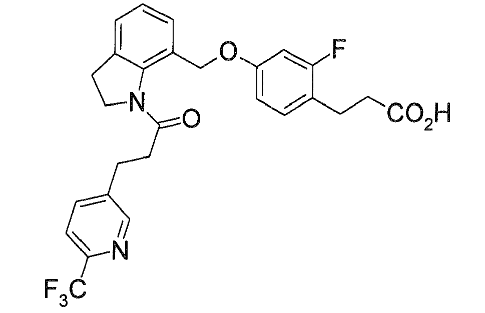 |

**[Table 75]**

| | |
|---|---|
| 286 | 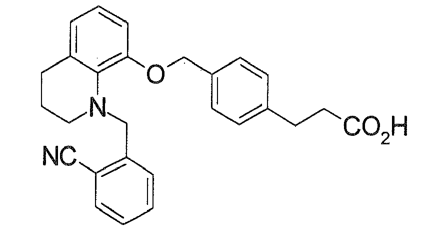 |
| 287 | 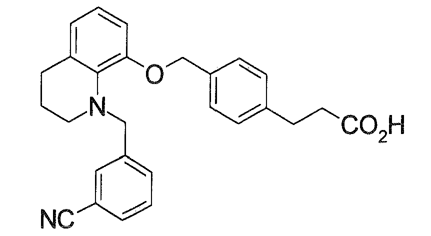 |
| 288 | 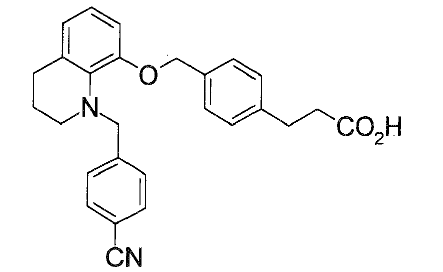 |
| 289 | 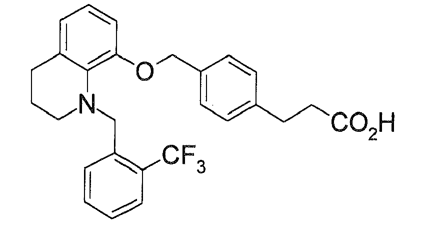 |
| 290 | 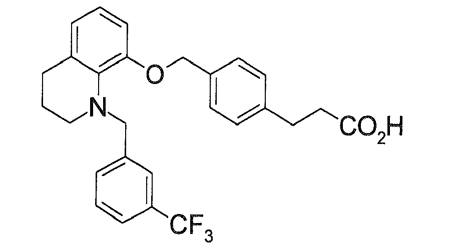 |

**[Table 76]**

| | |
|---|---|
| 291 | 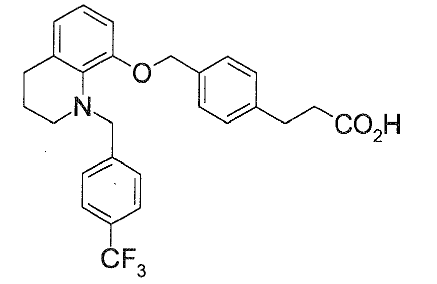 |
| 292 | 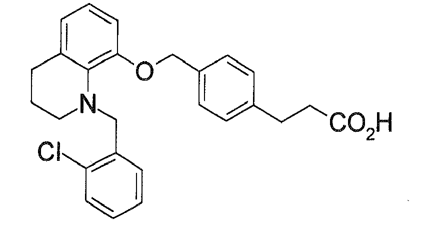 |
| 293 | 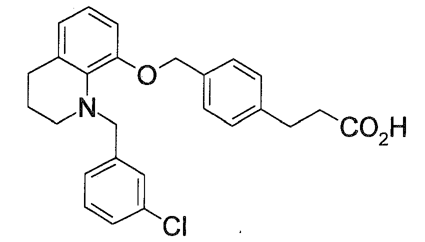 |
| 294 | 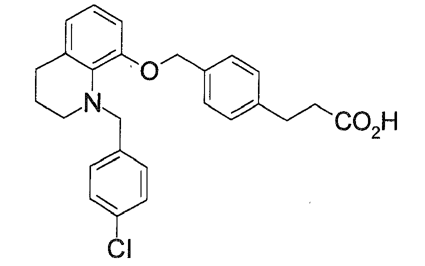 |
| 295 | 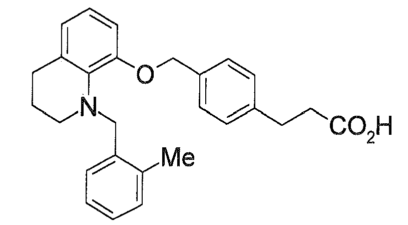 |

**[Table 77]**

| | |
|---|---|
| 296 | 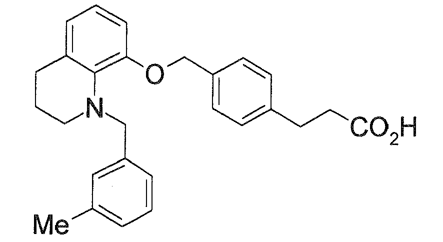 |
| 297 | 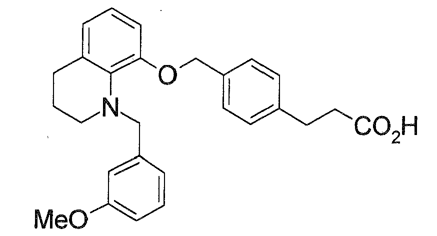 |
| 17 | 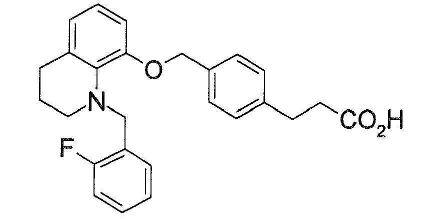 |
| 298 | 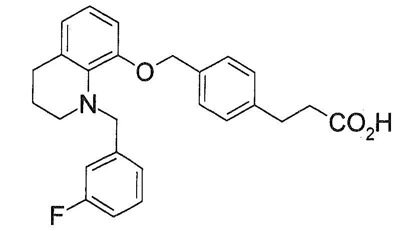 |
| 299 | 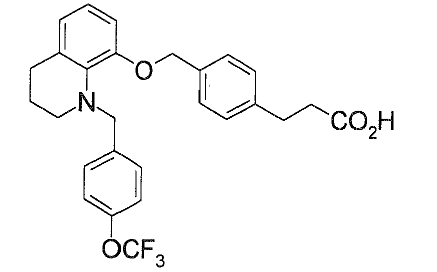 |

**[Table 78]**

| | |
|---|---|
| 300 | 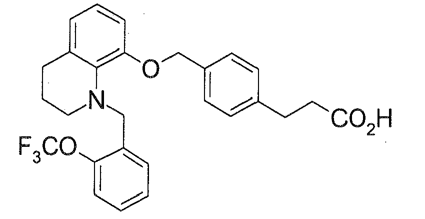 |
| 301 | 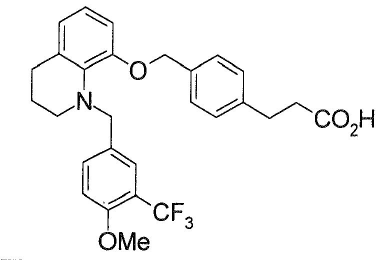 |
| 302 | 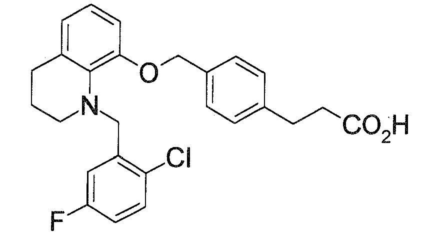 |
| 303 | 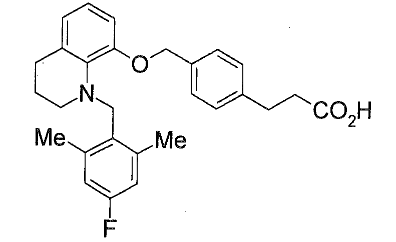 |
| 304 | 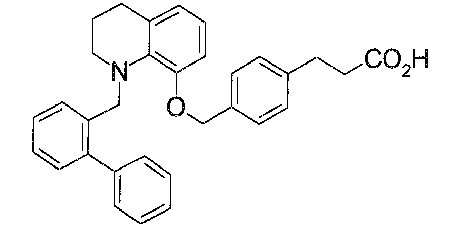 |

**[Table 79]**

| | |
|---|---|
| 305 | 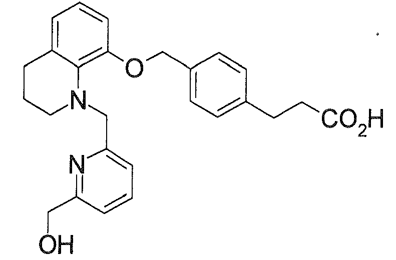 |
| 306 | 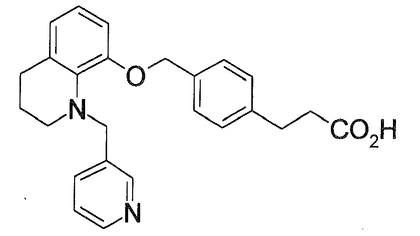 |
| 307 | 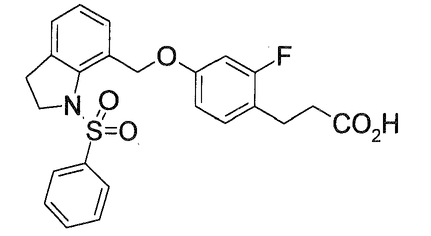 |
| 308 | 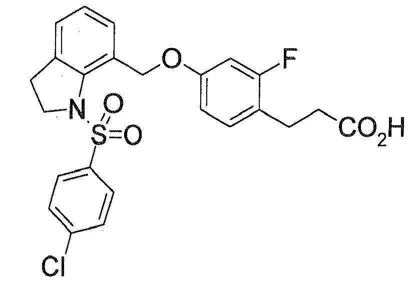 |
| 309 | 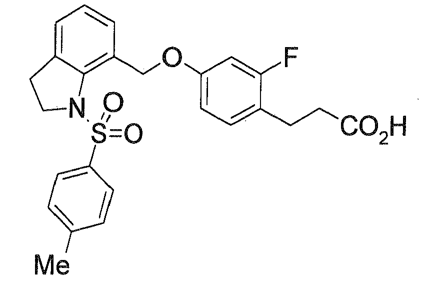 |

**[Table 80]**

| | |
|---|---|
| 310 | 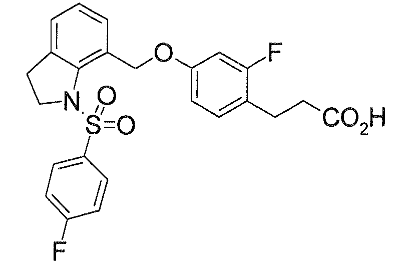 |
| 311 | 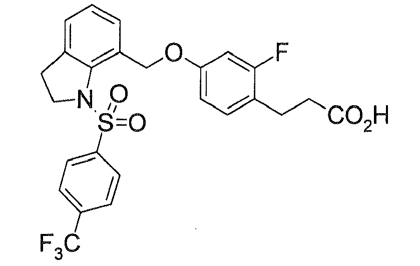 |
| 312 | 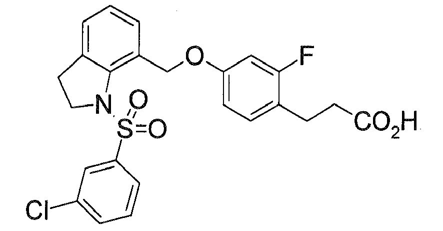 |
| 18 | 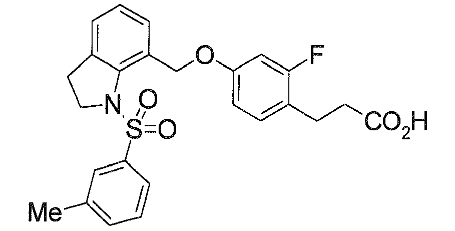 |
| 313 | 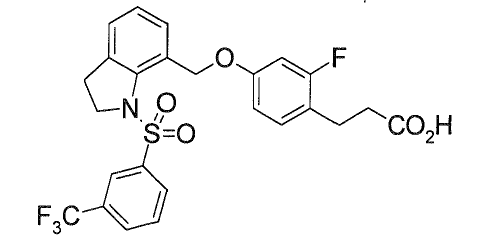 |

**[Table 81]**

| | |
|---|---|
| 314 | 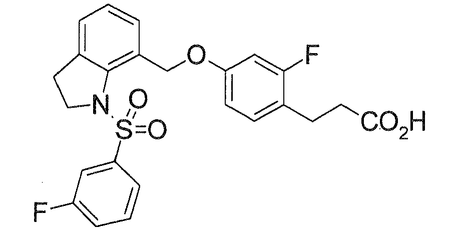 |
| 315 | 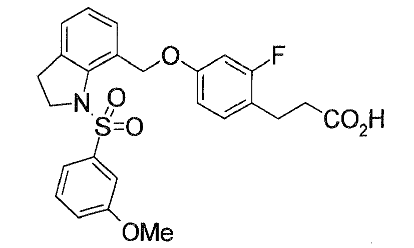 |
| 316 | 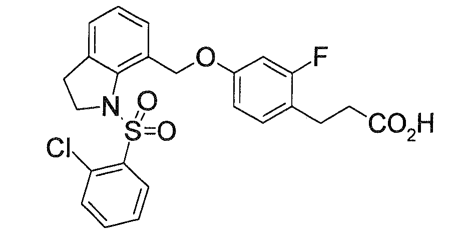 |
| 317 | 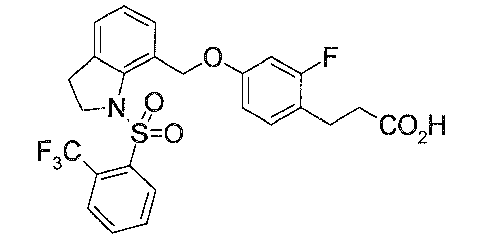 |
| 318 | 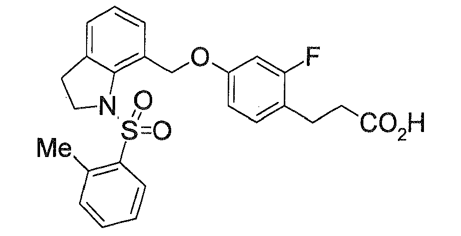 |

**[Table 82]**

| | |
|---|---|
| 319 | 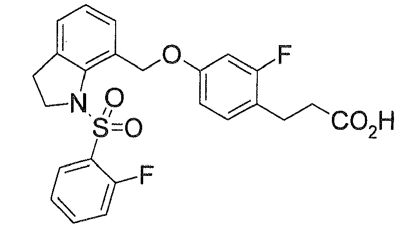 |
| 320 | 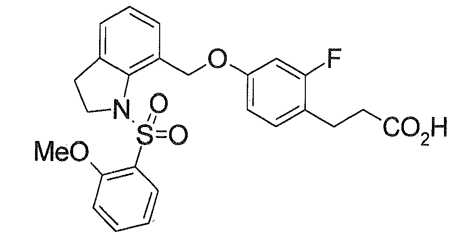 |
| 321 | 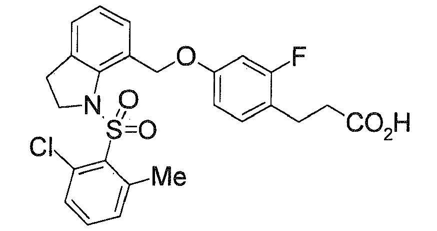 |
| 322 | 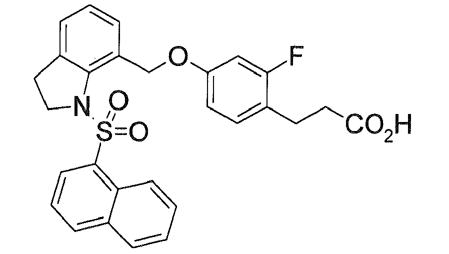 |
| 323 | 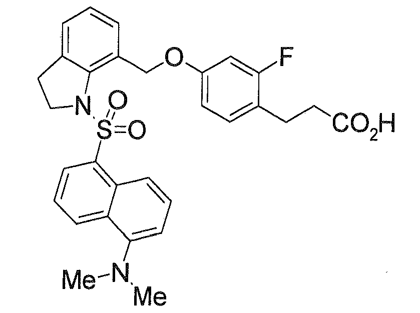 |

**[Table 83]**

| | |
|---|---|
| 324 | 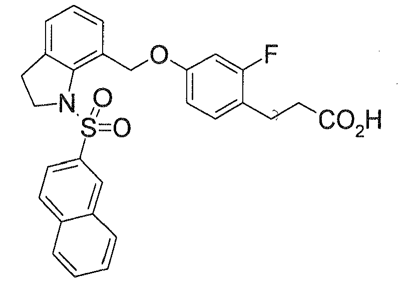 |
| 325 | 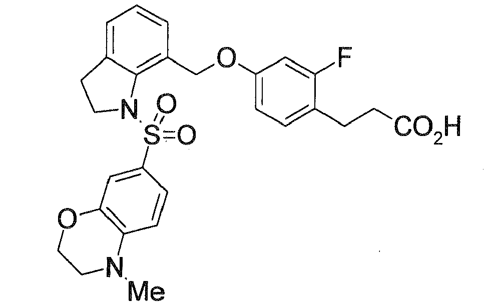 |
| 326 | 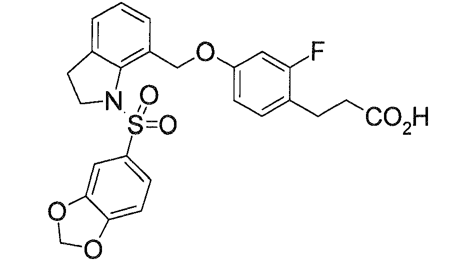 |
| 327 | 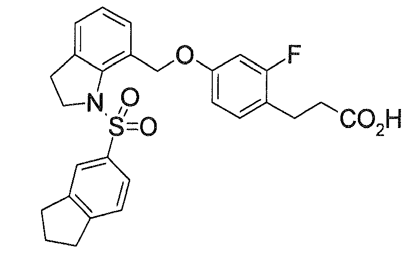 |

**[Table 84]**

| | |
|---|---|
| 328 | 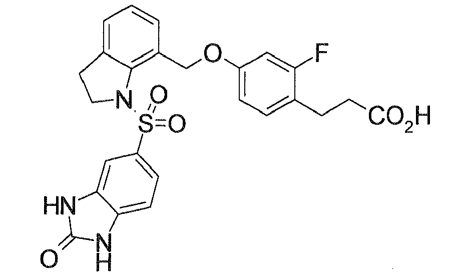 |
| 19 | 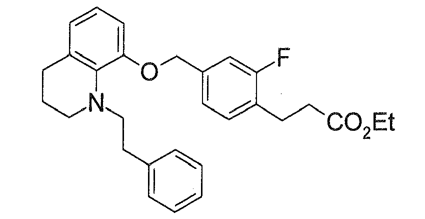 |
| 329 | 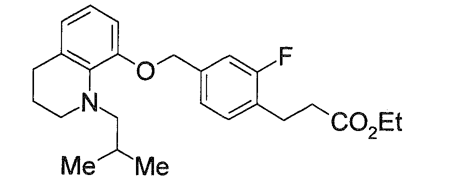 |
| 330 | 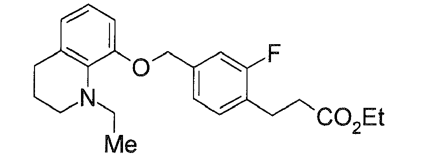 |
| 331 | 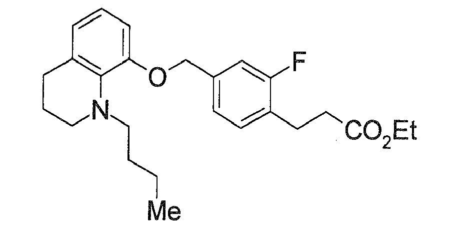 |
| 332 | 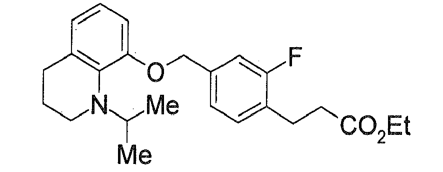 |

**[Table 85]**

| | |
|---|---|
| 20 | 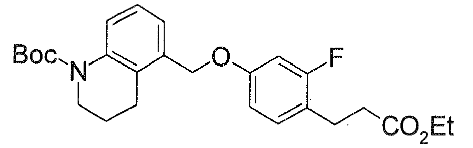 |
| 333 | 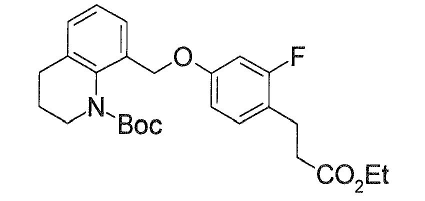 |
| 334 | 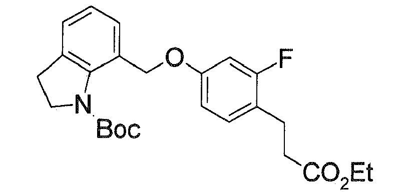 |
| 335 | 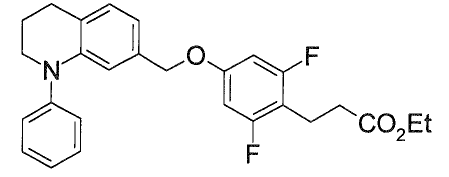 |
| 336 | 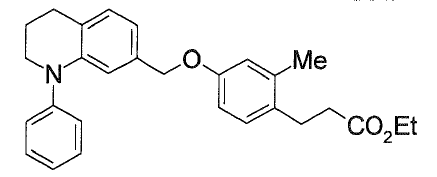 |
| 337 | 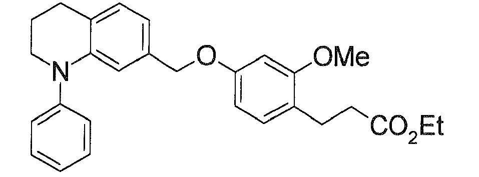 |
| 338 | 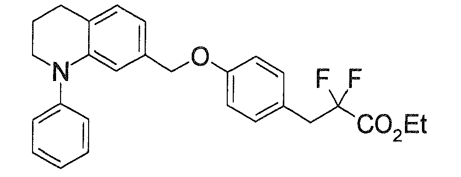 |

**[Table 86]**

| | |
|---|---|
| 21 | 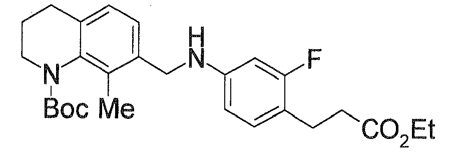 |
| 22 | 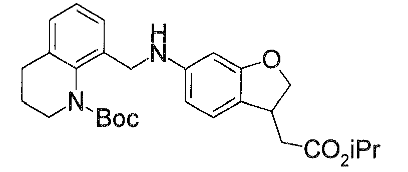 |
| 339 | 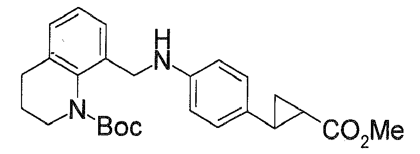 |
| 340 | 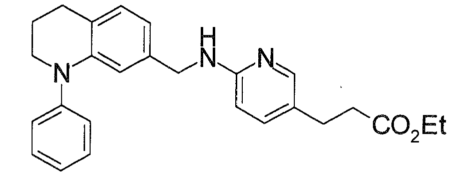 |
| 341 | 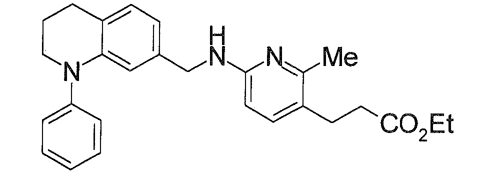 |
| 23 | 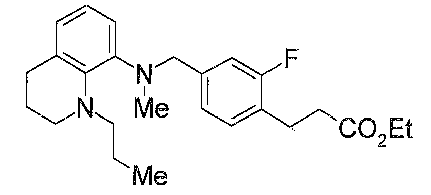 |
| 342 | 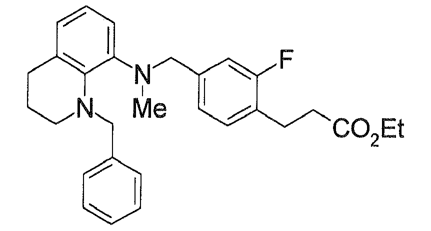 |

**[Table 87]**

| | |
|---|---|
| 24 | 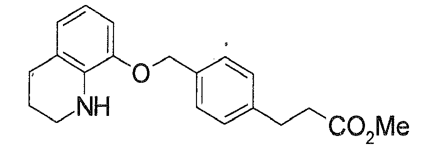 |
| 343 | 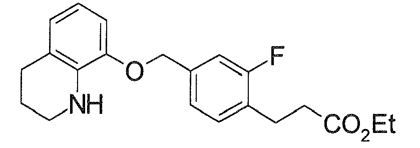 |
| 344 | 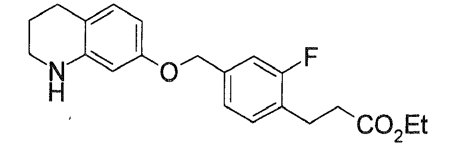 |
| 25 | 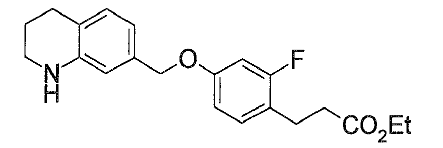 |
| 345 | 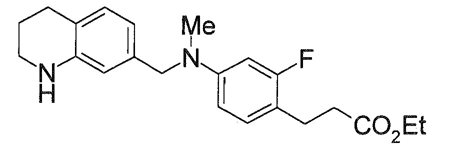 |
| 26 | 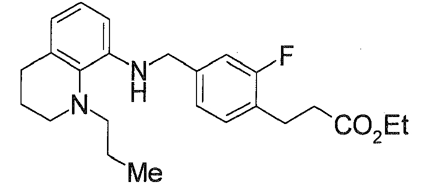 |
| 346 | 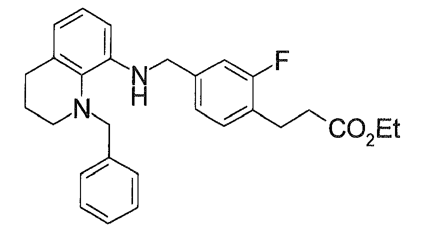 |

**[Table 88]**

| | |
|---|---|
| 347 | 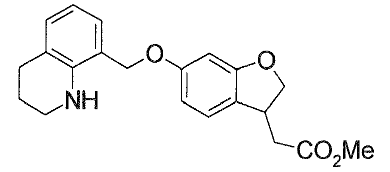 |
| 27 | 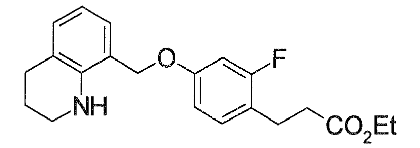 |
| 348 | 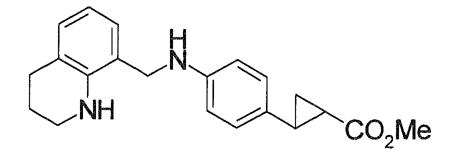 |
| 349 | 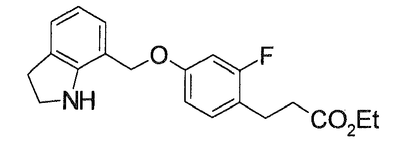 |
| 350 | 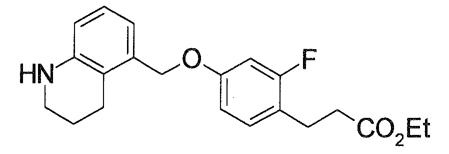 |
| 28 | 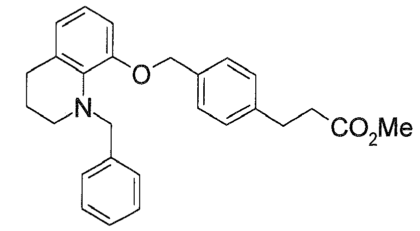 |
| 351 | 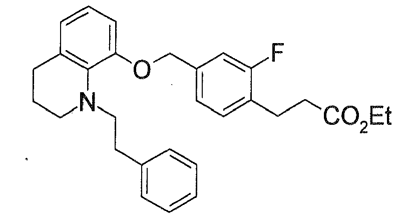 |

**[Table 89]**

| | |
|---|---|
| 352 | 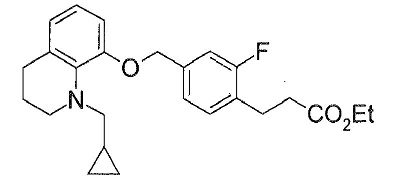 |
| 353 | 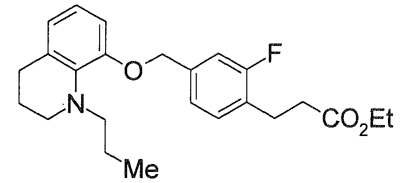 |
| 354 | 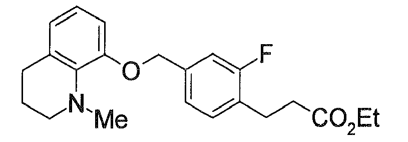 |
| 355 | 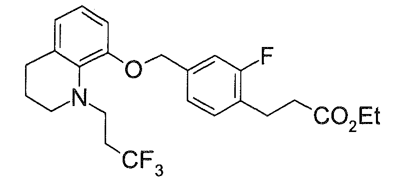 |
| 356 | 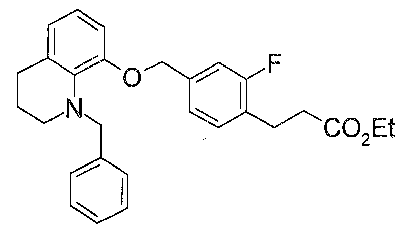 |
| 357 | 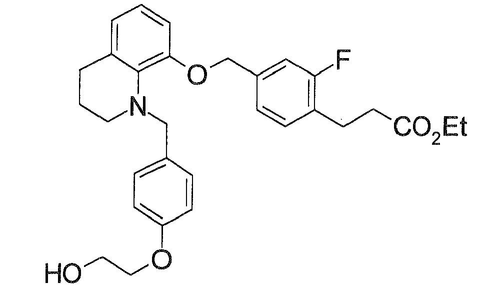 |

**[Table 90]**

| | |
|---|---|
| 358 | 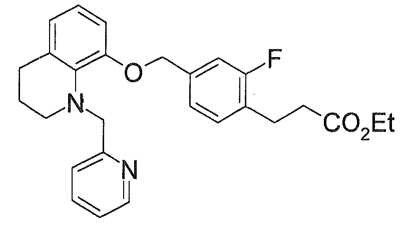 |
| 359 | 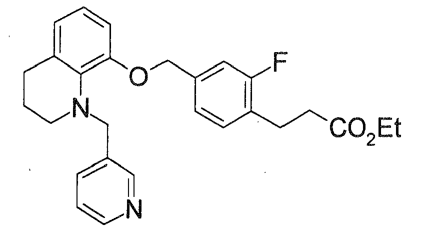 |
| 360 | 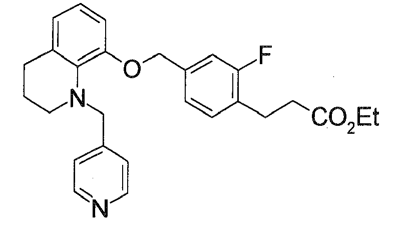 |
| 361 | 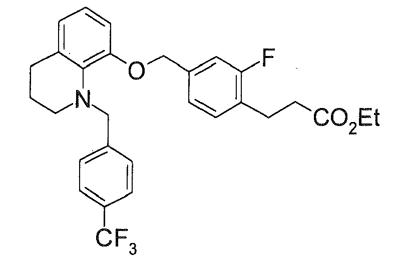 |
| 362 | 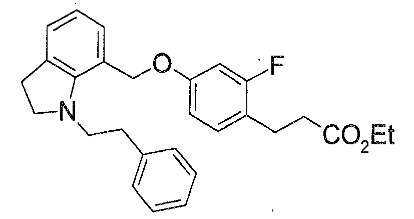 |
| 363 | 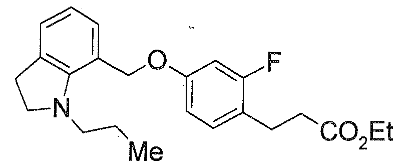 |

**[Table 91]**

| | |
|---|---|
| 364 | 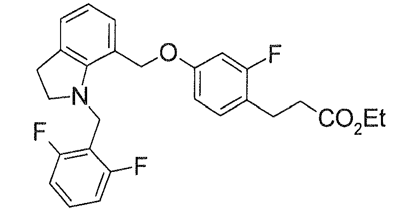 |
| 365 | 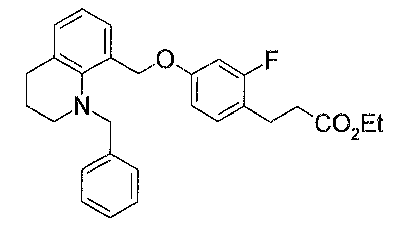 |
| 366 | 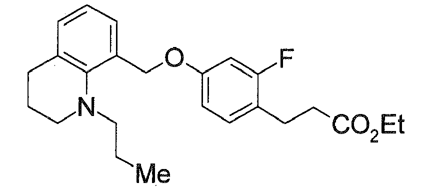 |
| 367 | 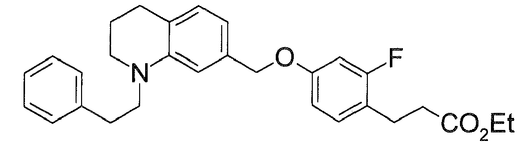 |
| 368 | 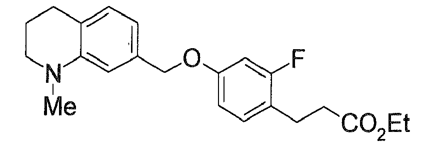 |
| 369 | 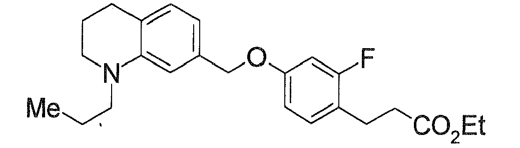 |
| 370 | 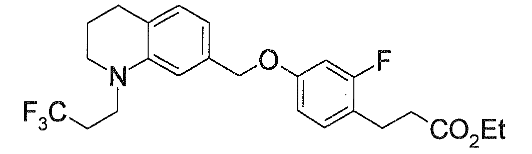 |

**[Table 92]**

| | |
|---|---|
| 371 | 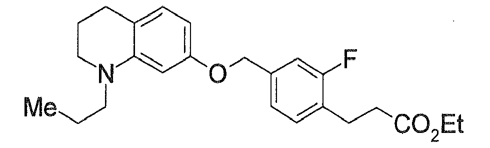 |
| 372 | 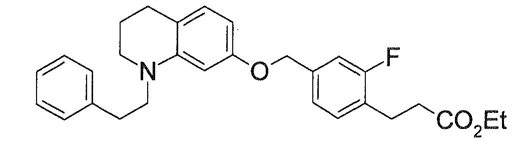 |
| 373 | 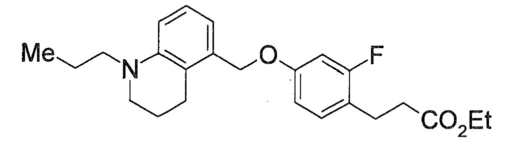 |
| 374 | 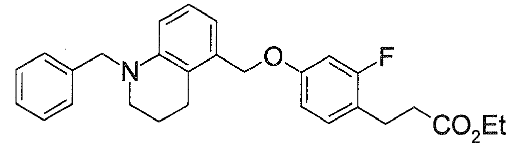 |
| 375 | 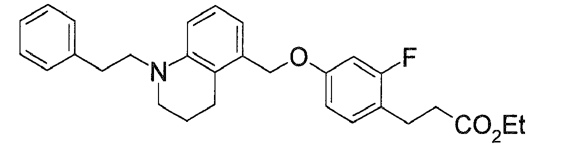 |
| 29 | 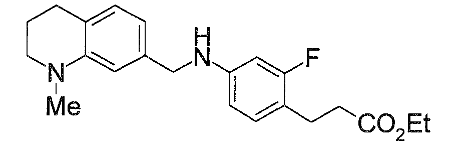 |
| 376 | 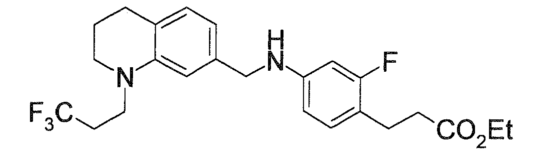 |
| 377 | 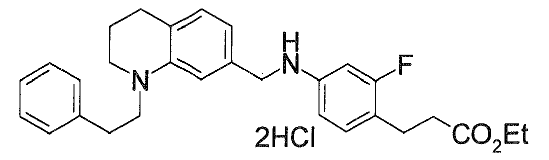 |

**[Table 93]**

| | |
|---|---|
| 378 | 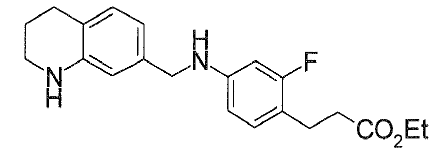 |
| 379 | 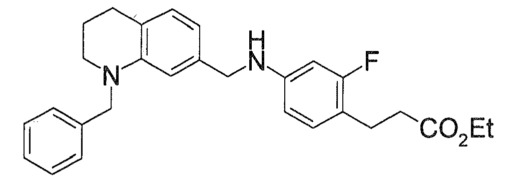 |
| 380 | 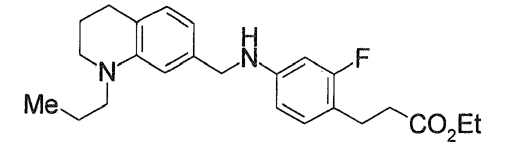 |
| 381 | 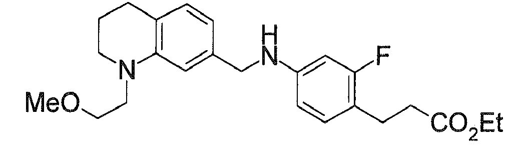 |
| 382 | 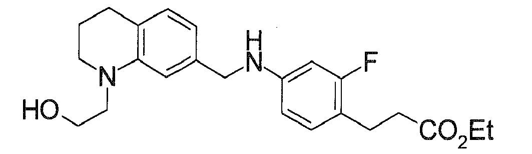 |
| 383 | 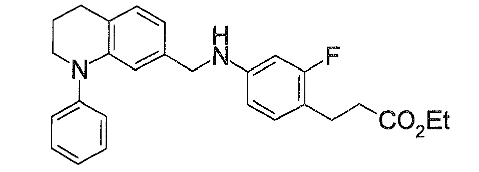 |
| 384 | 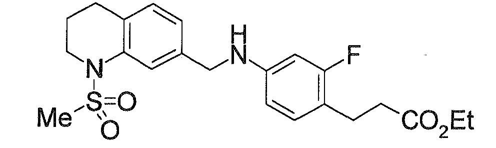 |
| 385 | 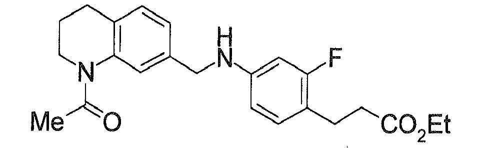 |

**[Table 94]**

| | |
|---|---|
| 386 | 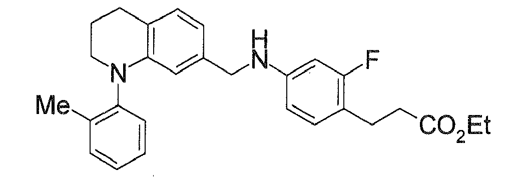 |
| 387 | 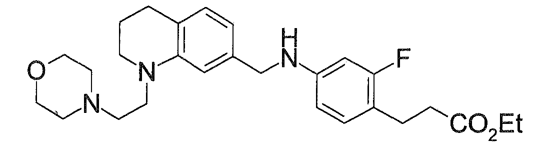 |
| 30 | 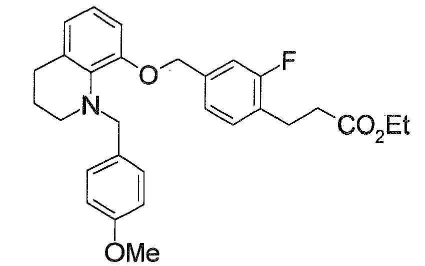 |
| 388 | 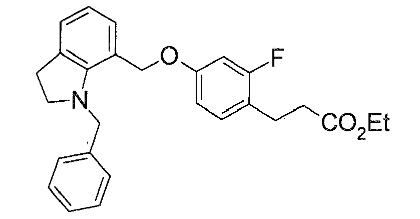 |
| 389 | 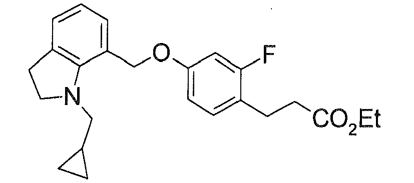 |
| 390 | 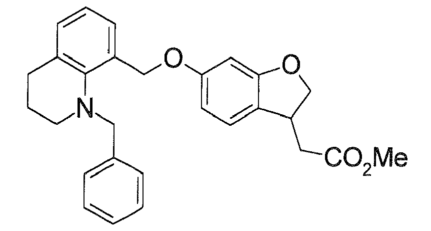 |

**[Table 95]**

| | |
|---|---|
| 391 | 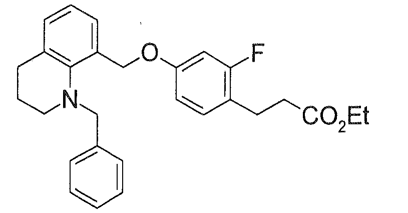 |
| 392 | 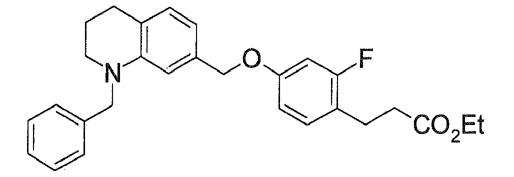 |
| 393 | 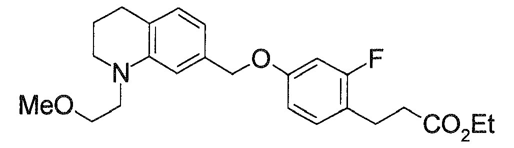 |
| 394 | 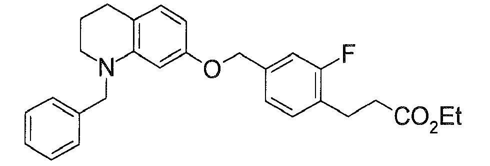 |
| 395 | 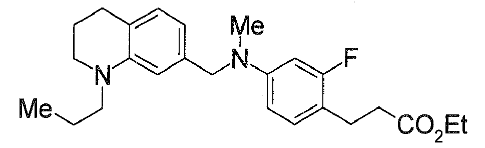 |
| 31 | 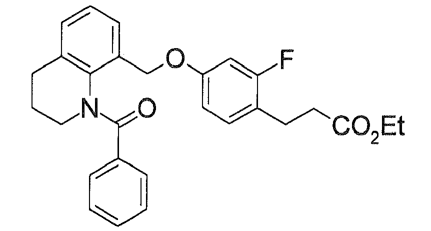 |
| 396 | 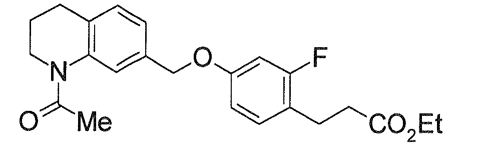 |

**[Table 96]**

| | |
|---|---|
| 397 | 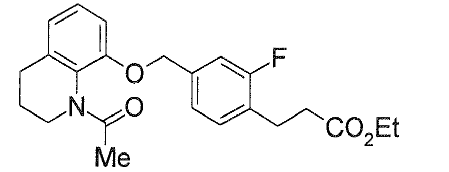 |
| 398 | 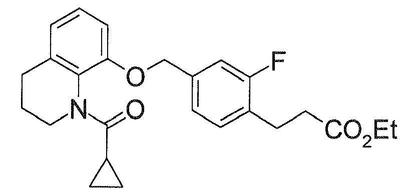 |
| 399 | 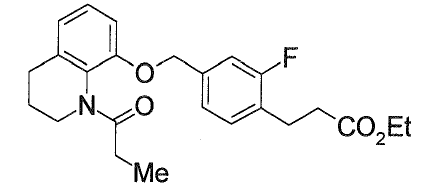 |
| 400 | 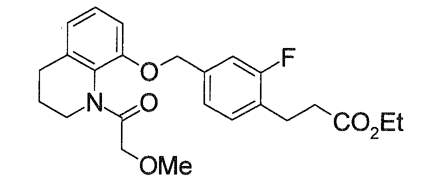 |
| 401 | 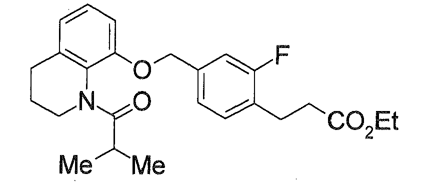 |
| 402 | 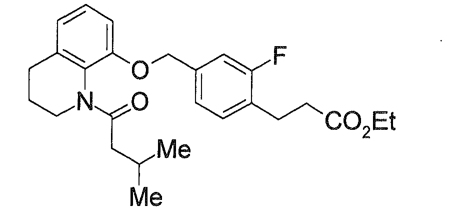 |

**[Table 97]**

| | |
|---|---|
| 403 | 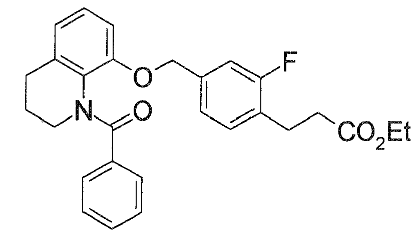 |
| 404 | 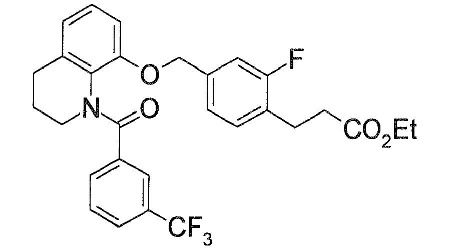 |
| 32 | 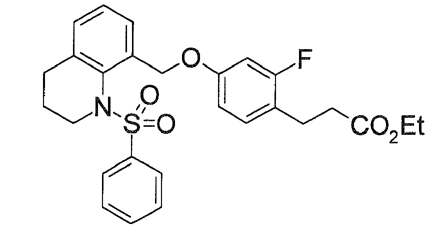 |
| 405 | 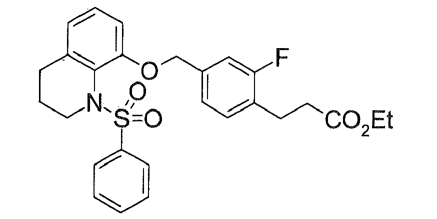 |
| 406 | 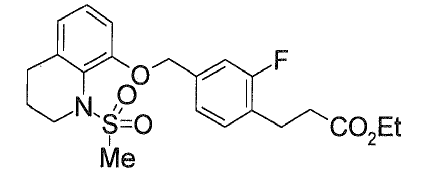 |
| 407 | 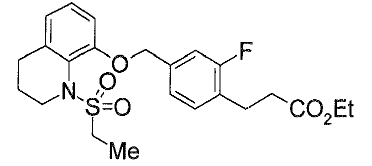 |

**[Table 98]**

| | |
|---|---|
| 408 | 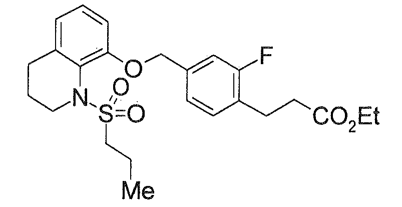 |
| 33 | 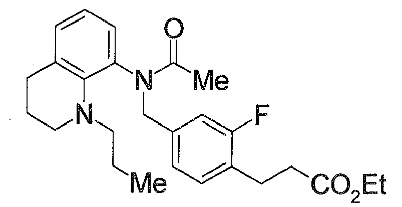 |
| 409 | 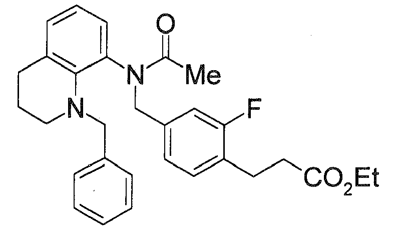 |
| 34 | 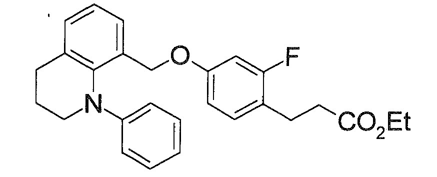 |
| 410 | 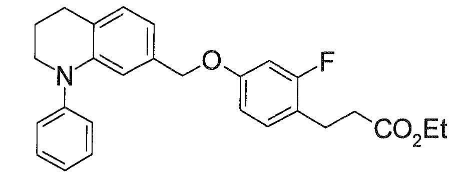 |
| 411 | 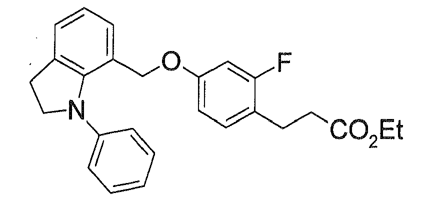 |

**[Table 99]**

| | |
|---|---|
| 412 | 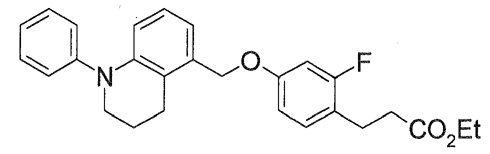 |
| 35 | 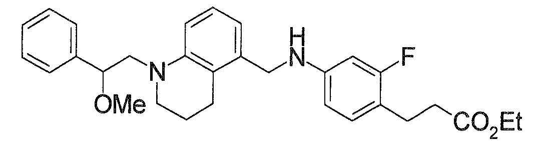 |
| 413 | 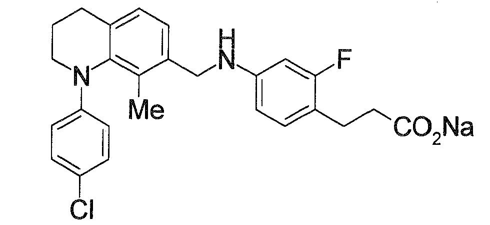 |
| 414 | 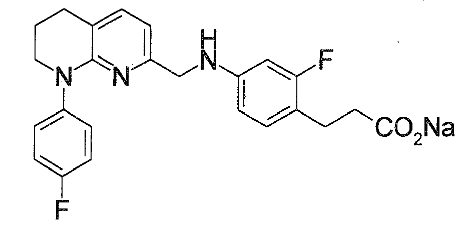 |
| 415 | 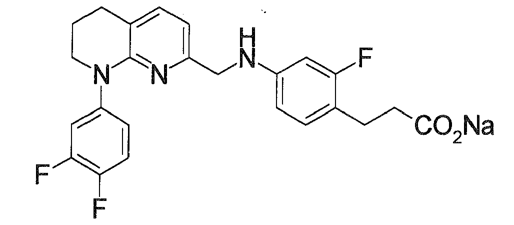 |
| 416 | 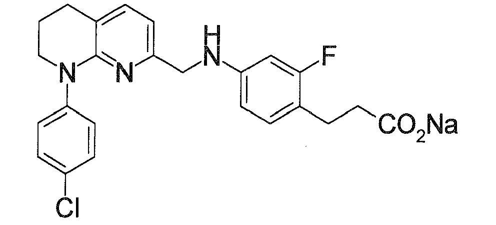 |

**[Table 100]**

| | |
|---|---|
| 417 | 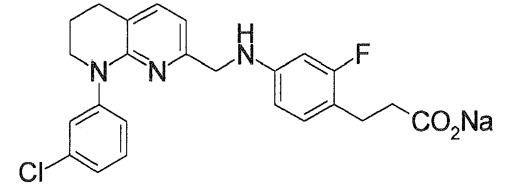 |
| 418 | 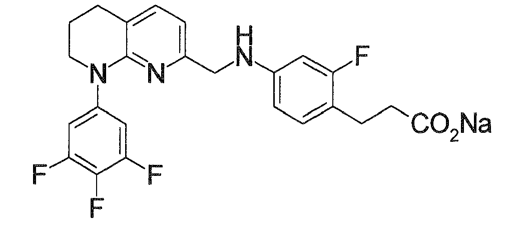 |
| 419 | 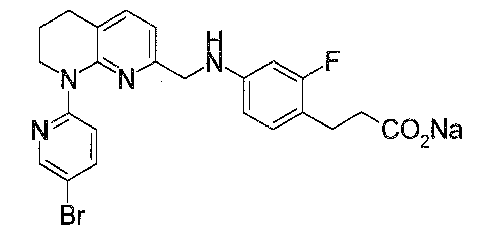 |
| 420 | 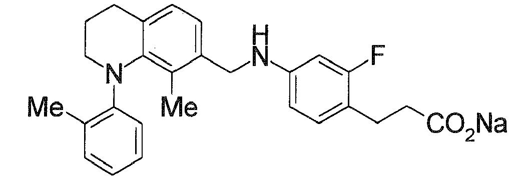 |
| 421 | 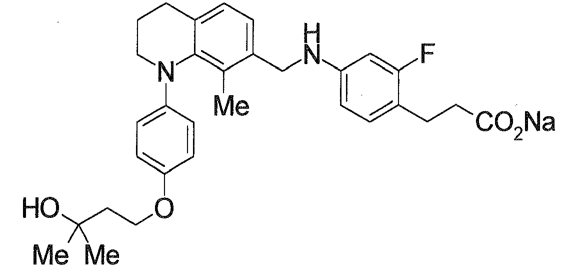 |

**[Table 101]**

| | |
|---|---|
| 422 | 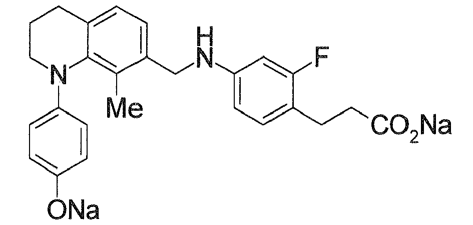 |
| 423 | 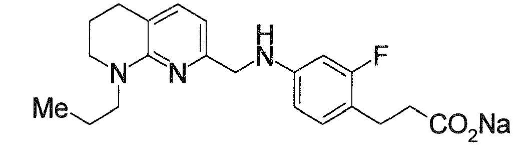 |
| 424 | 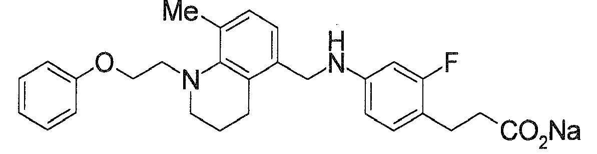 |
| 425 | 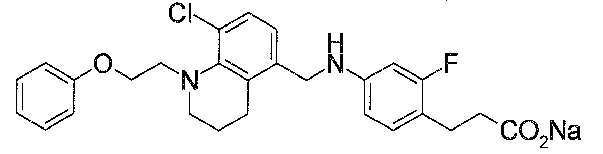 |
| 426 | 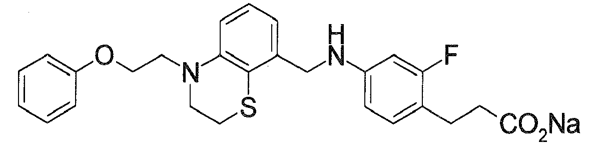 |
| 427 | 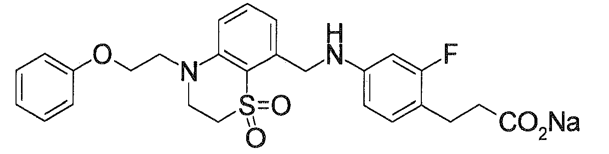 |
| 428 | 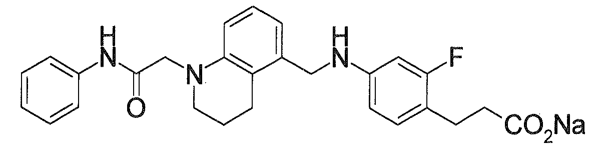 |

**[Table 102]**

| | |
|---|---|
| 429 | 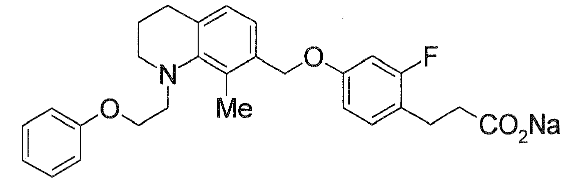 |
| 430 | 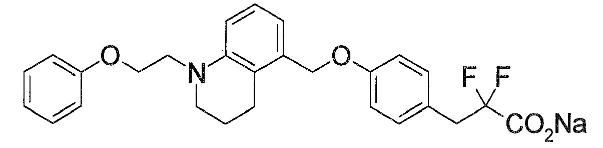 |
| 39 | 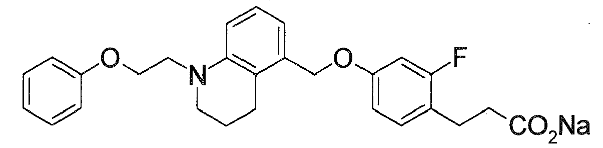 |
| 431 | 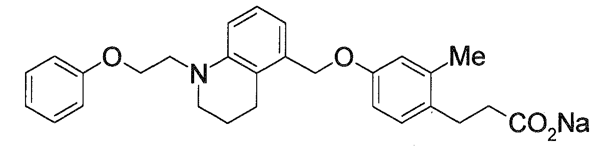 |
| 432 | 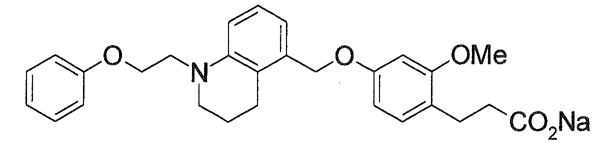 |
| 433 | 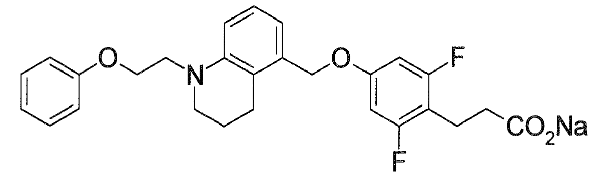 |
| 434 | 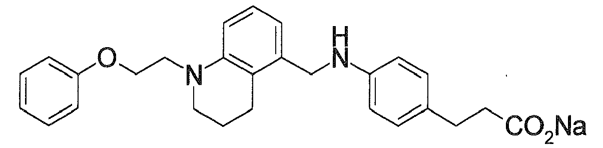 |
| 435 | 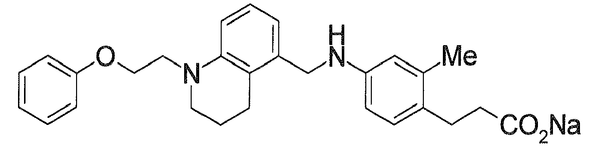 |

**[Table 103]**

| | |
|---|---|
| 436 | 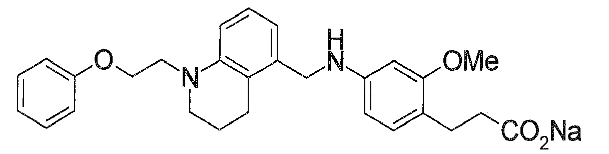 |
| 437 | 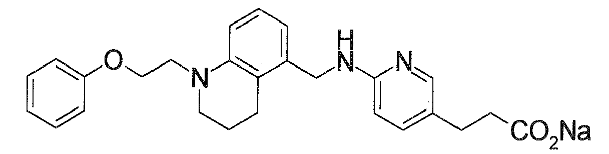 |
| 438 | 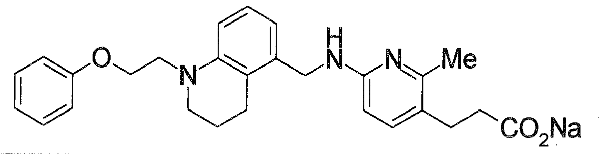 |
| 439 | 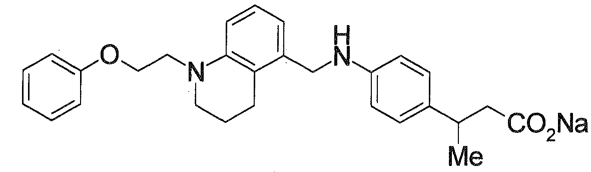 |
| 36 | 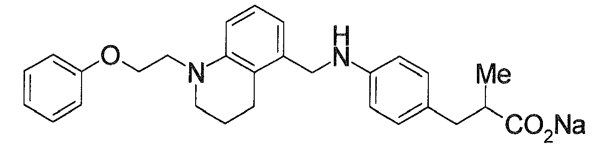 |
| 440 | 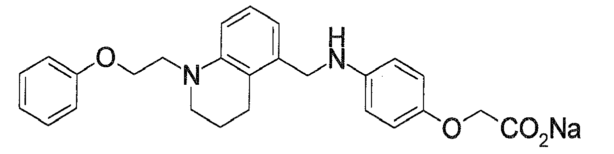 |
| 441 | 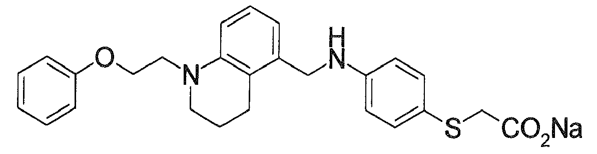 |
| 442 | 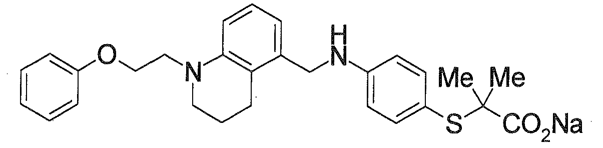 |

**[Table 104]**

| | |
|---|---|
| 443 | 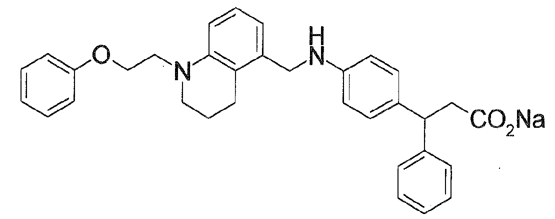 |
| 444 | 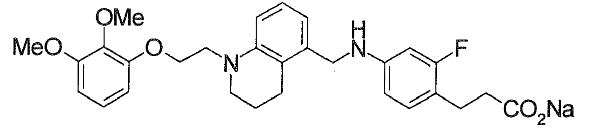 |
| 445 | 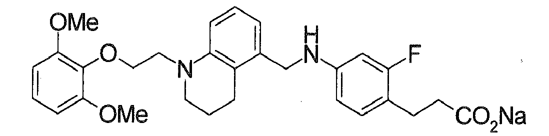 |
| 446 | 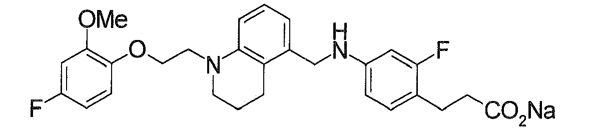 |
| 447 | 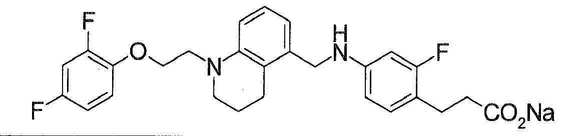 |
| 448 | 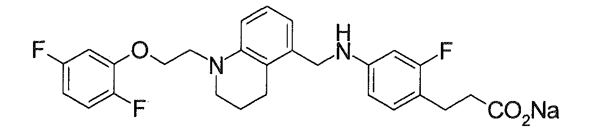 |
| 449 | 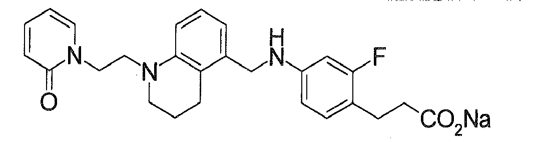 |
| 450 | 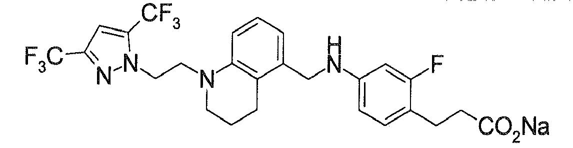 |
| 451 | 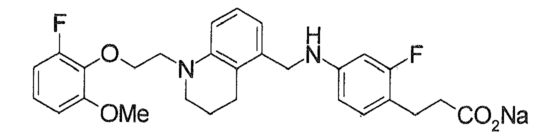 |

**[Table 105]**

| | |
|---|---|
| 452 | 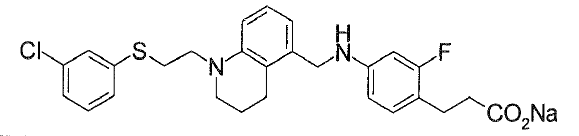 |
| 453 | 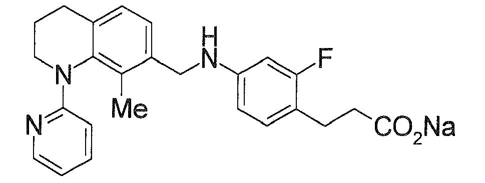 |
| 454 | 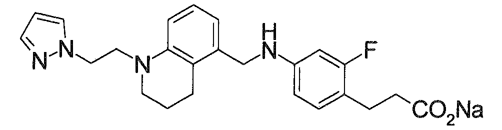 |
| 455 | 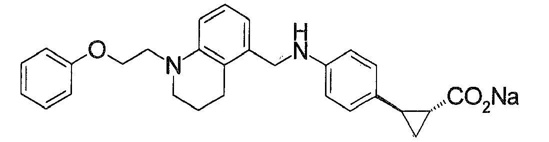 |
| 456 | 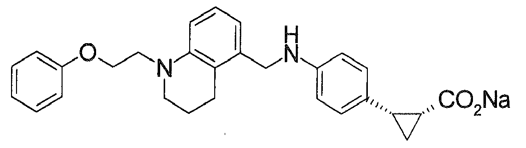 |
| 37 | 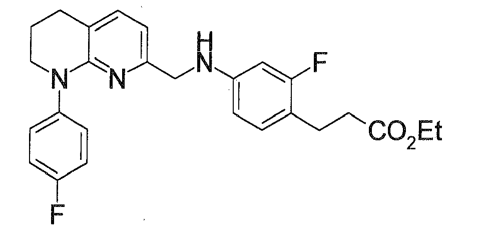 |
| 457 | 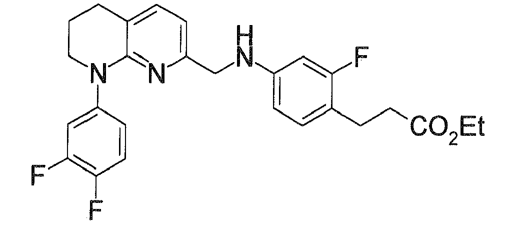 |

**[Table 106]**

| | |
|---|---|
| 458 | 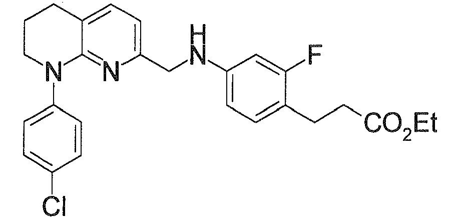 |
| 459 | 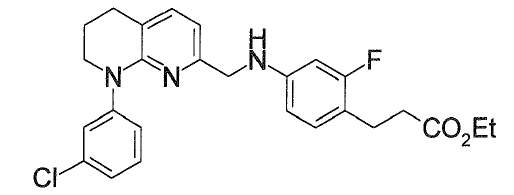 |
| 460 | 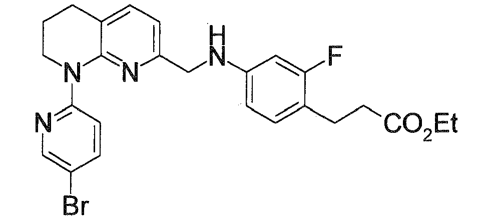 |
| 461 | 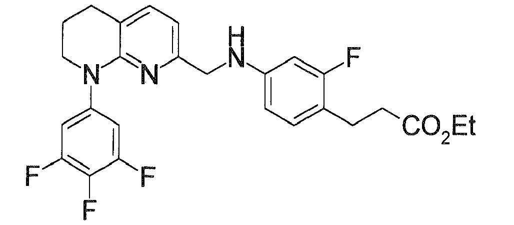 |
| 462 | 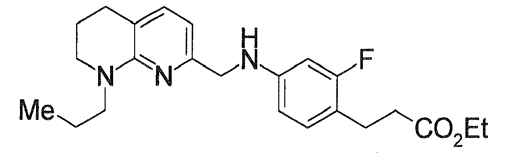 |
| 463 | 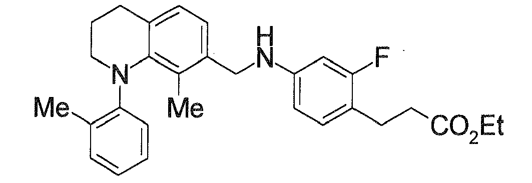 |

**[Table 107]**

| | |
|---|---|
| 464 | 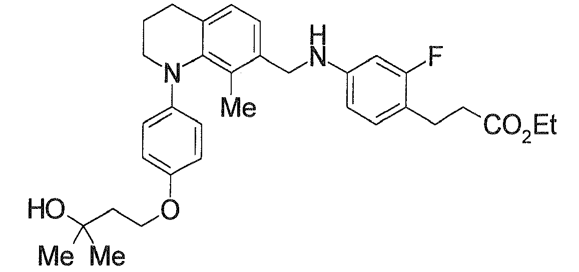 |
| 465 | 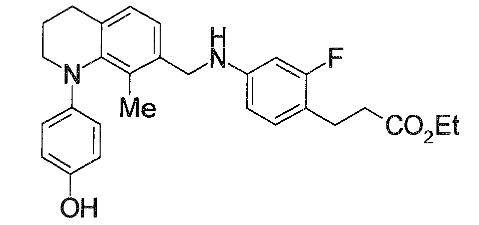 |
| 466 | 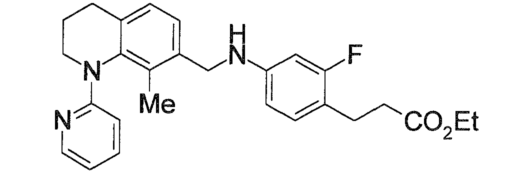 |
| 467 | 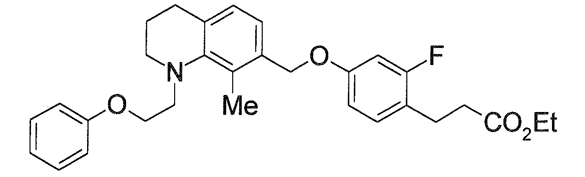 |
| 38 | 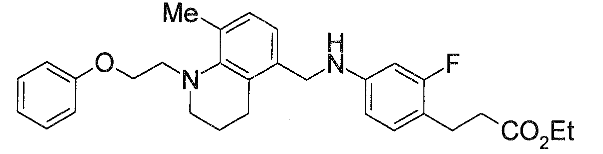 |
| 468 | 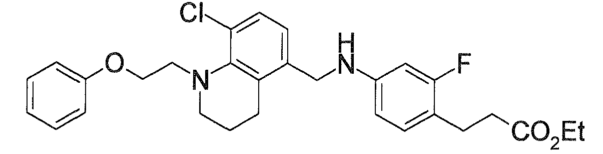 |

**[Table 108]**

| | |
|---|---|
| 469 | 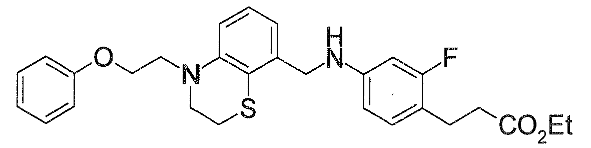 |
| 470 | 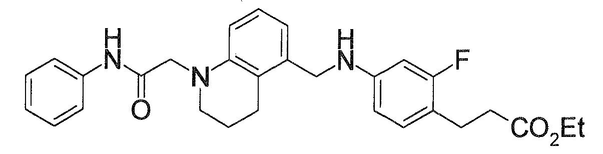 |
| 471 | 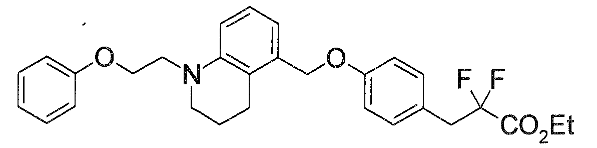 |
| 472 | 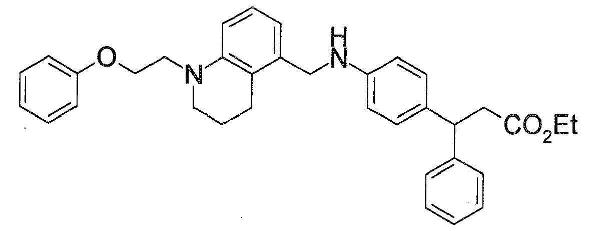 |
| 473 | 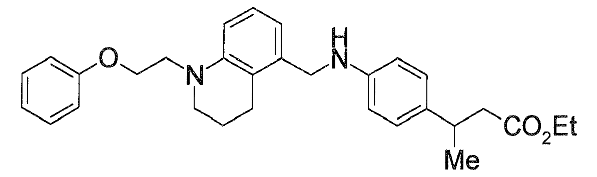 |
| 474 | 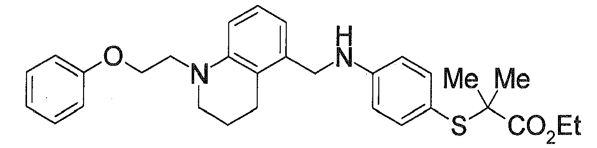 |
| 475 | 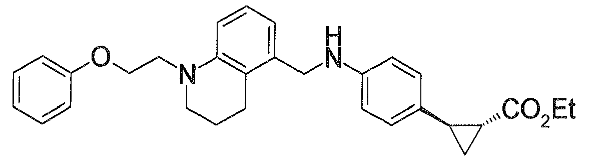 |

**[Table 109]**

| | |
|---|---|
| 476 | 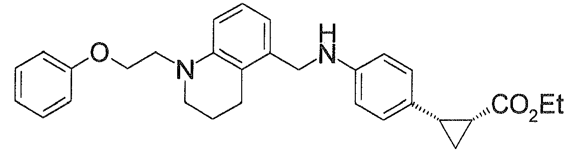 |
| 477 | 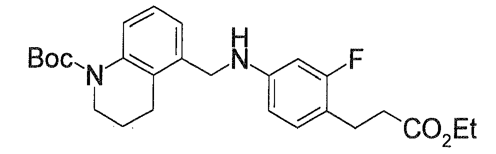 |
| 40 | 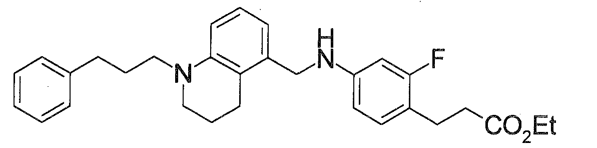 |
| 478 | 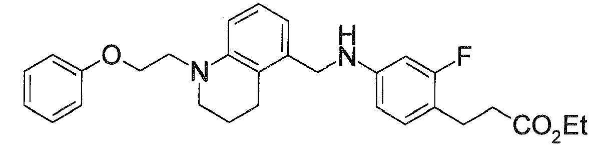 |

**[Table 110]**

| Ex | Syn | Data |
|---|---|---|
| 41 | 1 | FAB+:330 |
| 14 | 14 | FAB+:434 |
| 42 | 1 | NMR1:1.55-2.00(2H,m),2.05(2H,t,J=7.8Hz),2.64(2H,t,J=7.8Hz),2.68-2.93(2H,m),3.27-3.55(1H,m),3.69-4.20(1H,m),4.37-5.22(2H,m),6.26-6.76(2H,m),6.93-7.72(9H,m) FAB+:434 |
| 43 | 14 | NMR1:1.16-1.30(1H,m),1.38-1.54(1H,m),1.82-1.97(1H,m),2.05(2H,t,J=8.0Hz),2.25-2.40(1H,m),2.64(2H,t,J=8.0Hz),3.29-3.57(1H,m),3.95-4.17(1H,m),5.19(1H,d,J=13.0Hz),5.32(1H,d,J=13.0Hz),6.56-6.66(2H,m),7.05(1H,d,J=6.8Hz),7.17(1H,t,J=8.9Hz),7.26(1H,t,J=7.6Hz),7.45(1H,d,J=7.1H z),7.54-7.59(4H,m)7.69-7.76(1H,m) ESI-:468 |
| 44 | 1 | NMR1:1.73-1.86(2H,m),2.03(2H,t,J=8.0Hz),2.62(2H,t,J=8.0Hz),2.80(2H,t,J=6.6Hz),2.90-3.02(2H,m),4.10(2H,s),5.03(2H,s),6.49-6.60(2H,m),6.93(1H,t,J=7.5Hz),7.03(1H,d,J=7.1Hz),7.08(1H,t,J=9.0Hz),7.21-7.29(2H,m),7.34(2H,t,J=7.4Hz),7.43(2H,d,J=7.4Hz) FAB+:420 |
| 45 | 14 | FAB+:426 |
| 46 | 1 | FAB+:372 |
| 47 | 1 | FAB-:404 |
| 48 | 1 | FAB-:428 |
| 1 | 1 | NMR1:1.73-1.89(2H,m),2.04(2H,t,J=8.0Hz),2.58-2.69(4H,m),2.75(2H,t,J=7.6Hz),3.19(2H,t,J=5.7Hz),3.43(2H,t,J=7.7Hz),4.98(2H,s),6.53(1 H,d,J=7.5Hz),6.64-6.80(3H,m),6.86(1H,d,J=7.5Hz),7.16(1H,t,J=8.8Hz),7.19-7.25(3H,m),7.25-7.32(2H,m) FAB+:434 |
| 49 | 1 | FAB+:420 |
| 10 | 10 | NMR1:1.16-1.25(2H,m),1.26-1:35(1H,m),1.57-1.66(1H,m),1.73-1.90(2H,m),2.21(ddd,J=4.2,6.4,9.8Hz),2.70-3.04(6H,m),3.12-3.22(2H,m),4.17(2H,m),6.47(2H,d,J=7.8Hz),6.78-6.97(4H,m),7.10-7.32(6H,m) FAB+:427 |
| 50 | 1 | FAB+:322 |
| 51 | 1 | FAB+:365 |
| 52 | 1 | FAB+:420 |
| 53 | 1 | FAB+:358 |
| 54 | 1 | NMR1:2.05(2H,t,J=8.0Hz),2.64(2H,t,J=8.0Hz),2.95(2H,t,J=8.7Hz),3.29-3.42(2H.m),4.43(2H,s),4.90(2H,s),6.58-6.69(3H,m),7.01-7.09(2H,m),7.13(1H,t,J=8.8Hz),7.20-7.34(5H,m) ESI+:406 |

**[Table 111]**

| | | |
|---|---|---|
| 55 | 1 | FAB-:411 |
| 56 | 1 | FAB+:329 |
| 3 | 3 | NMR1:1.73-1.86(2H,m),2.03(2H,t,J=8.1Hz),2.56(2H,t,J=8.1Hz),2.76(2H,t,J=6.6Hz),2.88-3.02(4H,m),3.11-321(2H,m),4,12(2H,d,J=5.6Hz),6.14-6.30(3H,m),6.82(1H,t,J=7.4Hz),6.86-6.94(2H,m),7.10-7.26(6H,m) FAB+:419 |
| 57 | 1 | NMR1:0.85(3H,t,J=7.3Hz,),1.60-1.82(4H,m),2.01(2H,t,J=8.1Hz),2.54(2H,t,J=8.1Hz),2.63-2.80(4H,m)3.00-3.11(2H,m),4.11(2H,d,J=5.7Hz),6.13-6.26(3H,m),6.77-6.92(3H,m),7.12(1H,d,J=7.2Hz) FAB+:371 |
| 58 | 1 | NMR1:1.72-1.86(2H,m),2.03(2H,t,J=8.1Hz),2.54(2H,t,J=8.1Hz),2.80(2H,t,J=6.6Hz),2.91-3.00(2H,m),4.05(2H,s),4.21(2H,d,J=5.8Hz),6.12-6.28(3H,m),6.81-6.97(3H,m),7.16(1H,d,J=7.2Hz),7.27(1H,t,J=7.3Hz),7.37(2H,t,J=7.6Hz),7.50(H,d,J=7.4Hz ) FAB+:419 |
| 59 | 1 | FAB+:442 |
| 60 | 1 | FAB+:392 |
| 61 | 1 | ESI+:430 |
| 62 | 1 | FAB+:439 |
| 63 | 10 | ESI+:381 |
| 7 | 7 | ESI+:381 |
| 13 | 13 | ESI+:479 |
| 12 | 12 | ESI+:407 |
| 64 | 12 | ESI+:395 |
| 65 | 1 | FAB+:370 |
| 66 | 1 | FAB+:399 |
| 67 | 1 | ESI+:330 |
| 68 | 1 | ESI+:344 |
| 69 | 1 | NMR1:0.86(3H,t,J=7.4Hz),1.40-1.55(2H,m),1.75-1.87(2H,m),2.01-2.12(2H,m),2.58-2.70(4H,m),3.11-3.19(2H,m),3.19-3.26(2H,m),4.93(2H,s),6.46-6.51(1H,m),6.54-6.59(1H,m),6.65-6.71(1H,m),6.71-6.78(1H,m),6.81-6.86(1H,m),7.16-7.19(1H,m) ESI+:372 |
| 70 | 1 | NMR1:1.80-1.93(2H,m),1,97-2.09(2H,m),2.46-2.60(2H,m),2.62-2.74(2H,m),3.22-3.36(2H,m),3.98(2H,d,J=5.7Hz),4.45(2H,s),6.11(1H,t,J=5.7Hz),6.13-6.26(2H,m),6.42-6.49(1H,m),6.54-6.62(1H,m),6.77-6.91(2H,m),7.17-7.32(5H,m) ESI+:372 |
| 71 | 1 | ESI+:406 |
| 72 | 1 | ESI+:388 |

**[Table 112]**

| | | |
|---|---|---|
| 73 | 1 | ESI+:426 |
| 74 | 1 | ESI+:372 |
| 75 | 1 | ESI-:327 |
| 76 | 1 | NMR1:1.81-1.90(2H,m),1.98-2.06(2H,m),2.47-2.59(2H,m),2.60-2.69(2H,m),2.80(3H,s),3.12-3.18(2H,m),4.07(2H,d,J=5.8Hz),6.16(1H,t,J=5.8Hz),6.20-6.27(1H,m),6.27-6.32(1H,m),6.46-6.52(1H,m),6.54-6.58(1H,m),6.77-6.82(1H,m),6.85-6.94(1H,m) ESI+:343 |
| 77 | 1 | NMR1:1.73-1.84(2H,m),1,96-2.06(2H,m),248-2.74(6H,m),3.14-322(2H,m),3.32-3.44(2H,m),4.12(2H,d,J=5.5Hz),6.22-6.37(3H,m),6.43-6.48(1H,m),6.60-6.66(1H,m),6.78-6.84(1H,m),6.87-6.95(1H,m),7.11-7.31(5H,m) ESI+:433 |
| 78 | 1 | NMR1:1.79-1.88(2H,m),1,96-2.05(2H,m),2.33-2.47(2H,m),2.48-2.57(2H,m),2.58-2.66(2H,m),3.18-3.26(2H,m),3.42-3.52(2H,m),4.09(2H,d,J=6.4Hz),6.15-6.32(3H,m),6.47-6.55(2H,m),6.79-6.92(2H,m) ESI+:425 |
| 79 | 1 | NMR1:0.90(3H,t,J=7.4Hz),1.52-1.69(2H,m),1.84-2.1(2H,m),2.48(2H,t,J=7.5Hz),2.68-2.82(4H,m),3.18-3.38(4H,m),4.99(2H,s),6.68-7.02(4H,m),7.03-7.15(1H,m),7.21(1H,t,J=8.8Hz) FAB+:372 |
| 80 | 1 | FAB+:420 |
| 81 | 1 | NMR1:1.77-1.91(2H,m),2.48(2H,t,J=7.5Hz),2.68(2H,t,J=6.4Hz),2.77(2H,t,J=7.6Hz),2.83(2H,t,J=7.7Hz ),3.23(2H,t,J=5.3Hz),3.49(2H,t,J=7.8Hz),4.96(2H,s),6.66-6.83(3H,m),6.86(1H,dd,J=2.5,12.2Hz),7.15-7.24(2H,m),7.25-7.35(4H,m) FAB+:434 |
| 82 | 1 | ESI+:406 |
| 83 | 1 | ESI+:330 |
| 84 | 1 | ESI-:405 |
| 85 | 1 | NMR1:0.83(3H,t,J=7:3Hz),1.34-1.51(2H,m),1,76-1.88(2H,m),1.96-2.07(2H,m),2.47-2.67(4H,m),3.08-3.1(2H,m),3.17-3.25(2H,m),4.06(2H,brs),6.13-6.33(3H,m),6.38-6.46(1H,m),6.48-6.55(1H,m),6.73-6.80(1H,m),6.84-6.93(1H,m) ESI+:371 |
| 86 | 1 | NMR1:1,84-1.95(2H,m),1.96-2.07(2H,m),2.47-2.59(2H,m),2.66-2.78(2H,m),3.51-3.60(2H,m),3.95-4.02(2H,m),6.08-6.22(3H,m),6.62-6.67(1H,m),6.68-6.72(1H,m),6.85-6.91(1H,m),6.94-7.05(2H,m),7.08-7.15(2H,m),7:22-7.30(2H,m) ESI+:405 |

**[Table 113]**

| | | |
|---|---|---|
| 87 | 1 | NMR1:0.91(3H,t,J=7.4Hz),1.56-1.72(2H,m),1.92-2.08(2H,m),2.42(2H,t,J=7.7Hz),2.67(2H,t,J=7.7Hz),2.72-2.81(2H,m),3.21-3.43(4H,m),4.14(2H,s),6.32-6.48(2H,m),6.77-7.27(4H,m) ESI+:371 |
| 88 | 1 | NMR1:1.91-2.04(2H,m),2.44(2H,t,J=7.71Hz),2.70(2H,t,J=7.7Hz),2.75(2H,t,J=6.3Hz),3.34(2H,t,J=5.5Hz ),4.15(2H,s),4.51(2H,s),6.39-6:76(4H,m),6.90(1H,t,J=7.7Hz),7.04(1H,t,J=8.7Hz),7.20-7.38(5H,m) ESI+:419 |
| 89 | 1 | NMR1:1.84-1.98(2H,m),2.43)2H,t,J=7.6Hz),2.63-2.77(4H,m),2.88(2H,t,J=7.6Hz),3.19-3.37(2H,m),3:52(2H,t,J=7.8Hz),4.15(2H,s),6.37-6.59(2H,m),6.66-6.97(2H,m),6.98-7.13(2H,m),7.17-7.35(5H,m) ESI+:433 |
| 90 | 1 | NMR1:1.90-2.01(2H,m),2.43(2H,t,J=7.6Hz),2.68(2H,t,J=7.6Hz),2.78(2H,t,J=6.5Hz),3.56(2H,t,J=5.6Hz ),4.15(2H,s),6.36-6.50(2H,m),2.57(1H,d,J=7.7Hz),6.72(1H,d,J=7.7Hz),6.85(1H,t,J=7.7Hz),6.99(1H,t,J=8.6H z),7.07(1H,t,J=73Hz),7.15-7.22(2H,m),735(2H,t,J=7.9Hz) ESI+:405 |
| 91 | 1 | ESI+:329 |
| 92 | 1 | ESI+:371 |
| 93 | 1 | ESI+:343 |
| 94 | 1 | ESI+:385 |
| 95 | 1 | NMR1:1.69-1:87(2H,m),2.04-2.20(2H,m),2.44-2.71(4H,m),3.14-3.53(9H,m),3.96-4.12(2H,m),6.11-6.34(3H,m),6.39-6.47(1H,m),6.51-6.59(1H,m),6.72-6.83(1H,m),6.84-6.98(1H,m) ESI+:387 |
| 96 | 1 | ESI-:371 |
| 97 | 1 | NMR1:1,84-2.02(5H,m),2.07-2.27(2H,m),2.54-2.64(2H,m),2.67-2.86(2H,m),3.22-3.54(2H,m),3.81-3.92(2H,m),5.84-5.90(1H,m),5.98-6.20(3H,m),6.45-6.53(1H,m),6.80-6.95(2H,m),7.08-7.32(4H,m) ESI+:419 |
| 98 | 1 | ESI+:418 |
| 99 | 1 | FAB+:402 |
| 100 | 1 | NMR1:1.89-2.06(4H,m),2.58-2.69(2H,m),2.77(2H,t,J=6.4Hz),3.57(2H,t,J=5.7Hz),4.87(2H,s),6.53(2H,m),6.64-6.74(2H,m),7.02(1H,d,J=7.7Hz),7.09(1H,t,J=7.3Hz),7.18(2H,d,J=7.8Hz),7.33(2H,t,J=7.8H z) FAB+:424 |

**[Table 114]**

| | | |
|---|---|---|
| 101 | 1 | NMR1:1.84-1.97(2H,m),2.04(2H,t,J=7.8Hz),2.53(2H,t,J=7.8Hz),2.72(2H,t,J=6.3Hz),3.54(2H,t,J=5.6Hz ),4.16(2H,d,J=5.9Hz),6.27(1H,d,J=8.6Hz),6.52(1H,t,J=6.0Hz),6.63(1H,d,J=7.7Hz),6.69(1H ,s),6.94(1H,d,J=7.7Hz),7.02(1H,t,J=7.3Hz),7.13(2H,d,J=7.8Hz),7.19(1H,dd,J=2.2,8.5Hz),7. 27(2H,t,J=7.8Hz),7.71(1H,d,J=2.2Hz) ESI+:388 |
| 102 | 1 | FAB+:402 |
| 103 | 1 | ESI+:44.7 |
| 104 | 1 | NMR1:1.84-1.95(2H,m),2.43(2H,t,J=7.6Hz),2.62-2.76(4H,m),3.39(2H,t,J=5.3Hz),3.69(2H,t,J=5.5Hz),4.13(2H,s),4.16(2H,t,J=5:6Hz),6.45-6.60(2H,m),6.67(1H,d,J=7.3Hz),6.74(1H,d,J=8.2Hz),6.89-6.96(3H,m),6.99(1H,t,J=7.9Hz),7.04(1H,t,J=8.7Hz),7.23-7.32(2H,m) ESI+:449 |
| 105 | 1 | NMR1:1,76-1.89(2H,m),2.40-2.48(2H,m),2.59-2.74(4H,m),3.05-3.18(2H,m),3.18-3.31(4H,m),3.37-3.51(2H,m),3.66-4.12(6H,m),4.22(2H,brs,),6.52-6.58(2H,m),6.67-6.78(2H,m),6.81-6.86(2H,m),6.96-7.01(2H,m),7.04-7.13(2H,m),11.4(brs,1H) ESI+:442 |
| 106 | 1 | NMR1:0.89(3H,t,J=7.4Hz),1.57-1.77(4H,m),1,97-2.08(2H,m),2.17(3H,s),2.51-2.74(6H,m),2.95-3.05(2H,m),4.03-4.11(2H,m),5.93-6.01(1H,m),6.19-6.34(2H,m),6.72-6.85(2H,m),6.87-6.96(1H,m) ESI+:385 |
| 107 | 1 | NMR1:1.85-1.96(2H,m),2.12-2.21(2H,m),2.32(3H,s),2.65-2.75(2H,m),2.80-2.90(2H,m),2.98-3.14(4H,m),3.23-3.32(2H,m),4.16-4.26(2H,m),6.07-6.15(1H,m),6.35-6.48(2H,m),6.89-6.99(2H,m),7.02-7.10(1H,m),7.30-7.37(1H,m),7.38-7.49(4H,m) ESI+:447 |
| 108 | 1 | NMR1:1.68-1.90(2H,m),2.43(2H,t,J=7.6Hz),2.60-2.74(4H,m),2.98-3.10(1H,m),3.28-3.47(3H,m),4.11(2H,s),4.86(1H,t,J=6.3Hz),6.40-6.52(2H,m),6.65(1H,d,J=7.3Hz),6.72(1H,d,J=8.1Hz),6.96-7.05(2H,m),7.23-7.29(1H,m),7.30-7.42(4H,m) ESI+:449 |
| 109 | 1 | NMR1:1.73(2H,tt,J=5.4,6.6Hz),1.82(3H,s),2:02(2H,t,J=7.9Hz),2.57(2H,t,J=7.9Hz),2.71(2 H,t,J=6.6Hz),3.66(2H,t,J=5.4Hz),4.07(2H,brs),6.03(1H,brs),6.18-6.35(2H,m),6.07-6.76(2H,m),6.78-6.86(1H,m),6.88-6.96(1H,m),6.96-7.06(2H,m),7.13-7.23(2H,m) ESI+:419 |

**[Table 115]**

| | | |
|---|---|---|
| 110 | 1 | NMR1:1.97-2.05(2H,m),2.47-2.59(2H,m),2.64-2.73(2H,m),2.95-3.03(2H,m),3.99(2H,d,5.4Hz),6.04-6.18(3H,m),6.19-6.24(1H,m),6.81-6.89(1H,m),6.91-6.99(1H,m),7.13-7.24(3H,m),7.37-7.51 (3H, m) ESI+:419 |
| 111 | 1 | NMR1:2.05(2H,tt,J=5.5,6.0Hz),2.44(2H,t,J=7.5Hz),2.68(2H,t,J=7.5Hz),2.89(2H,t,J=6.0Hz ),3.81(2H,t,J=5.5Hz),3.90-4.83(5H,m),6.25-6.35(2H,m),6.81-6.89(1H,m),6.95-7.04(1H,m),7.42-7.51(3H,m),7.51-7.60(2H,m),7.75-7.84(1H,m) ESI+:406 |
| 112 | 1 | NMR1:1.96-2.07(2H,m)2.47-2.61(2H,m),3.62-3.69(2H,m)3.95-4.04(2H,m),4.14-4.22(2H,m),6.08-625(3H,m),6.68-6.73(1H,m),6.75-6.80(1H,m),6.85-6.93(2H,m),7.05-7.07(1H,m),7.11-7.17(2H,m),7.25-7.32(2H,m) ESI-:405 |
| 113 | 1 | NMR1:1.78-1.88(2H,m),2.03(2H,t,J=8.1Hz),2.57(2H,t,J=8.1Hz),2.66(2H,t,J=6.3Hz),2.72(2H,t,J=7.7Hz ),3.18(2H,t,J=5.4Hz),3.40(2H,t,J=7.7Hz),3.72(3H,s),4.03(2H,d,J=5.3Hz),5.93(1H,t,J=5.3H z),6.26(1H,dd,J=2.1,13.1Hz),6.31(1H,dd,J=2.1,8.2Hz),6.54(1H,d,J=7.5Hz),6.60(1H,d,J=8. 2Hz),6.82-6.89(2H,m),6.92(1H,t,J=8.7Hz),6.95(1H,t,J=7.8Hz),7.12-7.25(2H,m) ESI+:463 |
| 114 | 1 | NMR1:1.79-1.89(2H,m),2.00-2.10(2H,m),2.58(2H,t,J=8.1Hz),2.66(2H,t,J=6.4Hz),2.76(2H,t,J=7.6Hz),3.19(2H,t,J=5.6Hz ),3.45(2H,t,J=7.6Hz),3.74(3H,s),4.03(2H,d,J=5.4Hz),5.94(1H,t,J=5.4Hz),6.27(1H,dd,J=2.1, 13.2Hz),6.31(1H,dd,J=2.1,8.3Hz),6.54(1H,d,J=7.4Hz),6.62(1H,d,J=8.2Hz),6.77(1H,dd,J=2 .3,7.7Hz),6.81-6.87(2H,m),6.92(1H,t,J=8.7Hz),6.96(1H,t,J=7.8Hz),7.21(1H,t,J=8.0Hz) ESI+:463 |
| 115 | 1 | NMR1:1.80-1.90(2H,m),1.97-2.07(2H,m),2.57(2H,t,J=8.0Hz),2.66(2H,t,J=6.3Hz),2:78(2H,t,J=7.7Hz),3.22(2H,t,J=5.4Hz ),3.37(2H,t,J=7.7Hz),3.84(3H,s),4.03(2H,s),6.27(1H,dd,J=2.2,13.1Hz),6.32(1H,dd,J=2.2,8. 3Hz),6.53(1H,d,J=7.3Hz),6.71(1H,d,J=8.2Hz),6.84-7.01(4H,m),7.13-7.25(2H,m) FAB+:463 |
| 116 | 1 | NMR1:1.63-1.77(2H,m),2.44-2.56(2H,m),2.70(2H,t,J=6.4Hz),2.74-2.87(4H,m),4.12(2H,s),5.04(2H,s),6.65(1H,d,J=7.7Hz),6.79(1H,t,J=7.7Hz),6.86(1H,d,J=7.7 Hz),7.14(2H,d,J=8.1Hz),7.16-7.21(3H,m),7.24-7.33(4H,m) FAB+:402 |

**[Table 116]**

| | | |
|---|---|---|
| 117 | 1 | NMR1:0.87(3H,t,J=7.3Hz),1.49-1.69(2H,m),1.69-1.84(2H,m),2.01-2.16(2H,m),2.59-2.77(6H,m),2.93-3.02(2H,m),4.26(2H,s),6.27(1H,d,J=8.0Hz),6.31(1H,d,J=7.5Hz),6.69(1H,t,J=7.7Hz),6.98-7.10(2H,m),7.24(1H,t,J=8.1Hz) ESI+:371 |
| 11 | 11 | NMR1:2.02-2.17(2H,m),2.63-2.78(2H,m),3.25-333(2H,m),3.97-4.11(4H,m),4.34(2H,s),5.57(1H,t,J=6.2Hz),5.76(1H,dd,J=2.4,8.4Hz),5.98(1H,d,J=2.4Hz),6. 40(1H,d,J=8.4Hz),6.93(1H,s),6.94-6.99(1H,m),7.10-7.36(6H,m) ESI+:421 |
| 118 | 11 | ESI+:373 |
| 119 | 1 | FAB+:434 |
| 120 | 1 | FAB+:386 |
| 121 | 1 | ESI+:358 |
| 122 | 1 | FAB+:386 |
| 123 | 1 | ESI+:372 |
| 124 | 1 | NMR1:0.67(3H,t,J=7.4Hz),138-1.52(2H,m),1.63-1.75(2H,m),2.07-2.18(2H,m),2.66(2H,t,J=6.4Hz),2.76(2H,t,J=8.0Hz),2.84-3.03(4H,m),4.98(2H,s),6.58(1H,d,J=7.7Hz),6.68(1H,t,J=7.7Hz),6.77(1H,d,J=7.7Hz),7.11-7.21(2H,m),7.30(1H,t,J=7.8Hz) FAB+:372 |
| 125 | 1 | FAB+:426 |
| 126 | 1 | FAB+:384 |
| 127 | 1 | FAB+:344 |
| 128 | 1 | FAB+:420 |
| 129 | 1 | FAB+:480 |
| 130 | 1 | ESI+:330 |
| 131 | 1 | FAB+:372 |
| 132 | 1 | FAB+:398 |
| 133 | 1 | FAB+:386 |
| 134 | 1 | FAB+:402 |
| 135 | 1 | FAB+:400 |
| 136 | 1 | FAB+:414 |
| 137 | 1 | NMR1:1.63-1.76(1H,m)2.05-2.25(3H,m),2.68-2.84(4H,m),3.19-3.50(1H,m),4.03-4.23(1H,m),4.47(1H,d,J=12.7Hz),4.79(1H,d,J=12.7Hz),6.65(1H,d,J=7.8Hz),6.73-6.85(2H,m),6.88(1Hd,J=7.8Hz),7.03(1H,t,J=7.8Hz),7.14-7.28(5H,m),7.32-7.40(1H,m) FAB+:434 |
| 138 | 1 | FAB+:502 |
| 139 | 1 | FAB+:408 |
| 140 | 1 | ESI-:468 |

**[Table 117]**

| | | |
|---|---|---|
| 141 | 1 | FAB-:420 |
| 142 | 1 | FAB+:436 |
| 143 | 1 | ESI+:372 |
| 144 | 1 | ESI+:434 |
| 145 | 1 | ESI+:420 |
| 146 | 1 | ESI+:421 |
| 147 | 1 | ESI+:421 |
| 148 | 1 | ESI+:421 |
| 149 | 1 | FAB+:488 |
| 150 | 1 | FAB+:450 |
| 151 | 1 | FAB+:385 |
| 152 | 1 | FAB+:413 |
| 153 | 1 | NMR1:1.84-229(2H,m),2.47-2.57(2H,m),2.73-2.93(4H,m),3.11-3.33(2H,m),4.39(2H,s),4.43-5.02(2H,bs),6.56-6.71(2H,m),7.06-7.34(4H,m),7.37-7.49(3H,m),7.57-7.69(2H,m) ESI+:419 |
| 154 | 1 | FAB+:433 |
| 155 | 1 | FAB-:459 |
| 156 | 1 | ESI+:501 |
| 157 | 1 | ESI+:501 |
| 158 | 1 | ESI+:501 |
| 159 | 1 | ESI+:467 |
| 160 | 1 | ESI+:467 |
| 161 | 1 | NMR1:1.81-1.92(2H,m),1.98-2.09(2H,m),2.53-2.61(2H,m),2.63-2.71(2H,m),3.24-3.47(2H,m),3.64(2H,t,J=5.7Hz),4.03(2H,d,J=5.4Hz),4.10(2H,t,J=5.7Hz),5.93(1H,t,J=5.4Hz ),6.25(1H,dd,J=2.1,13.1Hz),6.30(1H,dd,J=2.1,8.3Hz),6.54(1H,d,J=7.4Hz),6.61(1H,d,J=8.2 Hz),6.87-6.97(4H,m),7.05-7.14(2H,m) ESI+:467 |
| 162 | 1 | ESI+:483 |
| 163 | 1 | NMR1:1.80-1.92(2H,m),1.97-2.08(2H,m),2.53-2.61(2H,m)2.67(2H,t,J=6.3Hz),3.30-3.40(2H,m),3.65(2H,t,J=5.6Hz),4.03(2H,d,J=5.3Hz),4.16(2H,t,J=5.6Hz),5.93(1H,t,J=5.3Hz ),6.25(1H,dd,J=2.013.1Hz),6.30(1H,dd,J=2.0,8.3Hz),6.54(1H,d,J=7.5Hz),6.61(1H,d,J=8.6 Hz),6.86-7.04(5H,m),7.29(1H,t,J=8.1Hz) ESI+:483 |
| 164 | 1 | ESI+:483 |
| 165 | 1 | ESI+:434 |

**[Table 118]**

| | | |
|---|---|---|
| 166 | 1 | NMR1:1.70-1.78(2H,m),1.85(3H,s),2.41(2H,t,J=7.6Hz),2.64-2.74(4H,m),3.62-3.67(2H,m),3.64(3H,s),3.80-4.50(2H,br),4.13(2H,s),6.22-6.44(5H,m),6.93-7.10(4H,m) FAB+:449 |
| 167 | 1 | ESI+:463 |
| 168 | 1 | ESI+:448 |
| 169 | 3 | FAB+:439 |
| 170 | 3 | ESI+:399 |
| 171 | 3 | FAB+:401 |
| 172 | 3 | ESI+:371 |
| 173 | 3 | ESI+:399 |
| 174 | 3 | NMR1:1.10-1.44(6H,m),1.71-1.90(1H,m),2.09-2.29(1H,m),2.42(2H,t,J=8.0Hz),2.54(3H,s),2.67(2H,t,J=8.0Hz),2.82-2.90(2H,m),3.42-3.94(3H,m),4.18-4.30(2H,m),6.27-7.41(8H,m),11.6-11.9(1H,brs) ESI+ : 385 |
| 175 | 4 | NMR1:027-0.55(2H,m),0.60-0.77(2H,m),126-1.41(1H,m),1.87-2.33(2H,m),2.42(2H,t,J=8.0Hz),2.53(3H,s),2.68(2H,t,=8.0Hz),2.85-2.96(2H,m),3.21-3.50(3H,m),3.81-4.01(1H,m),4.15-4.31(2H,m),6.00-7.54(8H,m),11.9-12.3(1H,brs) ESI+ : 397 |
| 176 | 3 | ESI+:479 |
| 177 | 3 | ESI+:479 |
| 178 | 3 | ESI+:479 |
| 179 | 3 | ESI+:371 |
| 180 | 1 | NMR1:1.64-1.87(2H,m),2.42(2H,t,J=7.6Hz),2.59-2.73(4H,m),2.99-3.08(1H,m),3.14(3H,s),3.24-3.37(2H,m),3.50(1H,dd,J=7.8,15.0Hz),4.08(2H,s),4.47(1H,dd,J=4.8,7.8Hz),6.37-6.49(2H,m),6.54-6.65(2H,m),6.91-7.03(2H,m),7.28-7.43(5H,m) ESI+:463 |
| 181 | 1 | NMR1:1.87-1.99(2H,m)2.77(2H,t,J=6.4Hz),3.32(2H,t,J=17.3Hz),3.57(2H,t,J=11.4Hz),4.86(2H,s),6.67-6.74(2H,m),6.84(2H,d,J=8.7Hz),7.02(1H,d,J=7.5Hz),7.08(1H,t,J=7.3Hz),7.13(2H,t,J=8.7 Hz),7.17(2H,d,J=7.6Hz),7.33(2H,t,J=7.6Hz) ESI+:424 |
| 182 | 4 | NMR1:1.85-222(2H,m),2.34-2.54(7H,m),2.66(2H,t,J=7.6Hz),2.77-2.94(2H,m),3.09-3.64(4H,m),4.18(2H,s),6.26-6.42(2H,m),6.96(1H,t,J=8.6Hz),6.98-7.22(2H,m),7.27-7.39(3H,m),7.42-7.47(1H,m) ESI+:481 |
| 183 | 4 | ESI+:481 |

**[Table 119]**

| | | |
|---|---|---|
| 184 | 4 | NMR1:1.83-2.23(2H,m),2.41(2H,t,J=7.6Hz),2.44-2.53(5H,m),2.66(2H,t,J=7.6Hz),2.79-2.97(2H,m),3.11-3.92(4H,m),4.21(2H,s),6.27-6.45(2H,m),6.97(1H,t,J=8.6Hz),7.08(2H,dt,J=2.2,8.6Hz),7.13-7.29(3H,m),7.32-7.42(1H,m) ESI+:465 |
| 185 | 4 | ESI+:465 |
| 186 | 4 | NMR1:1.84-2.19(2H,m),2.29-2.55(7H,m),2.66(2H,t,J=7.7Hz),2.75-2.96(2H,m),3.09-3.72(4H,m),4.18(2H,s),6.23-6.43(2H,m),6.95(1H,t,J=8.7Hz),6.98-7.27(2H,m),7.49-7.78(4H,m) ESI+:515 |
| 187 | 4 | ESI+:515 |
| 188 | 4 | NMR1:1.84-2.25(2H,m),2.40(2H,t,J=7.7Hz),2.43-2.53(5H,m),2.65(2H,t,J=7.7Hz),2.77-2.97(2H,m),3.01-3.55(4H,m),3.82(3H,s),4.20(2H,s),6.23-6.42(2H,m),6.90(1H,t,J=7.4Hz),6.95(1H,t,J=8.7Hz),7.00(1H,d,J=8.1Hz),7.03-7.32(4H,m) ESI+:477 |
| 189 | 4 | NMR1:1.81-2.27(2H,m),2.40(2H,t,J=7.7Hz),2.43-2.52(5H,m),2.66(2H,t,J=7.7Hz),2.77-2.95(2H,m),3.06-3.55(4H,m),3.75(3H,s),4.20(2H,s),6.26-6.42(2H,m),6.78-6.85(1H,m),6.86-6.93(2H,m),6.95(1H,t,J=8.7Hz),7.01-7.21(2H,m),7.24(1H,t,J=7.8Hz) ESI+:477 |
| 190 | 4 | NMR1:1.82-2.29(2H,m),2.40(2H,t,J=7.6Hz),2.43-2.53(5H,m),2.66(2H,t,J=7.6Hz),2.77-2.97(2H,m),3.01-3.56(4H,m),3.73(3H,s),4.20(2H,s),6.24-6.42(2H,m),6.89(2H,d,J=8.6Hz),6.95(1H,t,J=8.6Hz),7.00-7.31(4H,m) ESI+:477 |
| 5 | 5 | NMR1:1.91-2.23(2H,m),2.42(2H,t,J=7.6Hz),2.45-2.53(5H,m),2.68(2H,t,J=7.6Hz),2.81-2.97(2H,m),3.34-3.91(2H,m),4.22(2H,s),4.45-4.68(2H,m),6.30-6.54(2H,m),6.93-7.10(3H,m),7.12-7.28(2H,m),7.35(1H,dt,J=1.5,7.8Hz),7.47(1H,dd,J=1.5,7.9Hz) ESI+:497 |
| 191 | 5 | ESI+:497 |
| 192 | 5 | ESI+:497 |
| 193 | 5 | NMR1:1.89-2.20(2H,m),2.36-2.54(7H,m),2.68(2H,t,J=7.6Hz),2.79-2.95(2H,m),3.30-3.87(2H,m),4.21(2H,s),4.42-4.61(2H,m),631-6.53(2H,m),6.92-7.33(7H,m) ESI+:481 |
| 194 | 5 | ESI+:481 |
| 195 | 5 | ESI+:481 |
| 196 | 5 | ESI+:531 |
| 197 | 5 | ESI+:531 |

**[Table 120]**

| | | |
|---|---|---|
| 198 | 5 | ESI+:531 |
| 199 | 5 | NMR1:1.80-1.91(2H,m),2.16(3H,s),2.43(2H,t,J=7.6Hz),2.69(2H,t,J=7.6Hz),2.77(2H,t,J=6.6Hz),3.14-3.25(2H,m),3.30-3.44(2H,m),4.18(2H,s),4.57(2H,t,J=6.6Hz),6.27(1H,t,J=2.1Hz),6.41-6.53(2H,m),6.93(1H,d,J=7.9Hz),6.97-7.07(2H,m),7.51(1H,d,J=2.1Hz),7.83(1H,d,J=2.1Hz) ESI+:437 |
| 200 | 5 | NMR1:2.01-2.14(2H,m),2.37-2.47(5H,m),2.68(2H,t,J=7.6Hz),2.87(2H,t,J=6.6Hz),3.47-3.57(2H,m),3.62-3.72(2H,m),4.22(2H,s),4.72(2H,t,J=5.2Hz),6.37-6.48(2H,m),6.92(1H,d,J=8.3Hz),7.00(1H,t,J=8.7Hz),7.03-7.09(2H,m),7.21(1H,d,J=7.9Hz),7.79(1H,ddd,J=2.0,7.1,8.8Hz),8.21(1H,dd,J=1.7,5.0Hz) ESI+:464 |
| 201 | 5 | NMR1:1.88-2.19(2H,m)2.35-2.54(7H,m),2.67(2H,t,J=7.6Hz),2.79-2.94(2H,m),3.28-3.72(2H,m),3.79(3H,s),4.20(2H,s),4.32-4.49(2H,s),6.29-6.44(2H,m),6.87-7.28(7H,m) ESI+:493 |
| 202 | 5 | NMR1:1.88-2.18(2H,m),2.35-2.54(7H,m),2.67(2H,t,J=7.7Hz),2.79-2.95(2H,m),3.28-3.70(2H,m),3.75(3H,s),4.20(2H,s),4.33-4.51(2H,m),6.31-6.46(2H,m),6.53-6.63(3H,m),6.93-7.28(4H,m) ESI+:493 |
| 203 | 5 | NMR1:1.89-2.18(2H,m),2.35-2.55(7H,m),2.67(2H,t,J=7.6Hz),2.79-2.93(2H,m),3.32-3.68(2H,m),3.71(3H,s),4.20(2H,s),4.29-4.44(2H,m),6.30-6.45(2H,m),6.84-7.28(7H,m) ESI+:493 |
| 204 | 5 | ESI+:547 |
| 205 | 5 | ESI+:547 |
| 206 | 5 | ESI+:547 |
| 207 | 3 | NMR1:1.93-2.05(2H,m),2.42(2H,t,J=7.6Hz),2.67(2H,t,J=7.6Hz),2.79(2H,t,J=6.5Hz),3.93(2H,t,J=6.1Hz ),4.25(2H,s),6.33-6.48(2H,m),6.98(1H,t,J=8.7Hz),7.09-7.22(3H,m),7.28(1H,dd,J=2.1,6.8Hz),7.38(1H,d,J=8.9Hz),7.93-8.03(1H,m),8.21(1H,dd,J=1.4,5.9Hz) ESI+:406 |
| 208 | 3 | NMR1:1.82-1.98(2H,m),2.42(2H,t,J=7.6Hz),2.67(2H,t,J=7.6Hz),2.73(2H,t,J=6.6Hz),3.70(2H,t,J=6.0Hz ),4.20(2H,s),4.94(2H,s),6.31(2H,m),6.73-6.88(2H,m),6.93(1H,t,J=7.3Hz),6.98(1H,t,J=8.6Hz),7.09-7.17(2H,m),7.22-7.30(2H,m),7.37-7.52(1H,m) FAB+:463 |

**[Table 121]**

| | | |
|---|---|---|
| 6 | 6 | NMR1:1.65-1.76(2H,m),1.78(3H,s),2.43(2H,t,J=7.8Hz),2.63-2.80(4H,m),3.55-3.68(2H,m),4.15(2H,s),6.35-6.56(2H,m),6.65-6.77(2H,m),6.94-7.10(5H,m) ESI+:437 |
| 209 | 6 | NMR1:1.71-1.82(2H,m),1.85(3H,s)2.45(2H,t,J=7.9Hz),2.62-2.80(4H,m),3.58-3.74(2H,m),4.24(2H,s),6.34-6.53(2H,m),6.54-6.74(3H,m),6.97-7.24(4H,m) ESI+:437 |
| 210 | 6 | NMR1:1.59-1.72(2H,m),1.76(3H,s),2.42(2H,t,J=7.5Hz),2.68(2H,t,J=7.8Hz),2.75(2H,t,J=6.7Hz),3.51-3.59(2H,m),3.69(3H,s),3.74-4.60(4H,m),6.29-6.48(2H,m),6.67(2H,d,J=8.9Hz),6.78(2H,d,J=8.9Hz),6.91-7.06(3H,m) ESI+:449 |
| 211 | 6 | ESI+:487 |
| 212 | 6 | ESI+:487 |
| 213 | 6 | ESI+:453 |
| 214 | 6 | NMR1:1.73-1.83(2H,m),1.84(3H,s),2.42(2H,t,J=7.7Hz)2.62-2.73(4H,m),3.64-3.80(2H,m),4.16(2H,s),6.31-6.47(2H,m),6.92(1H,d,J=7.9Hz),6.99(1H,t,J=8.7Hz),7.02-7.17(3H,m),7.21(1H,d,J=7.8Hz),7.35(1H,t,J=8.0Hz) ESI+:444 |
| 215 | 6 | NMR1:1.65-1.77(2H,m),1.80(3H,s)2.20(3H,s)2.44(2H,t,J=7.7Hz),2.65-2.77(4H,m),3.59-3.67(2H,m),4.20(2H,s),6.43(1H,d,J=8.0Hz),6.50-6.62(3H,m),6.64(1H,t,J=7.5Hz),6.95-7.13(4H,m) ESI+:433 |
| 216 | 6 | NMR1:1.76-1.87(2H,m),1.91(3H,s),2.45(2H,t,J=7.7Hz),2.66(2H,t,J=6.8Hz),2.72(2H,t,J=7.6Hz),3.59-3.78(2H,m),4.26(2H,s),6.10-b.41(2H,m),6.50-6.72(3H,m),7.00-7.13(2H,m),7.18(1H,d,J=7.8Hz) ESI+:455 |
| 217 | 6 | NMR1:1.69-1.80(2H,m),1.84(3H,s),2.44(2H,t,J=7.6Hz),2.64-2:76(4H,m),3.58-3.69(2H,m),4.20(2H,s),6.36-6.47(1H,m),6.48-6.64(2H,m),6.68-6.80(1H,m),6.99-7.14(3H,m),7.14-7.25(1H,m) ESI+:455 |
| 218 | 6 | NMR1:1.72-1.85(2H,m),1.90(3H,s),2.43(2H,t,J=7.7Hz),2.63-2.75(4H,m),3.58-3.73(2H,m),4.21(2H,s),6.40-6.60(4H,m),6.98-7.08(2H,m),7.13(1H,d,J=7.8Hz) ESI+:473 |

**[Table 122]**

| | | |
|---|---|---|
| 219 | 6 | NMR1:1.69-1.80(2H,m),1.83(3H,s),1.96-2.07(2H,m),2.48-2.62(2H,m),2.65-2.73(2H,m),3.61-3.70(2H,m),4.05-4.12(2H,m),5.98-6.06(1H,m),6.20-6.33(2H,m),6.70(2H,d,J=8.9Hz),6.87-7.06(3H,m),7.20(2H,d,J=8.9Hz) ESI-:451 |
| 220 | 7 | ESI+:385 |
| 221 | 7 | ESI+:415 |
| 8 | 8 | ESI+:463 |
| 222 | 4 | NMR1:1.17(3H,t,J=7.0Hz),1.88-224(2H,m),2.38-2.48(5H,m)2.67(2H,t,J=7.6Hz),2.84-2.94(2H,m),324-3.98(8H,m),4.23(2H,s),6.32-6.47(2H,m),6.94-7.03(1H,m),7.06-7.14(1H,m),7.21-7.34(1H,m) ESI+ : 415 |
| 223 | 4 | NMR1:1.85-2.32(7H,m),2,42(2H,t,J=7.7Hz),2.67(2H,t,J=7.7Hz),2.84-2.96(2H,m),3.13-3.82(9H,m),4.24(2H,s),6:30-6.46(2H,m),6.93-7.03(1H,m),7.07-7.18(1H,m),7.23-7:35(1H,m),11.9-12.3(1H,m) ESI+ : 415 |
| 224 | 4 | NMR1:1.23-1.44(2H,m),1.50-1.67(1H,m),1.84-2.54(7H,m),2.67(2H,t,J=7.6Hz),2.78-3.78(10H,m),3.81-3.94(2Hm),4.22(2H,s),6.29-6.44(2H,m),6.93-7.02(1H,m),7.05-7.16(1H,m),7.21-7.33(1H,m),11.4-11.9(1H,m) ESI+ : 441 |
| 225 | 4 | NMR1:1.44(9H,s),1.67-1.78(2H,m),2.13(3H,s),2.36-2.44(2H,m),2.61-2.72(4H,m),2.99-3.06(2H,m),3.49(2H,s),4.07(2H,d,J=5.0Hz),6.06(1H,t,J=5.0Hz),6.24-6.35(2H,m),6.75-6.83(2H,m),6:89-6.98(1H,m),12.0-12.1(1H,m) ESI+: 457 |
| 4 | 4 | NMR1:1.82-2.19(2H,m),2.32-2.47(5H,m),2.69(2H,t,J=7.6Hz),2.81-2.98(2H,m),3.08-3.38(2H,m),4.21-4.64(4H,m),5.53-6.71(5H,m),6.94-7.31(3H,m),7.37-7.49(3H,m),7.50-7.61(2H,m) ESI+ : 433 |
| 226 | 4 | NMR1:1.73-1.86(2H,m),2.14(3H,s),2.43(2H,t,J=7.7Hz),2.69(2H,t,J=7.7Hz),2.80(2H,t,J=6.7Hz),2.99-3.07(2H,m),4.18(2H,s),4.46(2H,s),6.42-6.53(2H,m),6.90-6.95(1H,m),6.97-7.06(2H,m),7.95-8.00(1H,m),8.19-8.25(1H,m),8.56-8.62(1H,m),8.85-8.90(1H,m) ESI+ : 434 |
| 227 | 4 | NMR1:1.72-1.85(2H,m),2.18(3H,s),2.39(2H,t,J=7.6Hz),2.69(2H,t,J=7.6Hz),2.79(2H,t,J=6.7Hz),2.92-3.00(2H,m),4.11(2H,s),4.13(2H,s),6.46-6.56(2H,m),6.89-6.94(1H,m),6.96-7.07(2H,m),8.10-8.16(1H,m),8.69-8.75(1H,m),8.88-8.93(1H,m),8.96-9.00(1H,m) ESI+ : 434 |

**[Table 123]**

| | | |
|---|---|---|
| 9 | 9 | NMR1:1.76-1.88(2H,m),2.28(3H,s),2.44(2H,t,J=7.4Hz),2.62-2.84(4H,m),3.01-3.54(10H,m),3.75-4.00(4H,m),4.14-4.26(2H,m),5.75-7.56(9H,m),11.5-11.9(1H,m) ESI+ : 456 |
| 2 | 2 | NMR1:1.41-1.57(2H,m),1.79-2.31(8H,m),2.42(2H,t,J=7.6Hz),2.67(2H,t,J=7.6Hz),2.85-2.96(2H,m),3.11- 3.81(6H,m),4.23(2H,s),6.32-6.45(2H,m),6.94-7.04(1H,m),7.09-7.10(1H,m),7.25-7.34(1H,m),11.8-12.2(1H,m) ESI+ : 415 |
| 228 | 5 | NMR1:1.71-2.32(7H,m),2.42(2H,t,J=7.6Hz),2.68(2H,t,J=7.6Hz),2.85-2.96(2H,m),3.18-3.82(6H,m),4.00(2H,t,J=6.2Hz),4.15-4.3(2H,m),5.50-6.89(5H,m),6.92-7.04(3H,m),7.07-7.17(3H,m),723-7.36(1H,m),11.9-12.4(1H,m) ESI+ : 509 |
| 229 | 4 | NMR1:0.95(6H,s),1.87-2.36(7H,m)2.42(2H,t,J=7.6Hz),2.67(2H,t,J=7.6Hz),2.85-2.96(2H,m),3.12(3H,s)3.14-3.83(4H,m),4.24(2H,s),6.31-6.44(2H,m),6.93-7.02(1H,m),7.09-7.17(1H,m),7.24-7.32(1H,m),11.8-12.3(1H,m) ESI+ : 443 |
| 230 | 4 | NMR1:1.14(6H,d,J=6.2Hz),1.70-2.30(2H,m),2.42(2H,t,J=7,7Hz),2.46(3H,s)2.68(2H,t,J=7.7Hz)2.83-2.93(2H,m),3.21-3.98(7H,m),4.23(2H,s),6.33-6.46(2H,m),6.95-7.03(1H,m),7.08-7,15(1H,m),7.25-7.32(1H,m) ESI+ : 429 |
| 231 | 2 | ESI+ : 454 |
| 232 | 2 | ESI+:414 |
| 233 | 4 | NMR1:1.89-2.02(2H,m),2.30(3H,s),2.44(2H,t,J=7.6Hz),2.70(2H,t,J=7.6Hz),2.82(2H,t,J=6.4Hz),3.25-3.35(2H,m),4.01(2H,s),4.23(2H,s),6.47-6.63(2H,m),6.94-7.01(1H,m),7.02-7.15(3H,m),7.29-7.39(2H,m),7.50-8.94(6H,m),10.6-10.8(1H,m) ESI+ : 476 |
| 234 | 4 | NMR1:1.07-1.39(5H,m),1.50-1.83(5H,m),1.88-2.00(2H,m),2.26(3H,s),2.43(2H,t,J=7.6Hz),2.69(2H,t,J=7.6Hz),2.75-2.87(2H,m),3.17-3.31(2H,m),3.60-3.88(3H,m),4.19(2H,s),6.08-8.03(9H,m),8.33-8.40(1H,m) ESI+ : 482 |
| 235 | 4 | NMR1:1.86-2.31(4H,m),2.35-2.46(5H,m),2.61-2.74(4H,m),2.82-2.95(6H,m),4.10-6.03(5H,m),6.29-6.44(2H,m),6.93-7.02(1H,m),7.07-7.14(1H,m),7.17-7.37(6H,m),11.7-12.6(1H,m) ESI+ : 461 |
| 236 | 2 | ESI+ : 470 |

**[Table 124]**

| | | |
|---|---|---|
| 237 | 2 | NMR1:1.66-1.79(2H,m),2.14(3H,s),2.40(2H,t,J=7.6Hz),2.61-2.75(4H,m),2.97-3.07(2H,m),3.13-3.65(4H,m),4.07(2H,d,J=3.8Hz),6.07(1H,t,J=3.8Hz),6.23-6.36(2H,m),6.74-6.85(2H,m),6.88-6.98(1H,m),11.7-12.8(2H,m) ESI+:401 |
| 238 | 8 | ESI+:401 |
| 239 | 8 | ESI+:444 |
| 240 | 4 | ESI+:441 |
| 241 | 4 | ESI+:411 |
| 242 | 15 | ESI+ : 479 |
| 243 | 15 | ESI+ : 557 |
| 15 | 15 | ESI+ : 459 |
| 244 | 15 | ESI+ : 468 |
| 245 | 15 | ESI+ : 470 |
| 246 | 15 | ESI+ : 479 |
| 247 | 15 | ESI+ : 480 |
| 248 | 16 | ESI+ : 448 (M+Na) |
| 249 | 16 | ESI+ : 394 (M+Na) |
| 250 | 16 | ESI+ : 455 |
| 251 | 16 | ESI+ : 457 (M+Na) |
| 252 | 16 | ESI+ : 457 (M+Na) |
| 253 | 16 | ESI+ : 487 (M+Na) |
| 254 | 16 | ESI+ : 456,478 (M+Na) |
| 255 | 16 | ESI+ : 464 (M+Na) |
| 256 | 16 | ESI+ : 498 (M+Na) |
| 257 | 16 | ESI+ : 436 (M+Na) |
| 258 | 16 | ESI+ : 499 (M+Na) |
| 259 | 16 | ESI+ : 490 |
| 260 | 16 | ESI+ : 442 (M+Na) |
| 16 | 16 | ESI+ : 476 (M+Na) |
| 261 | 16 | ESI+ : 472 (M+Na) |
| 262 | 16 | ESI+ : 458 (M+Na) |
| 263 | 16 | ESI+ : 510 (M+Na) |
| 264 | 16 | ESI+ : 456 (M+Na) |
| 265 | 16 | ESI+ : 485 (M+Na) |
| 266 | 16 | ESI+ : 472 (M+Na) |
| 267 | 16 | ESI+ : 458 (M+Na) |
| 268 | 16 | ESI+ : 456 (M+Na) |
| 269 | 16 | ESI+ : 526 (M+Na) |
| 270 | 16 | ESI+ : 476 (M+Na) |

**[Table 125]**

| | | |
|---|---|---|
| 271 | 16 | ESI+ : 499 (M+Na) |
| 272 | 16 | ESI+ : 485 (M+Na) |
| 273 | 16 | ESI+ : 526 (M+Na) |
| 274 | 16 | ESI+ : 472 (M+Na) |
| 275 | 16 | ESI+ : 553,575 (M+Na) |
| 276 | 16 | ESI+ : 519,541 (M+Na) |
| 277 | 16 | ESI+ : 486 (M+Na) |
| 278 | 16 | ESI+ : 470 (M+Na) |
| 279 | 16 | ESI+ : 524 (M+Na) |
| 280 | 16 | ESI+ : 499 (M+Na) |
| 281 | 16 | ESI+ : 470 (M+Na) |
| 282 | 16 | ESI+ : 470 (M+Na) |
| 283 | 16 | ESI+ : 490,512 (M+Na) |
| 284 | 16 | ESI+ : 471 (M+Na) |
| 285 | 16 | ESI+ : 539 (M+Na) |
| 286 | 17 | ESI+ : 427 |
| 287 | 17 | ESI+ : 427 |
| 288 | 17 | ESI+ : 427 |
| 289 | 17 | ESI+ : 470 |
| 290 | 17 | ESI+ : 470 |
| 291 | 17 | ESI+ : 470 |
| 292 | 17 | ESI+ : 436 |
| 293 | 17 | ESI+ : 436 |
| 294 | 17 | ESI+ : 436 |
| 295 | 17 | ESI+ : 416 |
| 296 | 17 | ESI+ : 416 |
| 297 | 17 | ESI+ : 432 |
| 17 | 17 | ESI+ : 420 |
| 298 | 17 | ESI+ : 420 |
| 299 | 17 | ESI+ : 486 |
| 300 | 17 | ESI+ : 486 |
| 301 | 17 | ESI+ : 500 |
| 302 | 17 | ESI+ : 454 |
| 303 | 17 | ESI+ : 448 |
| 304 | 17 | ESI+ : 478 |
| 305 | 17 | ESI+ : 433 |
| 306 | 17 | ESI+ : 403 |
| 307 | 18 | ESI+ : 478 (M+Na) |
| 308 | 18 | ESI+ : 512 (M+Na) |
| 309 | 18 | ESI+ : 492 (M+Na) |

**[Table 126]**

| | | |
|---|---|---|
| 310 | 18 | ESI+ : 496 (M+Na) |
| 311 | 18 | ESI+ : 546 (M+Na) |
| 312 | 18 | ESI+ : 512 (M+Na) |
| 18 | 18 | ESI+ : 492 (M+Na) |
| 313 | 18 | ESI+ : 546 (M+Na) |
| 314 | 18 | ESI+ : 496 (M+Na) |
| 315 | 18 | ESI+ : 508 (M+Na) |
| 316 | 18 | ESI+ : 512 (M+Na) |
| 317 | 18 | ESI+ : 546 (M+Na) |
| 318 | 18 | ESI+ : 492 (M+Na) |
| 319 | 18 | ESI+ : 496 (M+Na) |
| 320 | 18 | ESI+ : 508 (M+Na) |
| 321 | 18 | ESI+ : 526 (M+Na) |
| 322 | 18 | ESI+ : 528 (M+Na) |
| 323 | 18 | ESI+ : 549,571 (M+Na) |
| 324 | 18 | ESI+ : 528 (M+Na) |
| 325 | 18 | ESI+ : 549 (M+Na) |
| 326 | 18 | ESI+ : 522 (M+Na) |
| 327 | 18 | ESI+ : 518 (M+Na) |
| 328 | 18 | ESI+ : 534 (M+Na) |
| 19 | 19 | ESI+:462 |
| 329 | 19 | ESI+:414 |
| 330 | 19 | ESI+:386 |
| 331 | 19 | ESI+:414 |
| 332 | 19 | ESI+:400 |
| 20 | 20 | FAB+:458 |
| 333 | 20 | FAB+:458 |
| 334 | 20 | FAB+:444 |
| 335 | 20 | ESI+:452 |
| 336 | 20 | ESI+:430 |
| 337 | 20 | ESI+:446 |
| 338 | 20 | ESI+:452 |
| 21 | 21 | ESI+:471 |
| 22 | 22 | ESI+:481 |
| 339 | 22 | ESI+:437 |
| 340 | 22 | FAB+:416 |
| 341 | 22 | FAB+:430 |
| 23 | 23 | FAB+:413 |
| 342 | 23 | ESI+:461 |
| 24 | 24 | FAB+:326 |

**[Table 127]**

| | | |
|---|---|---|
| 343 | 24 | ESI+:358 |
| 344 | 24 | ESI+:358 |
| 25 | 25 | ESI+:358 |
| 345 | 25 | ESI+:371 |
| 26 | 26 | ESI+:399 |
| 346 | 26 | FAB+:447 |
| 347 | P29 | ESI+:354 |
| 27 | 27 | FAB+:358 |
| 348 | 27 | FAB+:336(M)⁺ |
| 349 | 27 | FAB+:344 |
| 350 | 27 | FAB+:358 |
| 28 | 28 | FAB+:416 |
| 351 | 28 | ESI+:462 |
| 352 | 28 | ESI+:412 |
| 353 | 28 | ESI+:400 |
| 354 | 28 | FAB+:372 |
| 355 | 28 | FAB+:454 |
| 356 | 28 | FAB+:448 |
| 357 | 28 | FAB+:508 |
| 358 | 28 | FAB+:449 |
| 359 | 28 | FAB+:449 |
| 360 | 28 | ESI+:449 |
| 361 | 28 | FAB+:516 |
| 362 | 28 | FAB+:448 |
| 363 | 28 | FAB+:386 |
| 364 | 28 | FAB+:470 |
| 365 | 28 | ESI+:448 |
| 366 | 28 | FAB+:400 |
| 367 | 28 | ESI+:462 |
| 368 | 28 | ESI+:372 |
| 369 | 28 | ESI+:400 |
| 370 | 28 | ESI+:454 |
| 371 | 28 | ESI+:400 |
| 372 | 28 | ESI+:462 |
| 373 | 28 | ESI+:400 |
| 374 | 28 | ESI+:448 |
| 375 | 28 | ESI+:462 |
| 29 | 29 | ESI+:371 |
| 376 | 29 | ESI+:453 |
| 377 | 29 | ESI+:461 |

**[Table 128]**

| | | |
|---|---|---|
| 378 | 29 | ESI+:357 |
| 379 | 29 | ESI+:447 |
| 380 | 29 | ESI+:399 |
| 381 | 29 | ESI+:415 |
| 382 | 29 | ESI+:401 |
| 383 | 29 | ESI+:433 |
| 384 | 29 | ESI+:435 |
| 385 | 29 | ESI+:399 |
| 386 | 29 | ESI+:447 |
| 387 | 29 | ESI+:470 |
| 30 | 30 | ESI+:478 |
| 388 | 30 | FAB+:434 |
| 389 | 30 | FAB+:398 |
| 390 | 30 | ESI+:444 |
| 391 | 30 | ESI+:448 |
| 392 | 30 | ESI+:448 |
| 393 | 30 | ESI+:416 |
| 394 | 30 | ESI+:448 |
| 395 | 30 | ESI+:413 |
| 31 | 31 | ESI+:462 |
| 396 | 31 | ESI+:400 |
| 397 | 31 | FAB+:400 |
| 398 | 31 | FAB+:426 |
| 399 | 31 | FAB+:414 |
| 400 | 31 | FAB+:430 |
| 401 | 31 | FAB+:428 |
| 402 | 31 | FAB+:442 |
| 403 | 31 | FAB+:462 |
| 404 | 31 | FAB+:530 |
| 32 | 32 | FAB+:498 |
| 405 | 32 | FAB+:498 |
| 406 | 32 | FAB+:436 |
| 407 | 32 | FAB+:450 |
| 408 | 32 | ESI+:464 |
| 33 | 33 | FAB+:441 |
| 409 | 33 | ESI+:489 |
| 34 | 34 | FAB+:434 |
| 410 | 34 | ESI+:434 |
| 411 | 34 | FAB+:420 |
| 412 | 34 | FAB+:434 |

**[Table 129]**

| | | |
|---|---|---|
| 35 | 35 | ESI+:491 |
| 413 | 2 | NMR1:1.69-1.80(2H,m),1.83(3H,s),1.96-2.07(2H,m)2.48-2.62(2H,m)2.65-2.73(2H,m),3.61-3.70(2H,m),4.05-4.12(2H,m),5.98-6.06(1H,m),6.20-6.33(2H,m),6.70(2H,d,J=8.9Hz),6.87-7.06(3H,m),7.20(2H,dJ=8.9Hz) ESI-:451 |
| 414 | 1 | NR4R1:1.94-2.07(4H,m)2.53-2.60(2H,m)2.74-2.81(2H,m),3.67-3.74(2H,m),3.90-3.97(2H,m),5.99-6.04(1H,m),6.13-6.23(2H,m),6.58(1H,d,J=7.4Hz),6.87-6.94(1H,m),7.11-7.19(2H,m),7.25(1H,d,J=7.4Hz),7.31-7.38(2H,m) ESI+:424 |
| 415 | 1 | NMR1:1.92-2.09(4H,m),2.53-2.61(2H,m),2.73-2.81(2H,m),3.68-3.75(2H,m),3.98(2H,d,J=5.4Hz),6.08(1H,t,J=5.4Hz),6.15-6.26(2H,m),6.61-6.68(1H,m),6.86-6.95(1H,m),7.13-7.20(1H,m),7.24-7.40(2H,m),7.41-7.51(1H,m) ESI+:442 |
| 416 | 1 | NMR1:1.91-2.08(4H,m),2.53-2.61(2H,m),2.72-2.81(2H,m),3.69-3.76(2H,m),3.97(2H,d,J=5.8Hz),6.07(1H,t,J=5.8Hz),6.14-6.25(2H,m),6.64(1H,d,J=7.4Hz),6.87-6.94(1H,m),7.28(1H,d,J=7.4Hz),7.32-7.39(4H,m) ESI+:440 |
| 417 | 1 | NMR1:1.91-2.08(4H,m),2.54-2.61(2H,m),2.73-2.80(2H,m),3.71-3.77(2H,m),3.98(2H,d,J=5.7Hz),6.05(1H,t,J=5.7Hz),6.15-6.27(2H,m),6.64-6.69(1H,m),6.88-6.95(1H,m),7.10-7.15(1H,m),7.28-7.36(3H,m),7.43-7.47(1H,m) ESI+:440 |
| 418 | 1 | NMR1:1.90-2.09(4H,m),2.52-2.63(2H,m),2.69-2.82(2H,m),3.67-3.77(2H,m),3.97-4.09(2H,m),6.08-6.29(3H,m),6.68-6.75(1H,m),6.85-6.95(1H,m),7.26-7.41(3H,m) ESI+:460 |
| 419 | 1 | NMR1:1.87-1.96(2H,m),1.97-2.06(2H,m),2.52-2.61(2H,m),2.73-2.80(2H,m),3.92-4.00(2H,m),4.10-4.18(2H,m),6.20-6.32(3H,m),6.83-6.88(1H,m),6.89-6.97(1H,m),7.40-7.47(1H,m),7.65-7.71(1H,m),7.77-7.84(1H,m),8.37-8.41(1H,m) ESI+: 485,487 |
| 420 | 1 | NMR3:1.60-1.69(4H,m)1.79-1.93(1H,m),2.31-2.39(2H,m),2.44(3H,s),2.71-2.79(2H,m),2.82-2.95(2H,m),3.33-3.44(2H,m),3.98-4.12(2H,m),6.15-6.22(1H,m),6.25-631(1H,m),6.41-6.48(1H,m)6.89-7.00(5H,m),7.19-7.26(1H,m) ESI+:433 |
| 421 | 1 | ESI+:521 |

**[Table 130]**

| | | |
|---|---|---|
| 422 | 1 | NMR1:1.66-1.74(2H,m),1.84(3H,s),1.99-2.08(2H,m),2.53-2.61(2H,m),2.65-2.74(2H,m),3.51-3.58(2H,m),4.00-4.07(2H,m),5.92-5.98(1H,m),5.99-6.05(2H,m),6.09-6.16(1H,m),6.21-6.32(2H,m),6.78-6.85(1H,m),6.88-6.96(3H,m) ESI+:435 |
| 423 | 1 | ESI+:372 |
| 424 | 1 | ESI-:461 |
| 425 | 1 | ESI-:481 |
| 426 | 1 | ESI-:465 |
| 427 | 2 | ESI-:497 |
| 428 | 1 | ESI+:462 |
| 429 | 1 | FAB+:464 |
| 430 | 1 | ESI+:468 |
| 39 | 39 | ESI+:450 |
| 431 | 39 | ESI+:446 |
| 432 | 39 | ESI+:462 |
| 433 | 39 | ESI+:468 |
| 434 | 11 | FAB+:431 |
| 435 | 11 | ESI+:445 |
| 436 | 11 | ESI+:461 |
| 437 | 11 | FAB+:432 |
| 438 | 11 | FAB+:446 |
| 439 | 1 | ESI+:445 |
| 36 | 36 | ESI+:445 |
| 440 | 11 | NMR1:1.76-2.00(2H,m),2.68(2H,t,J=8.0Hz),3.28-3.42(2H,m),3.65(2H,t,J=5.7Hz),3.93(2H,s),3.99(2H,d,J=5.4Hz),4.12(2H,t,J=5.7Hz),5.30(1 H,t,J=5.6Hz),6.46(2H,d,J=8.8Hz),6.52-6.66(4H,m),6.86-6.98(4H,m),722-7.32(2H,m) ESI+:433 |
| 441 | 21 | NMR1:1.76-1.96(2H,m),2.67(2H,t,J=6.4Hz),3.25(2H,s),3.65(2H,t,J=5.7Hz),4.04(2H,d,J=5.3Hz),4.12(2 H,t,J=5.7Hz),5.90(1H,t,J=5.4Hz),6.49(2H,d,J=8.6Hz),6.54(1H,d,J=7.4Hz),6.61(1H,d,J=8.2 Hz),6.85-6.97(4H,m),7.06(2H,d,J=8.5Hz),7.22-7.32(2H,m) ESI+:449 |
| 442 | 1 | ESI+:477 |
| 443 | 1 | ESI+:507 |
| 444 | 5 | ESI+:509 |
| 445 | 5 | ESI+:509 |

**[Table 131]**

| | | |
|---|---|---|
| 446 | 5 | NMR1:1.80-1.93(2H,m),2.14(2H,t,J=8.0Hz)2.59(2H,t,J=8.0Hz),2.66(2H,t,J=6.3Hz),3.34-3.41(2H,m),3.63(2H,t,J=5.8Hz),3.76(3H,s),4.03(1H,d,J=5.2Hz),4.07(2H,t,J=5.8Hz),5.96(1 H,t,J=5.2Hz),6.26(1H,dd,J=2.2,13.2Hz),6.31(1H,dd,J=2.2,8.3Hz),6.53(1H,d,J=7.4Hz),6.60 (1H,d,J=8.7Hz),6.61-6.67(1H,m),6.85-6.96(4H,m) ESI+:497 |
| 447 | 5 | NMR1:1.78-1.93(2H,m),2.10(2H,t,J=8.0Hz),2.59(2H,t,J=8.0Hz),2.67(2H,t,J=6.2Hz),3.33-3.39(2H,m),3.66(2H,t,J=5.5Hz),4.03(2H,d,J=5.1Hz),4.19(2H,t,J=5.5Hz),5.95(1H,t,J=5.1Hz ),6.26(1H,dd,J=1.9,13.1Hz),6.31(1H,dd,J=1.9,8.3Hz),6.54(1H,d,J=7.5Hz),6.61(1H,d,J=8.3 Hz),6.85-7.02(3H,m),7.18(1H,dt,J=5.4,9.4Hz),7.27(1H,ddd,J=3.0,8.8,11.7Hz) ESI+:485 |
| 448 | 5 | NMR1:1.81-1.93(2H,m),2.01-2.15(2H,m),2.54-2.61(2H,m),2.63-2.72(2H,m),3.27-3.42(2H,m),3.68(2H,t,J=5.5Hz),4.03(2H,d,J=5.2Hz),4.22(2H,t,J=5.5Hz),5.95(1H,t,J=5.2Hz ),6.25(1H,d,J=13.2Hz),6.30(1H,d,J=8.5Hz),6.54(1H,d,J=7.4Hz),6.61(1H,d,J=8.5Hz),6.74(1 H,tt,J=3.1,8.5Hz),6.87-6.96(2H,m),7.11(1H,ddd,J=3.1,7.1,10.1Hz),7.24(1H,ddd,J=5.4,9.1,11.1Hz) ESI+:485 |
| 449 | 5 | NMR1:1.79-1.91(2H,m),2.04-2.15(2H,m),2.55-2.62(2H,m),2.63-2.71(2H,m),3.29-3.37(2H,m),3.64(2H,t,J=6.0Hz),4.02(2H,d,J=5.1Hz),4.41(2H,t,J=6.0Hz),5.94(1H,t,J=5.1Hz ),6.26(1H,d,J=13,1Hz),6.31(1H,d,J=8.3Hz),6.53(1H,d,J=7.4Hz),6.67(1H,d,J=8.3Hz),6.80(1 H,d,J=8.4Hz),6.88-7.00(3H,m),7.66-7.73(1H,m),8.15(1H,dd,J=1.7,5.0Hz) ESI+:450 |
| 450 | 2 | NMR1:1.71-1.83(2H,m),1.98-2.10(2H,m),2.53-2.59(2H,m),2.60-2.66(2H,m),3.00-3.08(2H,m),3.74(2H,t,J=6.0Hz),4.02(2H,d,J=5.3Hz),4.49(2H,t,J=6.0Hz),5.95(1H,t,J=5.3Hz ),6.23(1H,dd,J=2.2,13.2Hz),6.29(1H,dd,J=2.2,8.3Hz),6.42(1H,d,J=8.3Hz),6.55(1H,d,J=7.3 Hz),6.84-6.95(2H,m),7.56(1H,s) ESI+:559 |
| 451 | 5 | NMR1:1.81-1.92(2H,m),2.02-2.11(2H,m),2.54-2.62(2H,m),2.63-.2.72(2H,m)3.33-3.38(2H,m),3.59(2H,t,J=5.9Hz),3.77(3H,s),4.03(2H,d,J=5.3Hz),4.11(2H,t,J=5.9Hz),5.95(1 H,t,J=5.3Hz),6.25(1H,dd,J=2.1,13.2Hz),6.30(1H,dd,J=2.1,8.3Hz),6.48-6.56(2H,m),6.79-6.95(4H,m),7.04(1H,dt,J=6.3,8.5Hz) ESI+:497 |

**[Table 132]**

| | | |
|---|---|---|
| 452 | 5 | NMR1:1.80-1.92(2H,m),2.02-2.12(2H,m),2.54-2.61(2H,m),2,62-2.69(2H,m),3.15-3.22(2H,m),3.23-3.29(2H,m),3.43-3.49(2H,m),4.03(2H,d,J=5.2Hz),5.95(1H,t,J=5.2Hz),6.25(1H,dd,J=2.0,13.1Hz),6.30(1H,dd ,J=2.0,8.3Hz),6.45(1H,d,J=8.2Hz),6.55(1H,d,J=7.4Hz),6.86-6.95(2H,m),7.22-7.29(1H,m),7.31-7.36(2H,m),7.40-7.46(1H,m) FAB-:497 |
| 453 | 1 | NMR1:1.77-1.87(2H,m),1.93(3H,s),1.99-2.08(2H,m),2.53-2.68(4H,m),3.82-4.08(2H,m),4.15(2H,d,J=5.4Hz),6.08(1H,t,J=5.4Hz),6.18-6.36(3H,m),6.68-6.76(1H,m),6.90-6.98(1H,m),7.03(1H,d,J=7.8Hz),7.09(1H,d,J=7.8Hz),7.41-7.49(1H,m),8.17-823 ESI+:420 |
| 454 | 2 | NMR1:1.69-1.81(2H,m),2.41(2H,t,J=7.6Hz),2.62(2H,t,J=6.4Hz),2.67(2H,t,J=7.6Hz),2.96-3.03(2H,m),3.62(2H,t,J=6.2Hz),4.05(2H,s),4.27(2H,t,J=6.2Hz),6.22(1H,t,J=2.0Hz),6.32-6.42(2H,m),6.49(1H,d,J=8.2Hz),6.55(1H,d,J=7.4Hz),6.88-7.00(2H,m),7.48(1H,d,J=2.0Hz),7.68(1H,d,J=2.0Hz) ESI-:421 |
| 455 | 1 | NMR1:0.60-0.68(1H,m),0.98-1.06(1H,m),1.22-1.30(1H,m),1.80-1.92(3H,m),2.67(2H,t,J=6.3Hz),3.32-3.42(2H,m),3.65(2H,t,J=5.7Hz),4.02(2H,d,J=5.3Hz),4.12(2H,t,J=5.7Hz),5.61(1H,t,J=5.5Hz ),6.45(2H,d,J=8.4Hz),6.54(1H,d,J=7.4Hz),6.60(1H,d,J=8.2Hz),6.71(2H,d,J=8.4Hz),6.87-6.95(4H,m),7.23-7.30(2H,m) ESI+:443 |
| 456 | 1 | NMR1:0.75-0.84(1H,m),1.05-1.12(1H,m),1.50-1.59(1H,m),1.79-1.92(3H,m),2.68(2H,t,J=6.3Hz),3.28-3.34(2H,m),3.65(2H,t,J=5.7Hz),4.01(2H,d,J=5.3Hz),4.12(2H,t,J=5.7Hz),5.49(1H,t,J=5.5Hz ),6.37(2H,d,J=8.4Hz),6.56(1H,d,J=7.4Hz),6:60(1H,d,J=8.2Hz),6.84-7.00(6H,m),7.22-7.31(2H,m) ESI+:443 |
| 37 | 37 | FAB+:452 |
| 457 | 37 | FAB+:470 |
| 458 | 37 | FAB+:468 |
| 459 | 37 | FAB+:468 |
| 460 | 37 | FAB+:513,515 |
| 461 | 37 | FAB+:488 |
| 462 | P33 | ESI+:400 |
| 463 | P33 | ESI+:461 |
| 464 | 21 | ESI+:549 |
| 465 | P34 | ESI+:463 |
| 466 | P33 | ESI+:448 |
| 467 | 20 | ESI+:492 |

**[Table 133]**

| | | |
|---|---|---|
| 38 | 38 | ESI+:491 |
| 468 | 38 | ESI+:511 |
| 469 | 38 | ESI+:495 |
| 470 | P33 | ESI+:490 |
| 471 | 20 | ESI+:496 |
| 472 | 21 | ESI+:535 |
| 473 | 21 | ESI+:473 |
| 474 | 21 | ESI+:505 |
| 475 | 21 | ESI+:471 |
| 476 | 21 | ESI+:471 |
| 477 | 21 | ESI+:457 |
| 40 | 40 | ESI+:475 |
| 478 | 40 | FAB-:475 |

**[Table 134]**

| No | Structure |
|---|---|
| 1 | 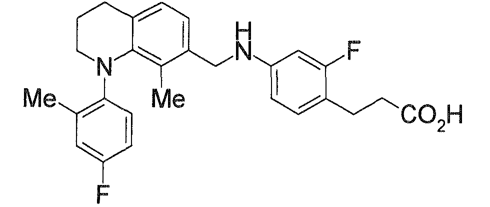 |
| 2 | 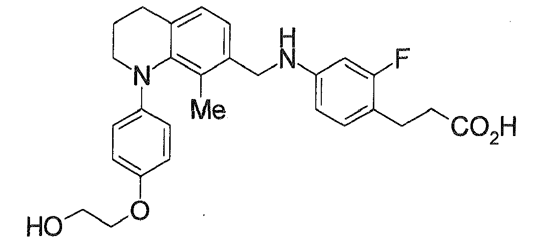 |
| 3 | 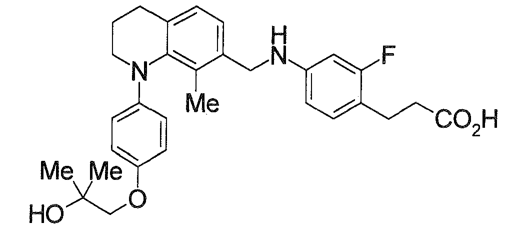 |
| 4 | 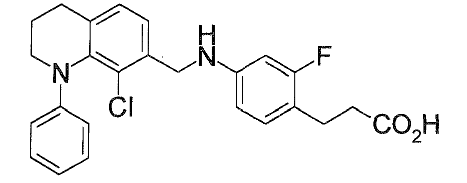 |
| 5 | 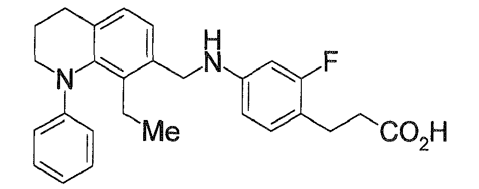 |
| 6 | 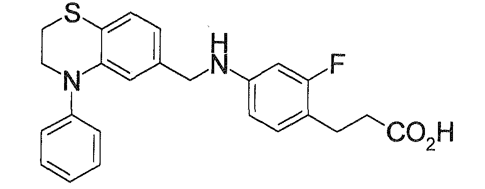 |

**[Table 135]**

| | |
|---|---|
| 7 | 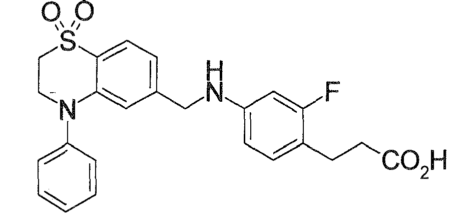 |
| 8 | 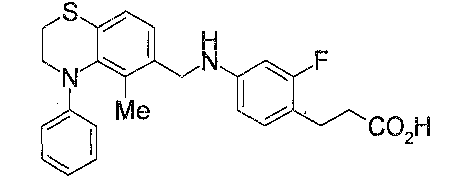 |
| 9 | 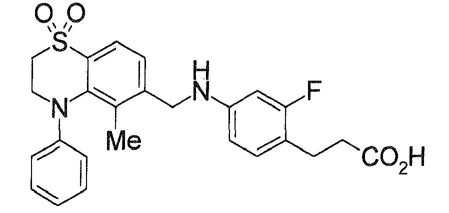 |
| 10 | 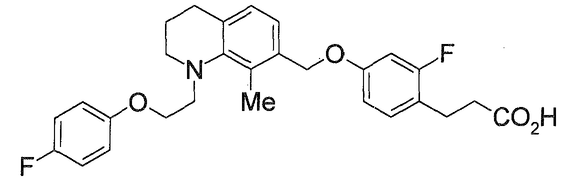 |
| 11 | 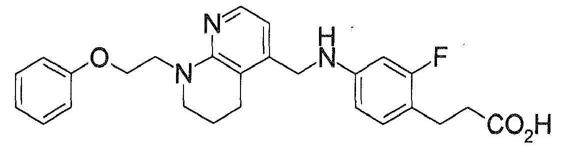 |
| 12 | 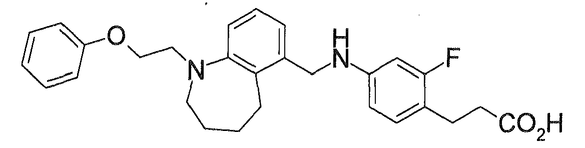 |
| 13 | 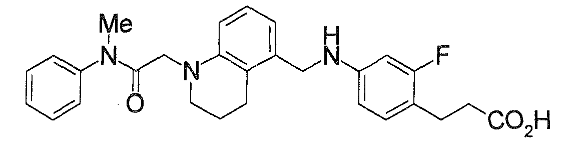 |

**[Table 136]**

| | |
|---|---|
| 14 | 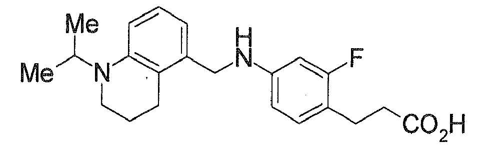 |
| 15 | 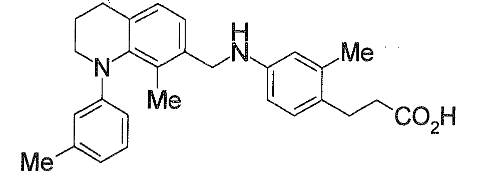 |
| 16 | 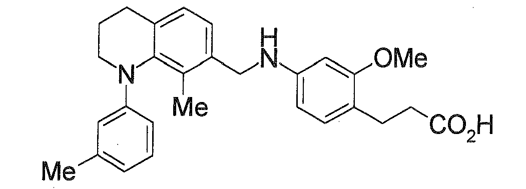 |

### INDUSTRIAL APPLICABILITY

Since the compounds of the invention have excellent GPR40 agonist action, they are useful as an insulin secretion promoter and a preventive or therapeutic agent for diabetes mellitus (insulin-dependent diabetes mellitus (IDDM), non insulin-dependent diabetes mellitus (NIDDM) and their boundary type (abnormal glucose resistance and fasting blood gulucose level) slight diabetes mellitus) and the like diseases in which GPR40 is concerned.

### SEQUENCE LISTING FREE TEXT

Explanation on the "Artificial Sequence" is described in the numerical index <223> in the following SEQUENCE LISTING. Illustratively, the nucleotide sequence represented by the sequence of SEQ ID NO:1 of the SEQUENCE LISTING is a nucleotide sequence of an artificially synthesized primer. Also, the nucleotide sequence represented by the sequence of SEQ ID NO:2 of the SEQUENCE LISTING is a nucleotide sequence of an artificially synthesized primer.

## Claims

1. A carboxylic acid derivative represented by the following formula (I) or a pharmaceutically acceptable salt thereof 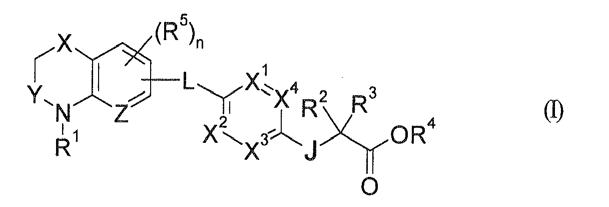 (symbols in the formula represent the following meanings;
R¹: -H, lower alkyl, halogeno-lower alkyl, cycloalkyl, aryl, heterocyclic group, lower alkylene-R^{A}, -C(O)R^{B}, -CO₂R^{B} or -S(O)ₚR^{B},
with the proviso that the lower alkylene, aryl and heterocyclic group in R¹ may be respectively substituted,
R^{A}: cycloalkyl, aryl, heterocyclic group, -S(O)ₚR⁰, -S(O)ₚ-aryl, -S(O)ₚ-heterocyclic group, -C(O)R⁰, -C(O)-aryl, -C(O)-heterocyclic group, -CO₂R⁰, -OR⁰, -O-aryl, -O-heterocyclic group, -N(R⁰)₂, -N(R⁰)-aryl, -N(R⁰)-heterocyclic group, -CO(R⁰)(aryl)₂, -C(O)N(R⁰)-cycloalkyl or -C(O)N(R⁰)-aryl,
with the proviso that the aryl and heterocyclic group in R^{A} may be respectively substituted, R^{B}: lower alkyl, halogeno-lower alkyl, cycloalkyl, aryl, heterocyclic group, lower alkylene-cycloalkyl, lower alkylene-aryl, lower alkylene-heterocyclic group, lower alkylene-OR⁰, lower alkylene-O-aryl or lower alkylene-S(O)₂NH₂,
with the proviso that the aryl and heterocyclic group in R^{B} may be respectively substituted,
R⁰: -H or lower alkyl,
n and p: the same or different from each other and each represents 0, 1 or 2,
J: -C(R⁶)(R⁷)-, -0- or -S-, R², R³, R⁶ and R⁷: the same or different from one another and each represents -H, halogen, lower alkyl, -OR⁰ or aryl,
with the proviso that R² and R³, R³ and R⁶ and R⁶ and R⁷ may together form a lower alkylene,
R⁴: -H or lower alkyl,
X: single bond, -CH₂-, -(CH₂)₂-, -O-, -S-, -S(O)- or -S(O)₂-,
Y: -CH₂- or -C(O)-,
Z: C(-*), C(R⁸), N or N(O), with the proviso that the * in Z means binding to L,
X¹ and X²: the same or different from each other and each represents C(R⁹), N or N(O),
X³ and X⁴: the same or different from each other and each represents C(R¹⁰), N or N(O),
R⁵: lower alkyl, halogen, halogeno-lower alkyl, -OR⁰ or -O-halogeno-lower alkyl,
R⁸, R⁹ and R¹⁰: the same or different from one another and each represents -H, lower alkyl, halogen, halogeno-lower alkyl, -OR⁰ or -O-halogeno-lower alkyl,
with the proviso that R⁶ and R¹⁰ may together form a lower alkylene, -O-lower alkylene or lower alkylene-O-,
L: -O-lower alkylene, lower alkylene-O-, -N(R¹¹)-lower alkylene, lower alkylene-N(R¹¹)-,
-O-lower alkylene-O-, -N(R¹¹)-lower alkylene-N(R¹¹)-, -O-lower alkylene-N(R¹¹)- or
-N(R¹¹)-lower alkylene-O-, and
R¹¹: -H, lower alkyl or -C(O)R⁰).

2. The compound described in claim 1, wherein J is -C(R⁶)(R⁷)-.

3. The compound described in claim 2, wherein X and Y are -CH₂-.

4. The compound described in claim 3, wherein L is *-CH₂-NH- or *-CH₂-O-(wherein * means binding to the nitrogen-containing bicyclic ring group to which R¹ is bonded).

5. The compound described in claim 4, wherein Z is CH, C(lower alkyl), C(-*) (wherein * means binding to L) or N.

6. The compound described in claim 5, wherein n is 0; or n is 1 and R⁵ is halogen or lower alkyl.

7. The compound described in claim 6, wherein X¹ and X² are the same or different from each other and each is CH or N, and X³ and X⁴ are the same or different from each other and each is CH or C(halogen).

8. The compound described in claim 7, wherein R², R³, R⁶ and R⁷ is H.

9. The compound described in claim 8, wherein R⁴ is -H.

10. The compound described in claim 9, wherein R¹ is lower alkyl, halogeno-lower alkyl, aryl, heterocyclic group, lower alkylene-OR⁰, lower alkylene-aryl, lower alkylene-heterocyclic group or lower alkylene-O-aryl (however, the aryl and heterocyclic group in R¹ may be respectively substituted by a group selected from halogen, -CN, lower alkyl, halogeno-lower alkyl, -OR⁰ and -O-halogeno-lower alkyl).

11. A compound described in claim 1, which is selected from the group consisting of 3-[2-fluoro-4-({[1-(2-phenylethyl)-1,2,3,4-tetrahydroquinolin-5-yl]methyl}amino)phenyl]propanoic acid,
3-[2-fluoro-4-({[1-(2-phenoxyethyl)-1,2,3,4-tetrahydroquinolin-5-yl]methyl}amino)phenyl]propanoic acid,
3-(2-fluoro-4-{[(8-methyl-1-propyl-1,2,3,4-tetrahydroquinolin-7-yl)methyl] amino }phenyl)propanoic acid,
3-[2-fluoro-4-({[8-methyl-1-(2-phenylethyl)-1,2,3,4-tetrahydroquinolin-7-yl]methyl}amino)phenyl]propanoic acid,
3-(2-fluoro-4-{[(8-methyl-1-phenyl-1,2,3,4-tetrahydroquinolin-7-yl)methyl]amino}phenyl)propanoic acid,
3-(2-fluoro-4-{[(8-phenyl-5,6,7,8-tetrahydro-1,8-naphthyridin-2-yl)methyl]amino}phenyl)propanoic acid,
3-{2-fluoro-4-[({1-[2-(4-methoxyphenyl)ethyl]-1,2,3,4-tetrahydroquinolin-5-yl}methyl)amino]phenyl}propanoic acid,
3-{2-fluoro-4-[({1-[2-(4-fluorophenoxy)ethyl]-1,2,3,4-tetrahydroquinolin-5-yl}methyl)amino]phenyl}propanoic acid,
3-{4-[({1-[2-(3-chlorophenoxy)ethyl]-1,2,3,4-tetrahydroquinolin-5-yl}methyl)amino]-2-fluorophenyl}propanoic acid,
3-[2-fluoro-4-({[1-(3-fluorophenyl)-8-methyl-1,2,3,4-tetrahydroquinolin-7-yl]methyl}amino)phenyl]propanoic acid,
3-{2-fluoro-4-({[8-methyl-1-(3-methylphenyl)-1,2,3,4-tetrahydroquinolin-7-yl]methyl}amino)phenyl}propanoic acid, and
3-[4-({[1-(3,4-difluorophenyl)-8-methyl-1,2,3,4-tetrahydroquinolin-7-yl]methyl}amino)-2-fluorophenyl]propanoic acid,
or a pharmaceutically acceptable salt thereof.

12. A pharmaceutical composition, which comprises a compound described in claim 1 or a pharmaceutically acceptable salt thereof and a pharmaceutically acceptable carrier.

13. The pharmaceutical composition described in claim 12, which is a GPR40 agonist.

14. The pharmaceutical composition described in claim 12, which is an insulin secretion promoter..

15. The pharmaceutical composition described in claim 12, which is an agent for preventing and/or treating diabetes mellitus.

16. Use of the compound described in claim 1 or a pharmaceutically acceptable salt thereof, for producing a GPR40 agonist, an insulin secretion promoter or a preventive and/or therapeutic agent for diabetes mellitus.

17. A method for preventing and/or treating diabetes mellitus, which comprises administering an effective amount of the compound described in claim 1 or a salt thereof to a patient.
